(19) Europäisches Patentamt
European Patent Office
Office européen des brevets

(11) EP 4 700 022 A1

# (12) EUROPEAN PATENT APPLICATION

published in accordance with Art. 153(4) EPC

(43) Date of publication:
**25.02.2026 Bulletin 2026/09**

(21) Application number: **24792116.6**

(22) Date of filing: **19.04.2024**

(51) International Patent Classification (IPC):
***C07D 405/14*** *(2006.01)* ***C07D 209/44*** *(2006.01)*
***C07D 211/98*** *(2006.01)* ***C07D 413/14*** *(2006.01)*
***A61K 31/4439*** *(2006.01)* ***A61K 31/351*** *(2006.01)*
***A61P 35/00*** *(2006.01)*

(52) Cooperative Patent Classification (CPC):
**A61K 31/351; A61K 31/4439; A61P 35/00; C07D 209/44; C07D 211/98; C07D 405/14; C07D 413/14**

(86) International application number:
**PCT/CN2024/088772**

(87) International publication number:
**WO 2024/217531 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**21.04.2023 CN 202310434656**
**06.06.2023 CN 202310661662**
**10.07.2023 CN 202310838513**
**09.08.2023 CN 202310998775**
**23.08.2023 CN 202311067100**
**22.09.2023 CN 202311231838**
**25.10.2023 CN 202311391781**
**22.01.2024 CN 202410087576**

(71) Applicant: **Xizang Haisco Pharmaceutical Co., Ltd.**
**Shannan, Xizang 856099 (CN)**

(72) Inventors:
- **LI, Yao**
  **Chengdu, Sichuan 611130 (CN)**
- **SHI, Zongjun**
  **Chengdu, Sichuan 611130 (CN)**
- **CHEN, Lei**
  **Chengdu, Sichuan 611130 (CN)**
- **ZHANG, Haoliang**
  **Chengdu, Sichuan 611130 (CN)**
- **HUANG, Shuai**
  **Chengdu, Sichuan 611130 (CN)**
- **WANG, Pengcheng**
  **Chengdu, Sichuan 611130 (CN)**
- **SHI, Shaohui**
  **Chengdu, Sichuan 611130 (CN)**
- **ZHANG, Xiaohai**
  **Chengdu, Sichuan 611130 (CN)**
- **YANG, Shuang**
  **Chengdu, Sichuan 611130 (CN)**
- **TANG, Pingming**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **AWA Denmark A/S**
**Strandgade 56**
**1401 Copenhagen K (DK)**

## (54) CYP11A1 INHIBITORS AND USE THEREOF

(57) The present invention relates to CYP11A1 inhibitors and the use thereof. Disclosed in the present invention are compounds represented by formula (I), or stereoisomers, deuterated substances, solvates, co-crystals or pharmaceutically acceptable salts thereof, a pharmaceutical composition of same, and the use thereof in preparation of drugs for treating/preventing CYP11A1-mediated diseases, each group in formula (I) being as defined in the description.

EP 4 700 022 A1

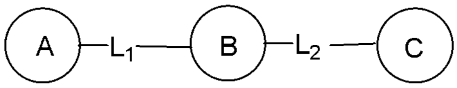


(I)

## Description

### Technical Field

The present invention belongs to the field of medicine and in particular relates to a small molecule compound, or a stereoisomer, a deuterated substance, a solvate, a co-crystal or a pharmaceutically acceptable salt thereof, which have a selective inhibitory activity against CYP11A1, and the use thereof in the preparation of a drug for treating a related disease.

### Background Art

Cytochrome p450 monooxygenase 11a1 (cyp11a1), also termed cholesterol side-chain cleavage enzyme, primarily participates in drug metabolism-related catalytic reactions, as well as the synthesis of cholesterol, steroids, and other lipids. CYP11A1 protein is localized within the inner mitochondrial membrane and catalyzes the conversion of cholesterol to pregnenolone, representing the first and rate-limiting step in the synthesis of steroid hormones. This reaction occurs in the mitochondria of the adrenal cortex, and is catalyzed by cytochrome CYP11A1 (also referred to as P450scc), which functions in conjunction with adrenodoxin (Adx) and adrenodoxin reductase (AdR). CYP11A1, Adx, and AdR belong to the cholesterol hydroxylase/lyase system (CH/L system), which catalyzes the initial step of steroid synthesis in mammals - the production of pregnenolone from cholesterol. Pregnenolone serves as a critical precursor for steroid hormones. The reaction process involves three consecutive monooxygenation reactions: production of 22R-hydroxycholesterol (22HC), production of 20R,22R-dihydroxycholesterol, and cleavage of the C20-C22 bond. Each monooxygenation reaction requires two electrons and one molecular oxygen. The electrons are supplied by NADPH and transferred to P450scc via NADPH-AdR and Adx. Adx forms a complex with P450scc and can function as a mobile electron shuttle.

CYP11A1 is primarily expressed in the placenta in response to the synthesis of placenta-derived hormones, such as progesterone and testosterone. It is also highly expressed in the adrenal glands and testes, but is almost absent in other tissues. By inhibiting cyp11a1 (a key enzyme upstream of cyp17a1 in steroid biosynthesis), complete blockade of the entire steroid biosynthesis pathway can be achieved. Therefore, cyp11a1 inhibitors hold great potential for treating steroid hormone-dependent cancers such as prostate cancer, even in advanced disease stages, especially in patients exhibiting hormone-refractory status. It has been recently confirmed that compounds having inhibitory effects on cyp11a1 significantly suppress tumor growth *in vivo* in mouse castration-resistant prostate cancer (crpc) xenograft models.

CYP11A1 inhibitors with good activity, high safety and minor side effects are found, which have a good clinical development prospect and can be used for treating cancers or other proliferative diseases or conditions.

### Summary of the Invention

The present invention provides a small molecule compound, or a stereoisomer, a deuterated substance, a solvate, a co-crystal or a pharmaceutically acceptable salt thereof, which have an inhibitory activity against CYP11A1, wherein the compound has the structure of formula (I), (I-a), (I-b), (I-c), (I-d), (I-e), (I-f), (II-a), (II-b), (II-c), (II-d), (II-e), (I-1), (I-2), (I-3), (I-4), or (I-5),

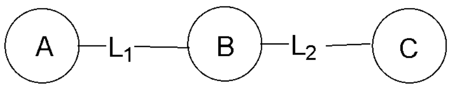

(I)

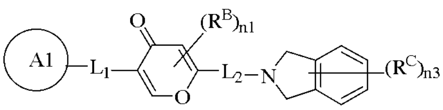

(I-a)

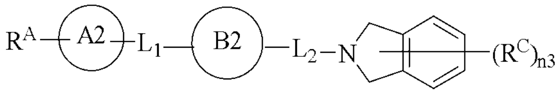

(I-b)

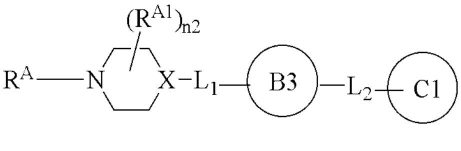
(I-c)

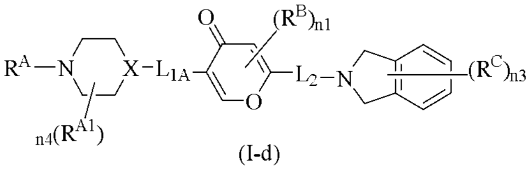
(I-d)

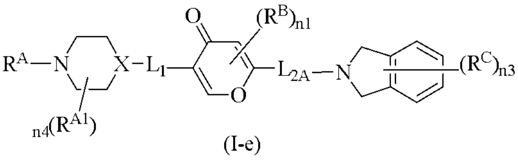
(I-e)

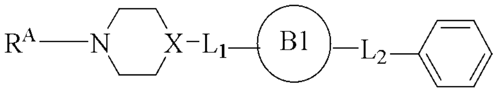
(I-f)

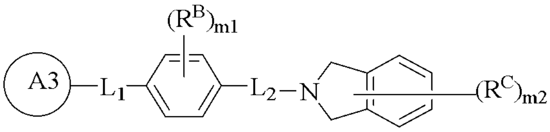
(II-a)

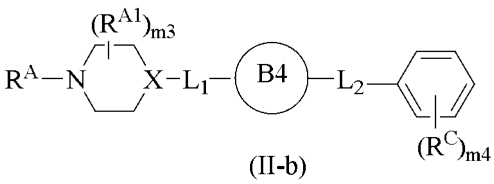
(II-b)

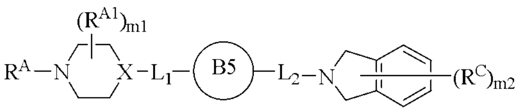
(II-c)

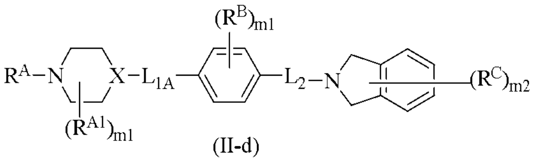
(II-d)

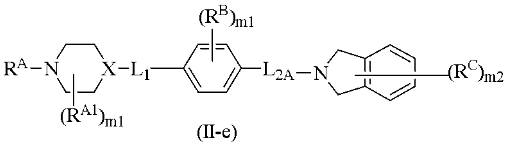
(II-e)

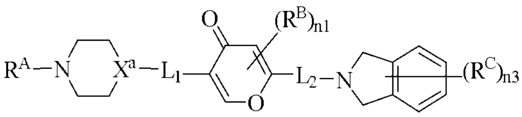
(I-1)

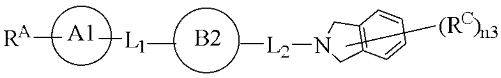
(I-2)

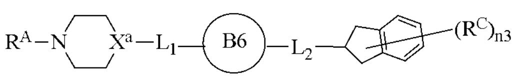
(I-3)

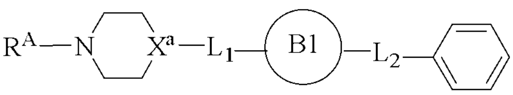
(I-4)

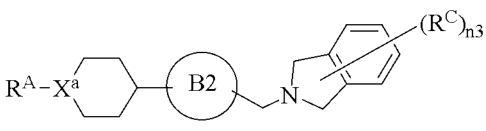
(I-5)

X is selected from CH or N;

$X^a$ is selected from CH or N;

A is selected from 4- to 14-membered heterocyclyl, 4- to 6-membered heterocycloalkylene, $(C_{3-4}$ cycloalkyl$)_{0-1}$-NH-$SO_2$-$C_{1-4}$ alkyl, or 5- or 6-membered cycloalkyl, wherein the heterocyclyl or heterocycloalkylene contains 1-3

heteroatoms selected from N, O or S; the cycloalkyl, heterocyclyl, heterocycloalkylene or alkyl is optionally substituted with 1-4 $R^A$;

in some embodiments, A is selected from 4- to 14-membered heterocyclyl, 5- or 6-membered cycloalkyl, or phenyl, wherein the heterocyclyl contains 1-3 heteroatoms selected from N, O or S; the heterocyclyl, cycloalkyl or phenyl is optionally substituted with 1-4 $R^A$;

in some embodiments, A is selected from 4- to 6-membered monocyclic heterocyclyl, 5- or 6-membered heterocyclyl fused 5- or 6-membered heterocyclyl, 5- or 6-membered heterocyclyl spiro 5- or 6-membered cycloalkyl, 5- or 6-membered heterocyclyl fused 5- or 6-membered cycloalkyl, 4- to 6-membered heterocycloalkyl spiro 5- or 6-membered heterocycloalkyl, 5- or 6-membered cycloalkyl fused 5- or 6-membered heterocycloalkyl, 5- or 6-membered cycloalkyl spiro 5- or 6-membered heterocycloalkyl, 10- to 14-membered partially unsaturated tricyclic heterocycloalkyl, cyclopropyl-$NHSO_2$-$C_{1-2}$ alkyl, -$NHSO_2$-$C_{1-2}$ alkyl, cyclohexyl, or 4-membered heterocycloalkylene, wherein the cycloalkyl, heterocycloalkyl or heterocyclyl is optionally substituted with 1-4 $R^A$;

in some embodiments, A is selected from 4- to 10-membered heterocycloalkyl, 4- to 6-membered heterocycloalkylene, ($C_{3-4}$ cycloalkyl)$_{0-1}$-$NHSO_2$-$C_{1-2}$ alkyl, or 5- or 6-membered cycloalkyl, wherein the heterocycloalkyl contains 1-3 heteroatoms selected from N, O or S; the cycloalkyl, heterocycloalkyl or alkyl is optionally substituted with 1-4 $R^A$;

in some embodiments, A is selected from 4- to 8-membered heterocycloalkyl, 4- to 6-membered heterocycloalkylene, ($C_{3-4}$ cycloalkyl)$_{0-1}$-$NHSO_2$-$C_{1-2}$ alkyl, or 5- or 6-membered cycloalkyl, wherein the heterocycloalkyl contains 1-3 heteroatoms selected from N, O or S; the cycloalkyl, heterocycloalkyl or alkyl is optionally substituted with 1-4 $R^A$;

in some embodiments, A is selected from 4- to 6-membered heterocycloalkyl, 4- to 6-membered heterocycloalkylene, ($C_{3-4}$ cycloalkyl)$_{0-1}$-$NHSO_2$-$C_{1-2}$ alkyl, or 5- or 6-membered cycloalkyl, wherein the heterocycloalkyl contains 1-3 heteroatoms selected from N, O or S; the cycloalkyl, heterocycloalkyl or alkyl is optionally substituted with 1-4 $R^A$;

in some embodiments, A is selected from 4- to 6-membered monocyclic heterocycloalkyl, 5- or 6-membered heterocyclyl fused 5- or 6-membered heterocyclyl, 5- or 6-membered heterocyclyl spiro 5- or 6-membered cycloalkyl, 5- or 6-membered heterocyclyl fused 5- or 6-membered cycloalkyl, 4- to 6-membered heterocycloalkyl spiro 5- or 6-membered heterocycloalkyl, 5- or 6-membered cycloalkyl fused 5- or 6-membered heterocycloalkyl, 5- or 6-membered cycloalkyl spiro 5- or 6-membered heterocycloalkyl, 10- to 14-membered partially unsaturated tricyclic heterocycloalkyl, cyclopropyl-$NHSO_2$-$C_{1-2}$ alkyl, -$NHSO_2$-$C_{1-2}$ alkyl, cyclohexyl, or 4-membered heterocycloalkylene, wherein the cycloalkyl, heterocycloalkyl or heterocyclyl is optionally substituted with 1-4 $R^A$;

in some embodiments, A is selected from azetidinyl, azacyclopentyl, azacyclohexyl, epoxybutane, epoxypentane, epoxyhexane, 4-membered heterocycloalkylene, cyclopropyl-$NHSO_2$-$C_{1-2}$ alkyl, cyclobutyl-$NHSO_2$-$C_{1-2}$ alkyl, cyclopentyl, or cyclohexyl, wherein the contains 1-3 heteroatoms selected from N, O or S; the cyclopropyl, cyclobutyl, cyclopentyl, cyclohexyl, epoxybutane, epoxypentane, epoxyhexane, heterocycloalkyl or alkyl is optionally substituted with 1-4 $R^A$;

in some embodiments, A is selected from 4- to 6-membered monocyclic heterocyclyl, wherein the heterocyclyl is optionally substituted with 1-4 $R^A$;

in some embodiments, A is selected from 7- to 9-membered bicyclic heterocyclyl, wherein the heterocyclyl is optionally substituted with 1-4 $R^A$;

in some embodiments, A is selected from one of the groups formed by the following structures optionally substituted with 1-4 $R^A$:

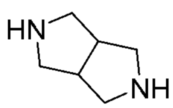, 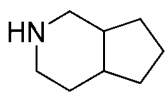, 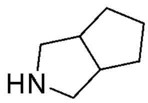, 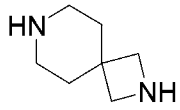, 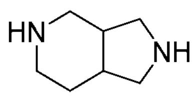,

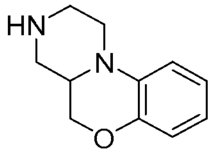, 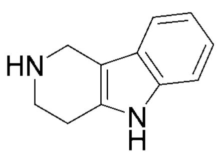, 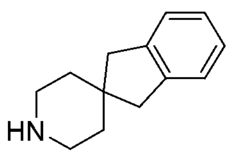, 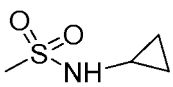, 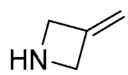,

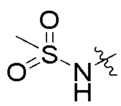, 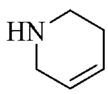, 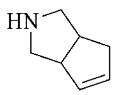, or 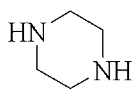;

in some embodiments, A is selected from one of the groups formed by the following structures optionally substituted with 1-4 $R^A$:

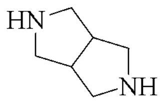, 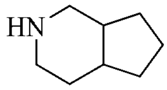,

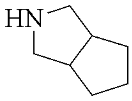, 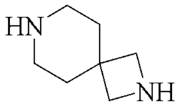, 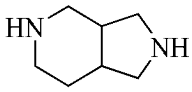, 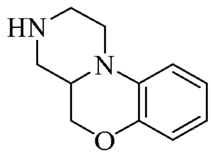, 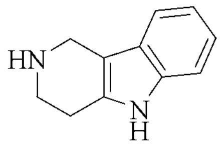,

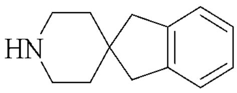, 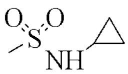, 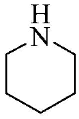, 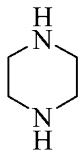, 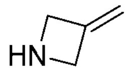, 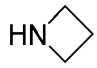, 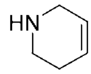, 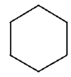,

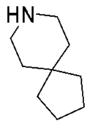, or 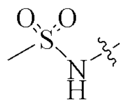;

in some embodiments, A is selected from one of the groups formed by the following structures optionally substituted with 1-4 $R^A$:

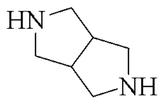, 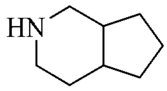,

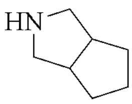, 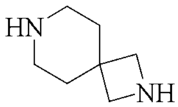, 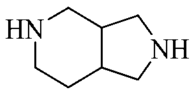, 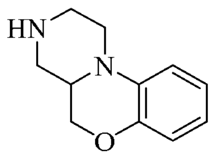, 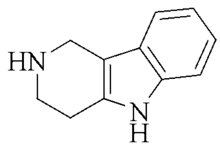,

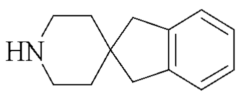, 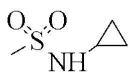, 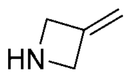, or 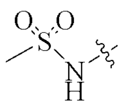;

in some embodiments, A is selected from one of the groups formed by the following structures:

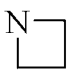, 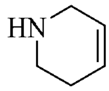, or 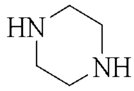;

in some embodiments, the ring A is selected from one of the groups formed by the following structures:

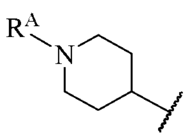, 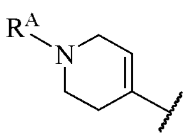, 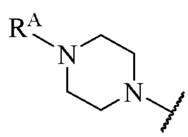,

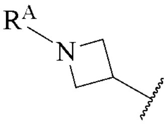, 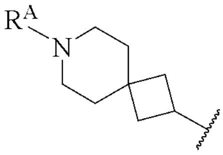, 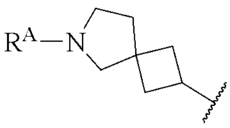, 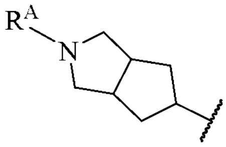,

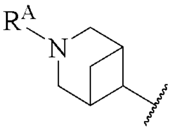, 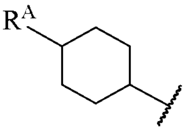, 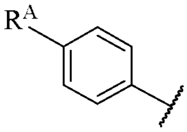, 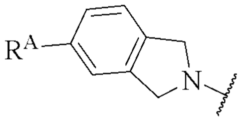, 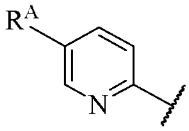,

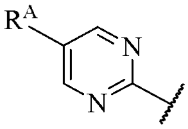, or 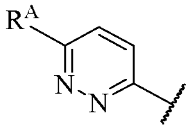;

in some embodiments, A1 is selected from the following groups:

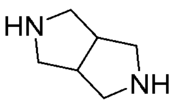,

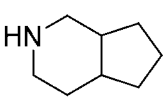, 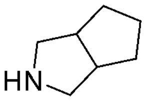, 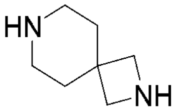, 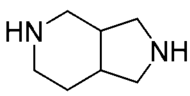, 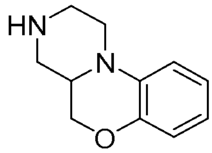,

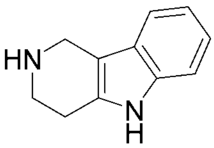, 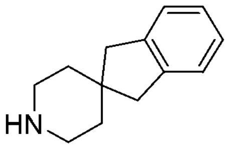, 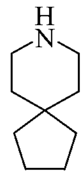, 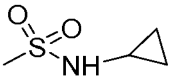, 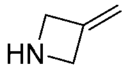, 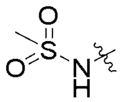,

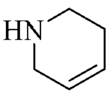, 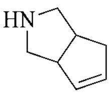, 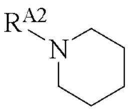, 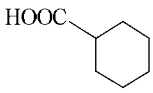 or 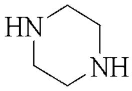,

or is selected from

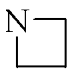,

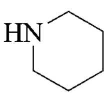, 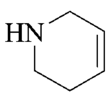, 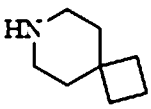, 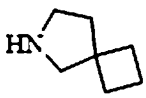, 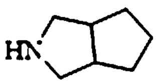 or 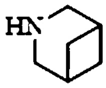,

or is selected from

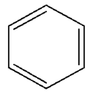,

or is selected from

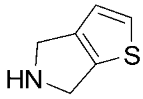, 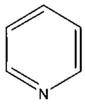, 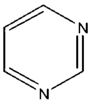 or 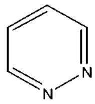;

in some embodiments, A1 is selected from the following groups:

: 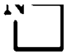,

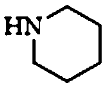, 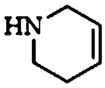, 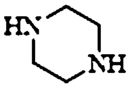, 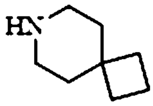, 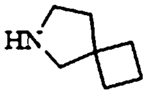, 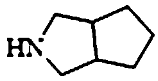, or 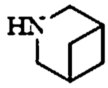;

in some embodiments, A1 is selected from the following groups:

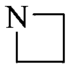,

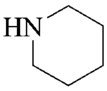, 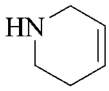, or 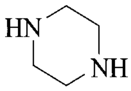;

in some embodiments, A1 is selected from the following groups:

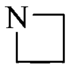,

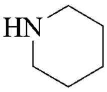, 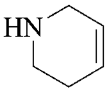, 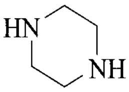, 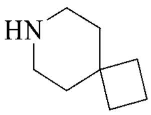, 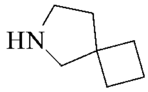, 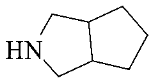, 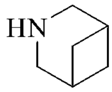,

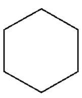, 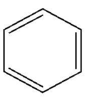, or 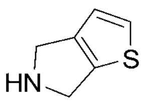;

in some embodiments, A1 is selected from the following groups:

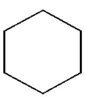;

$R^{A2}$ is selected from $-NH-S(O)_2-C_{1-4}$ alkyl, $-S(O)_2-(CH_2)_p-R^a$, $-P(O)-(C_{1-4}$ alkyl$)_2$, $-C(O)-OR^a$, or $-(CH_2)_p-C(O)-(CH_2)_p-R^a$, wherein the alkyl is optionally further substituted with 1-4 groups selected from halogen, OH, $NH_2$, CN, or -O-halo $C_{1-4}$ alkyl;

A2 is selected from 4- to 6-membered monocyclic heterocycloalkyl, 5- or 6-membered heterocyclyl fused 5- or 6-membered heterocyclyl, 5- or 6-membered heterocyclyl spiro 5- or 6-membered cycloalkyl, 5- or 6-membered heterocyclyl fused 5- or 6-membered cycloalkyl, 4- to 6-membered heterocycloalkyl spiro 5- or 6-membered heterocycloalkyl, 5- or 6-membered cycloalkyl fused 5- or 6-membered heterocycloalkyl, 5- or 6-membered cycloalkyl spiro 5- or 6-membered heterocycloalkyl, 10- to 14-membered partially unsaturated tricyclic heterocycloalkyl, cyclopropyl-$NHSO_2$-$C_{1-2}$ alkyl, -$NHSO_2$-$C_{1-2}$ alkyl, or 4-membered heterocycloalkylene, wherein the cycloalkyl, heterocycloalkyl or heterocyclyl is optionally substituted with 1-4 $R^A$;
in some embodiments, the A2 ring is selected from the following groups optionally substituted with 1-4 $R^A$:

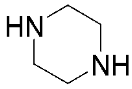 , or 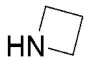 ;

A3 is selected from the following groups optionally substituted with 1-4 $R^A$:

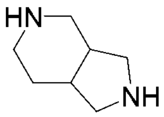 , 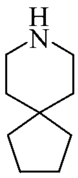 , 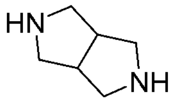 , 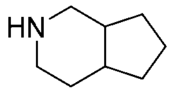 , 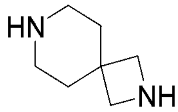 , or 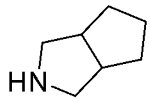 ;

in some embodiments,

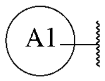

in the formula (I-a),

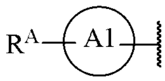

in the formula (1-2),

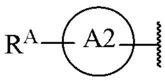

in the formula (I-b), and

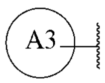

in the formula (II-a) are each independently selected from one of the groups formed by the following structures:

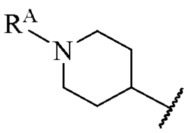 , 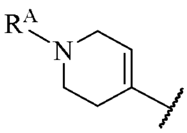 , 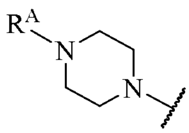 , 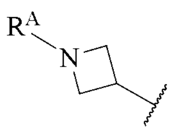 ,

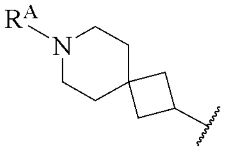, 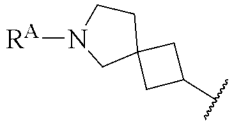, 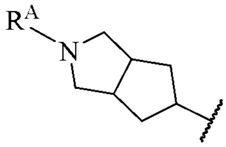, 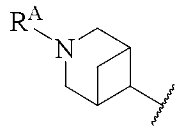,

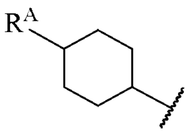, 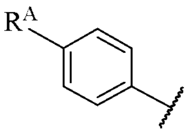, 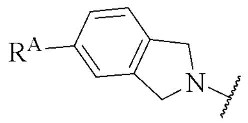, 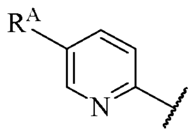,

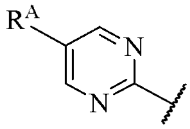, or 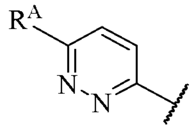;

B is selected from phenyl, 6- to 12-membered bicyclic heterocyclyl, or 6- to 12-membered heteroaryl, wherein the heterocyclyl or heteroaryl contains 1-3 heteroatoms selected from N, O or S; the heterocyclyl, heteroaryl or phenyl is optionally substituted with 1-4 $R^B$;

in some embodiments, B is selected from 6- to 12-membered bicyclic carbocyclyl, 6- to 12-membered bicyclic heterocyclyl, or 6- to 12-membered heteroaryl, wherein the heterocyclyl or heteroaryl contains 1-3 heteroatoms selected from N, O or S; the carbocyclyl, heterocyclyl or heteroaryl is optionally substituted with 1-4 $R^B$;

in some embodiments, B is selected from phenyl, 6- to 10-membered bicyclic heterocyclyl, or 6- to 10-membered heteroaryl, wherein the heterocyclyl or heteroaryl contains 1-3 heteroatoms selected from N, O or S; the heterocyclyl, heteroaryl or phenyl is optionally substituted with 1-4 $R^B$;

in some embodiments, B is selected from phenyl, 6- to 8-membered bicyclic heterocyclyl, or 6- to 8-membered heteroaryl, wherein the heterocyclyl or heteroaryl contains 1-3 heteroatoms selected from N, O or S; the heterocyclyl, heteroaryl or phenyl is optionally substituted with 1-4 $R^B$;

in some embodiments, B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, phenyl, benzo 5- or 6-membered heterocyclyl, or 5- or 6-membered heterocyclyl fused 5- or 6-membered heteroaryl, wherein the heterocyclyl, heteroaryl, carbocyclyl, phenyl or aryl is optionally substituted with 1-4 $R^B$;

in some embodiments, B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, or benzo 5- or 6-membered heteroaryl, wherein the heterocyclyl, heteroaryl, carbocyclyl or aryl is optionally substituted with 1-4 $R^B$;

in some embodiments, B is selected from the following groups optionally substituted with 1-4 $R^B$:

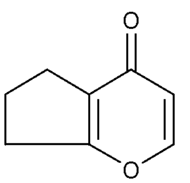, 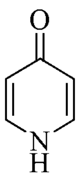, 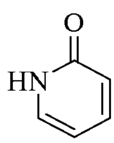, 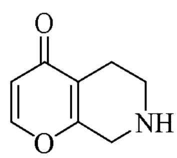,

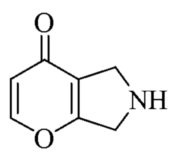, 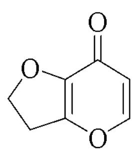, 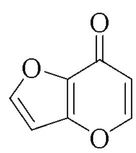, 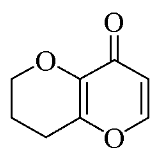, 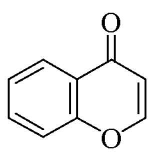, 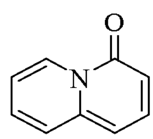,

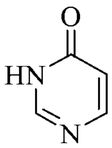, 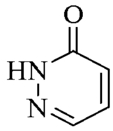, 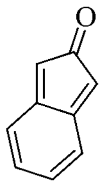, 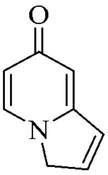, 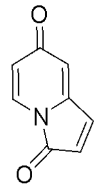, 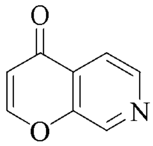, 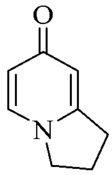, 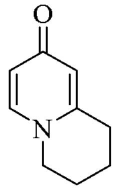,

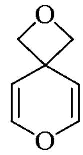, 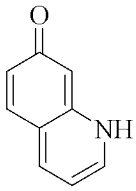, 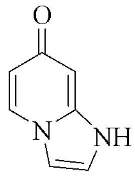, 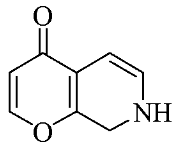, 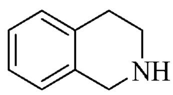, 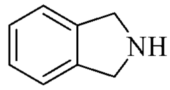,

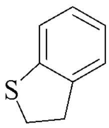, 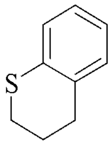, 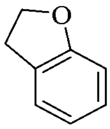, 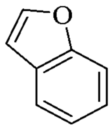, 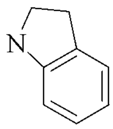, 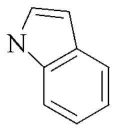, 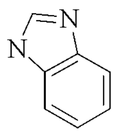, 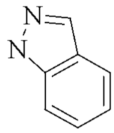,

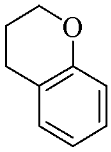, 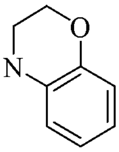, 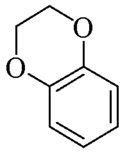, 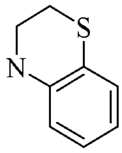, 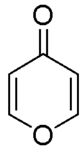, 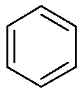, 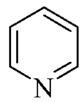, 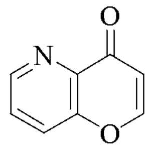,

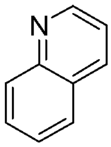 or 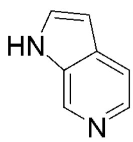, 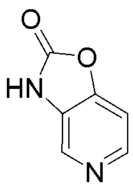, 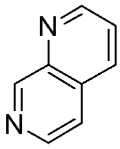, 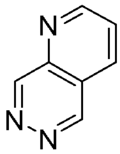, 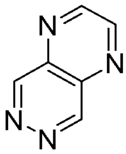, 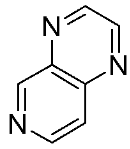,

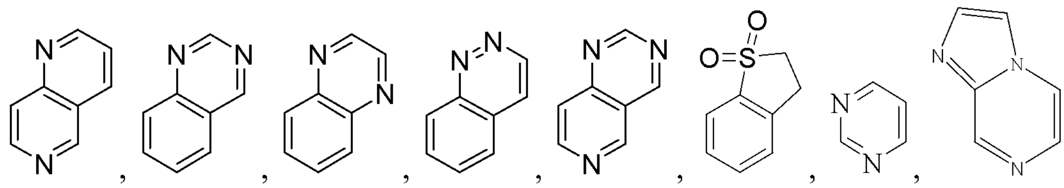, , , , , , , ,

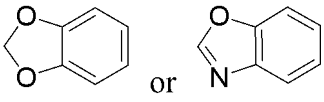 or ,

or is selected from

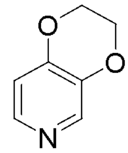,

or is selected from

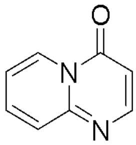 ;

in some embodiments, B is selected from the following groups optionally substituted with 1-4 $R^B$:

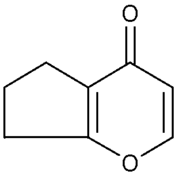 , 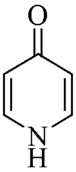 , 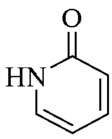 , 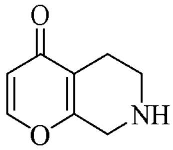 ,

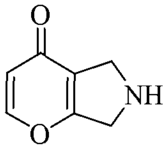 , 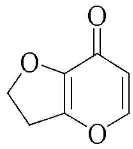 , 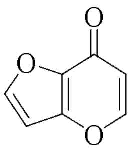 , 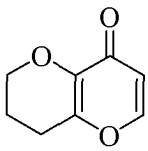 , 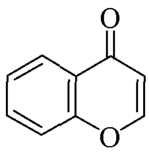 , 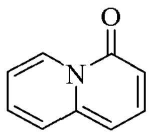 ,

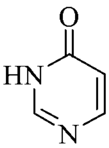 , 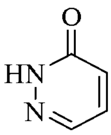 , 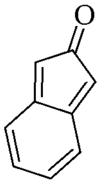 , 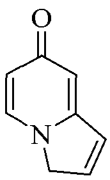 , 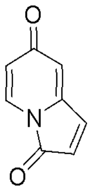 , 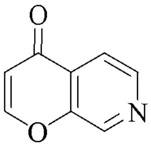 , 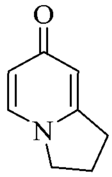 , 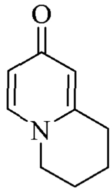 ,

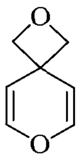 , 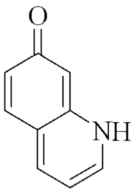 , 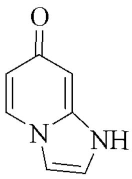 , 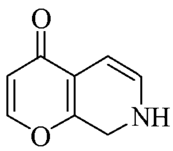 , 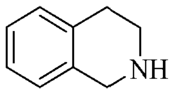 , 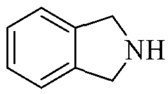 ,

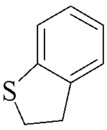 , 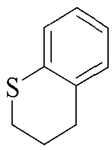 , 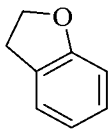 , 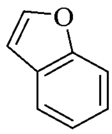 , 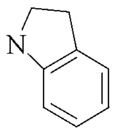 , 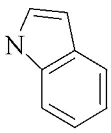 , 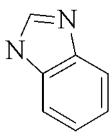 , 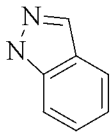 ,

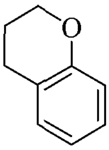 , 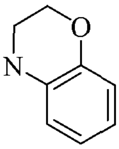 , 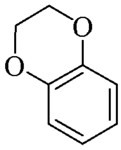 , 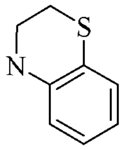 , 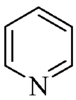 , or 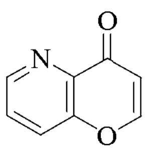 ;

in some embodiments, B is selected from one of the groups formed by the following structures optionally substituted with 1-4 $R^B$:

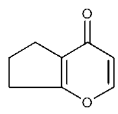, 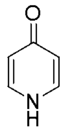, 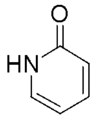,

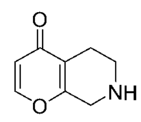, 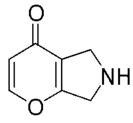, 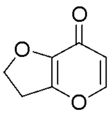, 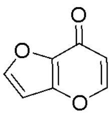, 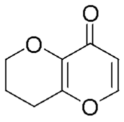, 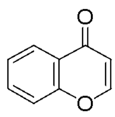, 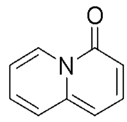, 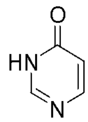,

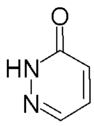, 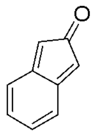, 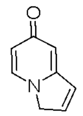, 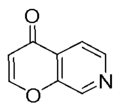, 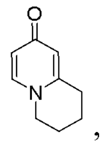, 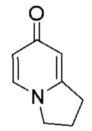, 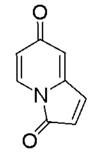, 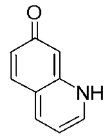, 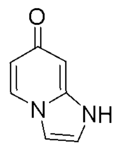,

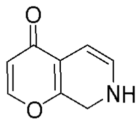, 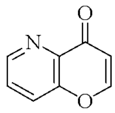, or 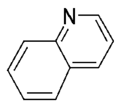;

in some embodiments,

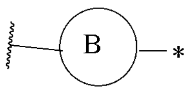

is selected from the following groups optionally substituted with 1-4 $R^B$:

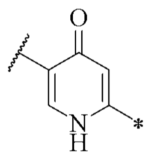, 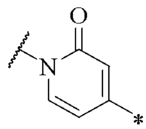, 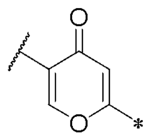,

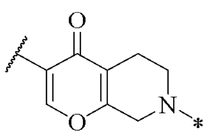, 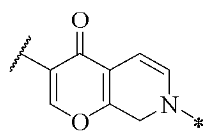, 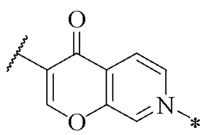, 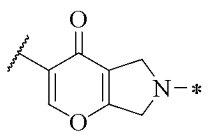,

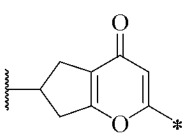, 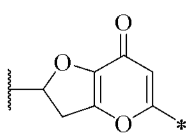, 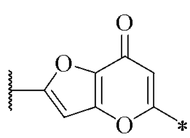, 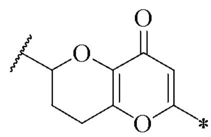,

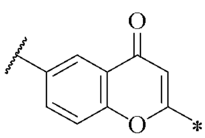, 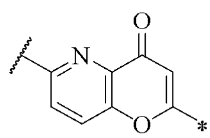, 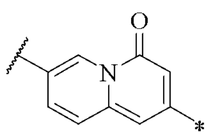, 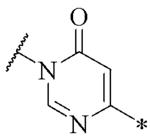, 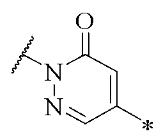,

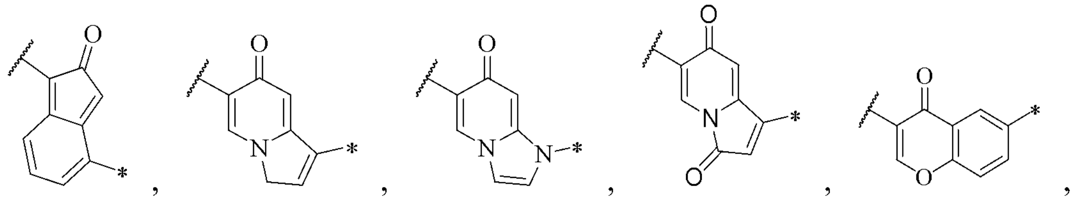
, , , , ,

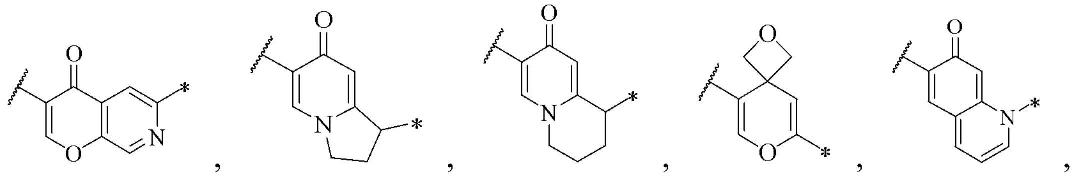
, , , , ,

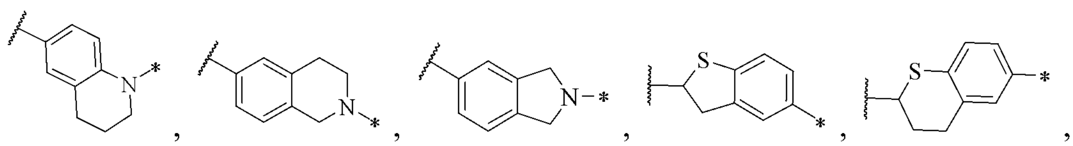
, , , , ,

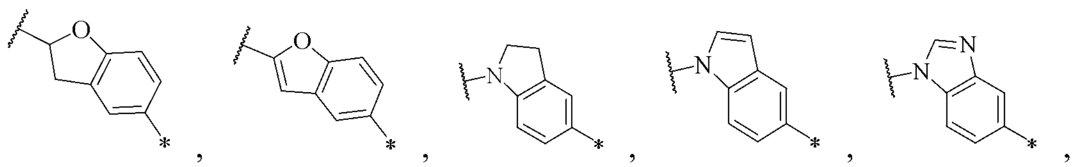
, , , , ,

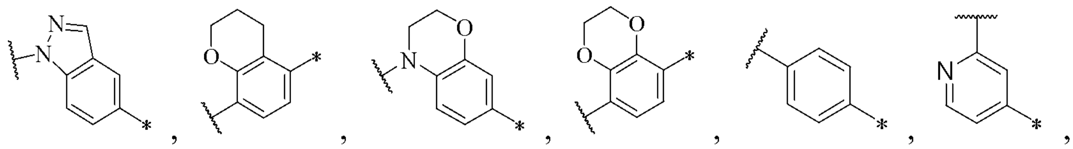
, , , , , ,

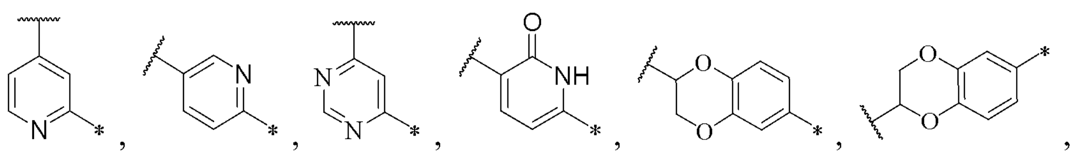
, , , , , ,

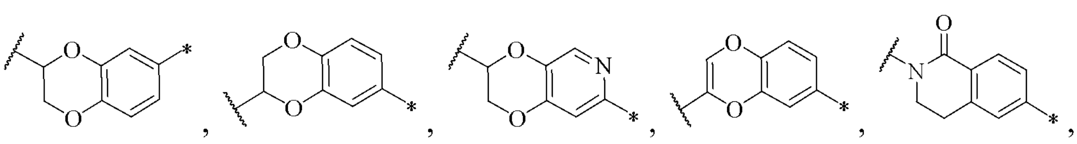
, , , , ,

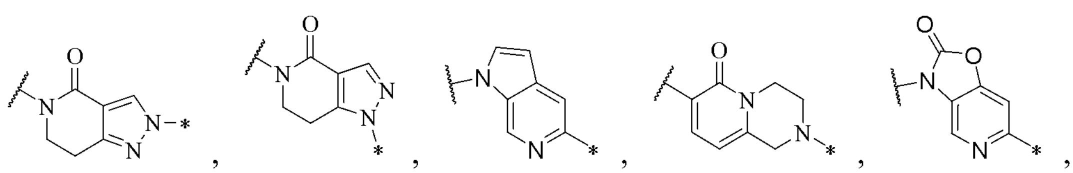
, , , , ,

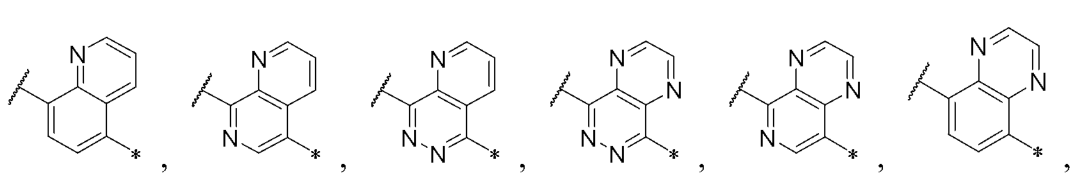
, , , , , ,

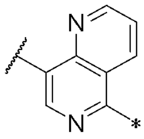, 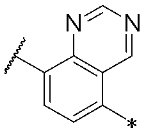, 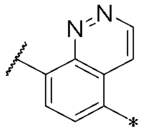, 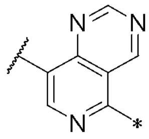, 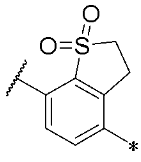, 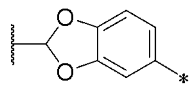,

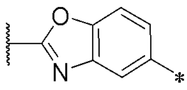, 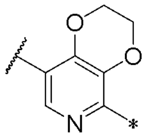, or 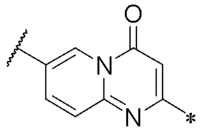,

wherein ~~~ represents the connection with $L_1$, and * represents the connection with $L_2$;
B1 is selected from the following groups optionally substituted with 1-4 $R^B$:

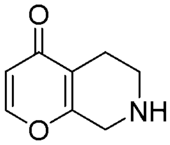, 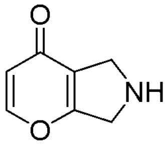, 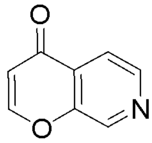, 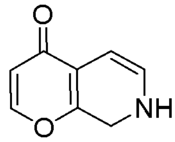, 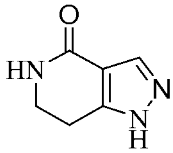,

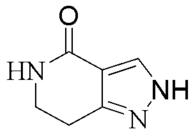, 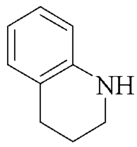, 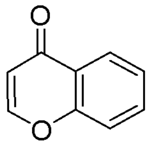, 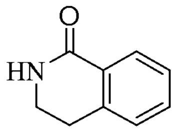, 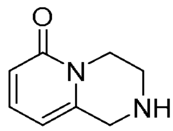,

or

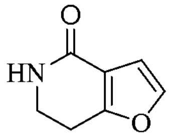;

in some embodiments, B1 is selected from the following groups optionally substituted with 1-4 $R^B$:

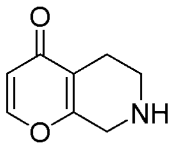, 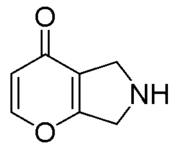, 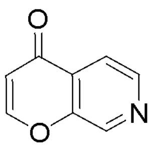 or 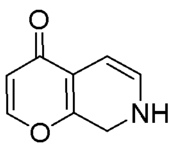,

or is selected from the following groups optionally substituted with 1-4 $R^B$:

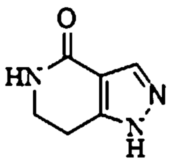, 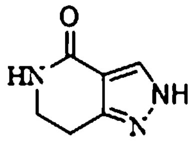, 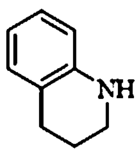, 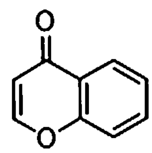, or 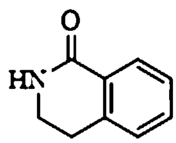;

in some embodiments,

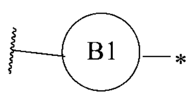

is selected from the following groups optionally substituted with 1-4 $R^B$:

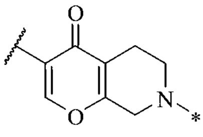 , 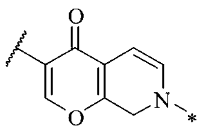 ,

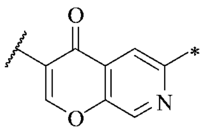 , 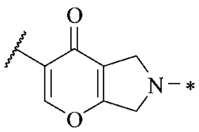 , 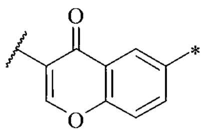 , 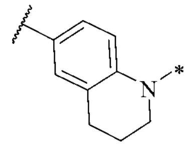 ,

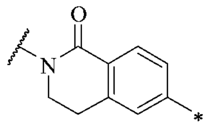 , 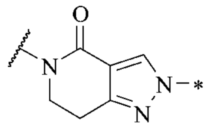 , 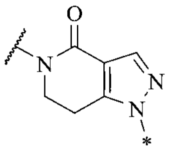 , 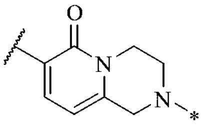 ,

wherein ~~~ represents the connection with $L_1$, and * represents the connection with $L_2$;
B2 is selected from the following groups optionally substituted with 1-4 $R^B$:

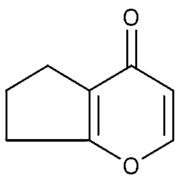 , 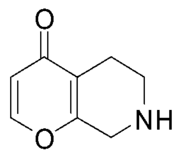 , 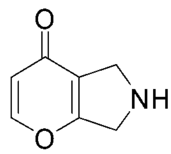 , 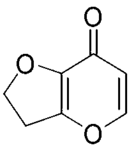 , 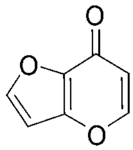 , 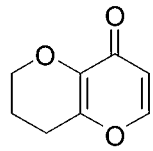 ,

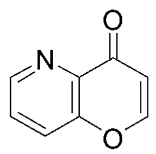 , 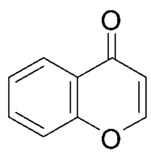 , 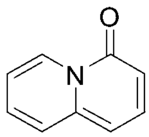 , 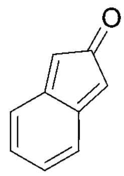 , 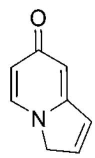 , 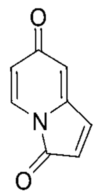 , 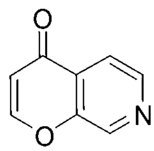 ,

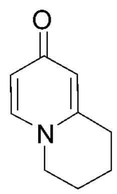 , 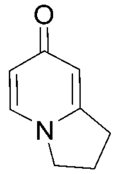 , 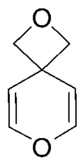 , 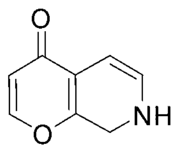 , 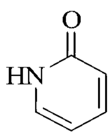 , 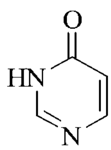 , 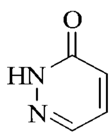 , 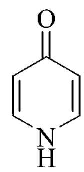 ,

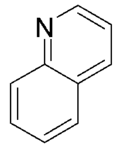 , 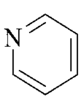 , 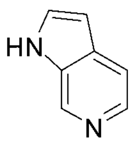 , 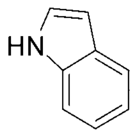 , 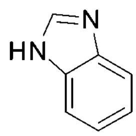 , 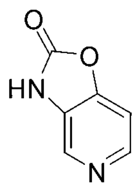 , 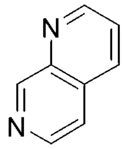 ,

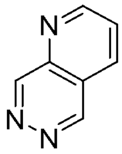, 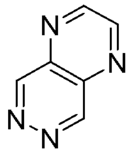, 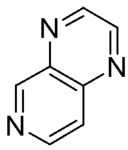, 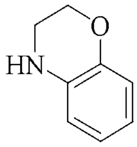, 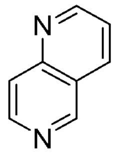, 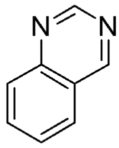, 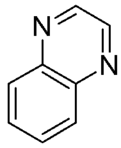,

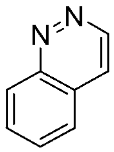, 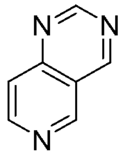, 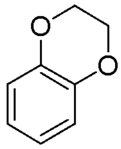, 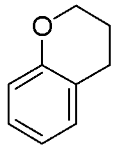, 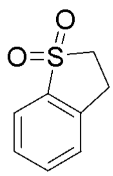, 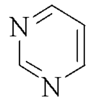 or 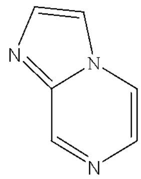,

or is selected from

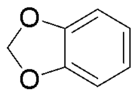, 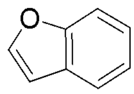, 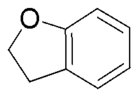 or 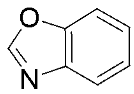;

in some embodiments, B2 is selected from the following groups optionally substituted with 1-4 $R^B$:

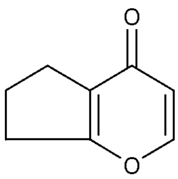, 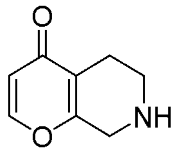, 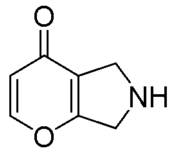, 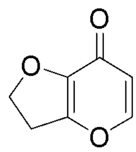,

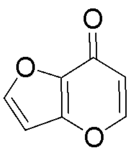, 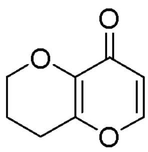, 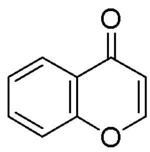, 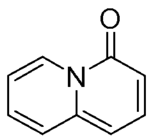, 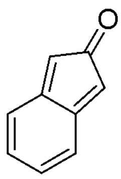, 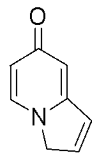, 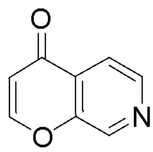,

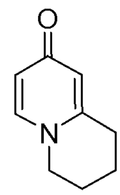, 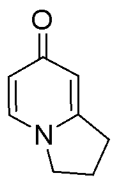, 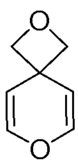, 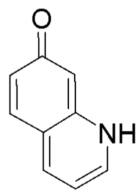, 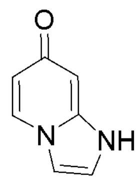, 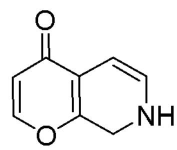, 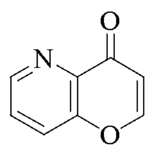,

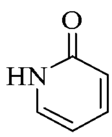, 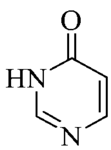, 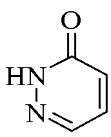, or 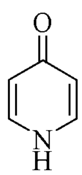;

in some embodiments, B2 is selected from the following groups optionally substituted with 1-4 $R^B$:

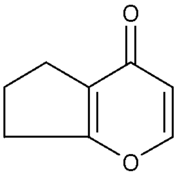, 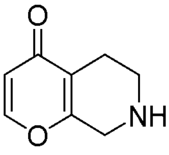, 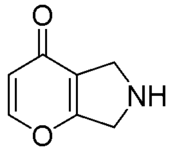, 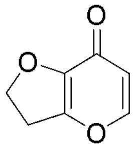,

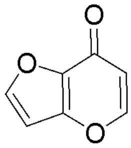, 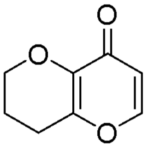, 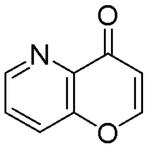, 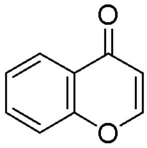, 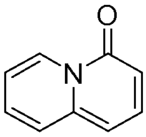, 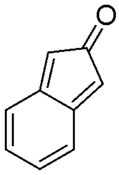, 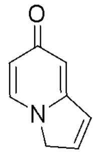,

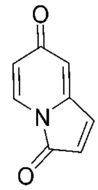, 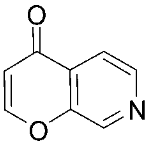, 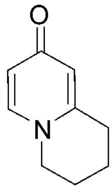, 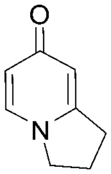, 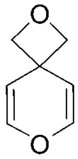, 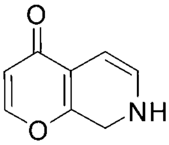, 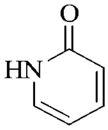, 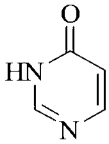,

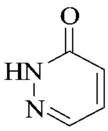, 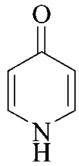 or 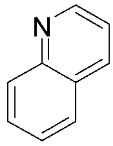,

or is selected from the following groups optionally substituted with 1-4 $R^B$:

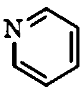;

in some embodiments, the B2 ring is selected from the following groups optionally substituted with 1-4 $R^B$:

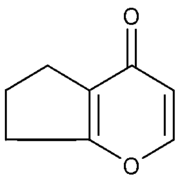, 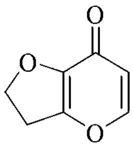, or 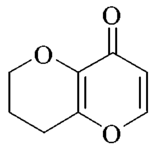;

in some embodiments, the B2 ring is selected from the following groups optionally substituted with 1-4 $R^B$:

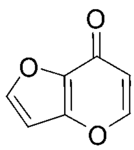, or 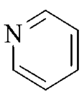;

in some embodiments,

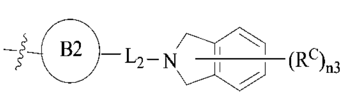 is 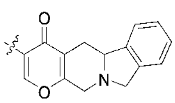;

in some embodiments,

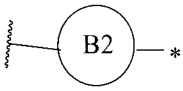

is selected from the following groups optionally substituted with 1-4 $R^B$:

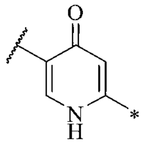, 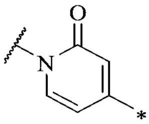, 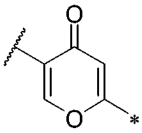,

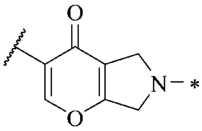, 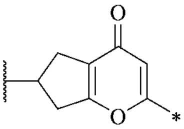, 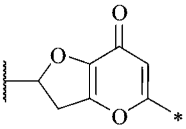, 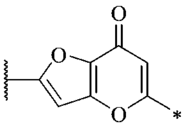, 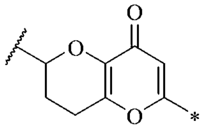,

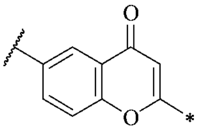, 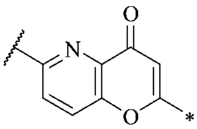, 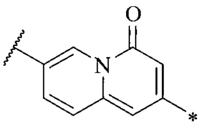, 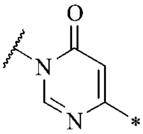, 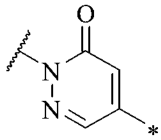,

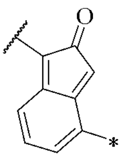, 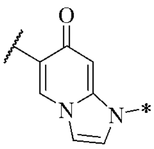, 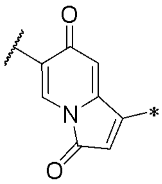, 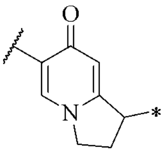, 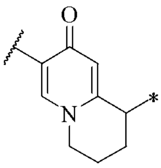, 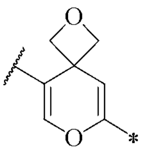,

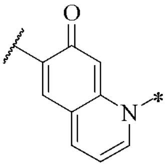, 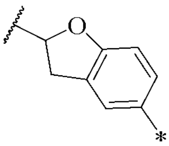, 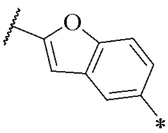, 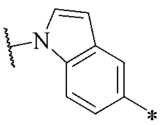, 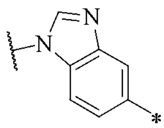,

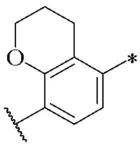, 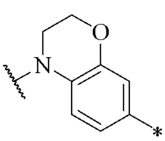, 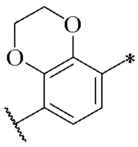, 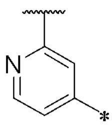, 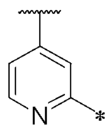, 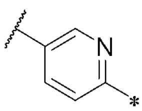, 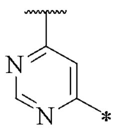,

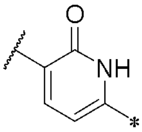, 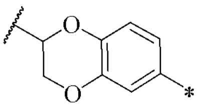, 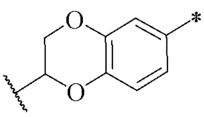, 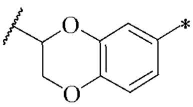, 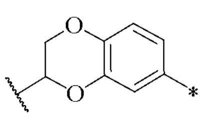,

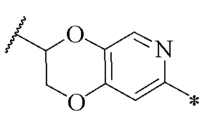, 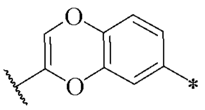, 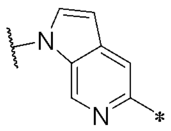, 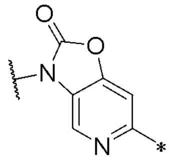, 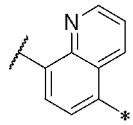,

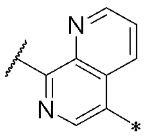, 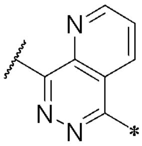, 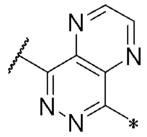, 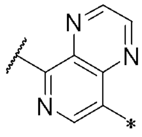, 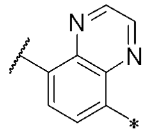, 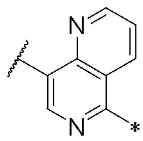,

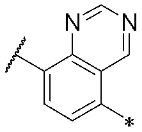, 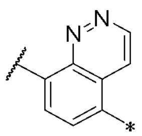, 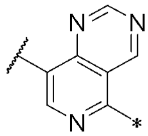, 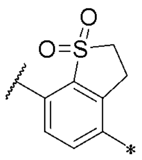, 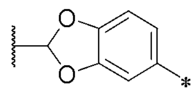, 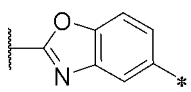,

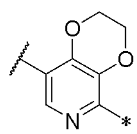, or 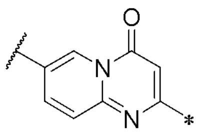,

wherein ~~~ represents the connection with $L_1$, and * represents the connection with $L_2$;
B3 is selected from the following groups optionally substituted with 1-4 $R^B$:

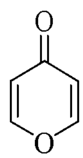, 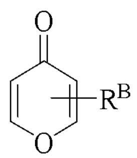

or B2;
in some embodiments, B3 is selected from the following groups optionally substituted with 1-4 $R^B$:

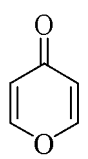, 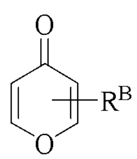, 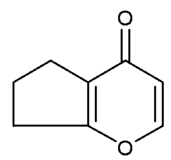, 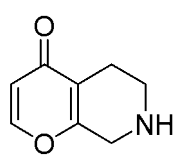,

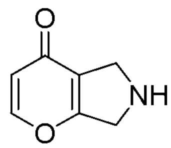, 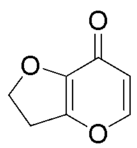, 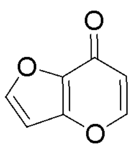, 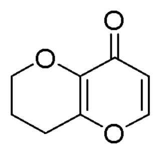, 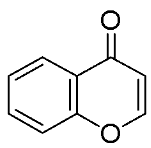, 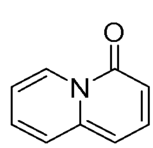,

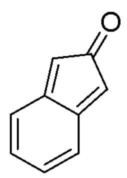, 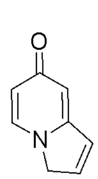, 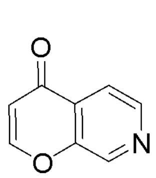, 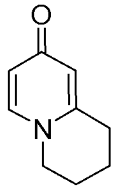, 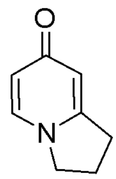, 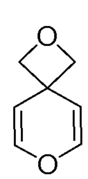, 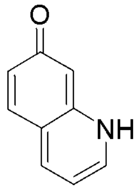, 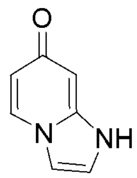,

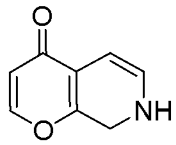, 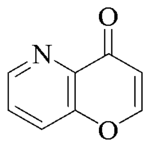, 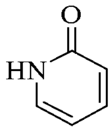, 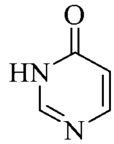, 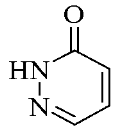, or 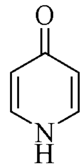;

in some embodiments, the B3 ring is selected from the following groups optionally substituted with 1-4 $R^B$:

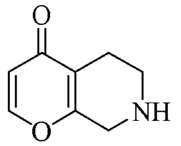, 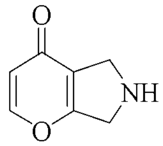, 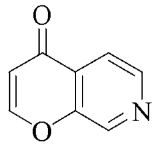,

or

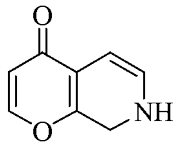;

in some embodiments,

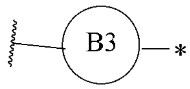

is selected from the following groups optionally substituted with 1-4 $R^B$:

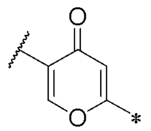, 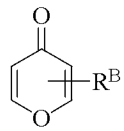, 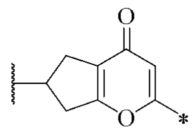,

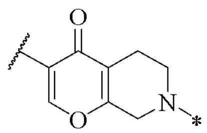, 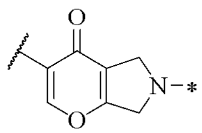, 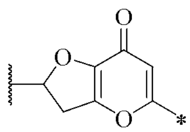, 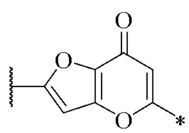,

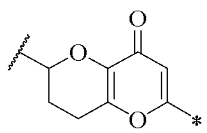, 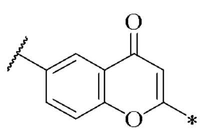, 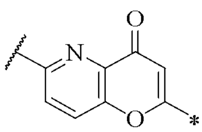, 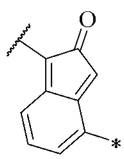, 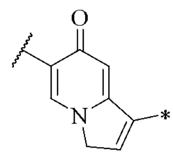,

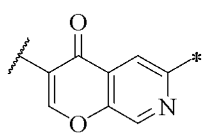, 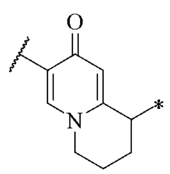, 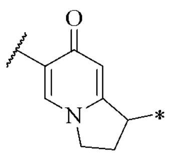, 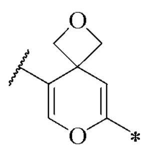, 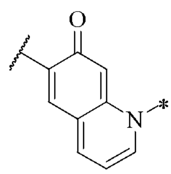,

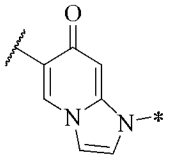, 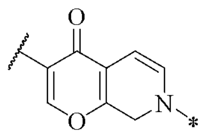, 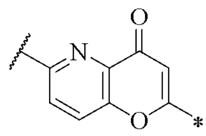, 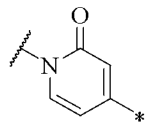, 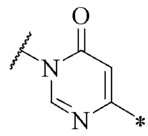,

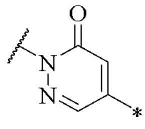, or 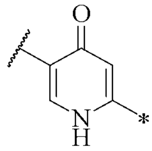,

wherein ~~~ represents the connection with $L_1$, and * represents the connection with $L_2$;
in some embodiments, the B4 ring is selected from the following groups optionally substituted with 1-4 $R^B$:

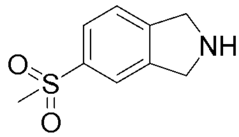, or 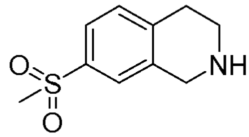;

the B5 ring the following groups optionally substituted with 1-4 $R^B$:

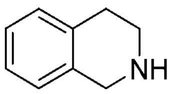, 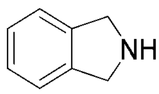, 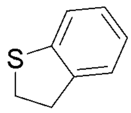, 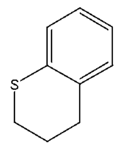, 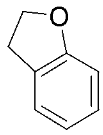, 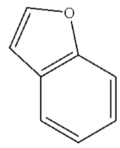, 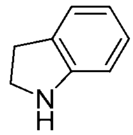,

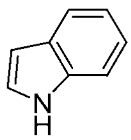, 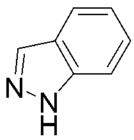, 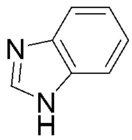, 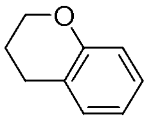, 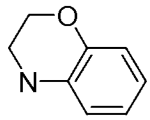, 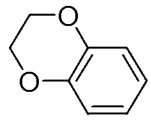,

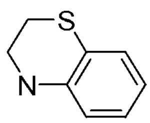, 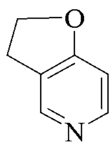, 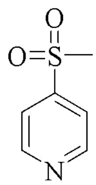, or 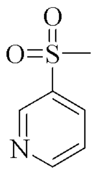;

in some embodiments,

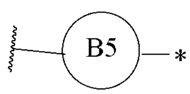

is selected from the following groups optionally substituted with 1-4 $R^B$:

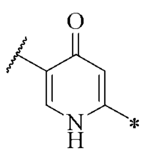, 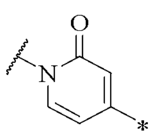, 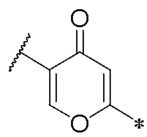,

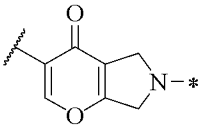 , 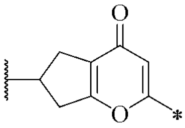 , 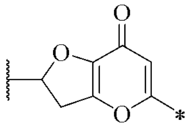 , 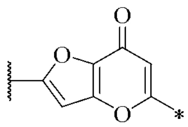 , 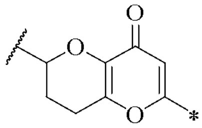 ,

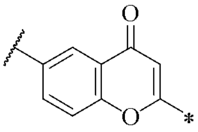 , 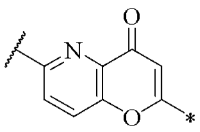 , 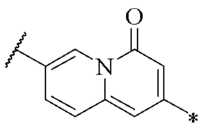 , 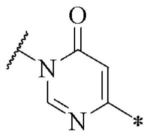 , 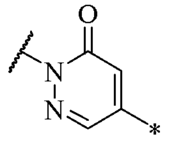 ,

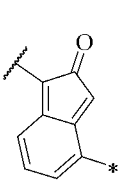 , 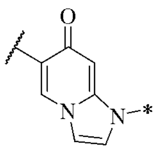 , 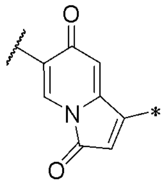 , 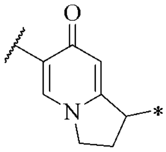 , 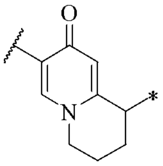 , 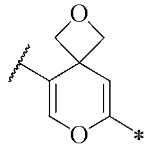 ,

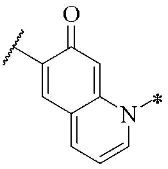 , 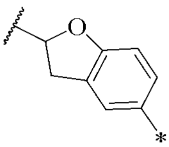 , 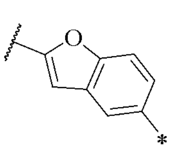 , 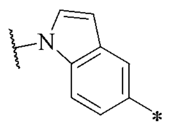 , 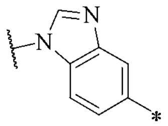 ,

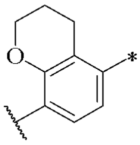 , 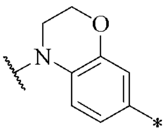 , 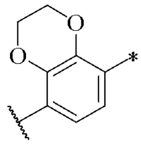 , 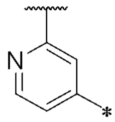 , 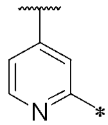 , 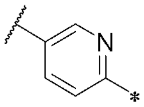 , 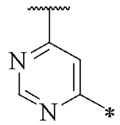 ,

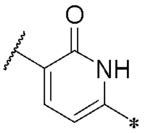 , 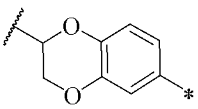 , 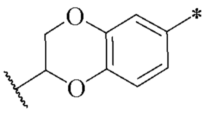 , 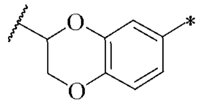 , 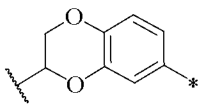 ,

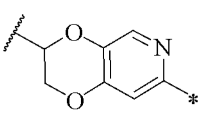 , 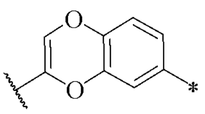 , 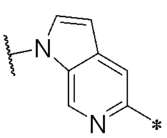 , 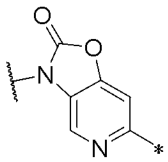 , 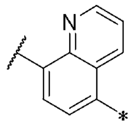 ,

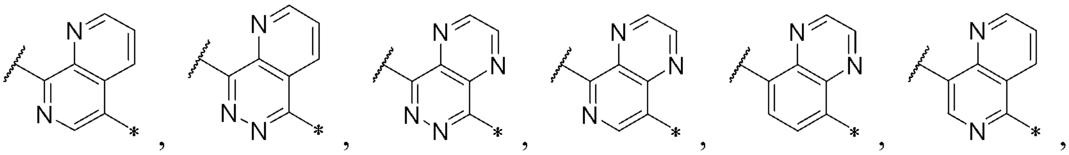 , , , , , ,

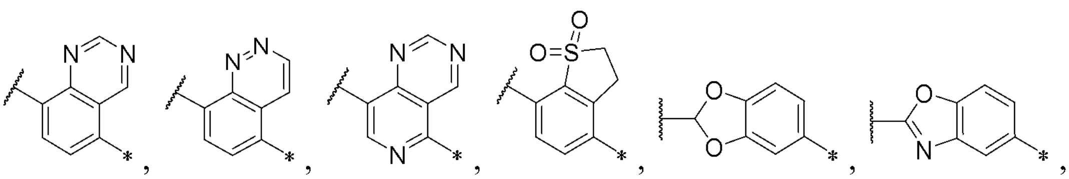 , , , , , ,

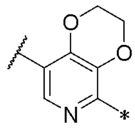 , or 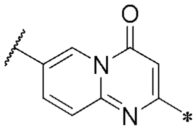 ,

wherein ~~~ represents the connection with $L_1$, and * represents the connection with $L_2$;
B6 is selected from the following groups optionally substituted with 1-4 $R^B$:

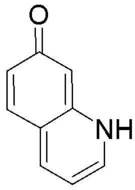 or 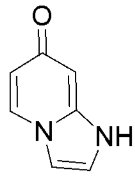 ;

in some embodiments,

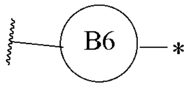

is selected from the following groups optionally substituted with 1-4 $R^B$:

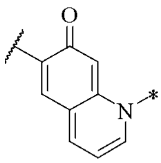 , or 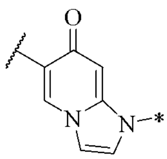 ,

wherein ~~~ represents the connection with $L_1$, and * represents the connection with $L_2$;
C is selected from phenyl, 6- to 12-membered partially unsaturated bicyclic heterocyclyl, 8- to 14-membered tricyclic heterocyclyl, $Y_1$-phenyl, or $Y_1$-5- to 10-membered heteroaryl, wherein the heterocyclyl or heteroaryl contains 1-3 heteroatoms selected from N, O or S; the heterocyclyl, heteroaryl or phenyl is optionally substituted with 1-4 $R^C$;
in some embodiments, C is selected from phenyl, 6- to 12-membered bicyclic carbocyclyl, 6- to 12-membered partially unsaturated bicyclic heterocyclyl, or 8- to 14-membered tricyclic heterocyclyl, wherein the heterocyclyl contains 1-3 heteroatoms selected from N, O or S; the cycloalkyl, heterocyclyl or phenyl is optionally substituted with 1-4 $R^C$;
in some embodiments, C is selected from phenyl, 6- to 10-membered partially unsaturated bicyclic heterocyclyl, 8- to 11-membered tricyclic heterocyclyl, $Y_1$-phenyl, or $Y_1$-5- to 8-membered heteroaryl, wherein the heterocyclyl or heteroaryl contains 1-3 heteroatoms selected from N, O or S; the heterocyclyl, heteroaryl or phenyl is optionally substituted with 1-4 $R^C$;
in some embodiments, C is selected from phenyl, 6- to 8-membered partially unsaturated bicyclic heterocyclyl, 8- to 10-membered tricyclic heterocyclyl, $Y_1$-phenyl, or $Y_1$-5- to 7-membered heteroaryl, wherein the heterocyclyl or heteroaryl contains 1-3 heteroatoms selected from N, O or S; the heterocyclyl, heteroaryl or phenyl is optionally substituted with 1-4 $R^C$;
in some embodiments, C is selected from phenyl, 6- or 7-membered partially unsaturated bicyclic heterocyclyl, 8- to 10-membered tricyclic heterocyclyl, $Y_1$-phenyl, or $Y_1$-5- or 6-membered heteroaryl, wherein the heterocyclyl or heteroaryl contains 1-3 heteroatoms selected from N, O or S; the heterocyclyl, heteroaryl or phenyl is optionally substituted with 1-4 $R^C$;
in some embodiments, C is selected from phenyl, 5- to 7-membered heterocyclyl fused phenyl, 5- or 6-membered carbocyclyl fused phenyl, 5- or 6-membered heterocyclyl fused 5- or 6-membered heteroaryl, 5- or 6-membered heterocycloalkyl fused 3- to 6-membered cycloalkyl, 9-12 membered tricyclic heterocyclyl, $Y_1$-phenyl, or $Y_1$-5- or 6-membered heteroaryl, wherein the heterocyclyl, heterocycloalkyl or heteroaryl contains 1-3 heteroatoms selected from N, O or S; the phenyl, heterocyclyl, carbocyclyl, heteroaryl, heterocycloalkyl or cycloalkyl is optionally substituted with 1-4 $R^C$;
in some embodiments, C is selected from phenyl, 5- to 7-membered heterocyclyl fused phenyl, 5- or 6-membered carbocyclyl fused phenyl, or 5- or 6-membered heterocyclyl fused 5- or 6-membered heteroaryl, wherein the

heterocyclyl contains 1-3 heteroatoms selected from N, O or S; the phenyl, heterocyclyl or carbocyclyl is optionally substituted with 1-4 $R^C$;

in some embodiments, C is selected from phenyl, 5- to 7-membered heterocyclyl fused phenyl, or 5- or 6-membered carbocyclyl fused phenyl, wherein the heterocyclyl contains 1-3 heteroatoms selected from N, O or S; the phenyl, heterocyclyl or carbocyclyl is optionally substituted with 1-4 $R^C$;

in some embodiments, C is selected from the following groups optionally substituted with 1-4 $R^C$:

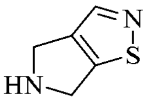, 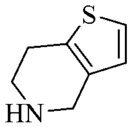, 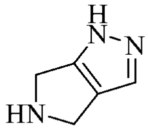, 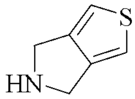,

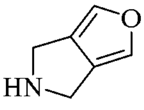, 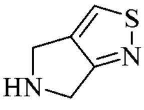, 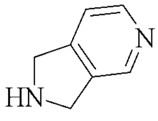, 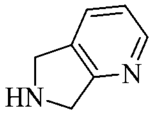, 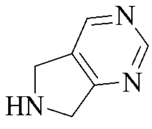, 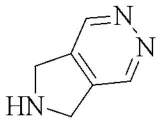,

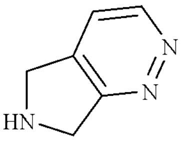, 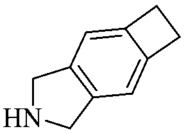, 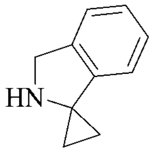, 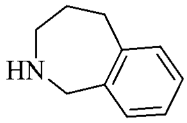, 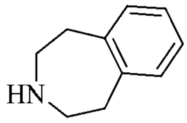,

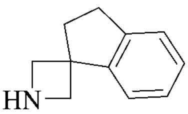, 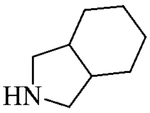, 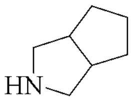, 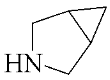, 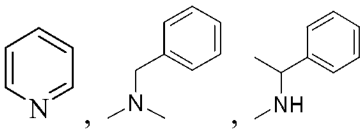,

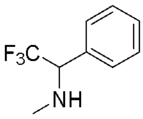, 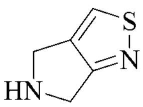, 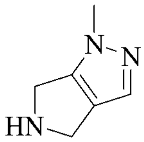, 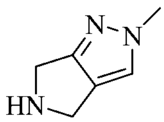, 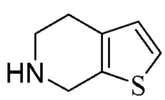, 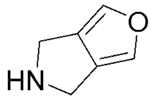,

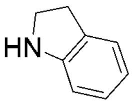, 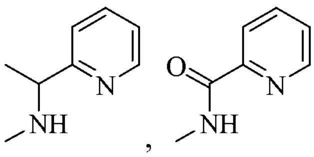, 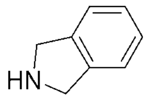, or 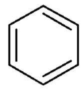;

in some embodiments, C is selected from the following groups optionally substituted with 1-4 $R^C$:

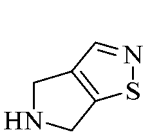, 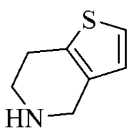, 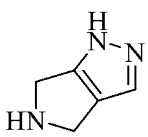, 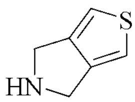,

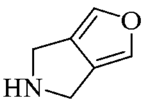, 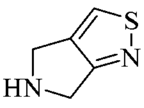, 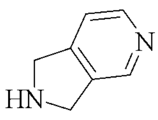, 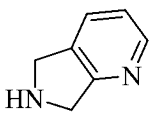, 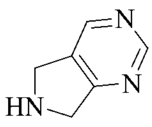, 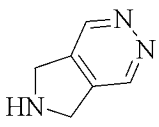,

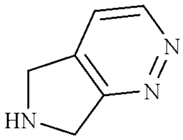, 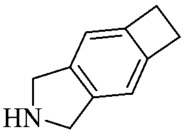, 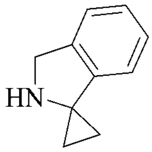, 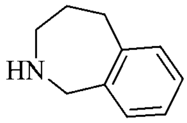, 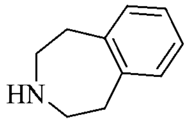,

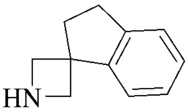, 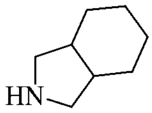, 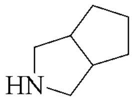, 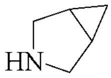, 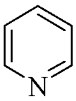, 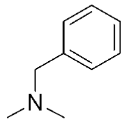, 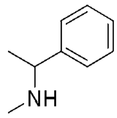,

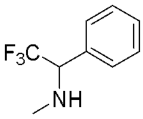, 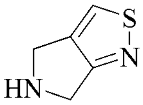, 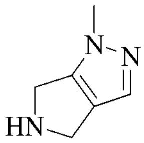, 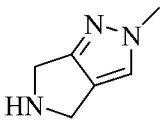, 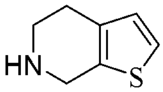, 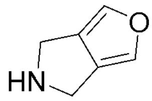,

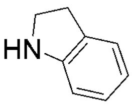, 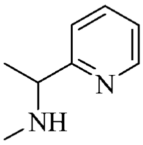, or 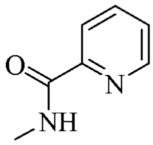;

in some embodiments, the C ring is selected from one of the following groups optionally substituted with 1-4 $R^C$:

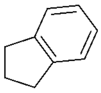 or 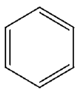;

C1 is selected from the following groups optionally substituted with 1-4 $R^C$:

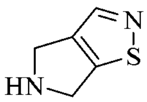, 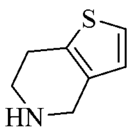, 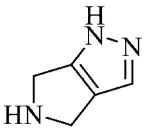, 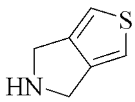, 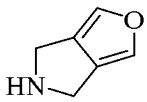, 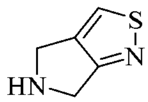,

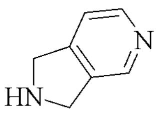, 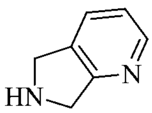, 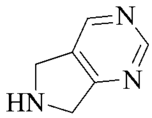, 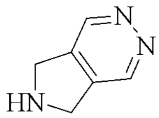, 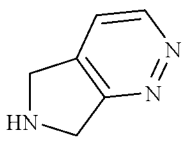,

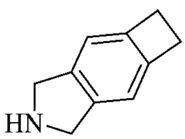, 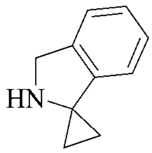, 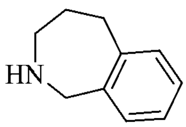, 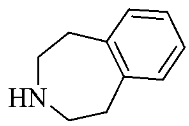, 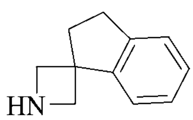,

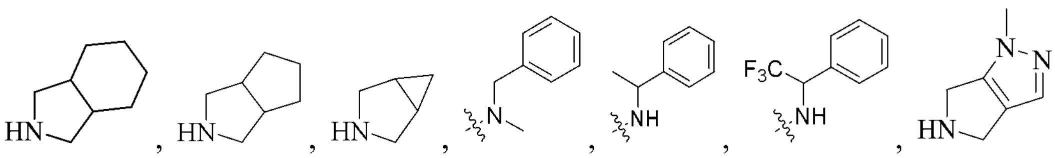,

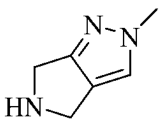, 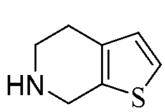, 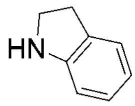, 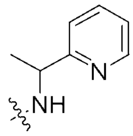, 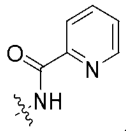, or 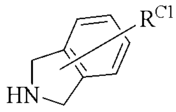;

in some embodiments, $R^{C1}$ is selected from -O-$C_{3-5}$ cycloalkyl, $C_{3-5}$ cycloalkyl, $C_{2-4}$ alkynyl, -$SCF_3$, -$SF_5$, =O, -P(O)($C_{1-4}$ alkyl)$_2$, -NH-P(O)($C_{1-4}$ alkyl)$_2$, or -$CH_2$-O-$C_{1-4}$ alkyl;
in some embodiments, $R^{C1}$ is selected from -O-$C_{3-5}$ cycloalkyl, $C_{3-5}$ cycloalkyl, $C_{2-4}$ alkynyl, -$SCF_3$, -$SF_5$, -$(NH)_p$-P(O)($C_{1-4}$ alkyl)$_2$, or -$(CH_2)_p$-O-$C_{1-4}$ alkyl;
in some embodiments, $R^{C1}$ is selected from cyclopropyl, - O-cyclopropyl, acetylene, -$SCF_3$, -$SF_5$, -NH-P(O)$(CH_3)_2$, -P(O)$(CH_3)_2$, -$CH_2$-O-$CH_3$;
in some embodiments, the C1 ring is selected from the following groups optionally substituted with 1-4 $R^C$:

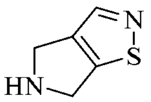, 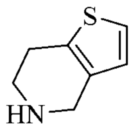, 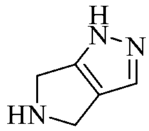, 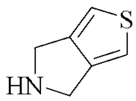,

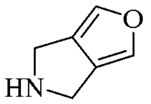, 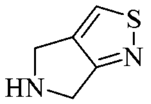, 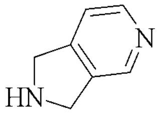, 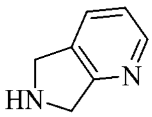, 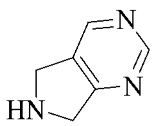, 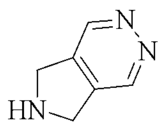,

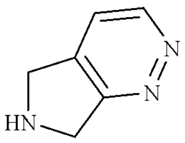, 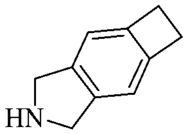, 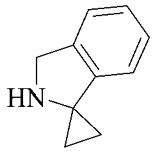, 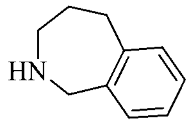, 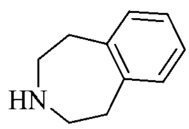,

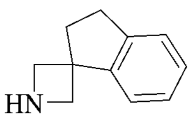, 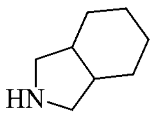, 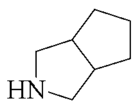, 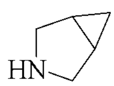, 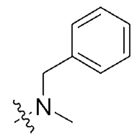, 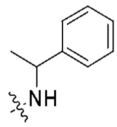,

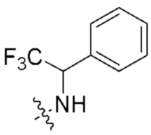, 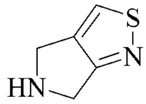, 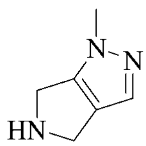, 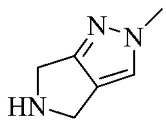, 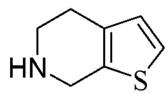, 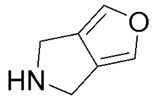,

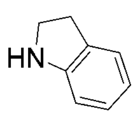, 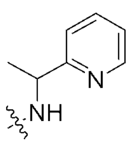, or 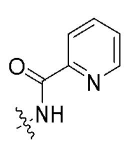;

each $R^A$ is independently selected from H, halogen, CN, OH, COOH, $C_{1-4}$ alkyl, $C_{2-6}$ alkenyl, $C_{2-6}$ alkynyl, -O$C_{1-4}$ alkyl, -NH-S(O)$_2$-$C_{1-4}$ alkyl, -S(O)$_2$-$C_{1-4}$ alkyl, - S(O)$_2$-$(CH_2)_p$-$R^a$, -P(O)-($C_{1-4}$ alkyl)$_2$, -C(O)-O$R^a$, -$(CH_2)_p$-C(O)-$(CH_2)_p$-$R^a$, -NHC(O)-$R^a$, -C(O)N($C_{1-4}$ alkyl)$_2$, -NHC(O)-$C_{1-4}$ alkyl, 3- to 6-membered heterocycloalkyl, or -NH$R^a$, wherein the alkyl, alkenyl, alkynyl or heterocycloalkyl is optionally further substituted with 1-4 groups selected from halogen, OH, $NH_2$, CN, -O-halo $C_{1-4}$ alkyl, deuterium or $C_{1-4}$ alkyl; the heterocycloalkyl contains 1-3 heteroatoms selected from N, O or S;
in some embodiments, each $R^A$ is independently selected from H, halogen, CN, OH, COOH, $C_{1-4}$ alkyl, $C_{2-4}$ alkenyl, $C_{2-4}$ alkynyl, -O$C_{1-4}$ alkyl, -NH-S(O)$_2$-$C_{1-2}$ alkyl, -S(O)$_2$-$C_{1-2}$ alkyl, -S(O)$_2$-$(CH_2)_p$-$R^a$, -P(O)-($C_{1-2}$ alkyl)$_2$, -C(O)-O$R^a$,

$-(CH_2)_p-C(O)-(CH_2)_p-R^a$, $-NHC(O)-R^a$, $-C(O)N(C_{1-4}$ alkyl$)_2$, or $-NHC(O)-C_{1-4}$ alkyl, wherein the alkyl, alkenyl, or alkynyl is optionally further substituted with 1-4 groups selected from halogen, OH, $NH_2$, CN, -O-halo $C_{1-2}$ alkyl or deuterium;

in some embodiments, $R^A$ is selected from $-S(O)_2-(CH_2)_p-R^a$, $-P(O)-(C_{1-2}$ alkyl$)_2$, $-C(O)-OR^a$, $-(CH_2)_p-C(O)-(CH_2)_p-R^a$, $-S(O)_2-C_{1-2}$ alkyl, $-NHC(O)-R^a$, - $C(O)N(C_{1-4}$ alkyl$)_2$, or $-NHC(O)-C_{1-2}$ alkyl, wherein the alkyl is optionally further substituted with 1-4 groups selected from halogen, OH, $NH_2$, CN, -O-halo $C_{1-2}$ alkyl or deuterium;

in some embodiments, $R^A$ is selected from $-S(O)_2-(CH_2)_p-R^a$, $-S(O)_2-C_{1-2}$ alkyl, $-C(O)-OR^a$, $-C(O)-CH_2-R^a$, $-NHC(O)-R^a$, $-C(O)N(C_{1-4}$ alkyl$)_2$, or $-NHC(O)-C_{1-2}$ alkyl, wherein the alkyl is optionally further substituted with 1-4 groups selected from F, Cl, Br, OH, $NH_2$, CN or D;

in some embodiments, each $R^A$ is independently selected from H, halogen, CN, OH, $NO_2$, COOH, $C_{1-4}$ alkyl, $C_{2-6}$ alkenyl, $C_{2-6}$ alkynyl, $-OC_{1-4}$ alkyl, $-NH-S(O)_2-C_{1-4}$ alkyl, $-S(O)_2-C_{1-4}$ alkyl, $-S(O)_2-(CH_2)_p-R^a$, $-P(O)-(C_{1-4}$ alkyl$)_2$, $-C(O)-OR^a$, - $(CH_2)_p-C(O)-(CH_2)_p-R^a$, or $-NHC(O)-R^a$, or $-C(O)N(C_{1-4}$ alkyl$)_2$, wherein the alkyl, alkenyl, or alkynyl is optionally further substituted with 1-4 groups selected from halogen, OH, $NH_2$, CN, -O-halo $C_{1-4}$ alkyl or deuterium;

in some embodiments, each $R^A$ is independently selected from H, halogen, CN, OH, COOH, $C_{1-2}$ alkyl, $C_{2-4}$ alkenyl, $C_{2-4}$ alkynyl, $-OC_{1-2}$ alkyl, $-NH-S(O)_2-C_{1-2}$ alkyl, $-S(O)_2-C_{1-4}$ alkyl, $-S(O)_2-(CH_2)_p-R^a$, $-P(O)-(C_{1-2}$ alkyl$)_2$, $-C(O)-OR^a$, $-(CH_2)_p-C(O)-(CH_2)_p-R^a$, or $-NHC(O)-R^a$, wherein the alkyl, alkenyl, or alkynyl is optionally further substituted with 1-4 groups selected from halogen, OH, $NH_2$, CN or -O-halo $C_{1-4}$ alkyl;

in some embodiments, each $R^A$ is independently selected from COOH, $-OC_{1-4}$ alkyl, $-NH-S(O)_2-C_{1-2}$ alkyl, $-S(O)_2-C_{1-2}$ alkyl, $-S(O)_2-C_{1-2}$ alkyl-$R^a$, $-P(O)-(C_{1-2}$ alkyl$)_2$, $-C(O)-OR^a$, $-(CH_2)_p-C(O)-(CH_2)_p-R^a$, or $-NHC(O)-R^a$, wherein the alkyl, alkenyl, or alkynyl is optionally further substituted with 1-4 groups selected from halogen, OH, $NH_2$, CN or -O-halo $C_{1-2}$ alkyl;

in some embodiments, each $R^A$ is independently selected from COOH, $-OC_{1-2}$ alkyl, $-NH-S(O)_2-C_{1-2}$ alkyl, $-S(O)_2-C_{1-2}$ alkyl, $-S(O)_2-C_{1-2}$ alkyl-$R^a$, $-P(O)-(C_{1-2}$ alkyl$)_2$, $-C(O)-OR^a$, $-(CH_2)_p-C(O)-(CH_2)_p-R^a$, or $-NHC(O)-R^a$, wherein the alkyl, alkenyl, or alkynyl is optionally further substituted with 1-4 groups selected from halogen, OH, $NH_2$, CN or -O-halo $C_{1-2}$ alkyl;

in some embodiments, each $R^A$ is independently selected from COOH, $-OCH_3$, $-OCH_2CH_3$, $-NH-S(O)_2-CH_3$, $-NH-S(O)_2-CH_2CH_3$, $-S(O)_2-CH_3$, $-S(O)_2-CH_2CH_3$, - $S(O)_2-CH_3-R^a$, $-S(O)_2-CH_2CH_3-R^a$, $-P(O)-(CH_2CH_3)_2$, $-P(O)-(CH_3)_2$, $-C(O)-OR^a$, - $CH_2-C(O)-R^a$, $-C(O)-CH_2-R^a$, or $-NHC(O)-R^a$;

in some embodiments, each $R^A$ is independently selected from: $-P(O)-(C_{1-4}$ alkyl$)_2$, $-NH-S(O)_2-C_{1-4}$ alkyl, -COOH, $-C_{1-4}$ alkyl-C(O)-4- to 6-membered heterocycloalkyl, $-C(O)-C_{1-4}$ alkyl-4- to 6-membered heterocycloalkyl, $-C(O)-C_{1-4}$ alkyl-$C_{3-6}$ cycloalkyl, $-C_{1-4}$ alkyl-C(O)-O-4- to 6-membered heterocycloalkyl, - $S(O)_2-C_{1-4}$ alkyl-4- to 6-membered heterocycloalkyl, $-S(O)_2-C_{1-4}$ alkyl-$C_{2-4}$ alkynyl, $-S(O)_2-C_{1-4}$ alkyl-$C_{3-5}$ cycloalkyl, $-S(O)_2-C_{1-4}$ alkyl-5- or 6-membered heteroaryl, - $S(O)_2-C_{1-4}$ alkyl-O-halo $C_{1-4}$ alkyl, or $-NHC(O)-C_{3-5}$ cycloalkyl;

in some embodiments, each $R^A$ is independently selected from $-S(O)_2-C_{1-2}$ alkyl-$R^a$, $-P(O)-(C_{1-2}$ alkyl$)_2$, $-C(O)-OR^a$, $-(CH_2)_p-C(O)-(CH_2)_p-R^a$, or $-NHC(O)-C_{3-5}$ cycloalkyl, wherein the alkyl is optionally further substituted with 1-4 groups selected from halogen, OH, $NH_2$, CN or -O-halo $C_{1-2}$ alkyl;

in some embodiments, each $R^A$ is independently selected from $-S(O)_2-C_{1-2}$ alkyl, $-S(O)_2-C_{1-2}$ alkyl-$R^a$, $-P(O)-(C_{1-2}$ alkyl$)_2$, or $-NHC(O)-C_{3-5}$ cycloalkyl, wherein the alkyl is optionally further substituted with 1-4 groups selected from halogen, OH, $NH_2$, CN or -O-halo $C_{1-2}$ alkyl;

in some embodiments, $R^A$ is selected from $-S(O)_2-C_{1-2}$ alkyl-$R^a$, $-P(O)-(C_{1-2}$ alkyl$)_2$, $-C(O)-OR^a$, $-(CH_2)_p-C(O)-(CH_2)_p-R^a$, $-S(O)_2-C_{1-2}$ alkyl, or $-NHC(O)-C_{3-5}$ cycloalkyl, wherein the alkyl is optionally further substituted with 1-4 groups selected from halogen, OH, $NH_2$, CN or -O-halo $C_{1-2}$ alkyl;

in some embodiments, $R^A$ is selected from $-S(O)_2-C_{1-2}$ alkyl-$R^a$, $-S(O)_2-C_{1-2}$ alkyl, $-C(O)-OR^a$, $-C(O)-CH_2-R^a$, or $-NHC(O)-C_{3-5}$ cycloalkyl, wherein the alkyl is optionally further substituted with 1-4 groups selected from F, Cl, Br, OH, NH2 or CN;

in some embodiments, $R^A$ is selected from $-S(O)_2-C_{1-2}$ alkyl-$R^a$, or $-S(O)_2-C_{1-2}$ alkyl, wherein the alkyl is optionally further substituted with 1-4 groups selected from F, Cl, Br, OH, $NH_2$ or CN;

in some embodiments, $R^A$ is selected from $-S(O)_2-C_{1-2}$ alkyl, wherein the alkyl is optionally further substituted with 1-4 groups selected from F, Cl, Br, OH, $NH_2$ or CN;

in some embodiments, $R^A$ is $-NHC(O)-R^a$;

in some embodiments, each $R^A$ is independently selected from $-S(O)_2-CH_3$, - $P(O)-(CH_3)_2$,

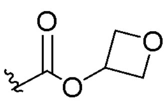 , 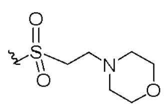 , 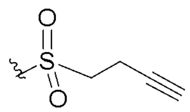 , 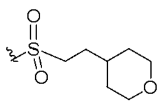 , 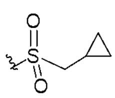 ,

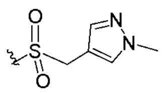, 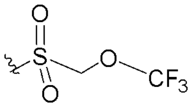, 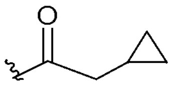, 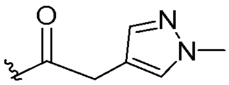, 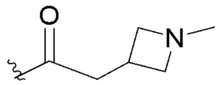,

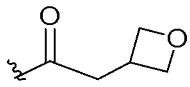, 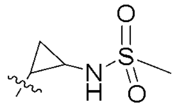, 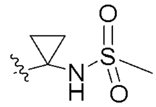 or 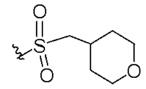,

or is selected from

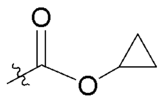, 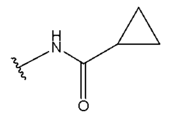, 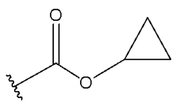, 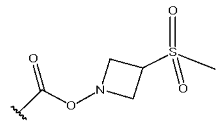, 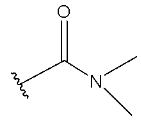,

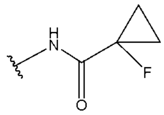, 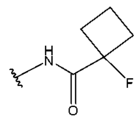, 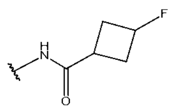, 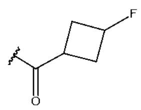, 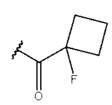, 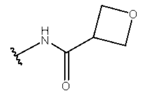,

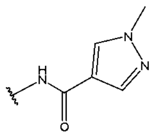, 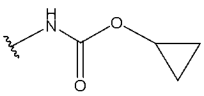, 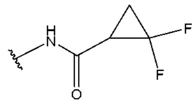, 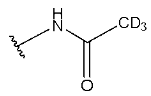, 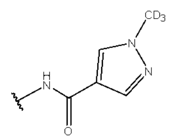,

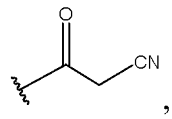, 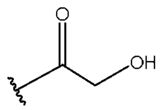, 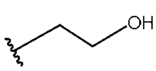, 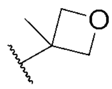,

$-NHS(O)_2-CH_3$ or

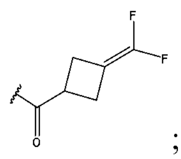;

in some embodiments, $R^A$ is selected from

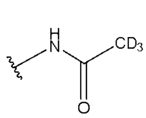, 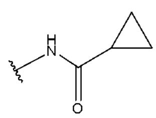,

or

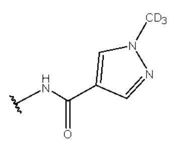;

in some embodiments, each $R^A$ is independently selected from $-CF_3$, $-S(O)_2CH_3$, $-COCD_3$, $-CON(CH3)_2$,

$-COCH_2CN$, $-CH_2CH_2OH$, $-NHCOCD_3$, $-NHCOCH_3$, - $NHS(O)_2CH_3$, $-NHCOCF_2CH_3$, $-NHCOCH_2CN$, $-NHCOCH_2CF_3$,

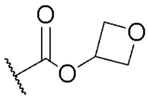,

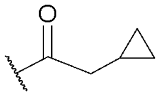, 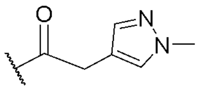, 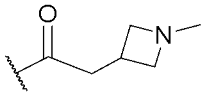, 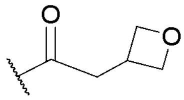, 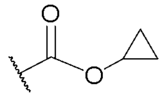,

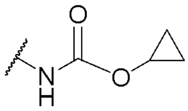, 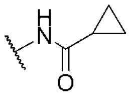, 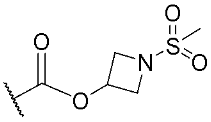, 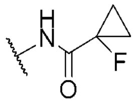, 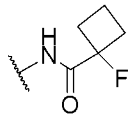, 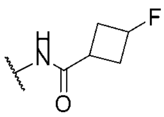,

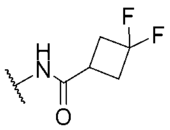, 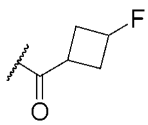, 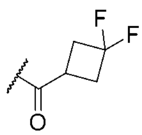, 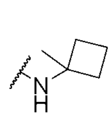, 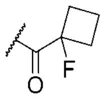, 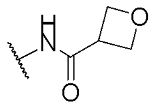,

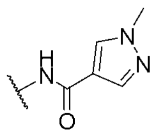, 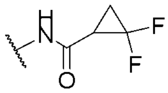, 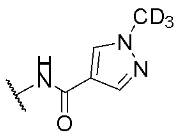, 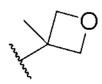, or 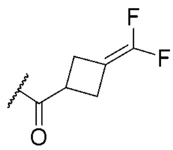;

in some embodiments, each $R^{A1}$ is independently selected from H, halogen, CN, OH, $NO_2$, COOH, $C_{1-2}$ alkyl, $C_{2-4}$ alkenyl, $C_{2-4}$ alkynyl, $-OC_{1-2}$ alkyl, $-NH-S(O)_2-C_{1-2}$ alkyl, $-S(O)_2-C_{1-2}$ alkyl, $-S(O)_2-C_{1-2}$ alkyl-$R^a$, $-P(O)-(C_{1-2}$ alkyl$)_2$, $-C(O)-OR^a$, or - $(CH_2)_p-C(O)-(CH_2)_p-R^a$, wherein the alkyl, alkenyl, or alkynyl is optionally further substituted with 1-4 groups selected from halogen, OH, $NH_2$, CN or -O-halo $C_{1-2}$ alkyl;

$R^a$ is selected from $C_{2-4}$ alkynyl, 3- to 5-membered cycloalkyl, 3- to 6-membered heterocycloalkyl, 5- or 6-membered heteroaryl, $C_{1-4}$ alkoxy, $-O-C_{3-6}$ cycloalkyl, deuterated $C_{1-4}$ alkyl, or $C_{1-4}$ alkyl, wherein the alkynyl, cycloalkyl, heterocycloalkyl, heteroaryl, or alkoxy is optionally further substituted with 1-4 groups selected from halogen, $C_{1-4}$ alkyl, halo $C_{1-4}$ alkyl, deuterated $C_{1-4}$ alkyl, 6-membered heterocycloalkyl, 5- or 6-membered heteroaryl, $C_{2-4}$ alkynyl, 3- to 5-membered cycloalkyl, -O-halo $C_{1-4}$ alkyl, OH, $-S(O)_2-CH_3$, or $=CH_2$, $=CHF$ or $=CF_2$, wherein the alkyl is further substituted with 1-3 groups selected from OH or CN, and the heterocycloalkyl or heteroaryl contains 1-3 heteroatoms selected from N, O or S;

in some embodiments, $R^a$ is selected from $C_{2-4}$ alkynyl, cyclopropyl, cyclobutyl, 4- to 6-membered heterocycloalkyl, pyrazolyl, $C_{1-4}$ alkoxy, $-O-C_{3-6}$ cycloalkyl, deuterated $C_{1-2}$ alkyl, or $C_{1-2}$ alkyl, wherein the cyclopropyl, cyclobutyl, heterocycloalkyl, pyrazolyl, or alkoxy is optionally further substituted with a group selected from halogen, $C_{1-2}$ alkyl, halo $C_{1-2}$ alkyl, deuterated $C_{1-2}$ alkyl, OH or - $S(O)_2-CH_3$, and the alkyl is further substituted with 1-3 groups selected from OH or CN;

in some embodiments, $R^a$ is selected from $C_{2-4}$ alkynyl, cyclopropyl, cyclobutyl, 4- to 6-membered heterocycloalkyl, pyrazolyl, $C_{1-4}$ alkoxy, -O-cyclopropyl, $-CD_3$, - $CH_2D$, $-CHD_2$, methyl, or ethyl, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH or - $S(O)_2-CH_3$, and the methyl or ethyl is further substituted with 1-3 groups selected from OH or CN;

in some embodiments, $R^a$ is selected from ethynyl, propynyl, cyclopropyl, cyclobutyl, 4- to 6-membered heterocycloalkyl, pyrazolyl, methoxy, ethoxy, -O-cyclopropyl, $-CD_3$, methyl, or ethyl, wherein the cyclopropyl, cyclobutyl, heterocycloalkyl, pyrazolyl, methoxy or ethoxy is optionally further substituted with a group selected from F, Cl, Br, methyl, ethyl, $-CD_3$, $-CH_2D$, $-CHD_2$, OH or $-S(O)_2-CH_3$, and the methyl or ethyl is further substituted with 1-3 groups selected from OH or CN;

in some embodiments, $R^a$ is selected from $C_{2-4}$ alkynyl, 3- to 5-membered cycloalkyl, 3- to 6-membered heterocycloalkyl, 5- or 6-membered heteroaryl, $C_{1-4}$ alkoxy, $-O-C_{3-6}$ cycloalkyl, or deuterated $C_{1-4}$ alkyl, wherein the alkynyl,

cycloalkyl, heterocycloalkyl, heteroaryl, alkyl or alkoxy is optionally further substituted with 1-4 groups selected from halogen, $C_{1-4}$ alkyl, halo $C_{1-4}$ alkyl, deuterated $C_{1-4}$ alkyl, 6-membered heterocycloalkyl, 5- or 6-membered heteroaryl, $C_{2-4}$ alkynyl, 3- to 5-membered cycloalkyl, -O-halo $C_{1-4}$ alkyl, OH or $-S(O)_2-CH_3$, and the heterocycloalkyl or heteroaryl contains 1-3 heteroatoms selected from N, O or S;

in some embodiments, $R^a$ is selected from $C_{2-4}$ alkynyl, cycloprpoyl, cyclobutyl, 4- to 6-membered heterocycloalkyl, pyrazolyl, $C_{1-4}$ alkoxy, $-O-C_{3-6}$ cycloalkyl, or deuterated $C_{1-2}$ alkyl, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH or $-S(O)_2-CH_3$;

in some embodiments, $R^a$ is selected from $C_{2-4}$ alkynyl, cyclopropyl, cyclobutyl, 4- to 6-membered heterocycloalkyl, pyrazolyl, $C_{1-4}$ alkoxy, -O-cyclopropyl, $-CD_3$, $-CH_2D$, or $-CHD_2$, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH or $-S(O)_2-CH_3$;

in some embodiments, $R^a$ is selected from ethynyl, propynyl, cyclopropyl, cyclobutyl, 4- to 6-membered heterocycloalkyl, pyrazolyl, methoxy, ethoxy, -O-cyclopropyl, or $-CD_3$, wherein the cyclopropyl, cyclobutyl, heterocycloalkyl, pyrazolyl, methoxy or ethoxy is optionally further substituted with a group selected from F, Cl, Br, methyl, ethyl, $-CD_3$, $-CH_2D$, $-CHD_2$, OH or $-S(O)_2-CH_3$;

in some embodiments, $R^a$ is selected from $C_{2-4}$ alkynyl, 3- to 5-membered cycloalkyl, 3- to 6-membered heterocycloalkyl, 5- or 6-membered heteroaryl, $C_{1-4}$ alkoxy, $-O-C_{3-6}$ cycloalkyl, or deuterated $C_{1-4}$ alkyl, wherein the alkynyl, cycloalkyl, heterocycloalkyl, heteroaryl or alkyl is optionally further substituted with 1-4 groups selected from 6-membered heterocycloalkyl, 5- or 6-membered heteroaryl, $C_{2-4}$ alkynyl, 3- to 5-membered cycloalkyl, -O-halo $C_{1-4}$ alkyl, OH or $-S(O)_2-CH_3$, and the heterocycloalkyl or heteroaryl contains 1-3 heteroatoms selected from N, O or S;

in some embodiments, $R^a$ is selected from $C_{2-4}$ alkynyl, cycloprpoyl, 4- to 6-membered heterocycloalkyl, pyrazolyl, $C_{1-4}$ alkoxy, $-O-C_{3-6}$ cycloalkyl, or deuterated $C_{1-2}$ alkyl, wherein the cyclopropyl, heterocycloalkyl, pyrazolyl or alkoxy is optionally further substituted with a group selected from halogen, $C_{1-2}$ alkyl, OH or $-S(O)_2-CH_3$;

in some embodiments, $R^a$ is selected from $C_{2-4}$ alkynyl, cyclopropyl, 4- to 6-membered heterocycloalkyl, pyrazolyl, $C_{1-4}$ alkoxy, -O-cyclopropyl, $-CD_3$, $-CH_2D$, or $-CHD_2$, wherein the cyclopropyl, heterocycloalkyl, pyrazolyl or alkoxy is optionally further substituted with a group selected from halogen, $C_{1-2}$ alkyl, OH or $-S(O)_2-CH_3$;

in some embodiments, $R^a$ is selected from ethynyl, propynyl, cyclopropyl, 4-to 6-membered heterocycloalkyl, pyrazolyl, methoxy, ethoxy, -O-cyclopropyl, or - $CD_3$, wherein the cyclopropyl, heterocycloalkyl, pyrazolyl, methoxy or ethoxy is optionally further substituted with a group selected from F, Cl, Br, methyl, ethyl, OH or $-S(O)_2-CH_3$;

in some embodiments, $R^a$ is selected from $C_{2-4}$ alkynyl, 3- to 5-membered cycloalkyl, 3- to 6-membered heterocycloalkyl, or 5- or 6-membered heteroaryl, wherein the heterocycloalkyl or heteroaryl is optionally further substituted with 1-4 groups selected from 6-membered heterocycloalkyl, 5- or 6-membered heteroaryl, $C_{2-4}$ alkynyl, 3- to 5-membered cycloalkyl, -O-halo $C_{1-4}$ alkyl, OH or $-S(O)_2-CH_3$, and the heterocycloalkyl or heteroaryl contains 1-3 heteroatoms selected from N, O or S;

in some embodiments, $R^a$ is selected from $C_{2-4}$ alkynyl, 3- to 5-membered cycloalkyl, 3- to 6-membered heterocycloalkyl, or 5- or 6-membered heteroaryl, wherein the heterocycloalkyl or heteroaryl is optionally further substituted with 1-4 groups selected from 6-membered heterocycloalkyl, 5- or 6-membered heteroaryl, $C_{2-4}$ alkynyl, 3- to 5-membered cycloalkyl, -O-halo $C_{1-2}$ alkyl, OH or $-S(O)_2-CH_3$, and the heterocycloalkyl or heteroaryl contains 1-3 heteroatoms selected from N, O or S;

in some embodiments, $R^a$ is selected from $C_{2-4}$ alkynyl, cycloprpoyl, 4- to 6-membered heterocycloalkyl, pyrazolyl, or $C_{1-4}$ alkoxy, wherein the cyclopropyl, heterocycloalkyl, pyrazolyl or alkoxy is optionally further substituted with a group selected from halogen, $C_{1-2}$ alkyl, OH or $-S(O)_2-CH_3$;

in some embodiments, $R^a$ is selected from ethynyl, propynyl, butynyl, cyclopropyl, 4- to 6-membered heterocycloalkyl, or pyrazolyl, wherein the ethynyl, propynyl, butynyl, cyclopropyl, heterocycloalkyl or pyrazolyl is optionally further substituted with a group selected from $C_{1-2}$ alkyl or OH;

in some embodiments, $R^a$ is selected from $C_{2-4}$ alkynyl, cycloprpoyl, 4- to 6-membered heterocycloalkyl, pyrazolyl, or $C_{1-4}$ alkoxy, wherein the cyclopropyl, heterocycloalkyl, pyrazolyl or alkoxy is optionally further substituted with a group selected from halogen, $C_{1-2}$ alkyl or OH;

in some embodiments, $R^a$ is selected from ethynyl, propynyl, cyclopropyl, 4-to 6-membered heterocycloalkyl, pyrazolyl, methoxy, or ethoxy, wherein the cyclopropyl, heterocycloalkyl, pyrazolyl, methoxy or ethoxy is optionally further substituted with a group selected from F, Cl, Br, methyl, ethyl or OH;

in some embodiments, $R^a$ is selected from cyclopropyl, cyclobutyl, pyrazolyl, or $-CD_3$, wherein the cyclopropyl, cyclobutyl or pyrazolyl is optionally further substituted with a group selected from F, Cl, Br, methyl, ethyl, $-CD_3$, $-CH_2D$ or - $CHD_2$;

each $R^B$ is independently selected from H, halogen, CN, =O, OH, $NO_2$, $-SF_5$, $C_{1-4}$ alkyl, $-P(O)-(CH_3)_2$, $-S(O)_2-CH_3$, $-S(O)_2-CH_2R^a-$, $NH_2$, $-C_{1-4}$ alkoxy, $C_{2-6}$ alkenyl, $C_{2-6}$ alkynyl, 3- to 5-membered cycloalkyl, halo $C_{1-2}$ alkyl, 5- or 6-membered heteroaryl containing 1-3 heteroatoms selected from N, O or S, or 3- to 6-membered heterocycloalkyl

containing 1-3 heteroatoms selected from N, O or S, wherein the alkoxy, cycloalkyl, heteroaryl or heterocycloalkyl is optionally further substituted with a $C_{1-2}$ alkyl group;

in some embodiments, each $R^B$ is independently selected from H, halogen, CN, =O, OH, $-SF_5$, $C_{1-2}$ alkyl, $-P(O)-(CH_3)_2$, $-S(O)_2-CH_3$, $-S(O)_2-CH_2R^a-$, $NH_2$, $-C_{1-2}$ alkoxy, $C_{2-4}$ alkenyl, $C_{2-4}$ alkynyl, 3- to 5-membered cycloalkyl, halo $C_{1-2}$ alkyl, 5- or 6-membered heteroaryl containing 1-3 heteroatoms selected from N, O or S, or 3- to 6-membered heterocycloalkyl containing 1-3 heteroatoms selected from N, O or S, wherein the alkoxy, cycloalkyl, heteroaryl or heterocycloalkyl is optionally further substituted with a $C_{1-2}$ alkyl group;

in some embodiments, each $R^B$ is independently selected from halogen, CN, $-P(=O)(C_{1-2}\ alkyl)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;

in some embodiments, each $R^B$ is independently selected from F, Cl, Br, CN, =O, $-SF_5$, $CH_3$, $CH_2CH_3$, $-S(O)_2-CH_3$, $-S(O)_2-CH_2CH_3$, $NH_2$, $-OCH_3$, $-OCH_2CH_3$, $C_{2-4}$ alkenyl, $C_{2-4}$ alkynyl, cyclopropyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2Cl$, $-CHCl_2$, $-CCl_3$, $-CH_2CH_2Cl$, $-CH_2CHCl_2$, $-CH_2CCl_3$, $-CHClCH_2Cl$, $-CHClCHCl_2$, $-CHClCCl_3$, $-CCl_2CH_2Cl$, $-CCl_2CHCl_2$, $-CCl_2CCl_3$, or 5- or 6-membered heteroaryl, wherein the CH, $CH_2$, $CH_3$, $CH_2CH_3$, alkenyl, alkynyl, cyclopropyl or heteroaryl is optionally further substituted with a $C_{1-2}$ alkyl group;

in some embodiments, each $R^B$ is independently selected from F, Cl, Br, =O, CN, methyl, ethyl, methoxy, ethoxy, vinyl, propenyl, butenyl, ethynyl, propynyl, cyclopropyl, $-CF_3$, $-CHF_2$, $-CH_2F$, 5-membered heteroaryl, or 6-membered heteroaryl, wherein the methyl, ethyl, methoxy, ethoxy, cyclopropyl or heteroaryl is optionally further substituted with a group selected from methyl or ethyl;

in some embodiments, each $R^B$ is independently selected from F, Cl, Br, =O, methyl, ethyl, methoxy, ethoxy, vinyl, propenyl, butenyl, ethynyl, propynyl, cyclopropyl, $-CF_3$, $-CHF_2$, $-CH_2F$, 5-membered heteroaryl, or 6-membered heteroaryl, wherein the methyl, ethyl, methoxy, ethoxy, cyclopropyl or heteroaryl is optionally further substituted with a group selected from methyl or ethyl;

in some embodiments, each $R^B$ is independently selected from methoxy, ethoxy, cyclopropyl,

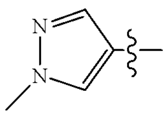 , 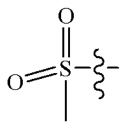 , 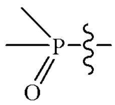 ,

acetylene, propyne, $-CH_3$, F, Cl, Br, ethylene, propylene, CN, $CF_3$, =O, or $SF_5$;

in some embodiments, each $R^B$ is independently selected from F, Cl, Br, =O, CN, methyl, ethyl, methoxy, or ethoxy, wherein the methyl, ethyl, methoxy or ethoxy is optionally further substituted with a group selected from methyl or ethyl;

each $R^C$ is independently selected from H, halogen, CN, =O, OH, $NO_2$, $C_{1-4}$ alkyl, halo $C_{1-4}$ alkyl, $C_{2-4}$ alkenyl, $-O-C_{3-5}$ cycloalkyl, $C_{3-5}$ cycloalkyl, $C_{2-4}$ alkynyl, $-SCF_3$, $-SF_5$, $-(NH)_p-P(O)(C_{1-4}\ alkyl)_2$, or $-(CH_2)_p-O-C_{1-4}$ alkyl;

in some embodiments, each $R^C$ is independently selected from H, halogen, CN, =O, OH, $C_{1-2}$ alkyl, halo $C_{1-2}$ alkyl, $-O-C_{3-5}$ cycloalkyl, $C_{3-5}$ cycloalkyl, $C_{2-4}$ alkenyl, $C_{2-4}$ alkynyl, $-SCF_3$, $-SF_5$, $-(NH)_p-P(O)(C_{1-2}\ alkyl)_2$, or $-(CH_2)_p-O-C_{1-2}$ alkyl;

in some embodiments, each $R^C$ is independently selected from CN, OH, $C_{1-2}$ alkyl, =O, -O-cyclopropyl, cyclopropyl, $C_{2-4}$ alkynyl, $-SCF_3$, $-SF_5$, $-(NH)_{0-1}-P(O)(C_{1-2}\ alkyl)_2$, or $C_{1-2}$ alkyl substituted with $C_{1-2}$ alkoxy;

in some embodiments, each $R^C$ is independently selected from CN, OH, $C_{1-2}$ alkyl, halo $C_{1-2}$ alkyl, $C_{2-4}$ alkenyl, or $C_{2-4}$ alkynyl;

in some embodiments, each $R^C$ is independently selected from methyl, ethyl, $-CF_3$, $-CHF_2$, $-CH_2CH_2F$, $-CH_2CF_3$, $-CH_2CHF_2$, $-CH_2CH_2F$, vinyl, propenyl, ethynyl or propynyl, or is selected from -O-cyclopropyl or cyclopropyl;

in some embodiments, each $R^C$ is independently selected from F, Cl, Br, CN, OH, $-CH_3$, $-CH_2CH_3$, =O, -O-cyclopropyl, cyclopropyl, $C_{2-4}$ alkynyl, $-SCF_3$, $-SF_5$, $-NH-P(O)(CH_3)_2$, $-NH-P(O)(CH_2CH_3)_2$, $-P(O)(CH_3)_2$, $-P(O)(CH_2CH_3)_2$, $-CH_2-O-CH_3$, or $-CH_2-O-CH_2CH_3$.

In some embodiments, each $R^C$ is independently selected from H, CN, OH, $-CH_3$, $-CH_2CH_3$, =O, -O-cyclopropyl, cyclopropyl, acetylene, propyne, $-SCF_3$, $-SF_5$, $-NH-P(O)(CH_3)_2$, $-NH-P(O)(CH_2CH_3)_2$, $-P(O)(CH_3)_2$, $-P(O)(CH_2CH_3)_2$, $-CH_2-O-CH_3$, or $-CH_2-O-CH_2CH_3$;

in some embodiments, each $R^C$ is independently selected from $-CF_3$, $-CHF_2$, $-CH_2CH_2F$, or cyclopropyl;

each $Y_1$ is independently selected from $NHC_{1-4}$ alkylene, or $N(C_{1-4}\ alkyl)C_{1-4}$ alkylene, wherein the alkylene is optionally substituted with 1-4 groups selected from halogen, =O, OH or CN;

in some embodiments, each $Y_1$ is independently selected from $NHC_{1-2}$ alkylene, or $N(C_{1-2}\ alkyl)C_{1-2}$ alkylene,

wherein the alkylene is optionally substituted with 1-4 groups selected from halogen, =O, OH or CN;

in some embodiments, each $Y_1$ is independently selected from $-NHCH_2-$, - $NHCH_2CH_2-$, $-N(CH_3)CH_2-$, $-N(CH_3)CH_2CH_2-$, $-N(CH_2CH_3)CH_2CH_2-$, or - $N(CH_2CH_3)CH_2-$, wherein the alkyl is optionally substituted with 1-4 groups selected from halogen, =O, OH or CN;

$L_1$ and $L_2$ are each independently selected from $W_1$-$R^L$-$W_2$, wherein $L_1$ is connected to A on the left side, and $L_2$ is connected to B on the left side; $L_1$ and $L_2$ are not both bonds;

$R^L$ is selected from a bond, $C_{1-4}$ alkylene, or $C_{2-4}$ alkenylene, wherein the alkylene or alkenylene is optionally further substituted with 1-4 $R^{L1}$;

in some embodiments, $R^L$ is selected from a bond, $C_{1-2}$ alkylene, $C_{2-4}$ alkenylene, or 3- to 6-membered cycloalkyl, wherein the alkylene, alkenylene or cycloalkyl is optionally further substituted with 1-4 $R^{L1}$;

in some embodiments, $R^L$ is selected from $-CH_2-$, $-CH_2CH_2-$, -CH=CH-, $-CH_2$-CH=CH-, $-CH_2CH_2$-CH=CH-, $-CH(CH_3)$-CH=CH-, $-CH_2$-CH=CH-$CH_2-$, cyclopropyl, cyclobutyl, cyclopentyl, or cyclohexyl, wherein the $CH_3$, $CH_2$, CH, cyclopropyl, cyclobutyl, cyclopentyl or cyclohexyl is optionally further substituted with 1-4 $R^{L1}$;

each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy or cycloalkyl is optionally further substituted with 1-4 substituents selected from halogen, CN, OH and $NH_2$;

in some embodiments, each $R^{L1}$ is independently selected from halogen, =O, $C_{1-2}$ alkyl, $C_{2-4}$ alkenyl, $C_{1-2}$ alkoxy, or 3- to 6-membered cycloalkyl, wherein the alkyl, alkoxy or cycloalkyl is optionally further substituted with 1-4 substituents selected from halogen, CN, OH and $NH_2$;

in some embodiments, each $R^{L1}$ is independently selected from F, Cl, Br, =O, $-CH_2-$, $-CH_2CH_2-$, $C_{2-4}$ alkenyl, $-O-CH_2-$, $-O-CH_2CH_2-$, or cyclopropyl, wherein the $CH_2$, alkenyl or cyclopropyl is optionally further substituted with 1-4 substituents selected from halogen, CN, OH and $NH_2$;

in some embodiments, each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$, - $OCHF_2$, $-OCH_2F$, cyclopropyl, or cyclobutyl;

$W_1$ and $W_2$ are each independently selected from a bond, -O-, -S-, $-NR^{W1}-$, - $CONR^{W1}-$, $-NR^{W1}CO-$, -C(=O)O-, or -OC(=O)-;

in some embodiments, $W_1$ and $W_2$ are each independently selected from -O-, - S-, $-NR^{W1}-$, $-CONR^{W1}-$, $-NR^{W1}CO-$, -C(=O)O-, or -OC(=O)-;

in some embodiments, $W_1$ and $W_2$ are each independently selected from -O-, - S-, -NH-, $-N(CH_3)-$, $-CON(CH_3)-$, $-N(CH_3)CO-$, -C(=O)O-, or -OC(=O)-;

$R^{W1}$ is selected from H, $C_{1-4}$ alkyl, or halogen;

in some embodiments, $R^{W1}$ is selected from H, $C_{1-2}$ alkyl, or halogen;

in some embodiments, $R^{W1}$ is selected from H, $-CH_3$, $-CH_2CH_3$, F, Cl, or Br;

in some embodiments, $L_1$ is selected from a bond, $C_{1-2}$ alkylene-O, $C_{1-2}$ alkylene, N($C_{1-2}$ alkylene)-$C_{1-2}$ alkylene, -O-, or $C_{2-4}$ alkenylene, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

in some embodiments, $L_1$ is selected from a bond, $-CH_2-O-$, $-CH_2CH_2-O-$, - $CH_2-$, -O-, $-CH_2CH_2-$, $-N(CH_3)-CH_2-$, ethylene, or propylene, wherein the $-CH_2-$ is optionally further substituted with 1-3 $R^{L1}$; each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$, $-OCHF_2$, $-OCH_2F$, cyclopropyl, or cyclobutyl;

in some embodiments, $L_1$ is selected from: a bond, halo $C_{1-4}$ alkoxy, $C_{1-4}$ alkyl, -C(O)-O-, $-O-CH_2$-CH=C-, $-CH_2-N(CH_3)-$, $-N(CH_3)$-C(O)-, -O-, -S-$C_{1-4}$ alkyl, $C_{2-4}$ alkenyl, or -CH($C_{3-4}$ cycloalkyl)-O-;

in some embodiments, $L_1$ is selected from a bond, $C_{1-2}$ alkylene-O, $C_{1-2}$ alkylene, $C_{1-2}$ alkylene-S, $C_{1-2}$ alkylene-N($C_{1-2}$ alkylene), N($C_{1-2}$ alkylene)-$C_{1-2}$ alkylene, -O-$C_{1-3}$ alkylene, $C_{2-4}$ alkenylene, -N($C_{1-2}$ alkylene)-C(=O), -C(=O)-N($C_{1-2}$ alkylene), -O-, -C(=O)-O-, or 3- to 6-membered cycloalkyl-O, wherein the alkylene, alkenylene or cycloalkyl is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

in some embodiments, $L_1$ is selected from a bond, $C_{1-2}$ alkylene-O, $C_{1-2}$ alkylene, N($C_{1-2}$ alkylene)-$C_{1-2}$ alkylene, or -O-, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

in some embodiments, $L_1$ is selected from a bond, $-CH_2-O-$, $-CH_2CH_2-O-$, - $CH_2-$, -O-, $-CH_2CH_2-$, or $-N(CH_3)-CH_2-$, wherein the $-CH_2-$ is optionally further substituted with 1-3 $R^{L1}$; each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$, - $OCHF_2$, $-OCH_2F$, cyclopropyl, or cyclobutyl;

in some embodiments, $L_1$ is selected from a bond, $-CH_2-O-$, $-CH_2CH_2-O-$, ethylene, or propylene, wherein the $-CH_2-$ is optionally further substituted with 1-3 $R^{L1}$; each $R^{L1}$ is independently selected from F, Cl, Br, methyl, ethyl, cyclopropyl, or cyclobutyl;

in some embodiments, $L_2$ is selected from: a bond, $-C_{1-4}$ alkyl-NH-C($C_{1-4}$ alkyl)-, $-C_{1-4}$ alkyl-NH-C(halo $C_{1-4}$ alkyl)-, $-C_{1-4}$ alkyl-NH-C(O)-, $-CH_2$-N($CH_3$)-$C_{1-4}$ alkyl or $-CH_2$-$C_{3-4}$ cycloalkyl, or is selected from -N($CH_3$)-;
in some embodiments, $L_2$ is selected from a bond, $C_{1-2}$ alkylene, -C(=O)-, 3- to 6-membered cycloalkyl, -NH- or -O-, or is selected from -N($CH_3$)-, wherein the alkylene, alkenylene or cycloalkyl is optionally further substituted with 1-3 $R^{L1}$;
each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;
in some embodiments, $L_2$ is selected from a bond or $C_{1-2}$ alkylene, or is selected from -N($CH_3$)-, wherein the alkylene is optionally further substituted with 1-3 $R^{L1}$;
each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

in some embodiments, $L_2$ is selected from a bond, $-CH_2$- or -CH($CH_3$)-, or is selected from-N($CH_3$)-, wherein the $-CH_2$- or -CH($CH_3$)- is optionally further substituted with 1-3 $R^{L1}$; each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$, - $OCHF_2$, $-OCH_2F$, cyclopropyl, or cyclobutyl;
in some embodiments, $L_2$ is selected from $-CH_2$-, wherein the $-CH_2$- is optionally further substituted with 1-3 $R^{L1}$; each $R^{L1}$ is independently selected from F, Cl, Br, methyl, ethyl, cyclopropyl, or cyclobutyl;
in some embodiments, $L_{1A}$ is selected from

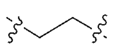, 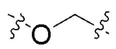, 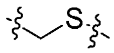,

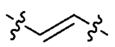, 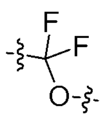, 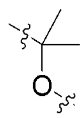, 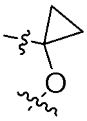, 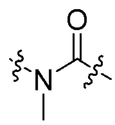, 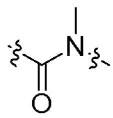, 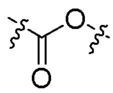, 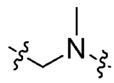,

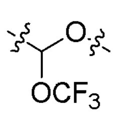, or 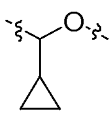;

the left end of $L_{1A}$ is connected to the piperidine ring;
$L_{2A}$ is selected from NH, O,

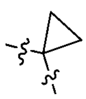, 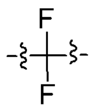, 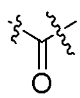, or 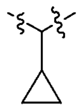;

the left end of $L_{2A}$ is connected to the pyrone ring;
p is selected from 0, 1, 2, 3 or 4; n1, n2, n3 and n4 are each independently selected from 0, 1, 2 or 3; m1, m2, m3 and m4 are each independently selected from 0, 1, 2 or 3;
in some embodiments, n1 is selected from 1, 2 or 3;
in some embodiments, n3 is selected from 0, 1, 2 or 3;
in some embodiments, n1 is selected from 1 or 2;
in some embodiments, n3 is selected from 0, 1 or 2;
in some embodiments, p is selected from 0, 1, 2 or 3; in some embodiments, p is selected from 0, 1 or 2; in some embodiments, p is selected from 0 or 1; in some embodiments, p is selected from 0;
alternatively, B, $L_2$ and C, together with atoms to which they are connected, form tetracyclic heterocyclyl, optionally substituted with a group selected from H, halogen, CN, OH, $NO_2$, =O, COOH, $C_{1-4}$ alkyl, $C_{2-6}$ alkenyl, $C_{2-6}$ alkynyl, $-OC_{1-4}$ alkyl or -O-halo $C_{1-4}$ alkyl;
optionally, the compound satisfies at least one of the following conditions:

A is selected from one of the groups formed by the following structures optionally substituted with 1-4 $R^A$:

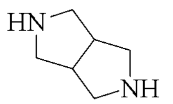, 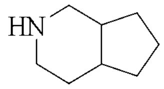, 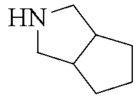,

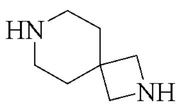, 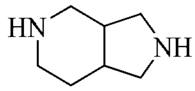, 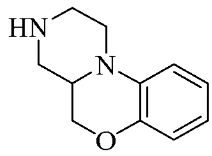, 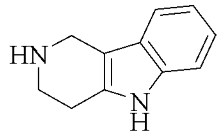,

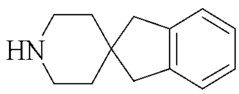, 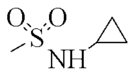, 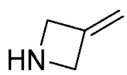, 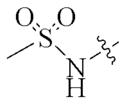, 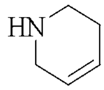, or 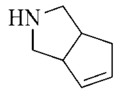;

2) B is selected from one of the groups formed by the following structures optionally substituted with 1-4 $R^B$:

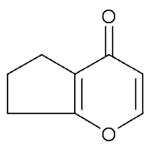, 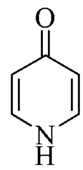, 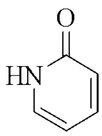, 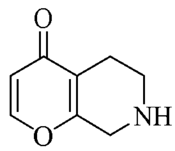,

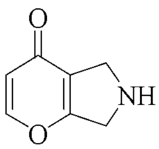, 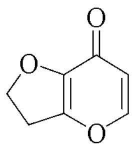, 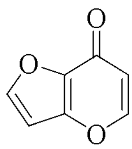, 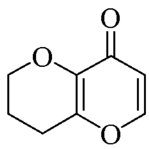, 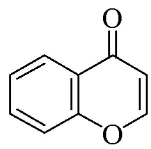, 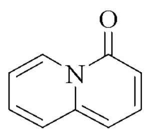, 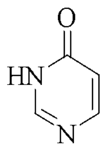,

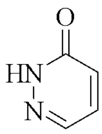, 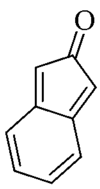, 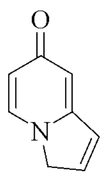, 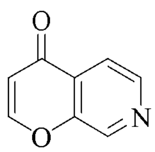, 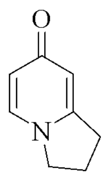, 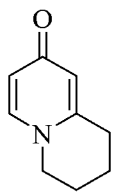, 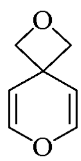, 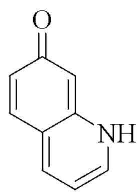,

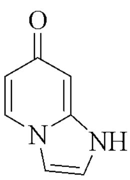, 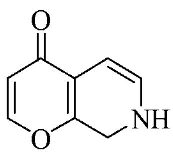, 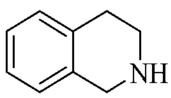, 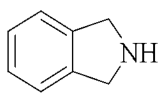, 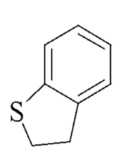, 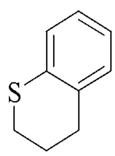, 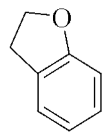,

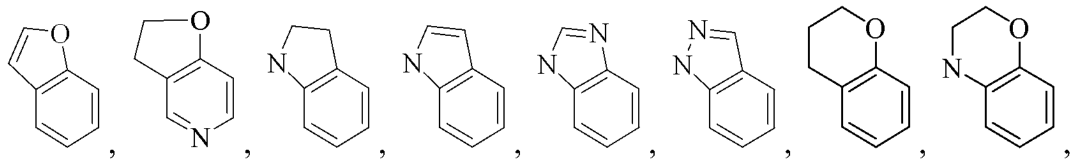

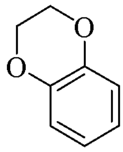, 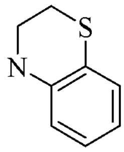, 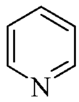, or 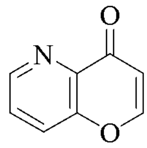;

3) the C ring is selected from one of the following groups optionally substituted with 1-4 $R^C$:

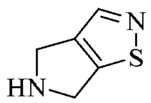, 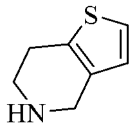, 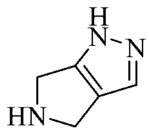, 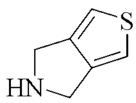, 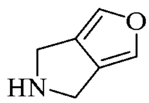,

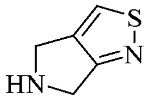, 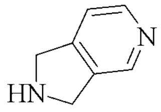, 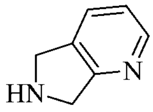, 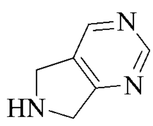, 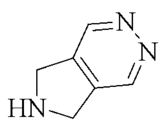, 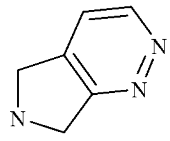,

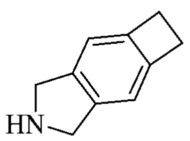, 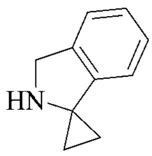, 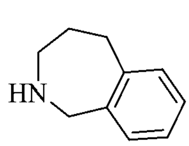, 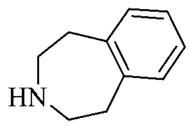, 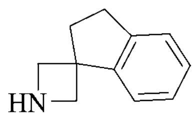,

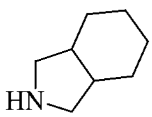, 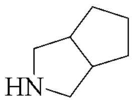, 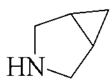, 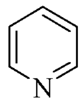, 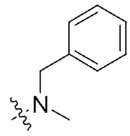, 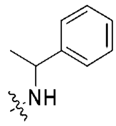, 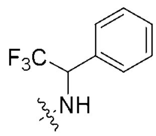,

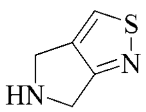, 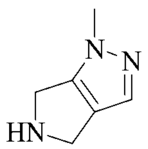, 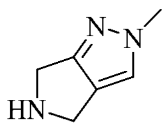, 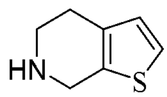, 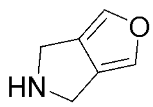, 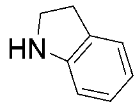,

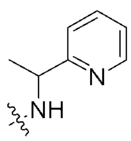, 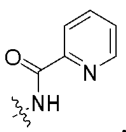, or 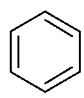;

4) B, $L_2$ and C, together with atoms to which they are connected, form a tetracyclic heterocycloalkyl, optionally substituted with a group selected from H, halogen, CN, OH, $NO_2$, =O, COOH, $C_{1-4}$ alkyl, $C_{2-6}$ alkenyl, $C_{2-6}$ alkynyl, $-OC_{1-4}$ alkyl or -O-halo $C_{1-4}$ alkyl;
5) at least one $R^A$ is selected from: $-P(O)-(C_{1-4}$ alkyl$)_2$, $-NH-S(O)_2-C_{1-4}$ alkyl, - COOH, $-C_{1-4}$ alkyl-C(O)-4- to 6-membered heterocycloalkyl, $-C(O)-C_{1-4}$ alkyl-4- to 6-membered heterocycloalkyl, $-C(O)-C_{1-4}$ alkyl-$C_{3-6}$ cycloalkyl, $-C_{1-4}$ alkyl-C(O)-O-4- to 6-membered heterocycloalkyl, $-S(O)_2-C_{1-4}$ alkyl-4- to 6-membered heterocycloalkyl, $-S(O)_2-C_{1-4}$ alkyl-$C_{2-4}$ alkynyl, $-S(O)_2-C_{1-4}$ alkyl-$C_{3-5}$ cycloalkyl, - $S(O)_2-C_{1-4}$ alkyl-5- or 6-membered heteroaryl, or $-S(O)_2-C_{1-4}$ alkyl-O-halo $C_{1-4}$ alkyl;
6) at least one $R^B$ is selected from: $C_{1-4}$ alkyl, halogen, $-C_{1-4}$ alkoxy, CN, halo $C_{1-4}$ alkyl, $SF_5$, $-P(O)-(C_{1-4}$ alkyl$)_2$, $C_{2-4}$ alkynyl, $C_{2-4}$ alkenyl, $C_{3-5}$ cycloalkyl, or $C_{5-6}$ heteroaryl;
7) at least one $R^C$ is selected from: $C_{1-4}$ alkyl, halogen, CN, $C_{2-4}$ alkynyl, $SCF_3$, $SF_5$, =O, $-(NH)_p-P(O)(C_{1-4}$ alkyl$)_2$, $-CH_2-O-C_{1-4}$ alkyl, $C_{3-5}$ cycloalkyl, or $-O-C_{3-5}$ cycloalkyl;
8) $L_1$ is selected from: a bond, halo $C_{1-4}$ alkoxy, $C_{1-4}$ alkyl, -C(O)-O-, $-O-CH_2-CH=C-$, $-CH_2-N(CH_3)-$,

$-N(CH_3)-C(O)-$, -O-, $-S-C_{1-4}$ alkyl, $C_{2-4}$ alkenyl, or $-CH(C_{3-4}$ cycloalkyl)-O-;

9) $L_2$ is selected from: a bond, $-C_{1-4}$ alkyl-NH-C($C_{1-4}$ alkyl)-, $-C_{1-4}$ alkyl-NH-C(halo $C_{1-4}$ alkyl)-, $-C_{1-4}$ alkyl-NH-C(O)-, $-CH_2-N(CH_3)-C_{1-4}$ alkyl_, or $-CH_2-C_{3-4}$ cycloalkyl.

A specific technical solution 1 of the present invention provides a compound represented by formula (I), or a stereoisomer, a deuterated substance, a solvate, a co-crystal or a pharmaceutically acceptable salt thereof, wherein

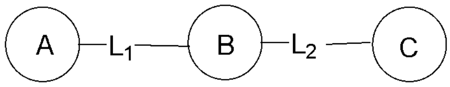

(I)

A is selected from 4- to 14-membered heterocyclyl, 5- or 6-membered cycloalkyl, or phenyl, wherein the heterocyclyl contains 1-3 heteroatoms selected from N, O or S; the heterocyclyl, cycloalkyl or phenyl is optionally substituted with 1-4 $R^A$;

B is selected from 6- to 12-membered bicyclic carbocyclyl, 6- to 12-membered bicyclic heterocyclyl, or 6- to 12-membered heteroaryl, wherein the heterocyclyl or heteroaryl contains 1-3 heteroatoms selected from N, O or S; the carbocyclyl, heterocyclyl or heteroaryl is optionally substituted with 1-4 $R^B$;

C is selected from phenyl, 6- to 12-membered bicyclic carbocyclyl, 6- to 12-membered partially unsaturated bicyclic heterocyclyl, or 8- to 14-membered tricyclic heterocyclyl, wherein the heterocyclyl contains 1-3 heteroatoms selected from N, O or S; the cycloalkyl, heterocyclyl or phenyl is optionally substituted with 1-4 $R^C$;

$L_1$ and $L_2$ are each independently selected from $W_1-R^L-W_2$, wherein $L_1$ is connected to A on the left side, and $L_2$ is connected to B on the left side; $L_1$ and $L_2$ are not both bonds;

$R^L$ is selected from a bond, $C_{1-4}$ alkylene, $C_{2-4}$ alkenylene, or 3- to 6-membered cycloalkyl, wherein the alkylene, alkenylene or cycloalkyl is optionally further substituted with 1-4 $R^{L1}$; each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy or cycloalkyl is optionally further substituted with 1-4 substituents selected from halogen, CN, OH and $NH_2$;

$W_1$ and $W_2$ are each independently selected from a bond, -O-, -S-, $-NR^{W1}-$, - $CONR^{W1}-$, $-NR^{W1}CO-$, -C(=O)O-, or -OC(=O)-;

$R^{W1}$ is selected from H, $C_{1-4}$ alkyl, or halogen;

alternatively, B, $L_2$ and C, together with atoms to which they are connected, form tetracyclic heterocyclyl, optionally substituted with 1-4 groups selected from halogen, CN, OH, $NO_2$, =O, COOH, $C_{1-4}$ alkyl, $C_{2-6}$ alkenyl, $C_{2-6}$ alkynyl, $-OC_{1-4}$ alkyl or -O-halo $C_{1-4}$ alkyl;

each $R^A$ is independently selected from H, halogen, CN, OH, COOH, $C_{1-4}$ alkyl, $C_{2-6}$ alkenyl, $C_{2-6}$ alkynyl, $-OC_{1-4}$ alkyl, $-NH-S(O)_2-C_{1-4}$ alkyl, $-S(O)_2-C_{1-4}$ alkyl, - $S(O)_2-(CH_2)_p-R^a$, $-P(O)-(C_{1-4}$ alkyl$)_2$, $-C(O)-OR^a$, $-(CH_2)_p-C(O)-(CH_2)_p-R^a$, - $NHC(O)-R^a$, $-C(O)N(C_{1-4}$ alkyl$)_2$, or $-NHC(O)-C_{1-4}$ alkyl, 3- to 6-membered heterocycloalkyl, or $-NHR^a$, wherein the alkyl, alkenyl, alkynyl or heterocycloalkyl is optionally further substituted with 1-4 groups selected from halogen, OH, $NH_2$, CN, -O-halo $C_{1-4}$ alkyl, deuterium or $C_{1-4}$ alkyl, and the heterocycloalkyl contains 1-3 heteroatoms selected from N, O or S; or

each $R^A$ is independently selected from H, halogen, CN, OH, COOH, $C_{1-4}$ alkyl, $C_{2-6}$ alkenyl, $C_{2-6}$ alkynyl, $-OC_{1-4}$ alkyl, $-NH-S(O)_2-C_{1-4}$ alkyl, $-S(O)_2-C_{1-4}$ alkyl, - $S(O)_2-(CH_2)_p-R^a$, $-P(O)-(C_{1-4}$ alkyl$)_2$, $-C(O)-OR^a$, $-(CH_2)_p-C(O)-(CH_2)_p-R^a$, - $NHC(O)-R^a$, or $-C(O)N(C_{1-4}$ alkyl$)_2$, or $-NHC(O)-(C_{1-4}$ alkyl$)_2$, wherein the alkyl, alkenyl, or alkynyl is optionally further substituted with 1-4 groups selected from halogen, OH, $NH_2$, CN, -O-halo $C_{1-4}$ alkyl or deuterium;

$R^a$ is selected from $C_{2-4}$ alkynyl, 3- to 5-membered cycloalkyl, 3- to 6-membered heterocycloalkyl, 5- or 6-membered heteroaryl, $C_{1-4}$ alkoxy, $-O-C_{3-6}$ cycloalkyl, deuterated $C_{1-4}$ alkyl, or $C_{1-4}$ alkyl, wherein the alkynyl, cycloalkyl, heterocycloalkyl, heteroaryl, or alkoxy is optionally further substituted with 1-4 groups selected from halogen, $C_{1-4}$ alkyl, halo $C_{1-4}$ alkyl, deuterated $C_{1-4}$ alkyl, 6-membered heterocycloalkyl, 5- or 6-membered heteroaryl, $C_{2-4}$ alkynyl, 3- to 5-membered cycloalkyl, -O-halo $C_{1-4}$ alkyl, OH, $-S(O)_2-CH_3$, or $=CH_2$, =CHF or $=CF_2$, wherein the alkyl is further substituted with 1-3 groups selected from OH or CN, and the heterocycloalkyl or heteroaryl contains 1-3 heteroatoms selected from N, O or S; or

$R^a$ is selected from $C_{2-4}$ alkynyl, 3- to 5-membered cycloalkyl, 3- to 6-membered heterocycloalkyl, 5- or 6-membered heteroaryl, $C_{1-4}$ alkoxy, $-O-C_{3-6}$ cycloalkyl, or deuterated $C_{1-4}$ alkyl, wherein the alkynyl, cycloalkyl, heterocycloalkyl, heteroaryl, alkyl or alkoxy is optionally further substituted with 1-4 groups selected from halogen, $C_{1-4}$ alkyl, halo $C_{1-4}$ alkyl, deuterated $C_{1-4}$ alkyl, 6-membered heterocycloalkyl, 5- or 6-membered heteroaryl, $C_{2-4}$ alkynyl, 3- to 5-membered cycloalkyl, -O-halo $C_{1-4}$ alkyl, OH or $-S(O)_2-CH_3$, and the heterocycloalkyl or heteroaryl contains 1-3 heteroatoms selected from N, O or S;

or $R^a$ is selected from $C_{2-4}$ alkynyl, 3- to 5-membered cycloalkyl, 3- to 6-membered heterocycloalkyl, 5- or 6-

membered heteroaryl, $C_{1-4}$ alkoxy, -O-$C_{3-6}$ cycloalkyl, or deuterated $C_{1-4}$ alkyl, wherein the alkynyl, cycloalkyl, heterocycloalkyl, heteroaryl or alkyl is optionally further substituted with 1-4 groups selected from 6-membered heterocycloalkyl, 5- or 6-membered heteroaryl, $C_{2-4}$ alkynyl, 3- to 5-membered cycloalkyl, -O-halo $C_{1-4}$ alkyl, OH or -$S(O)_2$-$CH_3$, and the heterocycloalkyl or heteroaryl contains 1-3 heteroatoms selected from N, O or S;
each $R^B$ is independently selected from H, halogen, CN, =O, OH, $NO_2$, -$SF_5$, $C_{1-4}$ alkyl, -P(O)-$(CH_3)_2$, -$S(O)_2$-$CH_3$, -$S(O)_2$-$CH_2R^a$-, $NH_2$, -$C_{1-4}$ alkoxy, $C_{2-6}$ alkenyl, $C_{2-6}$ alkynyl, 3- to 5-membered cycloalkyl, halo $C_{1-2}$ alkyl, 5- or 6-membered heteroaryl containing 1-3 heteroatoms selected from N, O or S, or 3- to 6-membered heterocycloalkyl containing 1-3 heteroatoms selected from N, O or S, wherein the alkoxy, cycloalkyl, heteroaryl or heterocycloalkyl is optionally further substituted with a $C_{1-2}$ alkyl group;
each $R^C$ is independently selected from H, halogen, CN, =O, OH, $NO_2$, $C_{1-4}$ alkyl, halo $C_{1-4}$ alkyl, -O-$C_{3-5}$ cycloalkyl, $C_{3-5}$ cycloalkyl, $C_{2-4}$ alkenyl, $C_{2-4}$ alkynyl, -$SCF_3$, -$SF_5$, -$(NH)_p$-P(O)($C_{1-4}$ alkyl$)_2$, or -$(CH_2)_p$-O-$C_{1-4}$ alkyl;
p is selected from 0, 1, 2, 3 or 4.

Further, a compound represented by formula (I), or a stereoisomer, a deuterated substance, a solvate, a co-crystal or a pharmaceutically acceptable salt thereof, wherein

A is selected from 4- to 14-membered heterocyclyl, 4- to 6-membered heterocycloalkylene, ($C_{3-4}$ cycloalkyl$)_{0-1}$-NH-$SO_2$-$C_{1-4}$ alkyl, or 5- or 6-membered cycloalkyl, wherein the heterocyclyl or heterocycloalkylene contains 1-3 heteroatoms selected from N, O or S; the cycloalkyl, heterocyclyl, heterocycloalkylene or alkyl is optionally substituted with 1-4 $R^A$;
B is selected from phenyl, 6- to 12-membered bicyclic heterocyclyl, or 6- to 12-membered heteroaryl, wherein the heterocyclyl or heteroaryl contains 1-3 heteroatoms selected from N, O or S; the heterocyclyl, heteroaryl or phenyl is optionally substituted with 1-4 $R^B$;
C is selected from phenyl, 6- to 12-membered partially unsaturated bicyclic heterocyclyl, 8- to 14-membered tricyclic heterocyclyl, $Y_1$-phenyl, or $Y_1$-5- to 10-membered heteroaryl, wherein the heterocyclyl or heteroaryl contains 1-3 heteroatoms selected from N, O or S; the heterocyclyl, heteroaryl or phenyl is optionally substituted with 1-4 $R^C$;
each $Y_1$ is independently selected from NH$C_{1-4}$ alkylene, or N($C_{1-4}$ alkyl)$C_{1-4}$ alkylene, wherein the alkylene is optionally substituted with 1-4 groups selected from halogen, =O, OH or CN;
$L_1$ and $L_2$ are each independently selected from $W_1$-$R^L$-$W_2$, wherein $L_1$ is connected to A on the left side, and $L_2$ is connected to B on the left side; $L_1$ and $L_2$ are not both bonds;
$R^L$ is selected from a bond, $C_{1-4}$ alkylene, $C_{2-4}$ alkenylene, or 3- to 6-membered cycloalkyl, wherein the alkylene, alkenylene or cycloalkyl is optionally further substituted with 1-4 $R^{L1}$; each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy or cycloalkyl is optionally further substituted with 1-4 substituents selected from halogen, CN, OH and $NH_2$;
$W_1$ and $W_2$ are each independently selected from a bond, -O-, -S-, -$NR^{W1}$-, - $CONR^{W1}$-, -$NR^{W1}$CO-, -C(=O)O-, or -OC(=O)-;
$R^{W1}$ is selected from H, $C_{1-4}$ alkyl, or halogen;
alternatively, B, $L_2$ and C, together with atoms to which they are connected, form tetracyclic heterocyclyl, optionally substituted with a group selected from H, halogen, CN, OH, $NO_2$, =O, COOH, $C_{1-4}$ alkyl, $C_{2-6}$ alkenyl, $C_{2-6}$ alkynyl, -O$C_{1-4}$ alkyl or -O-halo $C_{1-4}$ alkyl;
each $R^A$ is independently selected from H, halogen, CN, OH, $NO_2$, COOH, $C_{1-4}$ alkyl, $C_{2-6}$ alkenyl, $C_{2-6}$ alkynyl, -O$C_{1-4}$ alkyl, -NH-$S(O)_2$-$C_{1-4}$ alkyl, -$S(O)_2$-$(CH_2)_p$, -$S(O)_2$-$(CH_2)_p$-$R^a$, -P(O)-($C_{1-4}$ alkyl$)_2$, -C(O)-$OR^a$, or -$(CH_2)_p$-C(O)-$(CH_2)_p$-$R^a$, wherein the alkyl, alkenyl, or alkynyl is optionally further substituted with 1-4 groups selected from halogen, OH, $NH_2$, CN or -O-halo $C_{1-4}$ alkyl;
$R^a$ is selected from $C_{2-4}$ alkynyl, 3- to 5-membered cycloalkyl, 3- to 6-membered heterocycloalkyl, or 5- or 6-membered heteroaryl, wherein the heterocycloalkyl or heteroaryl is optionally further substituted with 1-4 groups selected from 6-membered heterocycloalkyl, 5- or 6-membered heteroaryl, $C_{2-4}$ alkynyl, 3- to 5-membered cycloalkyl, -O-halo $C_{1-4}$ alkyl or OH, and the heterocycloalkyl or heteroaryl contains 1-3 heteroatoms selected from N, O or S;
each $R^B$ is independently selected from H, halogen, CN, =O, OH, $NO_2$, -$SF_5$, $C_{1-4}$ alkyl, -P(O)-$(CH_3)_2$, -$S(O)_2$-$CH_3$, -$S(O)_2$-$CH_2R^a$-, $NH_2$, -$C_{1-4}$ alkoxy, $C_{2-6}$ alkenyl, $C_{2-6}$ alkynyl, 3- to 5-membered cycloalkyl, halo $C_{1-2}$ alkyl, 5- or 6-membered heteroaryl containing 1-3 heteroatoms selected from N, O or S, or 3- to 6-membered heterocycloalkyl containing 1-3 heteroatoms selected from N, O or S, wherein the alkoxy, cycloalkyl, heteroaryl or heterocycloalkyl is optionally further substituted with a $C_{1-2}$ alkyl group;
each $R^C$ is independently selected from H, halogen, CN, =O, OH, $NO_2$, $C_{1-4}$ alkyl, -O-$C_{3-5}$ cycloalkyl, $C_{3-5}$ cycloalkyl, $C_{2-4}$ alkynyl, -$SCF_3$, -$SF_5$, -$(NH)_p$-P(O)($C_{1-4}$ alkyl$)_2$, or -$(CH_2)_p$-O-$C_{1-4}$ alkyl;
p is selected from 0, 1, 2, 3 or 4;
the compound satisfies at least one of the following conditions:

A is selected from one of the groups formed by the following structures optionally substituted with 1-4 $R^A$:

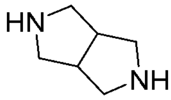, 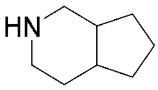, 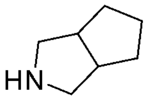, 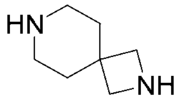, 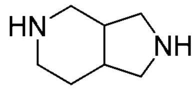,

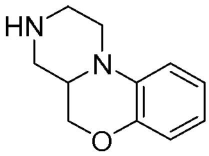, 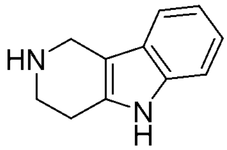, 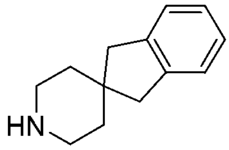, 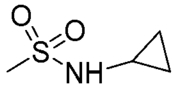, 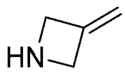,

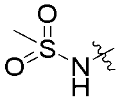, 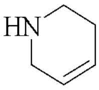, or 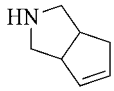;

2) B is selected from one of the groups formed by the following structures optionally substituted with 1-4 $R^B$:

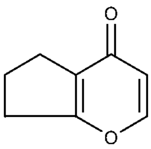, 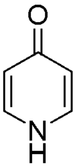, 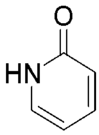, 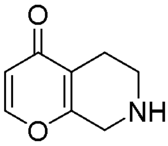,

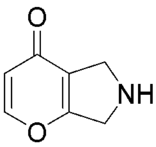, 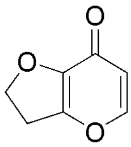, 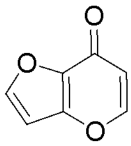, 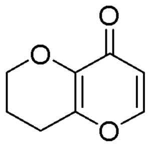, 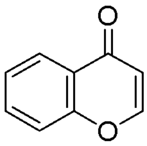, 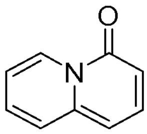, 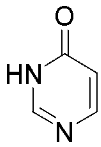,

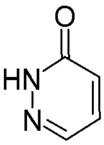, 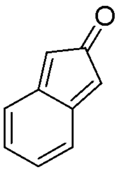, 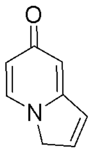, 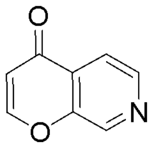, 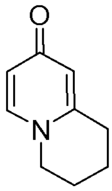, 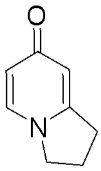, 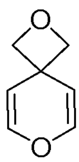, 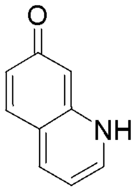,

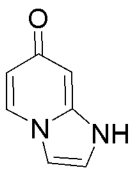, 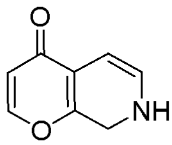, 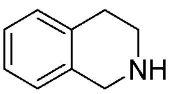, 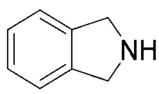, 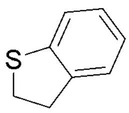, 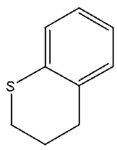,

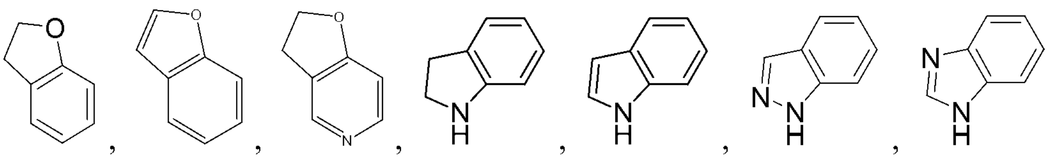, , , , , , ,

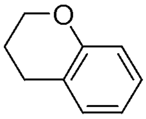, 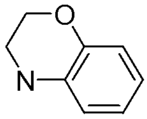, 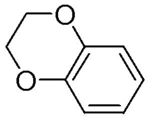, 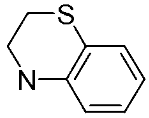, 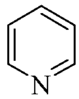, or 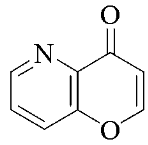;

3) the C ring is selected from one of the following groups optionally substituted with 1-4 $R^C$:

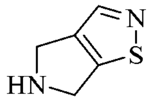, 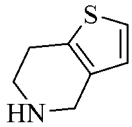, 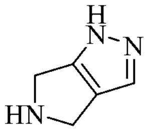, 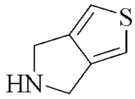, 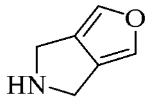,

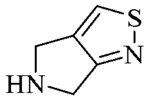, 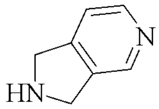, 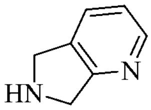, 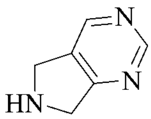, 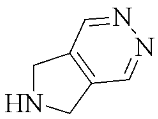, 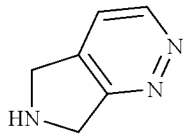,

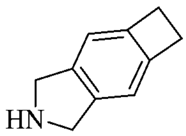, 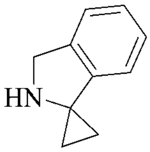, 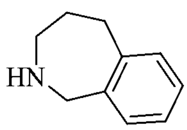, 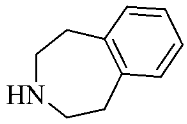, 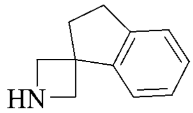,

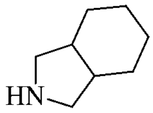, 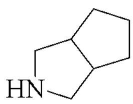, 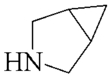, 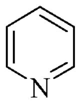, 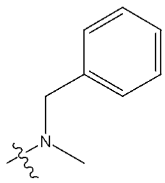, 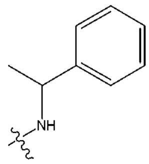, 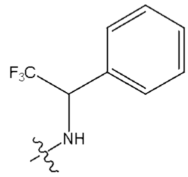,

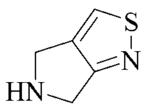, 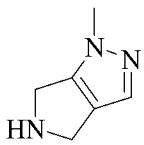, 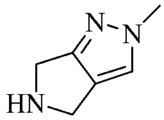, 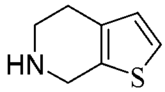, 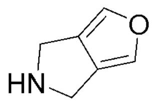, 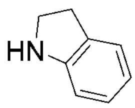,

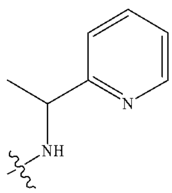, 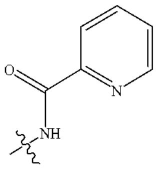, or 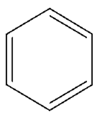;

4) B, $L_2$ and C, together with atoms to which they are connected, form a tetracyclic heterocycloalkyl, optionally substituted with a group selected from H, halogen, CN, OH, $NO_2$, =O, COOH, $C_{1-4}$ alkyl, $C_{2-6}$ alkenyl, $C_{2-6}$ alkynyl, -$OC_{1-4}$ alkyl or -O-halo $C_{1-4}$ alkyl;

5) at least one $R^A$ is selected from: -P(O)-($C_{1-4}$ alkyl)$_2$, -NH-S(O)$_2$-$C_{1-4}$ alkyl, - COOH, -$C_{1-4}$ alkyl-C(O)-4- to 6-membered heterocycloalkyl, -C(O)-$C_{1-4}$ alkyl-4- to 6-membered heterocycloalkyl, -C(O)-$C_{1-4}$ alkyl-$C_{3-6}$ cycloalkyl, -$C_{1-4}$ alkyl-C(O)-O-4- to 6-membered heterocycloalkyl, -S(O)$_2$-$C_{1-4}$ alkyl-4- to 6-membered heterocycloalkyl, -S(O)$_2$-$C_{1-4}$ alkyl-$C_{2-4}$ alkynyl, -S(O)$_2$-$C_{1-4}$ alkyl-$C_{3-5}$ cycloalkyl, - S(O)$_2$-$C_{1-4}$ alkyl-5- or 6-membered heteroaryl, or -S(O)$_2$-$C_{1-4}$ alkyl-O-halo $C_{1-4}$ alkyl;

6) at least one $R^B$ is selected from: $C_{1-4}$ alkyl, halogen, -$C_{1-4}$ alkoxy, CN, halo $C_{1-4}$ alkyl, $SF_5$, -P(O)-($C_{1-4}$ alkyl)$_2$, $C_{2-4}$ alkynyl, $C_{2-4}$ alkenyl, $C_{3-5}$ cycloalkyl, or $C_{5-6}$ heteroaryl;

7) at least one $R^C$ is selected from: $C_{1-4}$ alkyl, halogen, CN, $C_{2-4}$ alkynyl, $SCF_3$, $SF_5$, =O, $-(NH)_p-P(O)(C_{1-4}\ alkyl)_2$, $-CH_2-O-C_{1-4}$ alkyl, $C_{3-5}$ cycloalkyl, or $-O-C_{3-5}$ cycloalkyl;

8) $L_1$ is selected from: a bond, halo $C_{1-4}$ alkoxy, $C_{1-4}$ alkyl, -C(O)-O-, $-O-CH_2-CH=C-$, $-CH_2-N(CH_3)-$, $-N(CH_3)-C(O)-$, -O-, $-S-C_{1-4}$ alkyl, $C_{2-4}$ alkenyl, or $-CH(C_{3-4}$ cycloalkyl)-O-;

9) $L_2$ is selected from: a bond, $-C_{1-4}$ alkyl-NH-C($C_{1-4}$ alkyl)-, $-C_{1-4}$ alkyl-NH-C(halo $C_{1-4}$ alkyl)-, $-C_{1-4}$ alkyl-NH-C(O)-, $-CH_2-N(CH_3)-C_{1-4}$ alkyl-, or $-CH_2-C_{3-4}$ cycloalkyl.

A specific technical solution 2 of the present invention provides a compound represented by formula (I), or a stereoisomer, a deuterated substance, a solvate, a co-crystal or a pharmaceutically acceptable salt thereof, wherein

A is selected from 4- to 6-membered monocyclic heterocyclyl, 7- to 9-membered bicyclic heterocyclyl, 5- or 6-membered cycloalkyl, or phenyl, wherein the heterocyclyl, cycloalkyl or phenyl is optionally substituted with 1-4 $R^A$;

each $R^A$ is independently selected from H, halogen, CN, OH, COOH, $C_{1-4}$ alkyl, $C_{2-4}$ alkenyl, $C_{2-4}$ alkynyl, $-OC_{1-4}$ alkyl, $-NH-S(O)_2-C_{1-2}$ alkyl, $-S(O)_2-C_{1-2}$ alkyl, $-S(O)_2-(CH_2)_p-R^a$, $-P(O)-(C_{1-2}\ alkyl)_2$, $-C(O)-OR^a$, $-(CH_2)_p-C(O)-(CH_2)_p-R^a$, $-NHC(O)-R^a$, $-C(O)N(C_{1-4}\ alkyl)_2$, or $-NHC(O)-C_{1-4}$ alkyl, or 3- to 6-membered heterocycloalkyl, or $-NHR^a$, wherein the alkyl, alkenyl, alkynyl or heterocycloalkyl is optionally further substituted with 1-4 groups selected from halogen, OH, $NH_2$, CN, -O-halo $C_{1-2}$ alkyl, deuterium or $C_{1-4}$ alkyl, and the heterocycloalkyl contains 1-3 heteroatoms selected from N, O or S; or

each $R^A$ is independently selected from H, halogen, CN, OH, COOH, $C_{1-4}$ alkyl, $C_{2-4}$ alkenyl, $C_{2-4}$ alkynyl, $-OC_{1-4}$ alkyl, $-NH-S(O)_2-C_{1-2}$ alkyl, $-S(O)_2-C_{1-2}$ alkyl, $-S(O)_2-C_{1-2}$ alkyl-$R^a$, $-P(O)-(C_{1-2}\ alkyl)_2$, $-C(O)-OR^a$, $-(CH_2)_p-C(O)-(CH_2)_p-R^a$, $-NHC(O)-R^a$, or $-C(O)N(C_{1-4}\ alkyl)_2$, or $-NHC(O)-(C_{1-4}\ alkyl)_2$, wherein the alkyl, alkenyl, or alkynyl is optionally further substituted with 1-4 groups selected from halogen, OH, $NH_2$, CN, -O-halo $C_{1-2}$ alkyl or deuterium;

$R^a$ is selected from $C_{2-4}$ alkynyl, cyclopropyl, cyclobutyl, 4- to 6-membered heterocycloalkyl, pyrazolyl, $C_{1-4}$ alkoxy, $-O-C_{3-6}$ cycloalkyl, deuterated $C_{1-2}$ alkyl, or $C_{1-2}$ alkyl, wherein the cyclopropyl, cyclobutyl, heterocycloalkyl, pyrazolyl, or alkoxy is optionally further substituted with a group selected from halogen, $C_{1-2}$ alkyl, halo $C_{1-2}$ alkyl, deuterated $C_{1-2}$ alkyl, OH, $-S(O)_2-CH_3$, $=CH_2$, =CHF or $=CF_2$, and the alkyl is further substituted with 1-3 groups selected from OH or CN; or

$R^a$ is selected from $C_{2-4}$ alkynyl, cycloprpoyl, cyclobutyl, 4- to 6-membered heterocycloalkyl, pyrazolyl, $C_{1-4}$ alkoxy, $-O-C_{3-6}$ cycloalkyl, or deuterated $C_{1-2}$ alkyl, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH or $-S(O)_2-CH_3$;

or $R^a$ is selected from $C_{2-4}$ alkynyl, cycloprpoyl, 4- to 6-membered heterocycloalkyl, pyrazolyl, $C_{1-4}$ alkoxy, $-O-C_{3-6}$ cycloalkyl, or deuterated $C_{1-2}$ alkyl, wherein the cyclopropyl, heterocycloalkyl, pyrazolyl or alkoxy is optionally further substituted with a group selected from halogen, $C_{1-2}$ alkyl, OH or $-S(O)_2-CH_3$;

p is selected from 0, 1 or 2;

B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, or benzo 5- or 6-membered heterocyclyl, wherein the heterocyclyl, heteroaryl, carbocyclyl or aryl is optionally substituted with 1-4 $R^B$;

each $R^B$ is independently selected from halogen, CN, $-P(=O)(C_{1-2}\ alkyl)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;

C is selected from phenyl, 5- to 7-membered heterocyclyl fused phenyl, 5- or 6-membered carbocyclyl fused phenyl, or 5- or 6-membered heterocyclyl fused 5- or 6-membered heteroaryl, wherein the heterocyclyl or heteroaryl contains 1-3 heteroatoms selected from N, O or S; the phenyl, heterocyclyl, carbocyclyl or heteroaryl is optionally substituted with 1-4 $R^C$;

each $R^C$ is independently selected from CN, OH, $C_{1-2}$ alkyl, halo $C_{1-2}$ alkyl, -O-cyclopropyl, cyclopropyl, $C_{2-4}$ alkenyl, $C_{2-4}$ alkynyl, $-SCF_3$, $-SF_5$, $-(NH)_{0-1}-P(O)(C_{1-2}\ alkyl)_2$, or $C_{1-2}$ alkyl substituted with $C_{1-2}$ alkoxy;

$L_1$ is selected from a bond, $C_{1-2}$ alkylene-O, $C_{1-2}$ alkylene, N($C_{1-2}$ alkylene)-$C_{1-2}$ alkylene, -O-, or $C_{2-4}$ alkenylene, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

or $L_1$ is selected from a bond, $C_{1-2}$ alkylene-O, $C_{1-2}$ alkylene, N($C_{1-2}$ alkylene)-$C_{1-2}$ alkylene, or -O-, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

$L_2$ is selected from a bond, $C_{1-2}$ alkylene, or $-N(CH_3)-$, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo

$C_{1-2}$ alkoxy, or 3- to 6-membered cycloalkyl.

Further, a compound represented by formula (I), or a stereoisomer, a deuterated substance, a solvate, a co-crystal or a pharmaceutically acceptable salt thereof, wherein

A is selected from 4- to 14-membered heterocyclyl, 5- or 6-membered cycloalkyl, or phenyl, wherein the heterocyclyl contains 1-3 heteroatoms selected from N, O or S; the heterocyclyl, cycloalkyl or phenyl is optionally substituted with 1-4 $R^A$;
B is selected from 6- to 12-membered bicyclic carbocyclyl, 6- to 12-membered bicyclic heterocyclyl, or 6- to 12-membered heteroaryl, wherein the heterocyclyl or heteroaryl contains 1-3 heteroatoms selected from N, O or S; the carbocyclyl, heterocyclyl or heteroaryl is optionally substituted with 1-4 $R^B$;
C is selected from phenyl, 6- to 12-membered bicyclic carbocyclyl, 6- to 12-membered partially unsaturated bicyclic heterocyclyl, or 8- to 14-membered tricyclic heterocyclyl, wherein the heterocyclyl contains 1-3 heteroatoms selected from N, O or S; the cycloalkyl, heterocyclyl or phenyl is optionally substituted with 1-4 $R^C$;
$L_1$ and $L_2$ are each independently selected from $W_1$-$R^L$-$W_2$, wherein $L_1$ is connected to A on the left side, and $L_2$ is connected to B on the left side; $L_1$ and $L_2$ are not both bonds;
$R^L$ is selected from a bond, $C_{1-4}$ alkylene, $C_{2-4}$ alkenylene, or 3- to 6-membered cycloalkyl, wherein the alkylene, alkenylene or cycloalkyl is optionally further substituted with 1-4 $R^{L1}$; each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy or cycloalkyl is optionally further substituted with 1-4 substituents selected from halogen, CN, OH and $NH_2$;
$W_1$ and $W_2$ are each independently selected from a bond, -O-, -S-, -$NR^{W1}$-, - $CONR^{W1}$-, -$NR^{W1}CO$-, -C(=O)O-, or -OC(=O)-;
$R^{W1}$ is selected from H, $C_{1-4}$ alkyl, or halogen;
alternatively, B, $L_2$ and C, together with atoms to which they are connected, form tetracyclic heterocyclyl, optionally substituted with 1-4 groups selected from halogen, CN, OH, $NO_2$, =O, COOH, $C_{1-4}$ alkyl, $C_{2-6}$ alkenyl, $C_{2-6}$ alkynyl, -$OC_{1-4}$ alkyl or -O-halo $C_{1-4}$ alkyl;
each $R^A$ is independently selected from H, halogen, CN, OH, COOH, $C_{1-4}$ alkyl, $C_{2-6}$ alkenyl, $C_{2-6}$ alkynyl, -$OC_{1-4}$ alkyl, -NH-$S(O)_2$-$C_{1-4}$ alkyl, -$S(O)_2$-$C_{1-4}$ alkyl, - $S(O)_2$-$(CH_2)_p$-$R^a$, -P(O)-$(C_{1-4}$ alkyl$)_2$, -C(O)-$OR^a$, -$(CH_2)_p$-C(O)-$(CH_2)_p$-$R^a$, - NHC(O)-$R^a$, -C(O)N($C_{1-4}$ alkyl$)_2$, or -NHC(O)-($C_{1-4}$ alkyl$)_2$, wherein the alkyl, alkenyl, or alkynyl is optionally further substituted with 1-4 groups selected from halogen, OH, $NH_2$, CN, -O-halo $C_{1-4}$ alkyl or deuterium;
$R^a$ is selected from $C_{2-4}$ alkynyl, 3- to 5-membered cycloalkyl, 3- to 6-membered heterocycloalkyl, or 5- or 6-membered heteroaryl, wherein the heterocycloalkyl or heteroaryl is optionally further substituted with 1-4 groups selected from 6-membered heterocycloalkyl, 5- or 6-membered heteroaryl, $C_{2-4}$ alkynyl, 3- to 5-membered cycloalkyl, -O-halo $C_{1-4}$ alkyl, OH or -$S(O)_2$-$CH_3$, and the heterocycloalkyl or heteroaryl contains 1-3 heteroatoms selected from N, O or S;
each $R^B$ is independently selected from H, halogen, CN, =O, OH, $NO_2$, -$SF_5$, $C_{1-4}$ alkyl, -P(O)-$(CH_3)_2$, -$S(O)_2$-$CH_3$, -$S(O)_2$-$CH_2R^a$-, $NH_2$, -$C_{1-4}$ alkoxy, $C_{2-6}$ alkenyl, $C_{2-6}$ alkynyl, 3- to 5-membered cycloalkyl, halo $C_{1-2}$ alkyl, 5- or 6-membered heteroaryl containing 1-3 heteroatoms selected from N, O or S, or 3- to 6-membered heterocycloalkyl containing 1-3 heteroatoms selected from N, O or S, wherein the alkoxy, cycloalkyl, heteroaryl or heterocycloalkyl is optionally further substituted with a $C_{1-2}$ alkyl group;
each $R^C$ is independently selected from H, halogen, CN, =O, OH, $NO_2$, $C_{1-4}$ alkyl, halo $C_{1-4}$ alkyl, -O-$C_{3-5}$ cycloalkyl, $C_{3-5}$ cycloalkyl, $C_{2-4}$ alkenyl, $C_{2-4}$ alkynyl, -$SCF_3$, -$SF_5$, -$(NH)_p$-P(O)($C_{1-4}$ alkyl$)_2$, or -$(CH_2)_p$-O-$C_{1-4}$ alkyl;
p is selected from 0, 1, 2, 3 or 4.

A specific technical solution 3 of the present invention is the compound, or the stereoisomer, the deuterated substance, the solvate, the co-crystal or the pharmaceutically acceptable salt thereof described in solution 1, wherein

A is selected from 4- to 6-membered monocyclic heterocycloalkyl, 5- or 6-membered heterocyclyl fused 5- or 6-membered heterocyclyl, 5- or 6-membered heterocyclyl spiro 5- or 6-membered cycloalkyl, 5- or 6-membered heterocyclyl fused 5- or 6-membered cycloalkyl, 4- to 6-membered heterocycloalkyl spiro 5- or 6-membered heterocycloalkyl, 5- or 6-membered cycloalkyl fused 5- or 6-membered heterocycloalkyl, 5- or 6-membered cycloalkyl spiro 5- or 6-membered heterocycloalkyl, 10- to 14-membered partially unsaturated tricyclic heterocycloalkyl, cyclopropyl-$NHSO_2$-$C_{1-2}$ alkyl, -$NHSO_2$-$C_{1-2}$ alkyl, cyclohexyl, or 4-membered heterocycloalkylene, wherein the cycloalkyl, heterocycloalkyl or heterocyclyl is optionally substituted with 1-4 $R^A$;
each $R^A$ is independently selected from H, halogen, CN, OH, $NO_2$, COOH, $C_{1-4}$ alkyl, $C_{2-4}$ alkenyl, $C_{2-4}$ alkynyl, -$OC_{1-4}$ alkyl, -NH-$S(O)_2$-$C_{1-2}$ alkyl, -$S(O)_2$-$C_{1-2}$ alkyl, -$S(O)_2$-$C_{1-2}$ alkyl-$R^a$, -P(O)-($C_{1-2}$ alkyl$)_2$, -C(O)-$OR^a$, or -$(CH_2)_p$-C(O)-$(CH_2)_p$-$R^a$, wherein the alkyl, alkenyl, or alkynyl is optionally further substituted with 1-4 groups selected from halogen, OH, $NH_2$, CN or -O-halo $C_{1-2}$ alkyl;

$R^a$ is selected from $C_{2-4}$ alkynyl, cycloprpoyl, 4- to 6-membered heterocycloalkyl, pyrazolyl, or $C_{1-4}$ alkoxy, wherein the cyclopropyl, heterocycloalkyl, pyrazolyl or alkoxy is optionally further substituted with a group selected from halogen, $C_{1-2}$ alkyl or OH;
p is selected from 0, 1 or 2.

A specific technical solution 4 of the present invention is the compound, or the stereoisomer, the deuterated substance, the solvate, the co-crystal or the pharmaceutically acceptable salt thereof described in solution 1, wherein

B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, phenyl, benzo 5- or 6-membered heterocyclyl, or 5- or 6-membered heterocyclyl fused 5- or 6-membered heteroaryl, wherein the heterocyclyl, heteroaryl, carbocyclyl, phenyl or aryl is optionally substituted with 1-4 $R^B$;
each $R^B$ is independently selected from halogen, CN, $-P(=O)(C_{1-2}\ alkyl)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group.

A specific technical solution 5 of the present invention is the compound, or the stereoisomer, the deuterated substance, the solvate, the co-crystal or the pharmaceutically acceptable salt thereof described in solution 1, wherein

C is selected from phenyl, 5- to 7-membered heterocyclyl fused phenyl, 5- or 6-membered carbocyclyl fused phenyl, 5- or 6-membered heterocyclyl fused 5- or 6-membered heteroaryl, 5- or 6-membered heterocycloalkyl fused 3- to 6-membered cycloalkyl, 9-12 membered tricyclic heterocyclyl, $Y_1$-phenyl, or $Y_1$-5- or 6-membered heteroaryl, wherein the heterocyclyl, heterocycloalkyl or heteroaryl contains 1-3 heteroatoms selected from N, O or S; the phenyl, heterocyclyl, carbocyclyl, heteroaryl, heterocycloalkyl or cycloalkyl is optionally substituted with 1-4 $R^C$;
each $Y_1$ is independently selected from $NHC_{1-2}$ alkylene, or $N(C_{1-2}\ alkyl)C_{1-2}$ alkylene, wherein the alkylene is optionally substituted with 1-4 groups selected from halogen, =O, OH or CN;
each $R^C$ is independently selected from CN, OH, $C_{1-2}$ alkyl, =O, -O-cyclopropyl, cyclopropyl, $C_{2-4}$ alkynyl, $-SCF_3$, $-SF_5$, $-(NH)_{0-1}-P(O)(C_{1-2}\ alkyl)_2$, or $C_{1-2}$ alkyl substituted with $C_{1-2}$ alkoxy.

A specific technical solution 6 of the present invention is the compound, or the stereoisomer, the deuterated substance, the solvate, the co-crystal or the pharmaceutically acceptable salt thereof described in solution 1, wherein

$L_1$ is selected from a bond, $C_{1-2}$ alkylene-O, $C_{1-2}$ alkylene, $C_{1-2}$ alkylene-S, $C_{1-2}$ alkylene-N($C_{1-2}$ alkylene), N($C_{1-2}$ alkylene)-$C_{1-2}$ alkylene, -O-$C_{1-3}$ alkylene, $C_{2-4}$ alkenylene, -N($C_{1-2}$ alkylene)-C(=O), -C(=O)-N($C_{1-2}$ alkylene), -O-, -C(=O)-O-, or 3- to 6-membered cycloalkyl-O, wherein the alkylene, alkenylene or cycloalkyl is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;
$L_2$ is selected from a bond, $C_{1-2}$ alkylene, -C(=O)-, 3- to 6-membered cycloalkyl, -NH-, or -O-, wherein the alkylene, alkenylene or cycloalkyl is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl.

A specific technical solution 7 of the present invention is the compound, or the stereoisomer, the deuterated substance, the solvate, the co-crystal or the pharmaceutically acceptable salt thereof described in solution 1, wherein

A is selected from 4- to 6-membered monocyclic heterocyclyl, 5- or 6-membered heterocyclyl fused 5- or 6-membered heterocyclyl, 5- or 6-membered heterocyclyl spiro 5- or 6-membered cycloalkyl, 5- or 6-membered heterocyclyl fused 5- or 6-membered cycloalkyl, 4- to 6-membered heterocycloalkyl spiro 5- or 6-membered heterocycloalkyl, 5- or 6-membered cycloalkyl fused 5- or 6-membered heterocycloalkyl, 5- or 6-membered cycloalkyl spiro 5- or 6-membered heterocycloalkyl, 10- to 14-membered partially unsaturated tricyclic heterocycloalkyl, cyclopropyl-$NHSO_2-C_{1-2}$ alkyl, $-NHSO_2-C_{1-2}$ alkyl, cyclohexyl, or 4-membered heterocycloalkylene, wherein the cycloalkyl, heterocycloalkyl or heterocyclyl is optionally substituted with 1-4 $R^A$;
each $R^A$ is independently selected from H, halogen, CN, OH, $NO_2$, COOH, $C_{1-4}$ alkyl, $C_{2-4}$ alkenyl, $C_{2-4}$ alkynyl, $-OC_{1-4}$ alkyl, $-NH-S(O)_2-C_{1-2}$ alkyl, $-S(O)_2-C_{1-2}$ alkyl, $-S(O)_2-C_{1-2}$ alkyl-$R^a$, $-P(O)-(C_{1-2}\ alkyl)_2$, $-C(O)-OR^a$, or $-(CH_2)_p-C(O)-(CH_2)_p-R^a$, wherein the alkyl, alkenyl, or alkynyl is optionally further substituted with 1-4 groups selected from halogen, OH, $NH_2$, CN or -O-halo $C_{1-2}$ alkyl;
$R^a$ is selected from $C_{2-4}$ alkynyl, cycloprpoyl, 4- to 6-membered heterocycloalkyl, pyrazolyl, or $C_{1-4}$ alkoxy, wherein the

cyclopropyl, heterocycloalkyl, pyrazolyl or alkoxy is optionally further substituted with a group selected from halogen, $C_{1-2}$ alkyl or OH;
p is selected from 0, 1 or 2;
B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, phenyl, benzo 5- or 6-membered heterocyclyl, or 5- or 6-membered heterocyclyl fused 5- or 6-membered heteroaryl, wherein the heterocyclyl, heteroaryl, carbocyclyl, phenyl or aryl is optionally substituted with 1-4 $R^B$;
each $R^B$ is independently selected from halogen, CN, $-P(=O)(C_{1-2}\ alkyl)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;
C is selected from phenyl, 5- to 7-membered heterocyclyl fused phenyl, 5- or 6-membered carbocyclyl fused phenyl, 5- or 6-membered heterocyclyl fused 5- or 6-membered heteroaryl, 5- or 6-membered heterocycloalkyl fused 3- to 6-membered cycloalkyl, 9-12 membered tricyclic heterocyclyl, $Y_1$-phenyl, or $Y_1$-5- or 6-membered heteroaryl, wherein the heterocyclyl, heterocycloalkyl or heteroaryl contains 1-3 heteroatoms selected from N, O or S; the phenyl, heterocyclyl, carbocyclyl, heteroaryl, heterocycloalkyl or cycloalkyl is optionally substituted with 1-4 $R^C$;
each $Y_1$ is independently selected from $NHC_{1-2}$ alkylene, or $N(C_{1-2}\ alkyl)C_{1-2}$ alkylene, wherein the alkylene is optionally substituted with 1-4 groups selected from halogen, =O, OH or CN;
each $R^C$ is independently selected from CN, OH, $C_{1-2}$ alkyl, =O, -O-cyclopropyl, cyclopropyl, $C_{2-4}$ alkynyl, $-SCF_3$, $-SF_5$, $-(NH)_{0-1}-P(O)(C_{1-2}\ alkyl)_2$, or $C_{1-2}$ alkyl substituted with $C_{1-2}$ alkoxy;
$L_1$ is selected from a bond, $C_{1-2}$ alkylene-O, $C_{1-2}$ alkylene, $C_{1-2}$ alkylene-S, $C_{1-2}$ alkylene-$N(C_{1-2}$ alkylene), $N(C_{1-2}$ alkylene)-$C_{1-2}$ alkylene, $-O-C_{1-3}$ alkylene, $C_{2-4}$ alkenylene, $-N(C_{1-2}$ alkylene)-C(=O), -C(=O)-$N(C_{1-2}$ alkylene), -O-, -C(=O)-O-, or 3- to 6-membered cycloalkyl-O, wherein the alkylene, alkenylene or cycloalkyl is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;
$L_2$ is selected from a bond, $C_{1-2}$ alkylene, -C(=O)-, 3- to 6-membered cycloalkyl, -NH-, or -O-, wherein the alkylene, alkenylene or cycloalkyl is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl.

A specific technical solution 8 of the present invention is the compound, or the stereoisomer, the deuterated substance, the solvate, the co-crystal or the pharmaceutically acceptable salt thereof described in solution 2, wherein

A is selected from 4- to 6-membered monocyclic heterocyclyl, or is selected from 7- to 9-membered bicyclic heterocyclyl, or is selected from 5- or 6-membered cycloalkyl or phenyl, wherein the heterocyclyl, cycloalkyl or phenyl is optionally substituted with 1-4 $R^A$;
each $R^A$ is independently selected from H, halogen, CN, OH, COOH, $C_{1-4}$ alkyl, $C_{2-4}$ alkenyl, $C_{2-4}$ alkynyl, $-OC_{1-4}$ alkyl, $-NH-S(O)_2-C_{1-2}$ alkyl, $-S(O)_2-C_{1-2}$ alkyl, - $S(O)_2-C_{1-2}$ alkyl-$R^a$, $-P(O)-(C_{1-2}\ alkyl)_2$, $-C(O)-OR^a$, $-(CH_2)_p-C(O)-(CH_2)_p-R^a$, - $NHC(O)-R^a$, $-C(O)N(C_{1-4}\ alkyl)_2$, or $-NHC(O)-(C_{1-4}\ alkyl)_2$, wherein the alkyl, alkenyl, or alkynyl is optionally further substituted with 1-4 groups selected from halogen, OH, $NH_2$, CN, -O-halo $C_{1-2}$ alkyl or deuterium;
$R^a$ is selected from $C_{2-4}$ alkynyl, cycloprpoyl, 4- to 6-membered heterocycloalkyl, pyrazolyl, or $C_{1-4}$ alkoxy, wherein the cyclopropyl, heterocycloalkyl, pyrazolyl or alkoxy is optionally further substituted with a group selected from halogen, $C_{1-2}$ alkyl, OH or $-S(O)_2-CH_3$;
p is selected from 0, 1 or 2;
B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, or benzo 5- or 6-membered heterocyclyl, wherein the heterocyclyl, heteroaryl, carbocyclyl or aryl is optionally substituted with 1-4 $R^B$;
each $R^B$ is independently selected from halogen, CN, $-P(=O)(C_{1-2}\ alkyl)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;
C is selected from phenyl, 5- to 7-membered heterocyclyl fused phenyl or 5- or 6-membered carbocyclyl fused phenyl, or is selected from 5- or 6-membered heterocyclyl fused 5- or 6-membered heteroaryl, wherein the heterocyclyl or heteroaryl contains 1-3 heteroatoms selected from N, O or S; the phenyl, heterocyclyl, carbocyclyl or heteroaryl is optionally substituted with 1-4 $R^C$;
each $R^C$ is independently selected from CN, OH, $C_{1-2}$ alkyl, halo $C_{1-2}$ alkyl, -O-cyclopropyl, cyclopropyl, $C_{2-4}$ alkenyl,

$C_{2-4}$ alkynyl, $-SCF_3$, $-SF_5$, $-(NH)_{0-1}-P(O)(C_{1-2}\ alkyl)_2$, or $C_{1-2}$ alkyl substituted with $C_{1-2}$ alkoxy;
$L_1$ is selected from a bond, $C_{1-2}$ alkylene-O, $C_{1-2}$ alkylene, N($C_{1-2}$ alkylene)-$C_{1-2}$ alkylene, or -O-, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;
$L_2$ is selected from a bond or $C_{1-2}$ alkylene, or is selected from $-N(CH_3)-$, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl.

A specific technical solution 9 of the present invention is the compound, or the stereoisomer, the deuterated substance, the solvate, the co-crystal or the pharmaceutically acceptable salt thereof described in any one of technical solutions 1 or 3-7, having the structure of formula (I-a), (I-b), (I-c), (I-d), (I-e) or (I-f):

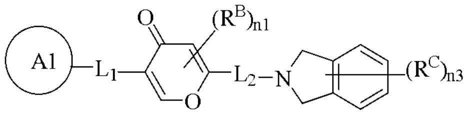
(I-a)

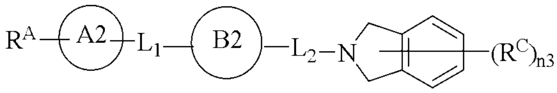
(I-b)

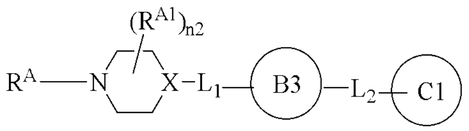
(I-c)

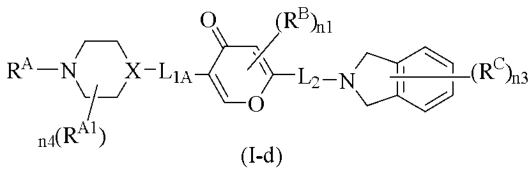
(I-d)

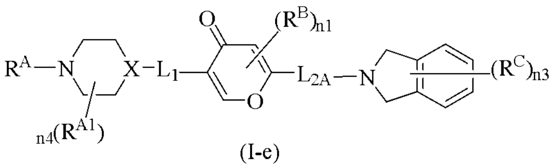
(I-e)

or

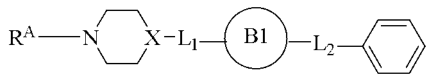
(I-f)

;

wherein A1 is selected from the following groups optionally substituted with 1-4 $R^{A1}$:

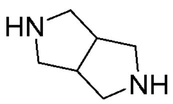
,
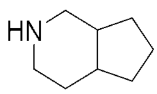
,
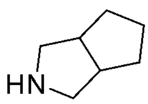
,
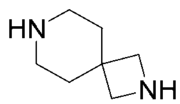
,
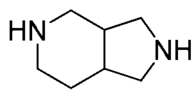
,
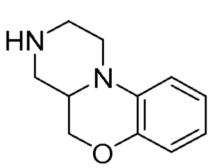
,
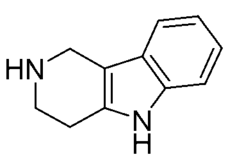
,
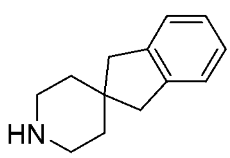
,
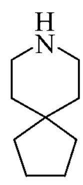
,
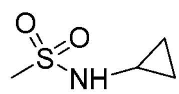
,
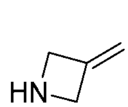
,
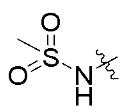
,
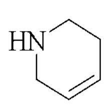
,
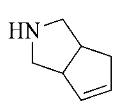
,
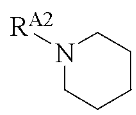
, or
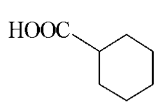
;

X is selected from CH or N;
$R^{A2}$ is selected from $-NH-S(O)_2-C_{1-4}$ alkyl, $-S(O)_2-(CH_2)_p-R^a$, $-P(O)-(C_{1-4}\ alkyl)_2$, $-C(O)-OR^a$, or $-(CH_2)_p-C(O)-(CH_2)_p-R^a$, wherein the alkyl is optionally further substituted with 1-4 groups selected from halogen, OH, $NH_2$, CN, or -O-halo $C_{1-4}$ alkyl;
A2 is selected from 4- to 6-membered monocyclic heterocycloalkyl, 5- or 6-membered heterocyclyl fused 5- or 6-membered heterocyclyl, 5- or 6-membered heterocyclyl spiro 5- or 6-membered cycloalkyl, 5- or 6-membered heterocyclyl fused 5- or 6-membered cycloalkyl, 4- to 6-membered heterocycloalkyl spiro 5- or 6-membered heterocycloalkyl, 5- or 6-membered cycloalkyl fused 5- or 6-membered heterocycloalkyl, 5- or 6-membered cycloalkyl spiro 5- or 6-membered heterocycloalkyl, 10- to 14-membered partially unsaturated tricyclic heterocycloalkyl, cyclopropyl-$NHSO_2$-$C_{1-2}$ alkyl, -$NHSO_2$-$C_{1-2}$ alkyl, or 4-membered heterocycloalkylene, wherein the cycloalkyl, heterocycloalkyl or heterocyclyl is optionally substituted with 1-4 $R^A$;

B1 is selected from the following groups optionally substituted with 1-4 $R^B$:

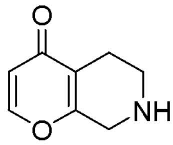, 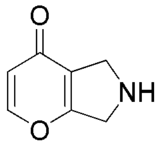, 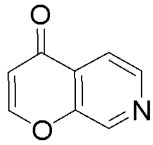, or 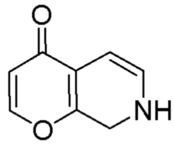;

B2 is selected from the following groups optionally substituted with 1-4 $R^B$:

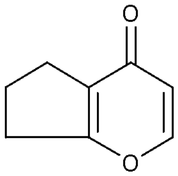, 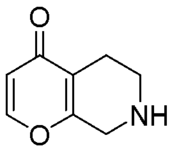, 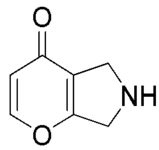, 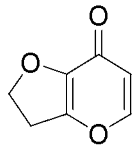, 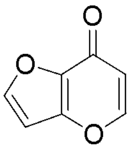, 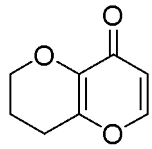,

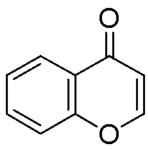, 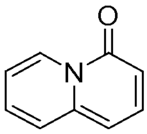, 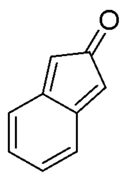, 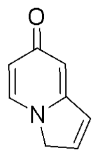, 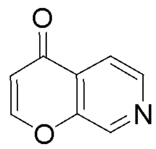, 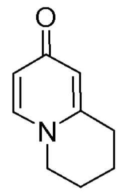, 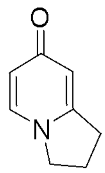, 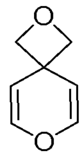,

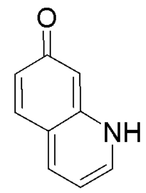, 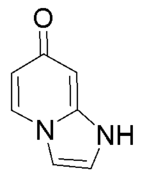, 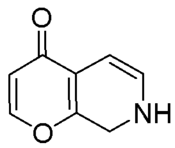, 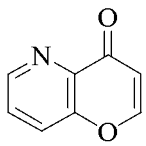, 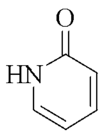, 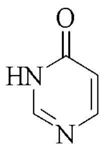, 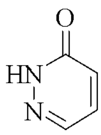, or

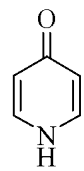;

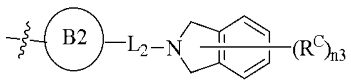 is 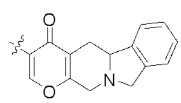;

B3 is selected from the following groups optionally substituted with 1-4 $R^B$:

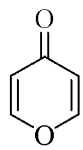, 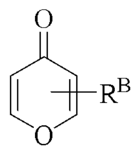

or B2;

C1 is selected from the following groups optionally substituted with 1-4 $R^C$:

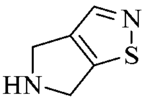, 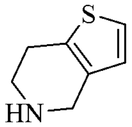, 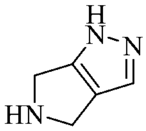, 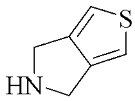, 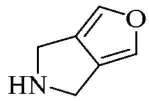, 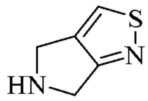,

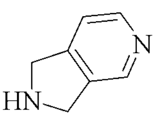, 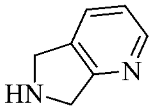, 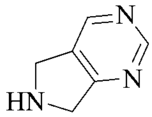, 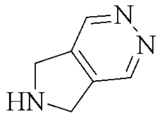, 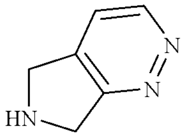,

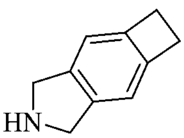, 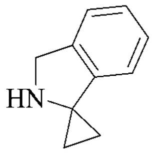, 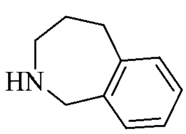, 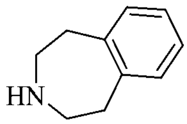, 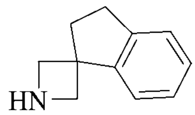,

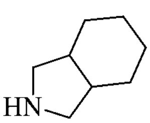, 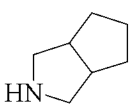, 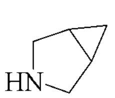, 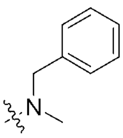, 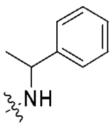, 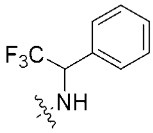, 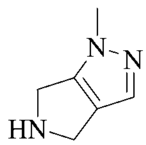,

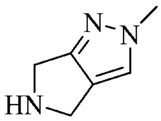, 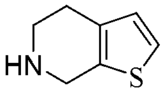, 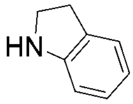, 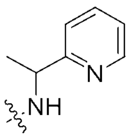, 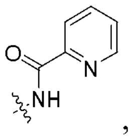, or 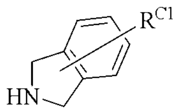;

$R^{C1}$ is selected from -O-$C_{3-5}$ cycloalkyl, $C_{3-5}$ cycloalkyl, $C_{2-4}$ alkynyl, -$SCF_3$, - $SF_5$, =O, -P(O)($C_{1-4}$ alkyl)$_2$, -NH-P(O)($C_{1-4}$ alkyl)$_2$, or -$CH_2$-O-$C_{1-4}$ alkyl;

each $R^A$ is independently selected from -S(O)$_2$-$C_{1-2}$ alkyl-$R^a$, -P(O)-($C_{1-2}$ alkyl)$_2$, -C(O)-O$R^a$, or -($CH_2$)$_p$-C(O)-($CH_2$)$_p$-$R^a$, wherein the alkyl is optionally further substituted with 1-4 groups selected from halogen, OH, $NH_2$, CN or -O-halo $C_{1-2}$ alkyl;

each $R^{A1}$ is independently selected from H, halogen, CN, OH, $NO_2$, COOH, $C_{1-2}$ alkyl, $C_{2-4}$ alkenyl, $C_{2-4}$ alkynyl, -O$C_{1-2}$ alkyl, -NH-S(O)$_2$-$C_{1-2}$ alkyl, -S(O)$_2$-$C_{1-2}$ alkyl, -S(O)$_2$-$C_{1-2}$ alkyl-$R^a$, -P(O)-($C_{1-2}$ alkyl)$_2$, -C(O)-O$R^a$, or -($CH_2$)$_p$-C(O)-($CH_2$)$_p$-$R^a$, wherein the alkyl, alkenyl, or alkynyl is optionally further substituted with 1-4 groups selected from halogen, OH, $NH_2$, CN or -O-halo $C_{1-2}$ alkyl;

each $R^B$ is independently selected from halogen, CN, -P(=O)($C_{1-2}$ alkyl)$_2$, =O, -$SF_5$, $C_{1-2}$ alkyl, -S(O)$_2$-$C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;

$R^a$ is selected from $C_{2-4}$ alkynyl, cycloprpoyl, 4- to 6-membered heterocycloalkyl, pyrazolyl, or $C_{1-4}$ alkoxy, wherein the cyclopropyl, heterocycloalkyl, pyrazolyl or alkoxy is optionally further substituted with a group selected from halogen, $C_{1-2}$ alkyl or OH;

n1, n2, n3 and n4 are each independently selected from 0, 1, 2 or 3;

$L_{1A}$ is selected from

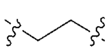, 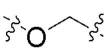, 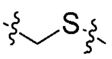, 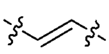, 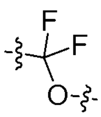, 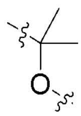,

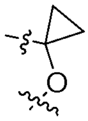, 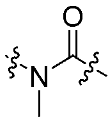, 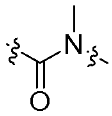, 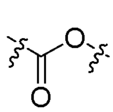, 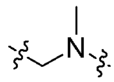, 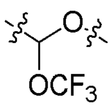, or 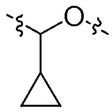;

the left end of $L_{1A}$ is connected to the piperidine ring and the right end of $L_{1A}$ is connected to the pyrone ring; $L_{2A}$ is selected from NH, O,

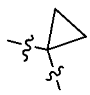, 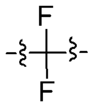, 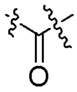, or 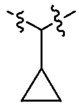;

the left end of $L_{2A}$ is connected to the pyrone ring.

A specific technical solution 10 of the present invention is the compound, or the stereoisomer, the deuterated substance, the solvate, the co-crystal or the pharmaceutically acceptable salt thereof described in any one of technical solutions 1 or 3-7, having the structure of formula (II-a), (II-b), (II-c), (II-d) or (II-e):

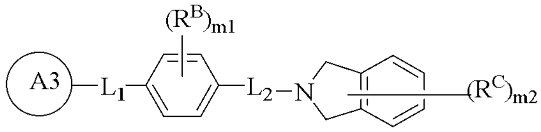
(II-a)

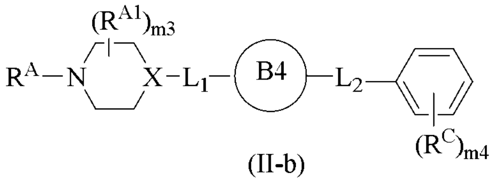
(II-b)

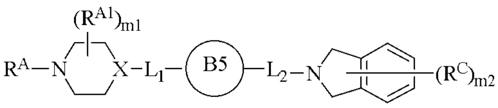
(II-c)

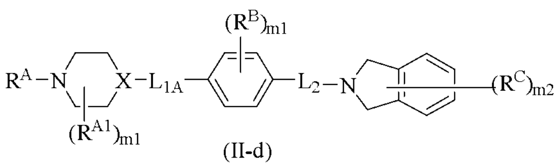
(II-d)

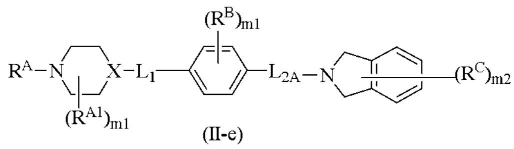
(II-e)

X is selected from CH or N;
A3 is selected from the following groups optionally substituted with 1-4 $R^A$:

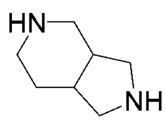, 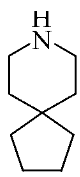, 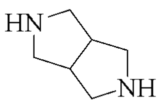, 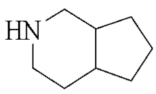, 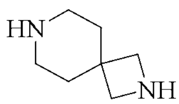, or 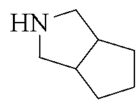;

B4 is selected from the following groups optionally substituted with 1-4 $R^B$:

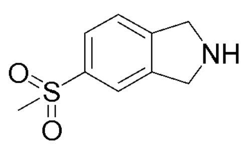, 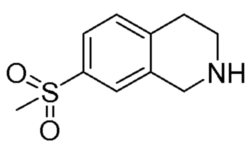;

B5 is selected from the following groups optionally substituted with 1-4 $R^B$:

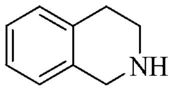, 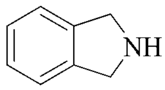, 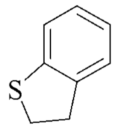, 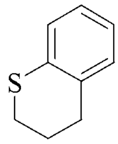, 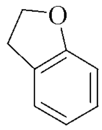, 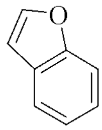, 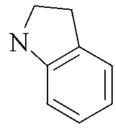,

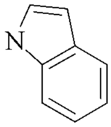, 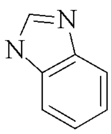, 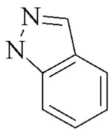, 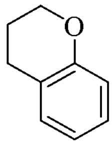, 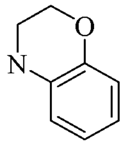, 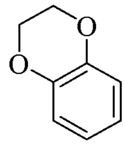, 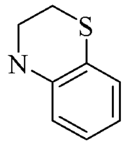, 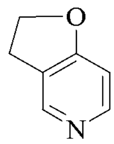,

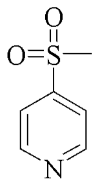, or 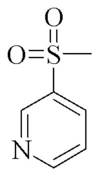;

$L_{1A}$ is selected from

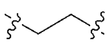, 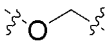, 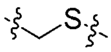, 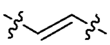, 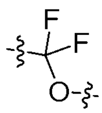, 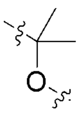,

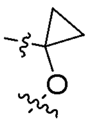, 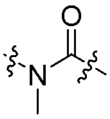, 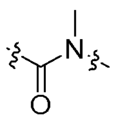, 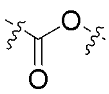, 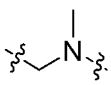, 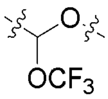, or 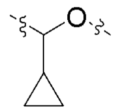;

the left end of $L_{1A}$ is connected to the piperidine ring and the right end of $L_{1A}$ is connected to the benzene ring;
$L_{2A}$ is selected from NH, O,

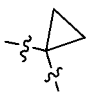, 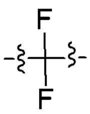, 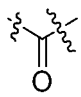, or 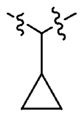;

the left end of $L_{2A}$ is connected to the benzene ring;
m1, m2, m3 and m4 are each independently selected from 0, 1, 2 or 3.

A specific technical solution 11 of the present invention is the compound, or the stereoisomer, the deuterated substance, the solvate, the co-crystal or the pharmaceutically acceptable salt thereof described in any one of technical solution 2 or 8, having the structure of formula (I-1), (I-2), (I-3) or (I-4):

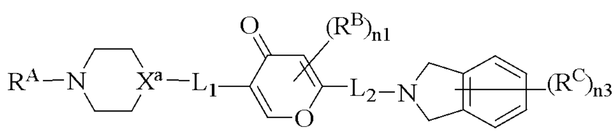

(I-1)

,

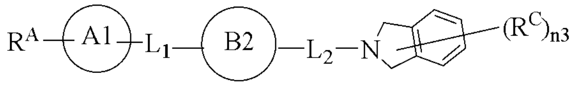

(I-2)

,

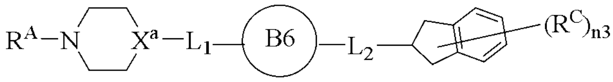

(I-3)

,

or

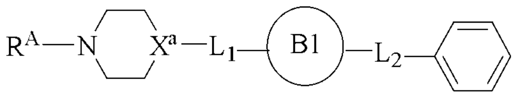

(I-4)

;

$X^a$ is selected from CH or N;

A1 is selected from the following groups:

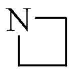 , 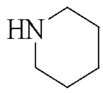 , 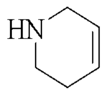 , 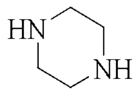 ,

or 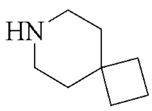 , 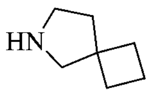 , 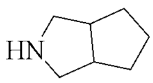 , 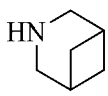 , or 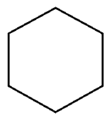 , or 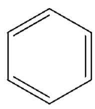 ,

or

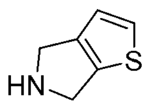 , or 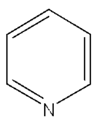 , 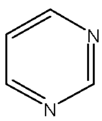 , or 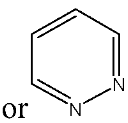 ;

B2 is selected from the following groups optionally substituted with 1-4 $R^B$:

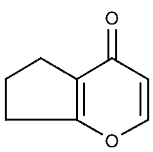 , 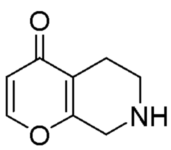 , 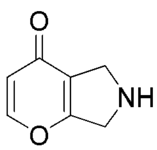 , 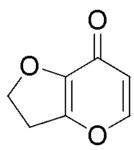 , 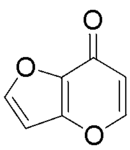 , 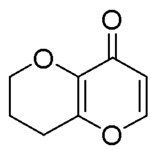 ,

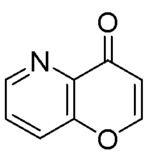 , 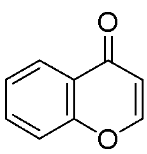 , 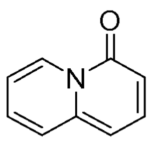 , 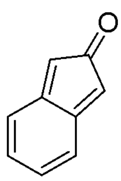 , 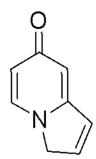 , 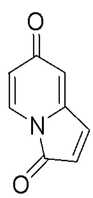 , 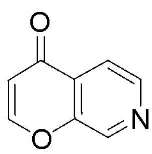 ,

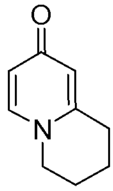, 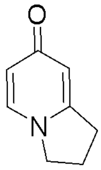, 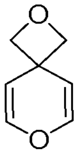, 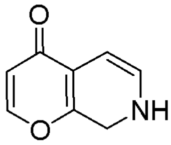, 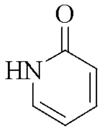, 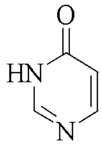, 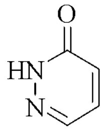, 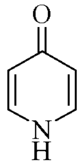

or

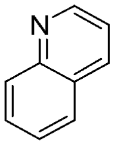,

or is selected from the following groups optionally substituted with 1-4 $R^B$.

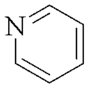

or is selected from the following groups optionally substituted with 1-4

$R^B$: 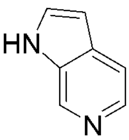, 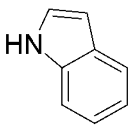, 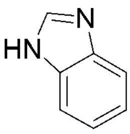, 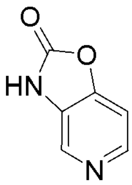, 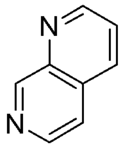, 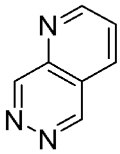, 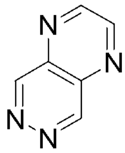,

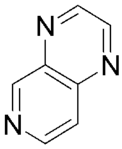, 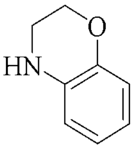, 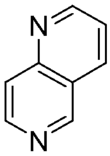, 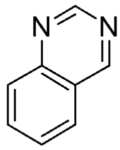, 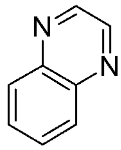, 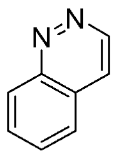, 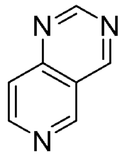,

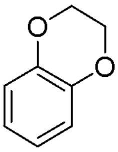, 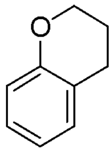, 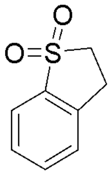, 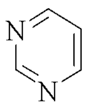 or 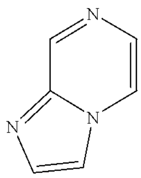,

or is selected from the following groups optionally substituted with 1-4 $R^B$:

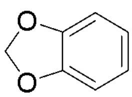, 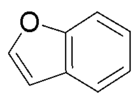,

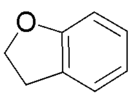 or 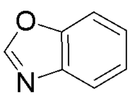,

or is selected from the following groups optionally substituted with 1-4 $R^B$:

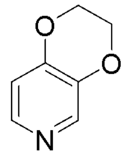

or is selected from the following groups optionally substituted with 1-4 $R^B$:

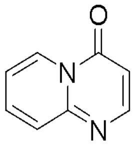 ;

B6 is selected from the following groups optionally substituted with 1-4 $R^B$:

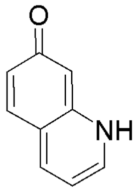 or 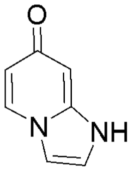 ;

B1 is selected from the following groups optionally substituted with 1-4 $R^B$:

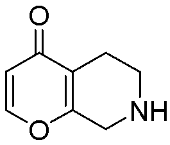 , 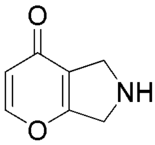 , 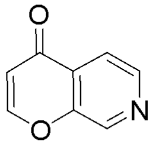 or 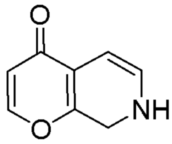 ,

or is selected from the following groups optionally substituted with 1-4 $R^B$:

: 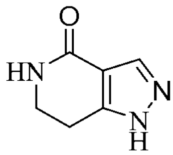 , 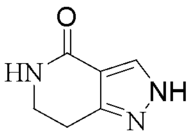 ,

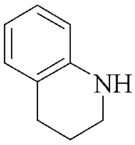 , 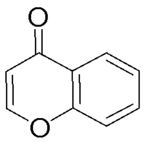 or 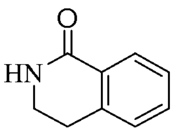 ,

or is selected from the following groups optionally substituted with 1-4 $R^B$:

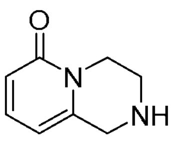 , or 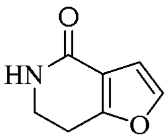 ;

alternatively,

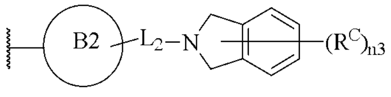 is 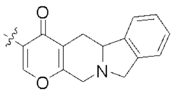 ;

$R^A$ is selected from $-S(O)_2-(CH_2)_p-R^a$, $-P(O)-(C_{1-2}$ alkyl$)_2$, $-C(O)-OR^a$, $-(CH_2)_p-C(O)-(CH_2)_p-R^a$, $-S(O)_2-C_{1-2}$ alkyl, $-NHC(O)-R^a$, $-C(O)N(C_{1-4}$ alkyl$)_2$, $-NHC(O)-C_{1-2}$ alkyl, 3- to 6-membered heterocycloalkyl, or $-NHR^a$, wherein the alkyl or heterocycloalkyl is optionally further substituted with 1-4 groups selected from halogen, OH, $NH_2$, CN, -O-halo $C_{1-2}$ alkyl, deuterium or $C_{1-2}$ alkyl, and the heterocycloalkyl contains 1-3 heteroatoms selected from N, O or S; or

$R^A$ is selected from $-S(O)_2-C_{1-2}$ alkyl-$R^a$, $-P(O)-(C_{1-2}$ alkyl$)_2$, $-C(O)-OR^a$, - $(CH_2)_p-C(O)-(CH_2)_p-R^a$, $-S(O)_2-C_{1-2}$ alkyl, $-NHC(O)-R^a$, or $-C(O)N(C_{1-4}$ alkyl$)_2$, wherein the alkyl is optionally further substituted with 1-4 groups selected from halogen, OH, $NH_2$, CN, -O-halo $C_{1-2}$ alkyl or deuterium;

each $R^B$ is independently selected from halogen, CN, $-P(=O)(C_{1-2}$ alkyl$)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;

each $R^C$ is independently selected from CN, OH, $C_{1-2}$ alkyl, halo $C_{1-2}$ alkyl, $C_{2-4}$ alkenyl or $C_{2-4}$ alkynyl, or is selected from -O-cyclopropyl or cyclopropyl, or is selected from $-SF_5$;

$R^a$ is selected from $C_{2-4}$ alkynyl, cyclopropyl, cyclobutyl, 4- to 6-membered heterocycloalkyl, pyrazolyl, $C_{1-4}$ alkoxy, -O-cyclopropyl, $-CD_3$, $-CH_2D$, $-CHD_2$, methyl, or ethyl, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH, $-S(O)_2-CH_3$, $=CH_2$, =CHF or $=CF_2$, and the methyl or ethyl is further substituted with 1-3 groups selected from OH or CN; or

$R^a$ is selected from $C_{2-4}$ alkynyl, cyclopropyl, 4- to 6-membered heterocycloalkyl, pyrazolyl, $C_{1-4}$ alkoxy, -O-cyclopropyl, $-CD_3$, $-CH_2D$, or $-CHD_2$, wherein the cyclopropyl, heterocycloalkyl, pyrazolyl or alkoxy is optionally further substituted with a group selected from halogen, $C_{1-2}$ alkyl, OH or $-S(O)_2-CH_3$; or

$R^a$ is selected from $C_{2-4}$ alkynyl, cyclopropyl, cyclobutyl, 4- to 6-membered heterocycloalkyl, pyrazolyl, $C_{1-4}$ alkoxy, -O-cyclopropyl, $-CD_3$, $-CH_2D$, or $-CHD_2$, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH or $-S(O)_2-CH_3$;

or $R^a$ is selected from $C_{2-4}$ alkynyl, cycloprpoyl, 4- to 6-membered heterocycloalkyl, pyrazolyl, or $C_{1-4}$ alkoxy, wherein the cyclopropyl, heterocycloalkyl, pyrazolyl or alkoxy is optionally further substituted with a group selected from halogen, $C_{1-2}$ alkyl, OH or $-S(O)_2-CH_3$;

n1 is selected from 1, 2 or 3;

n3 is selected from 0, 1, 2 or 3.

A specific technical solution 12 of the present invention is the compound, or the stereoisomer, the deuterated substance, the solvate, the co-crystal or the pharmaceutically acceptable salt thereof described in solution 11, wherein

$R^A$ is selected from $-S(O)_2-(CH_2)_p-R^a$, $-S(O)_2-C_{1-2}$ alkyl, $-C(O)-OR^a$, $-C(O)-CH_2-R^a$, $-NHC(O)-R^a$, $-C(O)N(C_{1-4}$ alkyl$)_2$, $-NHC(O)-(C_{1-4}$ alkyl$)_2$, $-NHC(O)-C_{1-2}$ alkyl, 3- to 6-membered heterocycloalkyl, or $-NHR^a$, wherein the alkyl or heterocycloalkyl is optionally further substituted with 1-4 groups selected from F, Cl, Br, OH, $NH_2$, CN, D, $-CH_3$ or $-CH_2CH_3$, and the heterocycloalkyl contains 1-3 heteroatoms selected from N, O or S; or

$R^A$ is selected from $-S(O)_2-C_{1-2}$ alkyl-$R^a$ or $-S(O)_2-C_{1-2}$ alkyl, or is selected from $-C(O)-OR^a$, $-C(O)-CH_2-R^a$, $-NHC(O)-R^a$, $-C(O)N(C_{1-4}$ alkyl$)_2$ or $-NHC(O)-(C_{1-4}$ alkyl$)_2$, wherein the alkyl is optionally further substituted with 1-4 groups selected from F, Cl, Br, OH, $NH_2$, CN or D;

each $R^B$ is independently selected from F, Cl, Br, =O, CN, methyl, ethyl, methoxy, ethoxy, vinyl, propenyl, butenyl, ethynyl, propynyl, cyclopropyl, $-CF_3$, - $CHF_2$, $-CH_2F$, 5-membered heteroaryl, or 6-membered heteroaryl, wherein the methyl, ethyl, methoxy, ethoxy, cyclopropyl or heteroaryl is optionally further substituted with a group selected from methyl or ethyl; or

each $R^B$ is independently selected from F, Cl, Br, =O, methyl, ethyl, methoxy, ethoxy, vinyl, propenyl, butenyl, ethynyl, propynyl, cyclopropyl, $-CF_3$, $-CHF_2$, - $CH_2F$, 5-membered heteroaryl, or 6-membered heteroaryl, wherein the methyl, ethyl, methoxy, ethoxy, cyclopropyl or heteroaryl is optionally further substituted with a group selected from methyl or ethyl;

each $R^C$ is independently selected from methyl, ethyl, $-CF_3$, $-CHF_2$, $-CH_2CH_2F$, $-CH_2CF_3$, $-CH_2CHF_2$, $-CH_2CH_2F$, vinyl, propenyl, ethynyl or propynyl, or is selected from -O-cyclopropyl or cyclopropyl, or is selected from $-SF_5$;

$R^a$ is selected from ethynyl, propynyl, cyclopropyl, cyclobutyl, 4- to 6-membered heterocycloalkyl, pyrazolyl, methoxy, ethoxy, -O-cyclopropyl, $-CD_3$, methyl, or ethyl, wherein the cyclopropyl, cyclobutyl, heterocycloalkyl, pyrazolyl, methoxy or ethoxy is optionally further substituted with a group selected from F, Cl, Br, methyl, ethyl, $-CD_3$, $-CH_2D$, $-CHD_2$, OH, $-S(O)_2-CH_3$, $=CH_2$, =CHF or $=CF_2$, and the methyl or ethyl is further substituted with 1-3 groups selected from OH or CN; or

$R^a$ is selected from ethynyl, propynyl, cyclopropyl, cyclobutyl, 4- to 6-membered heterocycloalkyl, pyrazolyl, methoxy, ethoxy, -O-cyclopropyl, or -$CD_3$, wherein the cyclopropyl, cyclobutyl, heterocycloalkyl, pyrazolyl, methoxy or ethoxy is optionally further substituted with a group selected from F, Cl, Br, methyl, ethyl, -$CD_3$, -$CH_2D$, -$CHD_2$, OH or -$S(O)_2$-$CH_3$;

or $R^a$ is selected from ethynyl, propynyl, cyclopropyl, 4- to 6-membered heterocycloalkyl, pyrazolyl, methoxy, ethoxy, -O-cyclopropyl, or -$CD_3$, wherein the cyclopropyl, heterocycloalkyl, pyrazolyl, methoxy or ethoxy is optionally further substituted with a group selected from F, Cl, Br, methyl, ethyl, OH or -$S(O)_2$-$CH_3$; or

$R^a$ is selected from ethynyl, propynyl, cyclopropyl, 4- to 6-membered heterocycloalkyl, pyrazolyl, methoxy, or ethoxy, wherein the cyclopropyl, heterocycloalkyl, pyrazolyl, methoxy or ethoxy is optionally further substituted with a group selected from F, Cl, Br, methyl, ethyl, OH or -$S(O)_2$-$CH_3$;

n1 is selected from 1 or 2;

n3 is selected from 0, 1 or 2;

$L_1$ is selected from a bond, -$CH_2$-O-, -$CH_2CH_2$-O-, -$CH_2$-, -O-, -$CH_2CH_2$-, - N($CH_3$)-$CH_2$-, ethylene, or propylene, wherein the -$CH_2$- is optionally further substituted with 1-3 $R^{L1}$; each $R^{L1}$ is independently selected from F, Cl, Br, =O, methyl, ethyl, -$CF_3$, -$CHF_2$, -$CH_2F$, vinyl, propenyl, methoxy, ethoxy, -$OCF_3$, - $OCHF_2$, -$OCH_2F$, cyclopropyl, or cyclobutyl;

or $L_1$ is selected from a bond, -$CH_2$-O-, -$CH_2CH_2$-O-, -$CH_2$-, -O-, -$CH_2CH_2$-, or -N($CH_3$)-$CH_2$-, wherein the -$CH_2$- is optionally further substituted with 1-3 $R^{L1}$. each $R^{L1}$ is independently selected from F, Cl, Br, =O, methyl, ethyl, -$CF_3$, -$CHF_2$, -$CH_2F$, vinyl, propenyl, methoxy, ethoxy, -$OCF_3$, -$OCHF_2$, -$OCH_2F$, cyclopropyl, or cyclobutyl;

$L_2$ is selected from a bond, -$CH_2$- or -CH($CH_3$)-, or is selected from-N($CH_3$)-, wherein the -$CH_2$- or -CH($CH_3$)- is optionally further substituted with 1-3 $R^{L1}$; each $R^{L1}$ is independently selected from F, Cl, Br, =O, methyl, ethyl, -$CF_3$, -$CHF_2$, -$CH_2F$, vinyl, propenyl, methoxy, ethoxy, -$OCF_3$, -$OCHF_2$, -$OCH_2F$, cyclopropyl, or cyclobutyl.

A specific technical solution 13 of the present invention is the compound, or the stereoisomer, the deuterated substance, the solvate, the co-crystal or the pharmaceutically acceptable salt thereof described in solution 11 or solution 12, wherein

A1 is selected from the following groups:

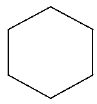;

B2 is selected from the following groups optionally substituted with 1-4 $R^B$:

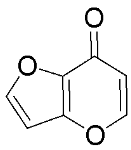, or 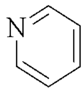;

$R^A$ is -NHC(O)-$R^a$;

each $R^B$ is independently selected from F, Cl, Br, =O, CN, methyl, ethyl, methoxy, or ethoxy, wherein the methyl, ethyl, methoxy or ethoxy is optionally further substituted with a group selected from methyl or ethyl;

each $R^C$ is independently selected from -$CF_3$, -$CHF_2$, -$CH_2CH_2F$, or cyclopropyl;

$R^a$ is selected from cyclopropyl, cyclobutyl, pyrazolyl, or -$CD_3$, wherein the cyclopropyl, cyclobutyl or pyrazolyl is optionally further substituted with a group selected from F, Cl, Br, methyl, ethyl, -$CD_3$, -$CH_2D$ or -$CHD_2$;

n3 is selected from 1 or 2;

$L_1$ is selected from a bond, -$CH_2$-O-, -$CH_2CH_2$-O-, ethylene, or propylene, wherein the -$CH_2$- is optionally further substituted with 1-3 $R^{L1}$; each $R^{L1}$ is independently selected from F, Cl, Br, methyl, ethyl, cyclopropyl, or cyclobutyl;

$L_2$ is selected from -$CH_2$-, wherein the -$CH_2$- is optionally further substituted with 1-3 $R^{L1}$; each $R^{L1}$ is independently selected from F, Cl, Br, methyl, ethyl, cyclopropyl, or cyclobutyl.

A specific technical solution 14 of the present invention is the compound, or the stereoisomer, the deuterated substance, the solvate, the co-crystal or the pharmaceutically acceptable salt thereof described in the present invention, having the structure of formula (I-5):

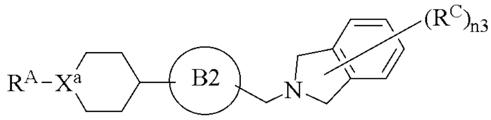

(I-5)

wherein:

$X^a$ is selected from CH or N;

each $R^A$ is independently selected from halogen, CN, OH, COOH, $C_{1-4}$ alkyl, - $OC_{1-4}$ alkyl, -NH-$S(O)_2$-$C_{1-4}$ alkyl, -$S(O)_2$-$C_{1-4}$ alkyl, -C(O)N($C_{1-4}$ alkyl)$_2$, -NHC(O)-$C_{1-4}$ alkyl, -C(O)-$OR^a$, -$(CH_2)_p$-C(O)-$(CH_2)_p$-$R^a$, -NHC(O)-$R^a$, -$NHR^a$, or 3- to 6-membered heterocycloalkyl, wherein the alkyl or heterocycloalkyl is optionally further substituted with 1-4 groups selected from halogen, OH, $NH_2$, CN, -O-halo $C_{1-4}$ alkyl, deuterium or $C_{1-4}$ alkyl, and the heterocycloalkyl contains 1-3 heteroatoms selected from N, O or S; in some embodiments, each $R^A$ is independently selected from halogen, CN, OH, COOH, $C_{1-2}$ alkyl, -$OC_{1-2}$ alkyl, - NH-$S(O)_2$-$C_{1-2}$ alkyl, -$S(O)_2$-$C_{1-2}$ alkyl, -C(O)N($C_{1-2}$ alkyl)$_2$, -NHC(O)-$C_{1-2}$ alkyl, - C(O)-$OR^a$, -$(CH_2)_p$-C(O)-$(CH_2)_p$-$R^a$, -NHC(O)-$R^a$, -$NHR^a$, or 3- or 4-membered heterocycloalkyl, wherein the alkyl or heterocycloalkyl is optionally further substituted with 1-4 groups selected from halogen, OH, $NH_2$, CN, -O-halo $C_{1-2}$ alkyl, deuterium or $C_{1-2}$ alkyl;

$R^a$ is selected from $C_{1-4}$ alkyl, deuterated $C_{1-4}$ alkyl, $C_{1-4}$ alkoxy, 3- to 5-membered cycloalkyl, 3- to 6-membered heterocycloalkyl, 5- or 6-membered heteroaryl, or -O-$C_{3-6}$ cycloalkyl, wherein the cycloalkyl, heterocycloalkyl or heteroaryl is optionally further substituted with 1-4 groups selected from OH, - $S(O)_2$-$CH_3$, halogen, $C_{1-4}$ alkyl, halo $C_{1-4}$ alkyl, deuterated $C_{1-4}$ alkyl or -O-halo $C_{1-4}$ alkyl, the alkyl is further substituted with 1-3 groups selected from OH or CN, and the heterocycloalkyl or heteroaryl contains 1-3 heteroatoms selected from N, O or S; in some embodiments, $R^a$ is selected from $C_{1-2}$ alkyl, deuterated $C_{1-2}$ alkyl, $C_{1-2}$ alkoxy, 3- or 4-membered cycloalkyl, 3- or 4-membered heterocycloalkyl, 5-membered heteroaryl, or -O-$C_{3-4}$ cycloalkyl, wherein the cycloalkyl, heterocycloalkyl or heteroaryl is optionally further substituted with 1-4 groups selected from OH, -$S(O)_2$-$CH_3$, halogen, $C_{1-2}$ alkyl, halo $C_{1-2}$ alkyl, deuterated $C_{1-2}$ alkyl or -O-halo $C_{1-2}$ alkyl, and the alkyl is further substituted with 1-3 groups selected from OH or CN;

each $R^C$ is independently selected from H, halogen, CN, OH, $C_{1-4}$ alkyl, halo $C_{1-4}$ alkyl, -O-$C_{3-5}$ cycloalkyl, or $C_{3-5}$ cycloalkyl; in some embodiments, each $R^C$ is independently H or halo $C_{1-2}$ alkyl; in some embodiments, each $R^C$ is independently H or $CF_3$;

B2 is selected from the following groups optionally substituted with 1-4 $R^B$:

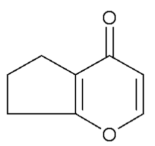, 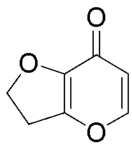, 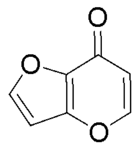, 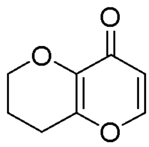, 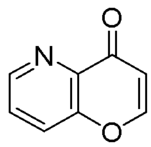, 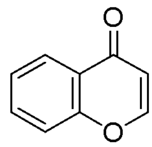,

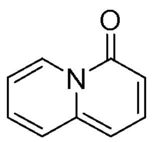, 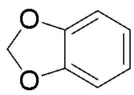, 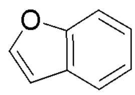, 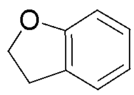, 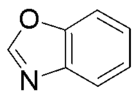, 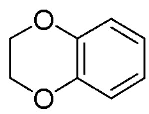,

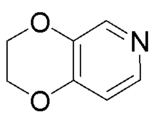, 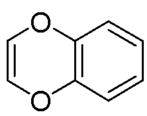, or 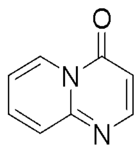;

each $R^B$ is independently selected from halogen, CN, =O, $C_{1-2}$ alkyl, $NH_2$, $C_{1-2}$ alkoxy, $C_{2-4}$ alkenyl, $C_{2-4}$ alkynyl, or halo $C_{1-2}$ alkyl;

n3 is selected from 0, 1 or 2;

p is selected from 0, 1 or 2.

A specific technical solution 15 of the present invention is the compound, or the stereoisomer, the deuterated substance, the solvate, the co-crystal or the pharmaceutically acceptable salt thereof described in solution 14, wherein:

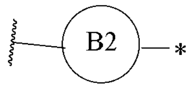

is selected from the following groups optionally substituted with 1-4 $R^B$:

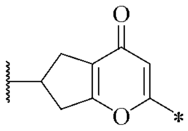 , 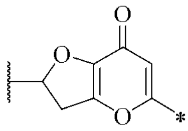 , 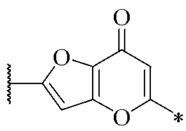 , 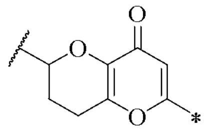 ,

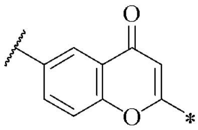 , 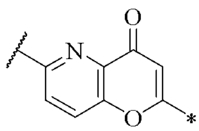 , 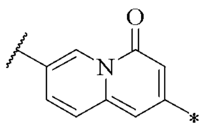 , 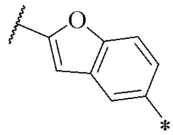 ,

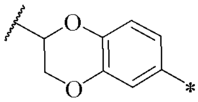 , 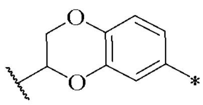 , 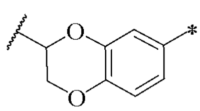 , 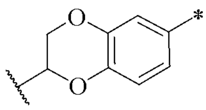 ,

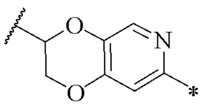 , 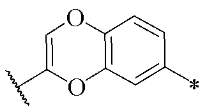 , 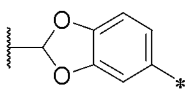 , 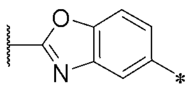 , or

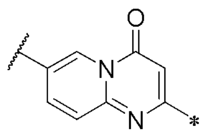 ,

wherein ~~~ represents the connection with the left ring.

The compound, or the stereoisomer, the deuterated substance, the solvate, the co-crystal or the pharmaceutically acceptable salt thereof of the present invention, wherein the compound is selected from, but not limited to, the structures in Table I below:

Table I:

| | | | |
|---|---|---|---|
| 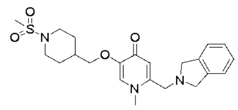 | 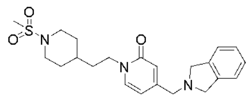 | 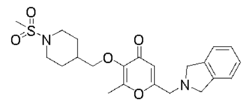 | 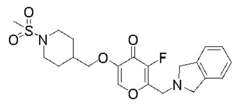 |
| 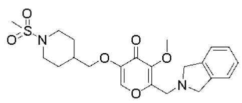 | 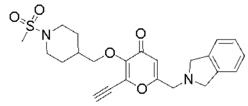 | 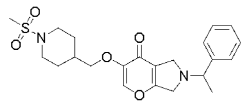 | 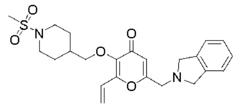 |
| 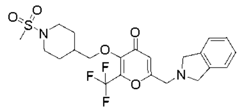 | 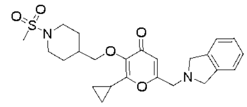 | 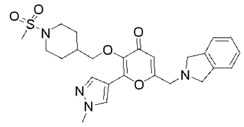 | 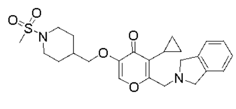 |
| 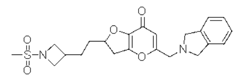 | 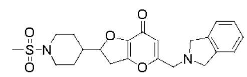 | 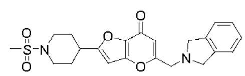 | 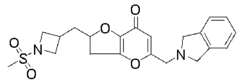 |

| | | | |
|---|---|---|---|
| 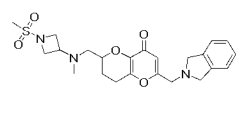 | 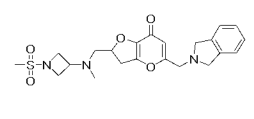 | 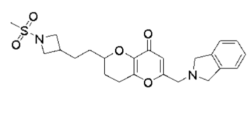 | 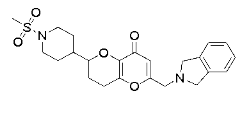 |
| 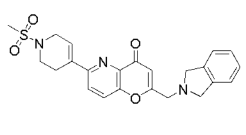 | 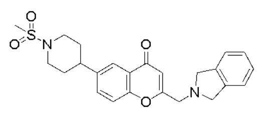 | 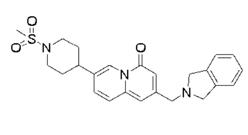 | 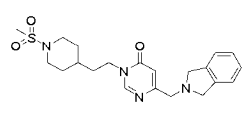 |
| 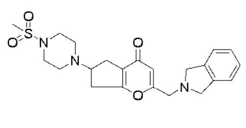 | 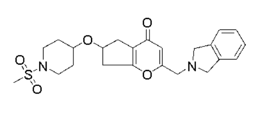 | 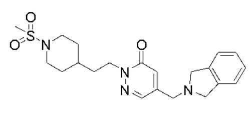 | 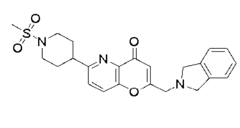 |
| 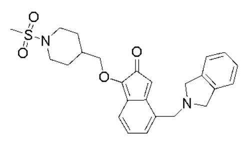 | 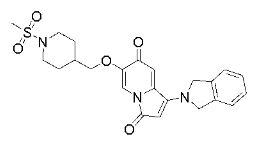 | 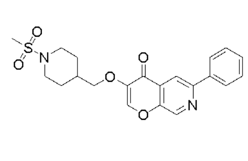 | 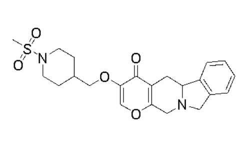 |
| 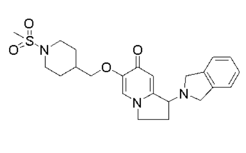 | 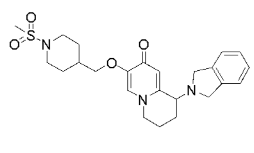 | 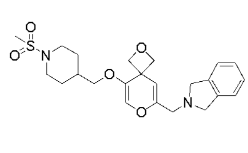 | 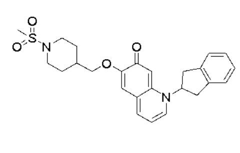 |
| 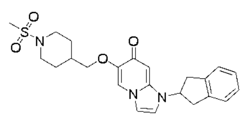 | 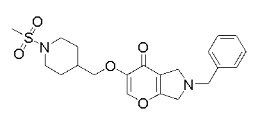 | 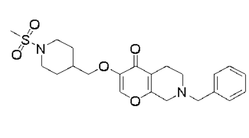 | 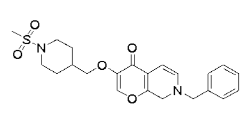 |
| 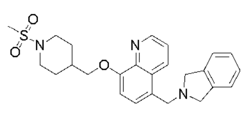 | 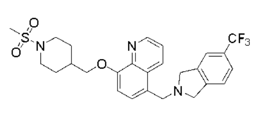 | 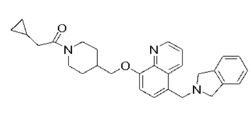 | 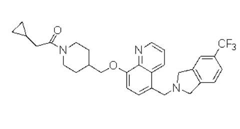 |
| 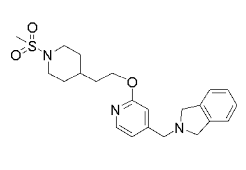 | 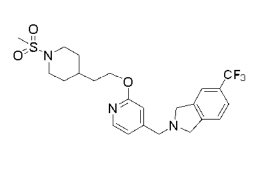 | 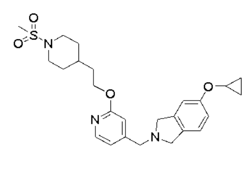 | 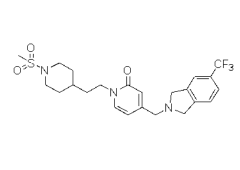 |
| 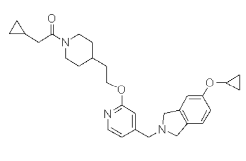 | 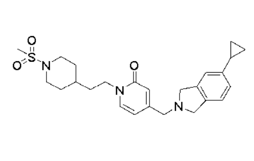 | 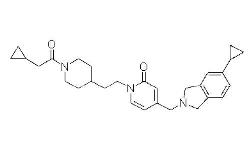 | 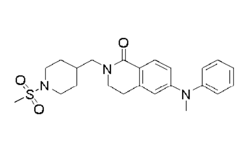 |
| 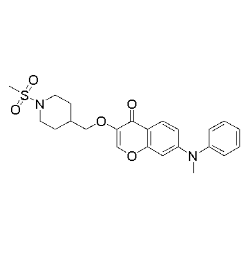 | 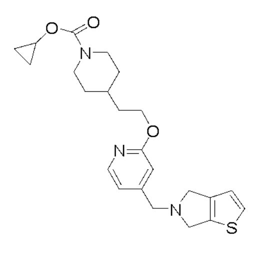 | 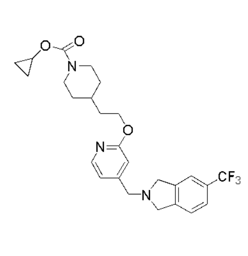 | 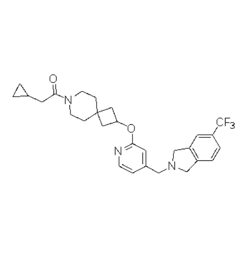 |

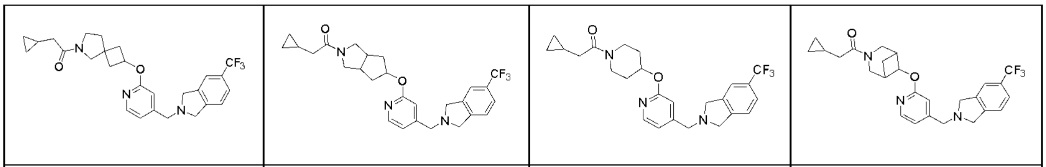

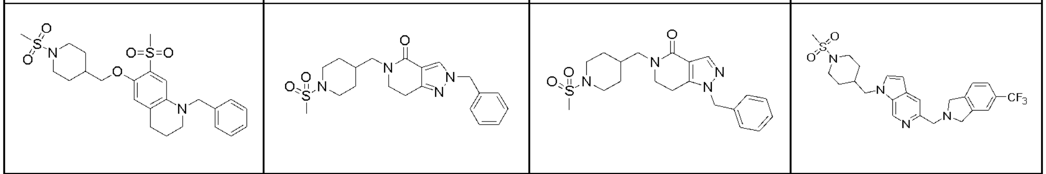

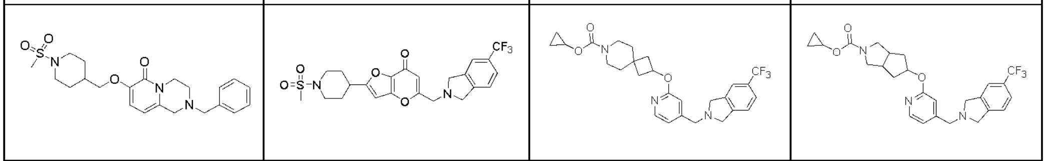

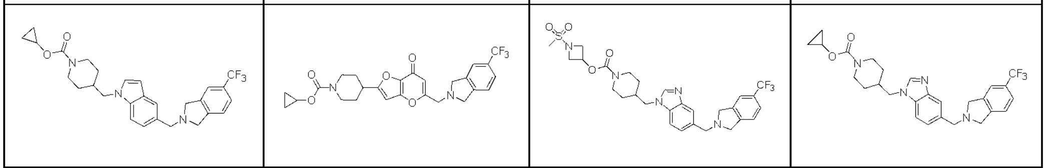

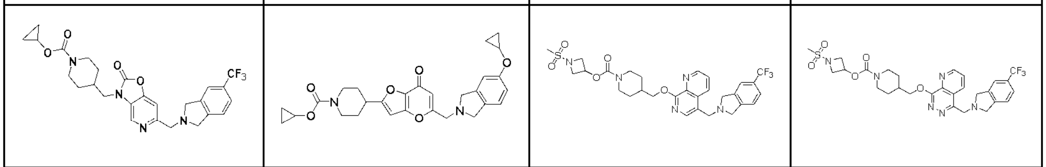

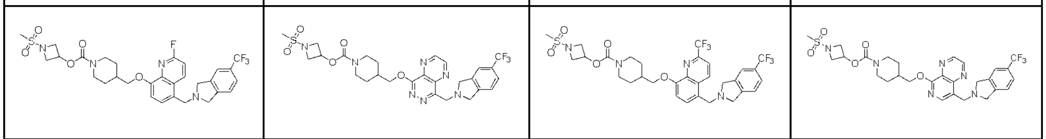

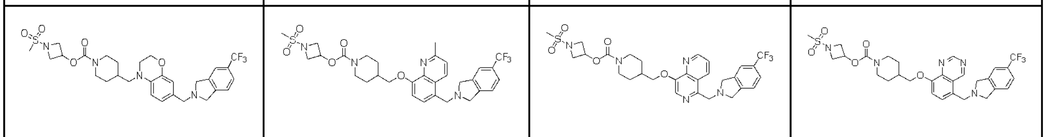

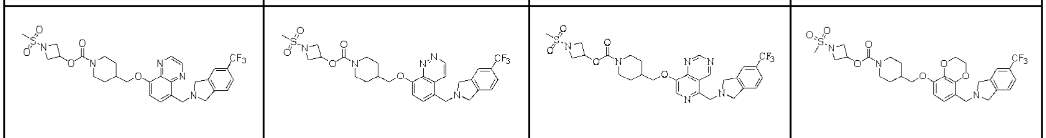

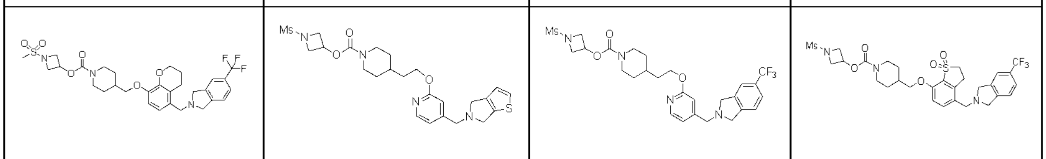

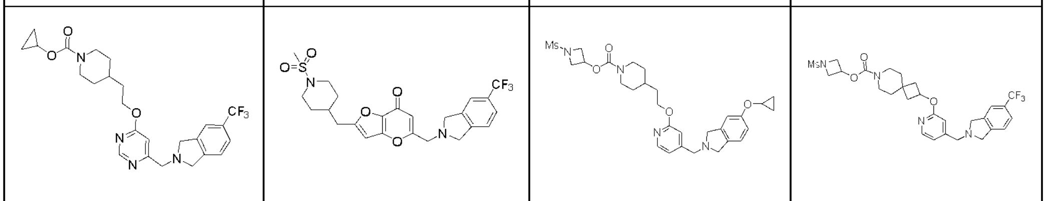

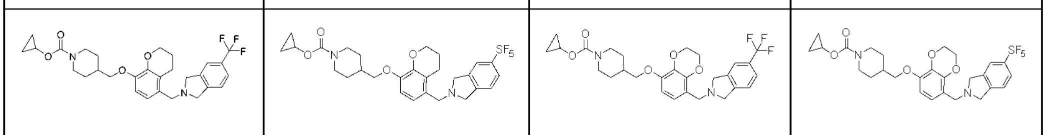

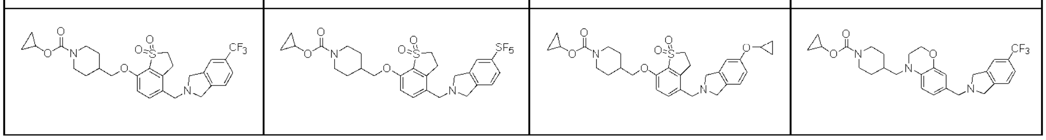

| | | | |
|---|---|---|---|
| 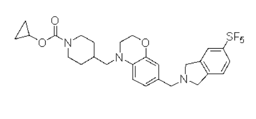 | 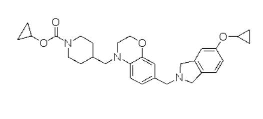 | 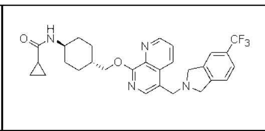 | 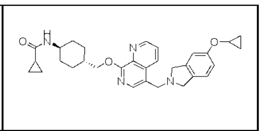 |
| 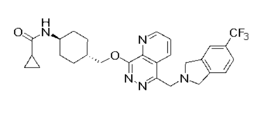 | 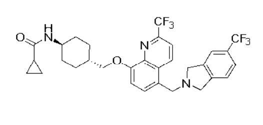 | 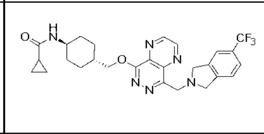 | 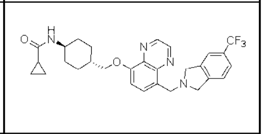 |
| 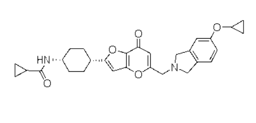 | 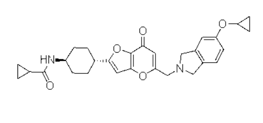 | 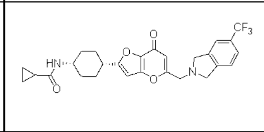 | 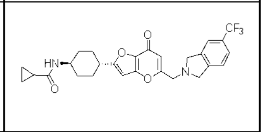 |
| 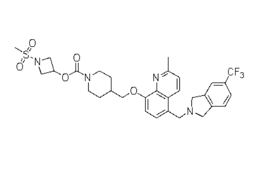 | 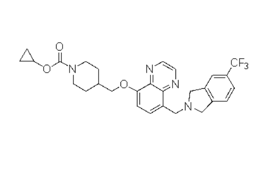 | 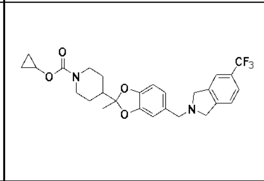 | 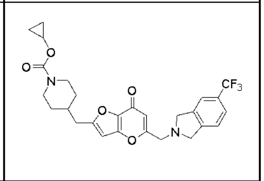 |
| 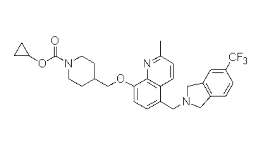 | 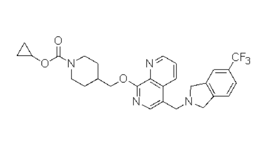 | 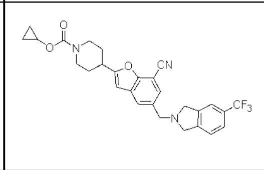 | 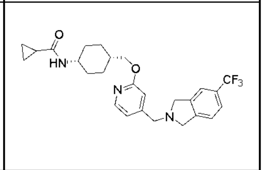 |
| 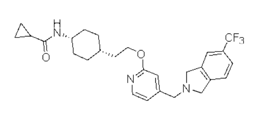 | 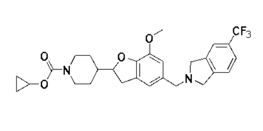 | 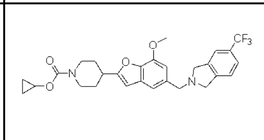 | 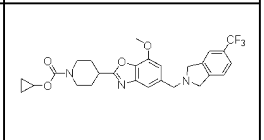 |
| 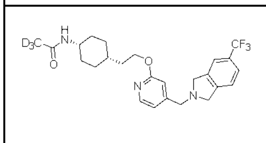 | 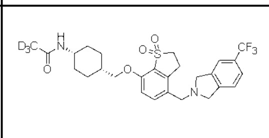 | 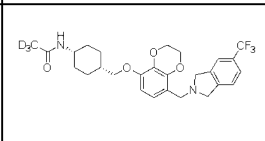 | 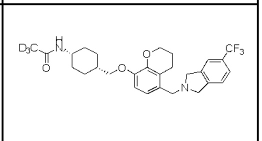 |
| 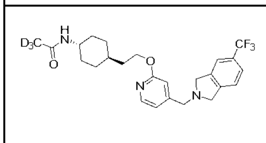 | 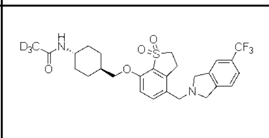 | 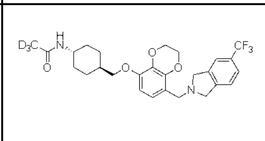 | 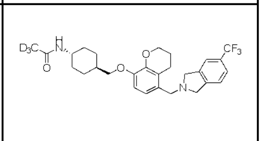 |
| 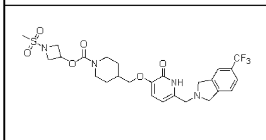 | 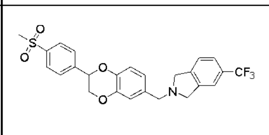 | 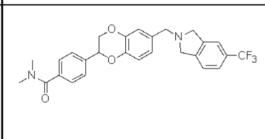 | 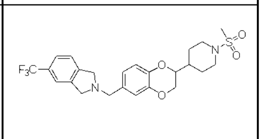 |
| 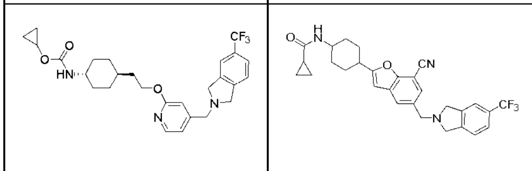 | | 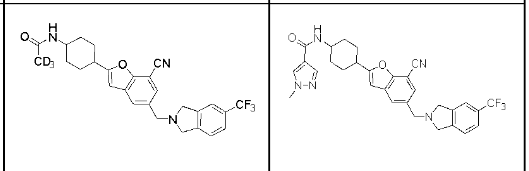 | |
| 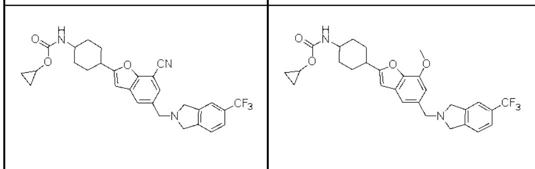 | | 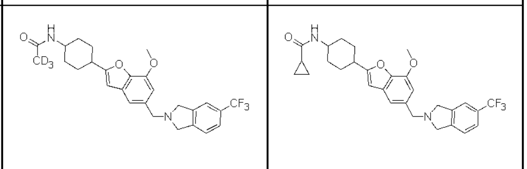 | |

| | | | |
|---|---|---|---|
| 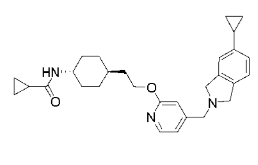 | 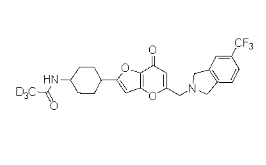 | 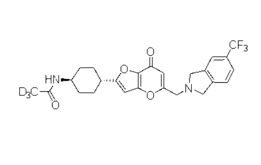 | 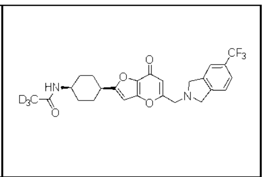 |
| 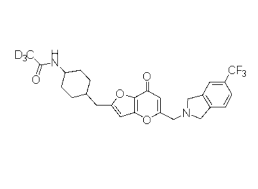 | 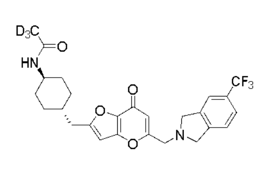 | 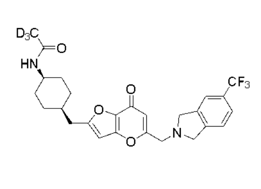 | 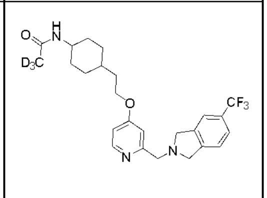 |
| 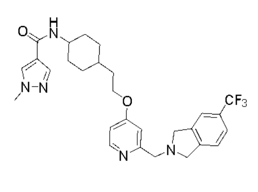 | 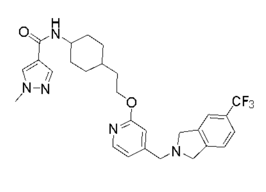 | 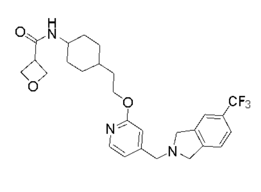 | 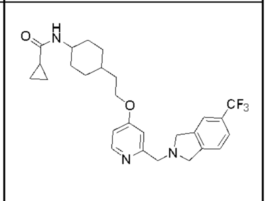 |
| 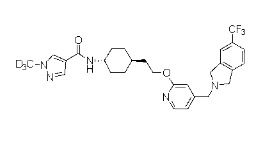 | 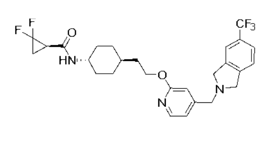 | 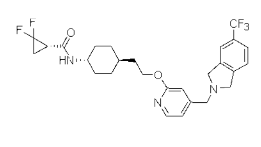 | 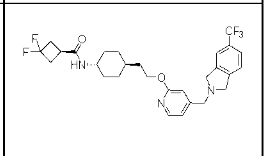 |
| 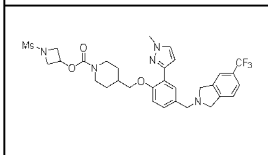 | 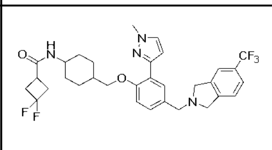 | 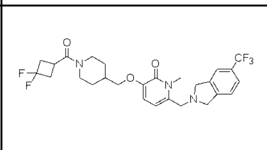 | 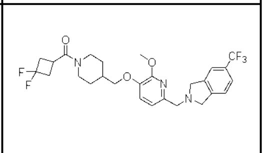 |
| 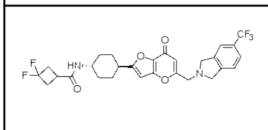 | 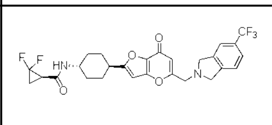 | 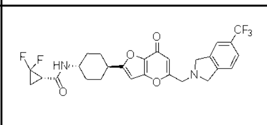 | 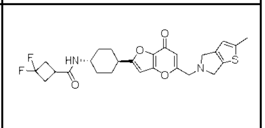 |
| 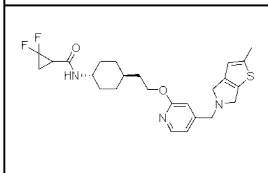 | 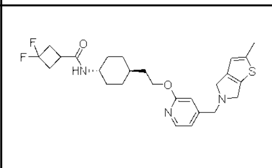 | 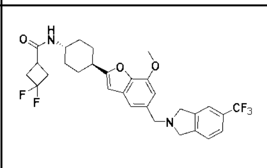 | 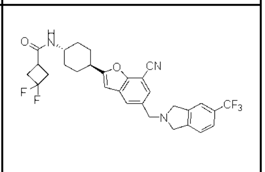 |
| 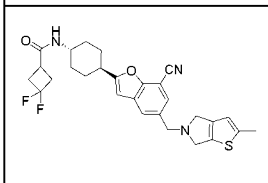 | 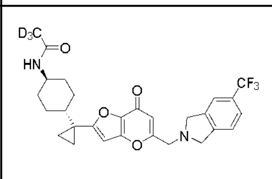 | 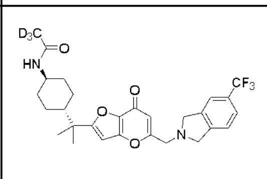 | 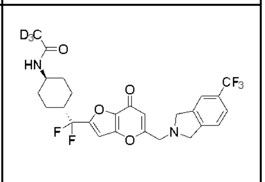 |
| 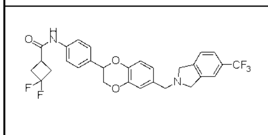 | 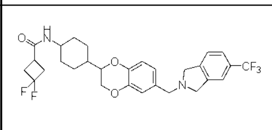 | 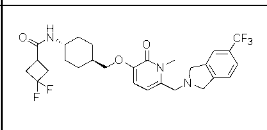 | 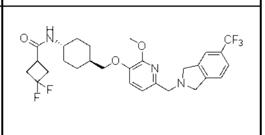 |
| 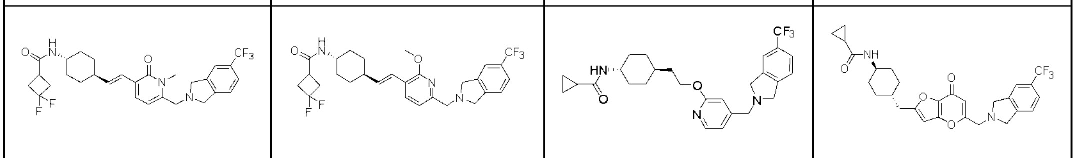 | | | |
| 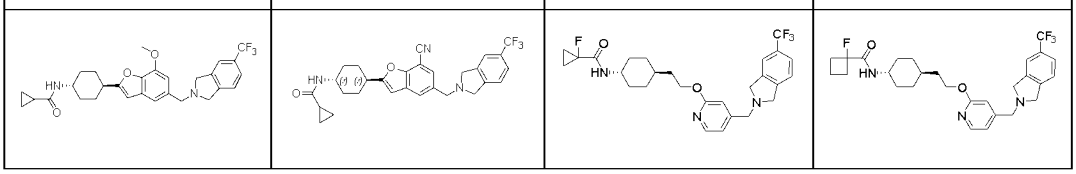 | | | |

| | | | |
|---|---|---|---|
| 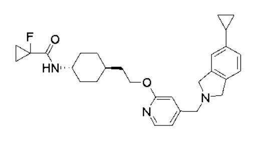 | 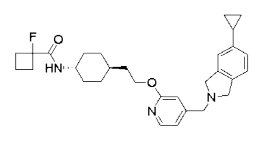 | 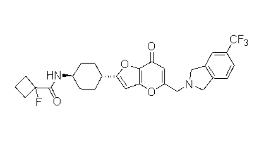 | 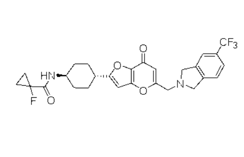 |
| 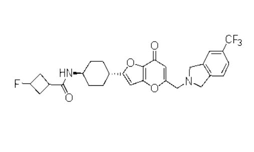 | 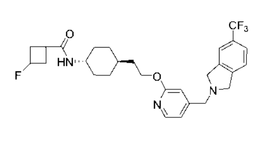 | 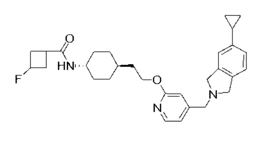 | 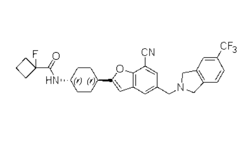 |
| 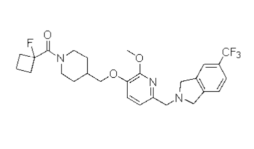 | 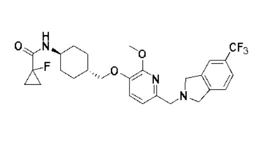 | 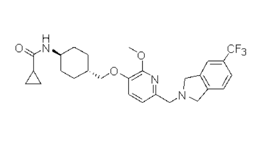 | 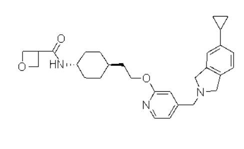 |
| 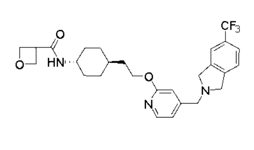 | 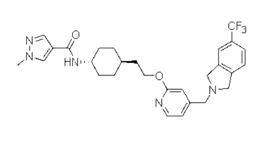 | 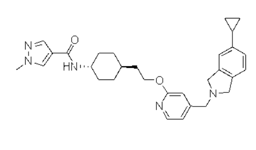 | 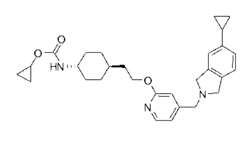 |
| 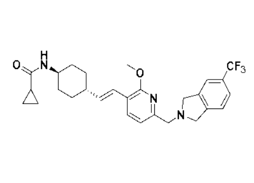 | 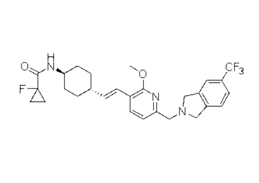 | 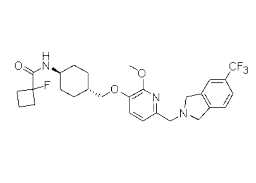 | 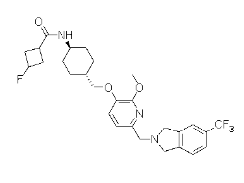 |
| 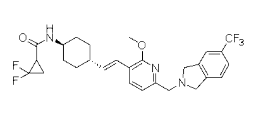 | 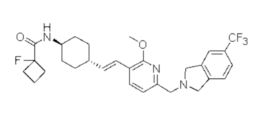 | 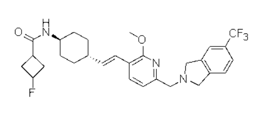 | 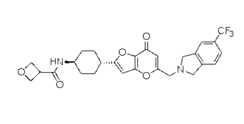 |
| 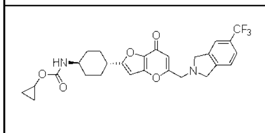 | 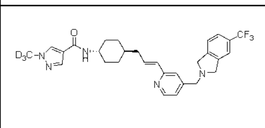 | 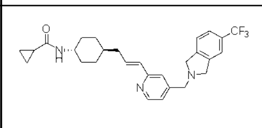 | 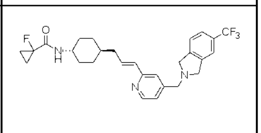 |
| 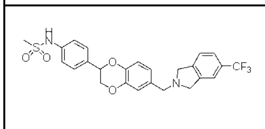 | 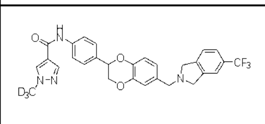 | 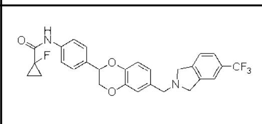 | 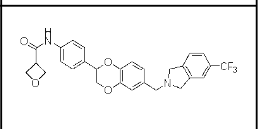 |
| 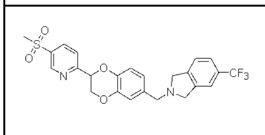 | 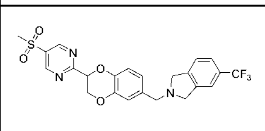 | 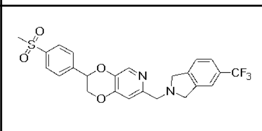 | 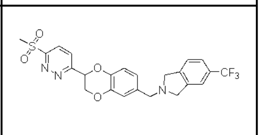 |
| 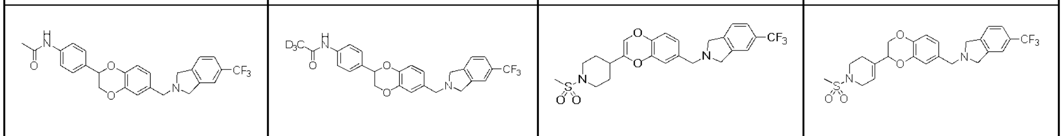 | | | |
| 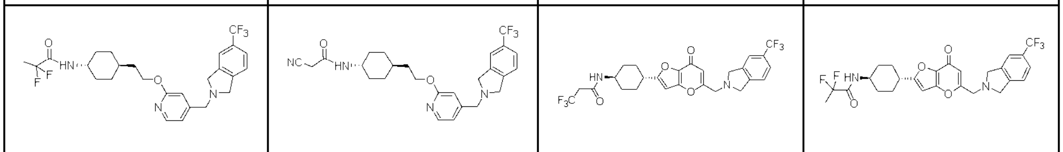 | | | |

| | | | |
|---|---|---|---|
| 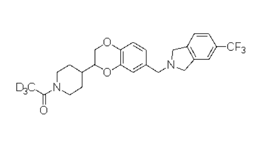 | 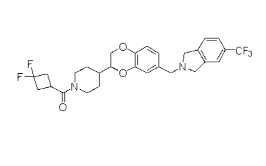 | 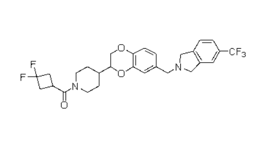 | 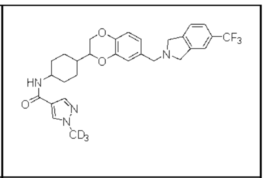 |
| 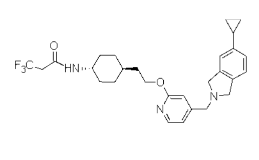 | 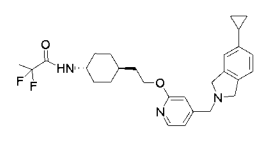 | 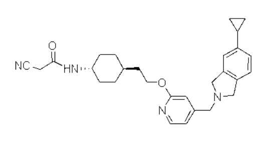 | 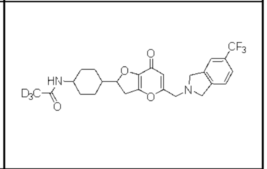 |
| 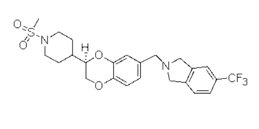 | 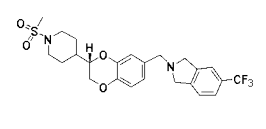 | 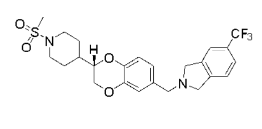 | 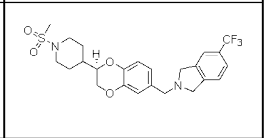 |
| 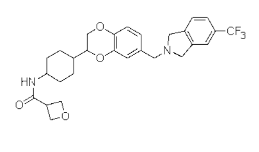 | 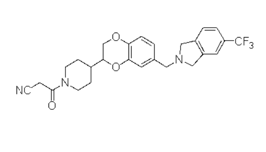 | 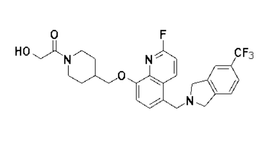 | 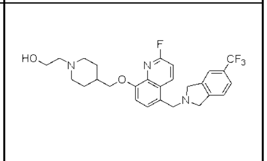 |
| 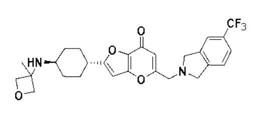 | 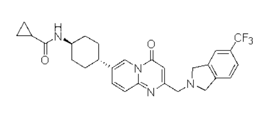 | 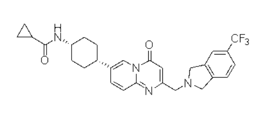 | 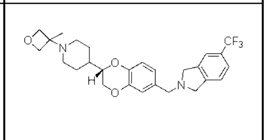 |
| 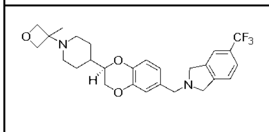 | 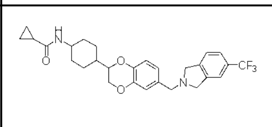 | 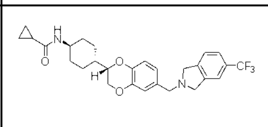 | 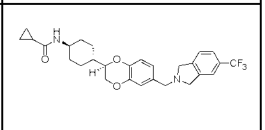 |
| 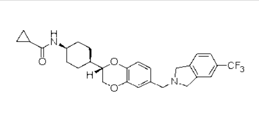 | 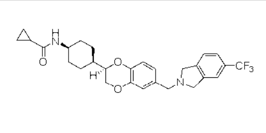 | 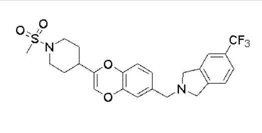 | 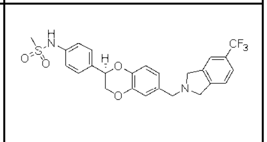 |
| 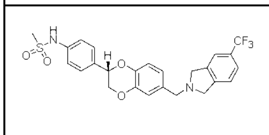 | 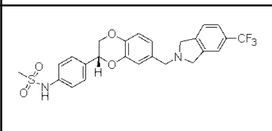 | 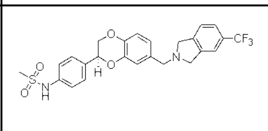 | 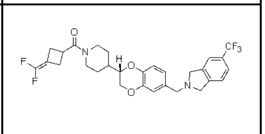 |
| 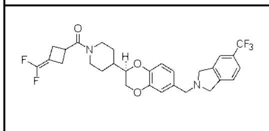 | 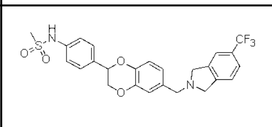 | 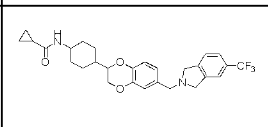 | 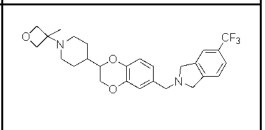 |
| 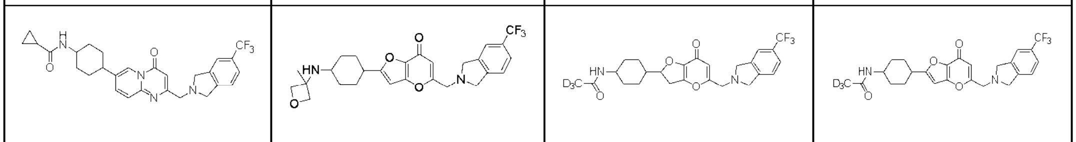 | | | |
| 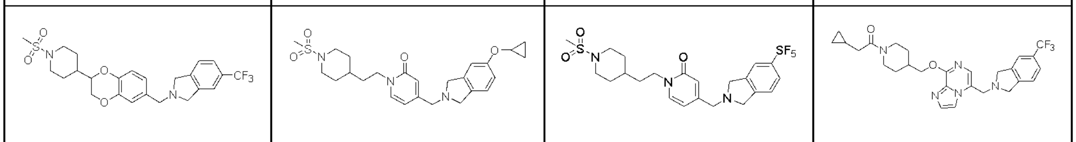 | | | |
| 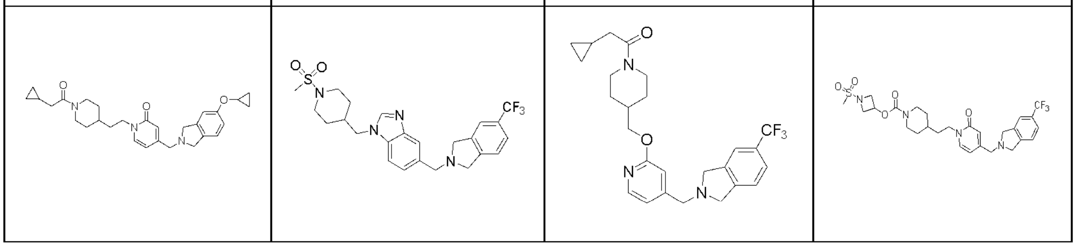 | | | |

| | | | |
|---|---|---|---|
| 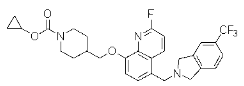 | | | |

.

The present invention further provides a pharmaceutical composition or pharmaceutical preparation comprising the compound, or the stereoisomer, the deuterated substance, the solvate, the co-crystal or the pharmaceutically acceptable salt thereof described in any one of the preceding solutions, and a pharmaceutically acceptable carrier and/or excipient.

Further, the composition or pharmaceutical preparation of the present invention contains 1-1500 mg of the compound, or the stereoisomer, the deuterated substance, the solvate, the co-crystal or the pharmaceutically acceptable salt thereof described in any one of the preceding solutions, and a carrier and/or excipient.

The present invention further provides the use of the compound, or the stereoisomer, the deuterated substance, the solvate, the co-crystal or the pharmaceutically acceptable salt thereof described in any one of the preceding solutions, or the composition described in any one of the preceding solutions in the preparation of a drug for treating/preventing a CYP11A1-mediated disease; further, the CYP11A1-mediated disease is steroid hormone-dependent cancer, further preferably, the cancer is prostate cancer.

The present invention further provides a method for treating a disease in a mammal, the method comprises administering to a subject a therapeutically effective amount of the compound, or the stereoisomer, the deuterated substance, the solvate, or the pharmaceutically acceptable salt thereof described in any one of the preceding solutions, wherein the therapeutically effective amount is preferably 1-1500 mg, and the disease is preferably prostate cancer. In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation comprising a therapeutically effective amount of the compound, or the stereoisomer, the deuterated substance, the solvate, the co-crystal or the pharmaceutically acceptable salt thereof according to the present invention, and a carrier and/or excipient. The pharmaceutical composition can be in a unit preparation form (the amount of the drug substance in the unit preparation is also referred to as the "preparation strength"). In some embodiments, the pharmaceutical composition comprises the compound, or the stereoisomer, the deuterated substance, the solvate, the co-crystal or the pharmaceutically acceptable salt thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

A method for treating a disease in a mammal or human, which comprises administering to a subject a therapeutically effective amount of the compound, or the stereoisomer, the deuterated substance, the solvate, the co-crystal or the pharmaceutically acceptable salt thereof according to the present invention, and a pharmaceutically

acceptable carrier and/or excipient, the therapeutically effective amount is preferably 1-1500 mg, and the disease is a CYP11A1-mediated disease, preferably prostate cancer.

A method for treating a disease in a mammal or human, which comprises administering to a subject the compound, or the stereoisomer, the deuterated substance, the solvate, the co-crystal or the pharmaceutically acceptable salt thereof according to the present invention, and a pharmaceutically acceptable carrier and/or excipient at a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, which may include a composition in single-dose or multiple-dose form, wherein the kit comprises the compound, or the stereoisomer, the deuterated substance, the solvate, the co-crystal or the pharmaceutically acceptable salt thereof according to the present invention, and the amount of the compound, or the stereoisomer, the deuterated substance, the solvate, the co-crystal or the pharmaceutically acceptable salt thereof according to the present invention is the same as that in the above-mentioned pharmaceutical composition.

In the present invention, the amount of the compound, or the stereoisomer, or the pharmaceutically acceptable salt thereof according to the present invention is calculated in the form of a free base in each case.

The term "preparation strength" refers to the weight of the active drug contained in each vial, tablet or other unit preparation.

**Synthetic route**

Patent documents such as WO 2018115591 A1 have introduced a method for preparing a CYP11A1 inhibitor, and those skilled in the art would have been able to prepare the compounds of the present invention in conjunction with this document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalog are optionally prepared by custom chemical synthesis plants, wherein many standard chemical supply plants provide custom synthesis services.

**Terms**

Unless otherwise specified in the present invention, the terms of the present invention have the following meanings.

The term "halogen" herein refers to F, Cl, Br, I, or isotopes thereof.

The term "halo" or "substituted with halogen" means that a hydrogen atom is substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen.

The term "deuterated" or "deuterated substance" refers to the case where a hydrogen atom on alkyl, cycloalkyl, alkylene, aryl, heteroaryl, mercapto, heterocycloalkyl, alkenyl, alkynyl and other groups is substituted with at least one isotope deuterium, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, more preferably 1-10 deuterium atoms, more preferably 1-6 deuterium atoms, and further preferably 1-3 deuterium atoms.

The term "alkyl" refers to a monovalent linear or branched saturated aliphatic hydrocarbon group. Unless otherwise specially specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples include methyl, ethyl, n-propyl, isopropyl, n-butyl, sec-butyl, neobutyl, tert-butyl, n-pentyl, isoamyl, neopentyl, n-hexyl, and various branched isomers thereof.

The term "alkylene" refers to a divalent linear or branched saturated alkyl, and examples of alkylene include, but are not limited to, methylene, ethylene, propylene, butylene, etc.

The term "cycloalkyl" refers to a monovalent non-aromatic, partially unsaturated or fully saturated, substituted or unsubstituted carbocyclic hydrocarbon group, which generally has 3 to 12 carbon atoms, preferably 3-10 carbon atoms, more preferably 3-6 carbon atoms, and further preferably 3-4 carbon atoms, unless otherwise specially specified. Non-limiting examples include cyclopropyl, cyclobutyl, cyclopentyl, cyclohexyl,

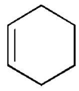,

cycloheptyl, etc.

The term "cycloalkylene" refers to a divalent group of "cycloalkyl", and non-limiting examples include cyclopropylene, cyclobutylene, etc.

The term "heterocycle" or "heterocyclyl" refers to a substituted or unsubstituted, saturated or unsaturated, aromatic or non-aromatic ring comprising 1 to 3 heteroatoms selected from N, O, or S unless otherwise specifically defined, including monocyclic heterocycles, bridged bicyclic heterocycles, fused bicyclic heterocycles, spiro bicyclic heterocycles, etc., which are 3- to 12-membered heterocycles, more preferably 4- to 12-membered heterocycles, more preferably 4-to 10-membered heterocycles, and further preferably 4- to 7-membered heterocycles unless otherwise specifically defined. The definition thereof comprises heterocycloalkyl and heteroaryl. The N and S in the heterocyclyl ring may be oxidized into various oxidation states. Heterocyclyl can be connected to a heteroatom or a carbon atom, and non-limiting examples of heterocyclyl include oxiranyl, aziridinyl, oxetanyl, azetidinyl, 1,3-dioxolanyl, 1,4-dioxolanyl, 1,3-dioxanyl, azepanyl, pyridinyl, furyl, thienyl, pyranyl, N-alkylpyrrolyl, pyrimidinyl, pyrazinyl, pyrazolyl, pyridazinyl, imidazolyl, piperidinyl, piperidinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridinyl, pyrrolopyridinyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl,

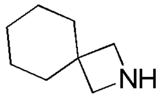, 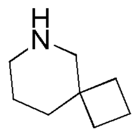, 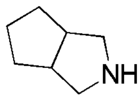, 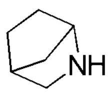,

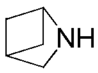, 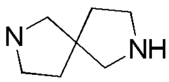, 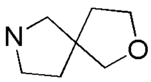, 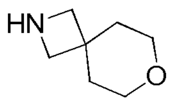, 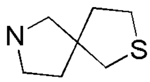, 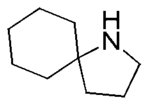,

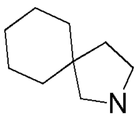,

etc.

The term "heterocyclylene" is a divalent group corresponding to "heterocyclyl", and non-limiting examples include imidazolylene, piperidinylene, aziridinylene, etc.

The term "carbocyclic" or "carbocyclyl" refers to a substituted or unsubstituted, saturated or unsaturated, aromatic or non-aromatic carbocyclic group, including monocyclic carbocyclic rings, bridged bicyclic rings, fused bicyclic rings, spiro bicyclic rings, etc., which have 3 to 12 carbon atoms, preferably 3-10 carbon atoms, and further preferably 3-6 carbon atoms unless otherwise specially specified. The definition thereof comprises cycloalkyl and aryl. In non-limiting embodiments, monocyclic carbocyclic rings include cyclopropyl, cyclobutyl, cyclopentyl, cyclohexyl, cycloheptyl or phenyl,

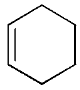,

etc., bridged bicyclic rings include

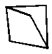,

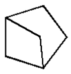, 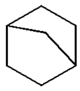,

etc., fused bicyclic rings include

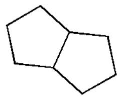, 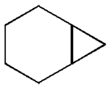, 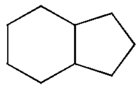,

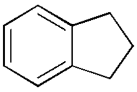, 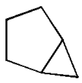,

etc., and spiro bicyclic rings include

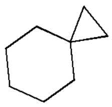, 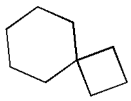,

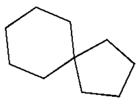, 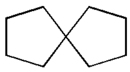, 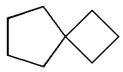, 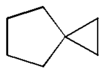,

etc.

The term "aryl" refers to an aromatic carbocyclic ring. Non-limiting examples include phenyl, naphthyl, etc.

The term "alkynyl" refers to a monovalent linear or branched unsaturated hydrocarbon group containing one or more carbon-carbon triple bonds. Unless otherwise specifically specified, the alkynyl contains 2-6 carbon atoms, preferably 2-4 carbon atoms, and non-limiting examples include ethynyl, propynyl, propargyl, etc.

The term "alkenyl" refers to a linear or branched monovalent unsaturated hydrocarbon group containing one or more carbon-carbon double bonds. Unless otherwise specifically specified, the alkynyl contains 2-6 carbon atoms, preferably contains 2-4 carbon atoms, and non-limiting examples include vinyl, propenyl, allyl, 2-butenyl, 1-butenyl, etc.

The term "alkoxy" or "alkyloxy" refers to -O-alkyl. Without particular limitation, alkoxy or alkyloxy is -O-$C_{1-8}$ alkyl, preferably -O-$C_{1-6}$ alkyl, more preferably -O-$C_{1-4}$ alkyl, and further preferably -O-$C_{1-2}$ alkyl. Non-limiting examples include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Without particular limitation, the haloalkoxy is -O-halo $C_{1-8}$ alkyl, preferably -O-halo $C_{1-6}$ alkyl, more preferably -O-halo $C_{1-4}$ alkyl, and further preferably -O-halo $C_{1-2}$ alkyl. Non-limiting examples of haloalkoxy include monofluoromethoxy, difluoromethoxy, trifluoromethoxy, difluoroethyloxy, *etc.*

The term "$C_{1-4}$ alkylacyl" refers to $C_{1-4}$ alkyl-C(O)-. Non-limiting examples include formyl, acetyl, and propionyl.

The term "$C_{1-4}$ alkylsulfonyl" refers to $C_{1-4}$ alkyl-$S(O)_2$-. Non-limiting examples include methylsulfonyl, ethylsulfonyl, and propylsulfonyl.

The term "heteroaromatic ring" or "heteroaryl" refers to an aromatic heterocycle. Non-limiting examples include pyrazolyl, pyrimidinyl, thiazolyl, pyridinyl, furyl, pyrone, pyridone, etc.

The term "heterocycloalkyl" refers to a non-aromatic, partially unsaturated or fully saturated heterocycle, which

generally has 4 to 12 ring members, preferably 4 to 10 ring members, more preferably 4 to 7 ring members, and further preferably 5 or 6 ring members. In addition to carbon atoms, heterocyclic alkyl groups also comprise 1-3 heteroatoms selected from N, S and O as ring members. Non-limiting examples include azetidinyl, morpholinyl, piperazinyl, piperidyl, tetrahydropyranyl, oxetanyl, etc.

"Heterocycloalkylene" refers to, but not limited to

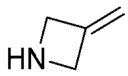.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but used as a diluent, adjuvant, binder and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colorant, a flavoring agent, and a sweetening agent. Examples of pharmaceutically acceptable excipients include, but are not limited to: (1) sugars, such as lactose, glucose, and sucrose; (2) starches, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffer solutions; (21) polyesters, polycarbonates and/or polyanhydrides; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

Detailed Description of Embodiments

The content of the present invention is described in detail by means of the following examples. In examples in which no specific conditions are indicated, experimental methods are carried out under conventional conditions. The listed examples are intended to better illustrate the content of the present invention but should not be construed as limiting the content of the present invention. Non-essential improvements and adjustments made to the embodiments by a person of

ordinary skill in the art according to the above Summary of the Invention still fall within the scope of protection of the present invention.

Dess-Martin reagent; 1,1,1-Triacetoxy-1,1-dihydro-1,2-benziodoxol-3-(1H)-one
DIPEA: N,N-diisopropylethylamine
NMP; N-methylpyrrolidone
TBDMSCl: Tert-butyl dimethylsilyl chloride
DMAP: 4-dimethyl $NH_2$ pyridine
HATU: 2-(7-azabenzotriazole)-N,N,N',N'-tetramethyluronium hexafluorophosphate
$T_3P$: Propylphosphonic cyclic anhydride

**Intermediate 1: 2-(chloromethyl)-5-hydroxy-(4H)-pyran-4-one (intermediate 1)**

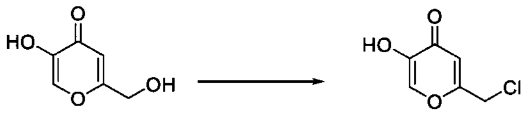

Intermediate 1

Kojic acid (20.0 g, 140.7 mmol) was dissolved in thionyl chloride (40 ml), and the resulting mixture was reacted at room temperature for 2 h. After the reaction was completed, the reaction solution was filtered, and the filter cake was slurried with petroleum ether (100 mL) and filtered. The resulting filter cake was dried to obtain **intermediate 1** (29.8 g, 90%).
LCMS (ESI): m/z= 161.2 [M+H]
$^1$H NMR (400 MHz, DMSO) δ 8.12 (s, 1H), 6.57(s, 1H), 4.66 (s, 2H).

**Intermediate 2: (1-(methylsulfonyl)piperidin-4-yl)methyl methanesulfonate (intermediate 2)**

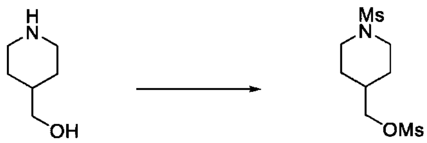

Intermediate 2

4-hydroxymethylpiperidine (20.0 g, 175.4 mmol) was dissolved in 200 ml of acetonitrile, potassium carbonate (53.6 g, 386.0 mmol) was added, and the resulting mixture was mechanically stirred and reacted at room temperature for 16 h. After the reaction was completed, the reaction solution was filtered, and the filtrate was concentrated, slurried with 100 mL of mixed solvents of ethyl acetate:petroleum ether-1:1 and filtered. The resulting filter cake was washed with water and dried to obtain **intermediate 2** (47 g, 40%).
LCMS (ESI): m/z= 272.3 [M+H]
$^1$H NMR (400 MHz, $CDCl_3$) δ 4.10 (d, 2H), 3.95 - 3.78 (m, 2H), 3.03 (s, 3H), 2.79 (s, 3H), 2.71-2.67 (m, 2H), 1.95 - 1.80 (m, 3H), 1.51 - 1.34 (m, 2H).

**Intermediate 3: 5-hydroxy-2-(isoindolin-2-ylmethyl)-4H-pyran-4-one (intermediate 3)**

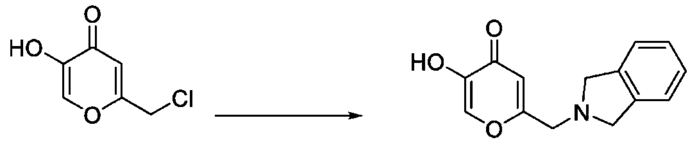

Intermediate 1 Intermediate 3

**Intermediate 1** (9.8 g, 61.0 mmol) and isoindole hydrochloride (19.0 g, 122.1 mmol) were dissolved in 200 ml of acetonitrile, DIPEA (30.8 g, 244.2 mmol) was added, and the resulting mixture was mechanically stirred and reacted at room temperature for 16 h. After the reaction was completed, the reaction solution was filtered, and the filter cake was slurried with 100 mL of water and 50 mL of ethyl acetate successively and filtered. The resulting filter cake was dried to obtain **intermediate 3** (47 g, 40%).

LCMS (ESI): m/z= 244.1 [M+H]

$^1$H NMR (400 MHz, DMSO) δ 9.06 (s, 1H), 8.06 (s, 1H), 7.39 - 7.04 (m, 4H), 6.41 (s, 1H), 3.95 (s, 4H), 3.78 (s, 2H).

**Intermediate 4: 6-(isoindolin-2-ylmethyl)-4-oxo-4H-pyran-3-yl trifluoromethanesulfonate**

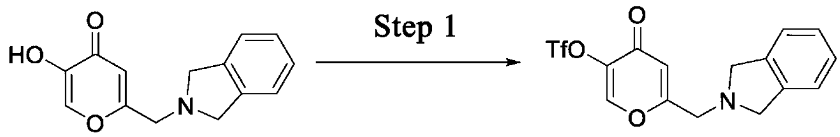


Intermediate 3 Intermediate 4

Step 1: At room temperature, the raw material substrate **intermediate 3** (1.0 g, 4.12 mmol) and triethylamine (1.26 g, 12.36 mmol) were dissolved in ultra-dry DCM (10 mL), the resulting mixture was cooled to 0°C under nitrogen protection, and then a solution of trifluoromethanesulfonic anhydride (1.75 g, 6.18 mmol) in ultra-dry DCM (10 mL) was added dropwise. After the dropwise addition was completed, the resulting mixture was naturally warmed to room temperature and reacted for 1 h. 30 mL of saturated sodium bicarbonate was added to quench the reaction, and the resulting mixture was extracted with DCM (50 mL × 3). The organic phases were combined, washed with water (50 mL × 3) and saturated sodium chloride (50 mL × 1), dried over anhydrous sodium sulfate, filtered, concentrated under reduced pressure, and then subjected to column chromatography (ethyl acetate: petroleum ether=0:1-1:1) to obtain **intermediate 4** (1.0 g, 65%).

LC-MS (ESI): m/z= 376.1 [M+H]$^+$.

**Example 1:**

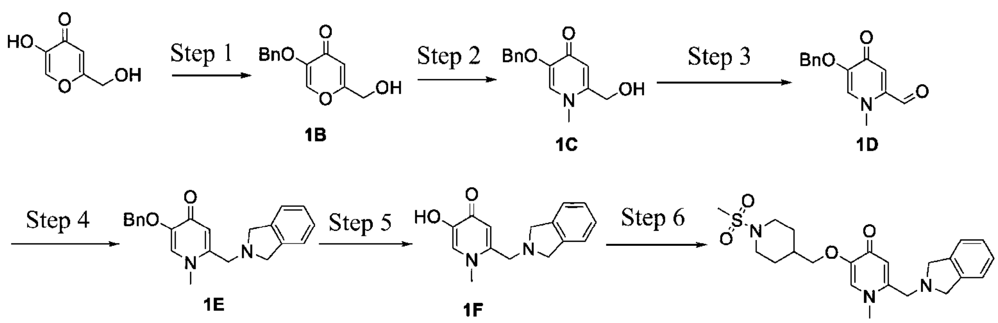


1B 1C 1D 1E 1F Compound 1

Step 1: Kojic acid (10.0 g, 70.4 mmol) and benzyl bromide (13.1 g, 77.4 mmol) were dissolved in 85 ml of methanol, 85 mL of a solution of sodium hydroxide in water (3.38 g, 84.4 mmol, about 1 M solution) was added, and the resulting mixture was reacted at 80°C for 16 h. After the reaction was completed, the reaction solution was dropwise added to 100 mL of ice water, and the resulting mixture was extracted with dichloromethane (200 mL x 3). The organic phases

were combined, concentrated, and purified by silica gel column chromatography (PE:EA = 1:1) to obtain the target compound **1B** (7.2 g, 41 %).
LCMS (ESI): m/z= 233.3 [M+H]

Step 2: Compound **1B** (7.20 g, 31.0 mmol) was suspended in 30 mL of aqueous methylamine solution, and the resulting mixture was stirred under reflux at 100°C for reaction overnight for 16 h, and filtered. The filter cake was suspended in 10 mL of ethyl acetate, stirred for 0.5 h, and filtered, and the resulting filter cake was dried to obtain the target compound **1C** (5.12 g, 67%).
LCMS (ESI): m/z= 246.2 [M+H]

Step 3: Compound **1C** (5.12 g, 20.9 mmol) was dissolved in 50 mL of ethyl acetate, IBX (17.5 g, 62.6 mmol) was added, and the resulting mixture was reacted at 80°C for 16 h. After the reaction was completed, the reaction product was filtered. The filtrate was concentrated, stirred at room temperature for 0.5 h, and separated by silica gel column chromatography (PE:EA = 2:1) to obtain the target compound **1D** (3.41 g, 51%).
LCMS (ESI): m/z= 262.1 [M+H]

Step 4: Compound **1D** (2.71 g, 11.1 mmol) and isoindole (1.46 g, 12.3 mmol) were dissolved in 15 mL of tetrahydrofuran, acetic acid (1.02 g, 22.2 mmol) was added, and the resulting mixture was reacted at 50°C for 3 h. After the reaction, the reaction product was cooled to room temperature, sodium triacetoxyborohydride (4.70 g, 22.2 mmol) was added with stirring, and the resulting mixture was reacted at room temperature for 16 h. After the reaction was completed, the reaction solution was dropped added to 20 mL of saturated sodium bicarbonate solution, and the resulting mixture was extracted with dichloromethane (50 mL x 3). The organic phases were combined, concentrated and separated by silica gel column chromatography (PE:EA =1:1) to obtain the target compound **1E** (1.36 g, 35%).
LCMS (ESI): m/z= 347.5 [M+H]

Step 5: Compound **1E** (1.36 g, 3.92 mmol) was dissolved in 50 mL of methanol, 680 mg of palladium on carbon was added, and the resulting mixture was subjected to hydrogen replacement three times and reacted at normal pressure and room temperature for 16 h. After the reaction was completed, the reaction product was filtered, and the filtrate was concentrated and dried to obtain the target compound **1F** (PE:EA = 2:1) (765 mg, 76%).
LCMS (ESI): m/z= 257.2 [M+H]

Step 6: Compound **1F** (400 mg, 1.56 mmol) and intermediate **2** (466 mg, 1.72 mmol) were dissolved in 2 mL of DMF, cesium carbonate (1.02 g, 3.12 mmol) was added, and the resulting mixture was reacted at 90°C for 2 h. After the reaction was completed as monitored by TLC, the reaction solution was dropwise added to ice water and filtered. The filter cake was purified by HPLC to obtain the target compound **1** (152 mg, 22%).

LCMS (ESI): m/z= 432.1 [M+H]
$^{1}$H NMR (400 MHz, DMSO) δ 7.53 (s, 1H), 7.28 - 7.14 (m, 4H), 6.25 (s, 1H), 3.88 (s, 4H), 3.78 (s, 2H), 3.77-3.75 (m, 2H), 3.72 (s, 3H), 3.62-3.56 (m, 2H), 2.86 (s, 3H), 2.77-2.68 (m, 2H), 1.89-1.80 (m, 3H), 1.35 - 1.22 (m, 2H).

**Example 2:**

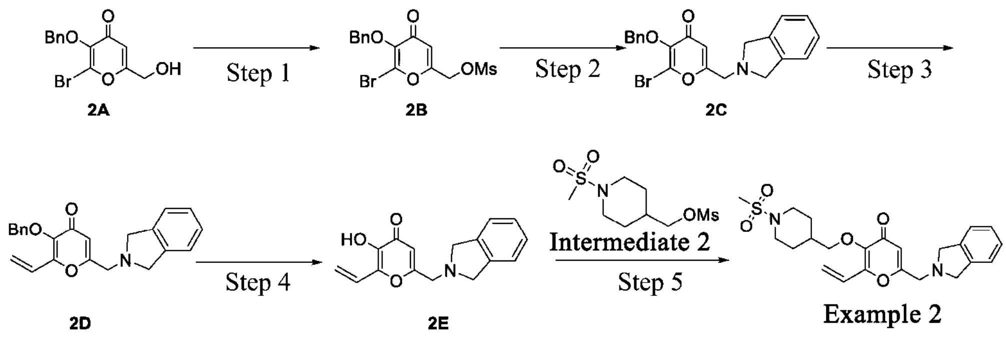


2A 2B 2C 2D 2E

Step 1: **2A** (7.00 g, 22.4 mmol) (synthesized according to patent CN114685415) and triethylamine (3.42 g, 33.6 mmol) were dissolved in dichloromethane (50 mL), and a solution of methanesulfonic anhydride (4.68 g, 26.9 mmol) in dichloromethane [trifluoromethanesulfonic anhydride was dissolved in dichloromethane (20 mL)] was added dropwise at room temperature. After the dropwise addition was completed, the resulting mixture was stirred and reacted at room temperature for 2 h. After the reaction was completed as monitored by LCMS, water (20 mL) was added to the reaction solution, and the resulting mixture was stirred at room temperature for 10 min, and then allowed to stand for layer separation. The organic phase was separated. The organic phase was dried over anhydrous $Na_2SO_4$, filtered and concentrated to obtain a crude product, which was purified by silica gel column chromatography (eluent: PE:EA = 1:1) to

obtain **compound 2B** (6.64 g, yield: 76%).

LCMS m/z =389.3 $[M +1]^+$

Step 2: **Compound 2B** (6.64 g, 17.0 mmol) and DIPEA (6.61 g, 51.2 mmol) were dissolved in acetonitrile (25 mL), 2,3-dihydroisoindole hydrochloride (3.97 g, 25.5 mmol) was added, and after the addition was completed, the resulting mixture was reacted at room temperature for 16 h. After the reaction was stopped as monitored by LCMS, the reaction solution was filtered, the filter cake was added to water (25 mL), slurried, and filtered, and then the filter cake was slurried with a mixed solution of ethyl acetate/petroleum ether (25 mL) (PE:EA = 1:1), filtered, and the filter cake was dried to obtain **compound 2C** (3.88 g, yield: 55%).

$^1H$ NMR (400 MHz, DMSO) δ 7.48 - 7.42 (m, 2H), 7.42 - 7.32 (m, 3H), 7.31 - 7.15 (m, 4H), 6.51 (s, 1H), 5.12 (s, 2H), 3.97 (s, 4H), 3.82 (s, 2H).

Step 3: Under nitrogen protection, **compound 2C** (0.412 g, 1.0 mmol), potassium vinyltrifluoroborate (0.16 g, 1.2 mmol) and sodium carbonate (0.21 g, 2.0 mmol) were weighed and added to 1,4-dioxane (10 mL) and water (2 mL), then tetrakis(triphenylphosphine)palladium (0.12 g, 0.1 mmol) was added. After the addition was completed, the resulting mixture was heated to reflux and reacted for 4 h, the reaction was monitored by TLC, and the disappearance of the raw materials was the end point of the reaction (developing agent: petroleum ether: ethyl acetate = 1:1). After the reaction was completed, the temperature was cooled to room temperature, and the reaction product was concentrated and directly purified by column chromatography (eluent: ethyl acetate: petroleum ether=1:1) to obtain the target **compound 2D** (0.323 g, yield: 89.87%).

LCMS m/z =360.4 $[M +1]^+$

Step 4: **Compound 2D** (0.313 g, 0.87 mmol) was weighed and dissolved in trifluoroacetic acid (5 mL), then the resulting mixture was heated to 30°C for reaction, and the reaction was monitored by TLC (developing agent: ethyl acetate: petroleum ether=1:1, the disappearance of the raw materials was the end point of the reaction). After the reaction was completed, the reaction product was concentrated to remove the solvent to obtain a residue, the residue was adjusted to pH = about 8 with saturated aqueous sodium bicarbonate solution, and the resulting mixture was extracted with ethyl acetate (50 mL*2). The organic phases were combined, dried over anhydrous sodium sulfate, filtered, and concentrated to obtain crude **compound 2E** (0.22 g, yield: 93.9%), which was directly used in the next reaction.

LCMS m/z =270.1 $[M +1]^+$

Step 5: **Compound 2E** (0.200 g, 0.74 mmol) was weighed and dissolved in sulfolane (10 mL) and then **intermediate 2** (0.24 g, 0.89 mmol) and cesium carbonate (0.36 g, 1.11 mmol) were added. After the addition was completed, the resulting mixture was heated to 80°C and reacted for 1 h. After the reaction was completed, the temperature was cooled to room temperature, water and ethyl acetate (80 mL*2) were added to the reaction solution, and the organic phases were combined, dried over anhydrous sodium sulfate, filtered, and concentrated to obtain a crude product, which was purified by a preparative plate (ethyl acetate:methanol=15:1) to obtain target **compound 2** (41 mg, yield: 12.46%).

LCMS m/z =445.3 $[M +1]^+$

$^1H$ NMR (400 MHz, DMSO) δ 7.26 - 7.18 (m, 4H), 6.91 - 6.84 (m, 1H), 6.39 (s, 1H), 6.04 - 6.00 (m, 1H), 5.69 - 5.66 (m, 1H), 4.00 (s, 4H), 3.93 - 3.91 (m, 2H), 3.85 (s, 2H), 3.59 - 3.56 (m, 2H), 2.85 (s, 3H), 2.75 - 2.69 (m, 2H), 1.86 - 1.79 (m, 3H), 1.36 - 1.26 (m, 2H).

**Example 3:**

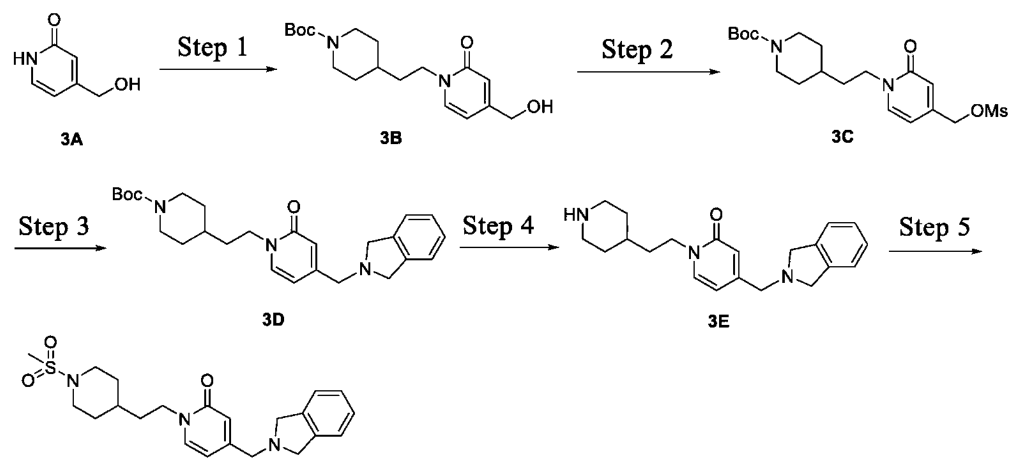


3A 3B 3C 3D 3E

Compound 3

Step 1: **3A** (1.0 g, 7.96 mmol), tert-butyl 4-(2-bromoethyl) piperidine-1-carboxylate (2.79 g, 9.55 mmol), and potassium carbonate (2.20 g, 15.92 mmol) were dissolved in N,N-dimethylformamide (20 mL), and the resulting mixture was reacted at 100°C overnight. After the reaction was cooled to room temperature, water (100 mL) was added to the reaction system to quench the reaction, and the resulting mixture was extracted with ethyl acetate (100 mL*2). The organic phase was dried, concentrated and separated by column chromatography (PE/EA = 80%/20%) to obtain **3B** (820 mg, 30%).

LC-MS (ESI): m/z= 337.2 $[M+H]^+$.

Step 2: **3B** (820 mg, 2.44 mmol) was dissolved in dichloromethane (10 mL), and triethylamine (617 mg, 6.10 mmol) and methylsulfonyl chloride (559 mg, 4.88 mmol) were added dropwise at 0-5°C. After the addition was completed, the resulting mixture was reacted at room temperature overnight. Water (20 mL) was added to the reaction system to quench the reaction, the resulting mixture was extracted with dichloromethane (20 mL*2), and the organic phase was dried to obtain crude product **3C.**

LC-MS (ESI): m/z= 359.3 $[M+H]^+$.

Step 3: **3C** (600 mg, 1.45 mmol) was dissolved in dichloromethane (5 mL), and triethylamine (293 mg, 2.90 mmol) and 2,3-dihydroisoindole hydrochloride (270 mg, 1.74 mmol) were added dropwise at 0-5°C. After the addition was completed, the resulting mixture was reacted at room temperature overnight. The reaction was concentrated and separated by column chromatography (PE/EA=70%/30%) to obtain **3D** (250 mg, 39%)

LC-MS (ESI): m/z= 438.2 $[M+H]^+$.

Step 4: **3D** (250 mg, 0.57 mmol) was dissolved in dichloromethane (6 mL), and trifluoroacetic acid (1.5 mL) was added dropwise at 0-5°C. After the addition was completed, the resulting mixture was reacted at room temperature for 2 h. The reaction was concentrated to obtain crude product **3E.**

LC-MS (ESI): m/z= 338.5 $[M+H]^+$.

Step 5: **3E** (190 mg, 0.56 mmol) was dissolved in dichloromethane (5 mL), and triethylamine (170 mg, 1.68 mmol) and methylsulfonyl chloride (128 mg, 1.12 mmol) were added dropwise at 0-5°C. After the addition was completed, the resulting mixture was reacted at room temperature for 2.5 h. After the reaction was concentrated, the residue was separated and purified by preparative liquid phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% trifluoroacetic acid); gradient: isocratic elution using 20%-70% acetonitrile; cycle time: 15 min) to obtain compound 3.

LC-MS (ESI): m/z= 416.2 $[M+H]^+$.

**Example 4:**

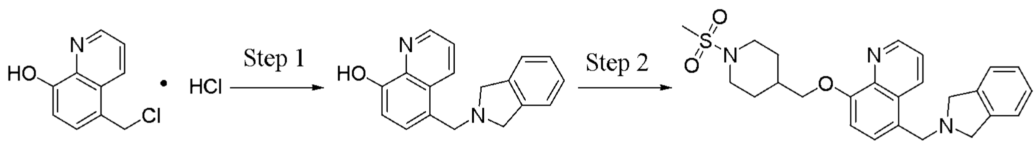


4A 4B Compound 4

Step 1: Compound 5-(chloromethyl)quinolin-8-ol hydrochloride (200 mg, 0.87 mmol) and potassium carbonate (720 mg, 5.21 mmol) were dissolved in acetonitrile (10 ml), then isoindoline hydrochloride (150 mg, 0.95 mmol) was added and the resulting mixture was reacted at 70°C for 20 h. After the reaction was completed as monitored by TLC, the reaction product was cooled to room temperature and filtered, and the filtrate was collected and concentrated to obtain compound 4B (240 mg, 99.90%).

LC-MS (ESI):m/z = 277.20 $[M+H]^+$.

Step 2: Compound 4B (100 mg, 0.36 mmol), intermediate 2 (100 mg, 0.36 mmol) and cesium carbonate (180 mg, 0.54 mmol) were added to the reaction flask, then dissolved in DMF (5 ml) and reacted at 100°C for 1 h. After the reaction was completed, the reaction product was cooled to room temperature, diluted by adding water (50 ml), then the resulting mixture was extracted twice with ethyl acetate (50 ml*2), and the organic phases were combined, dried over anhydrous sodium sulfate, concentrated, and purified by Pre-TLC (6 mg, 3.7%).

$^1H$ NMR (400 MHz, $CDCl_3$) δ 8.95 - 8.93 (m, 1H), 8.72 - 8.70 (m, 1H), 7.48 - 7.40 (m, 2H), 7.20 - 7.13 (m, 4H), 6.99 - 6.97 (m, 1H), 4.23 (s, 2H), 4.11 - 4.10 (m, 2H), 3.97 - 3.85 (m, 6H), 2.81 (s, 3H), 2.81 - 2.72 (m, 2H), 2.35 - 2.22 (m, 1H), 2.16 - 2.14 (m, 2H), 1.59 - 1.48 (m, 2H).

LC-MS (ESI):m/z = 452.30 $[M+H]^+$.

**Example 5:**

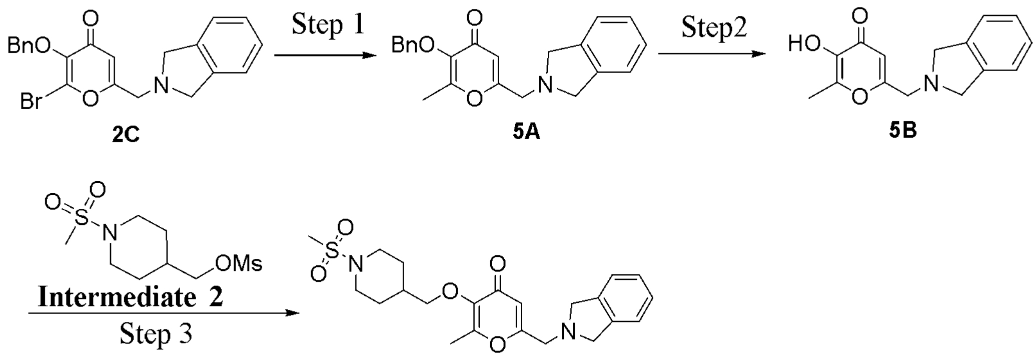

2C
5A
5B
**Compound 5**

Step 1:

Under nitrogen protection, compound **2C** (0.618 g, 1.5 mmol) was weighed and added to a mixed solution of 1,4-dioxane and water (35 mL, 1,4-dioxane:water=30:5), then methylboronic acid (0.885 g, 15 mmol), sodium carbonate (2.38 g, 15.0 mmol), and Pd(dppf)$Cl_2$ (0.219 g, 0.3 mmol) were successively added, then the resulting mixture was heated to 100°C and reacted for 5 h. The reaction was monitored by LCMS, after the reaction was completed, the reaction product was cooled to room temperature, and concentrated to remove the solvent, then ethyl acetate (100 mL) and water (50 mL) were added. The organic phase was separated, the aqueous phase was further extracted once with ethyl acetate (100 mL), and the organic phases were combined, dried over anhydrous sodium sulfate, filtered, and concentrated to obtain a crude product, which was purified by column chromatography (eluent: petroleum ether=1.5:1) to obtain the target compound **5A** (0.40 g, 76.92%).

LC-MS (ESI): m/z= 348.2 $[M+H]^+$.

Step 2:

**Compound 5A** (0.400 g, 1.15 mmol) was weighed and dissolved in trifluoroacetic acid (10 mL), then the resulting mixture was heated to 40°C for reaction, and the reaction was monitored by TLC (developing agent: ethyl acetate: petroleum ether=1:1.5, the disappearance of the raw materials was the end point of the reaction). After the reaction was completed, the reaction product was concentrated to remove the solvent to obtain a residue, which was adjusted to pH = about 8 with saturated aqueous sodium bicarbonate solution, and extracted with ethyl acetate (100 mL*2). The organic phases were combined, dried over anhydrous sodium sulfate, filtered, and concentrated to obtain crude **compound 5B** (0.29 g, yield: 97.97%), which was directly used in the next reaction.
LCMS m/z =258.2 $[M +1]^+$

Step 3:

**Compound 5B** (0.290 g, 1.13 mmol) was weighed and dissolved in DMF (20 mL) and then **intermediate 2** (0.36 g, 1.13 mmol) and cesium carbonate (0.554 g, 1.70 mmol) were added. After the addition was completed, the resulting mixture was heated to 80°C and reacted for 1 h. After the reaction was completed, the temperature was cooled to room temperature, water and ethyl acetate (100 mL*2) were added to the reaction solution, and the organic phases were combined, dried over anhydrous sodium sulfate, filtered, and concentrated to obtain a crude product, which was purified by a preparative plate (ethyl acetate:methanol=10:1) to obtain target **compound 5** (200 mg, yield: 40.98%).

LCMS m/z =445.3 $[M +1]^+$
$^1$H NMR (400 MHz, DMSO-$d_6$) δ 7.35 - 7.07 (m, 4H), 6.34 (s, 1H), 3.96 (s, 4H), 3.84 - 3.82 (m, 2H), 3.76 (s, 2H), 3.59- 3.56 (m, 2H), 2.85 (s, 3H), 2.75 - 2.69 (m, 2H), 2.29 (s, 3H), 1.86 - 1.77 (m, 3H), 1.35 - 1.25 (m, 2H).

**Example 6:**

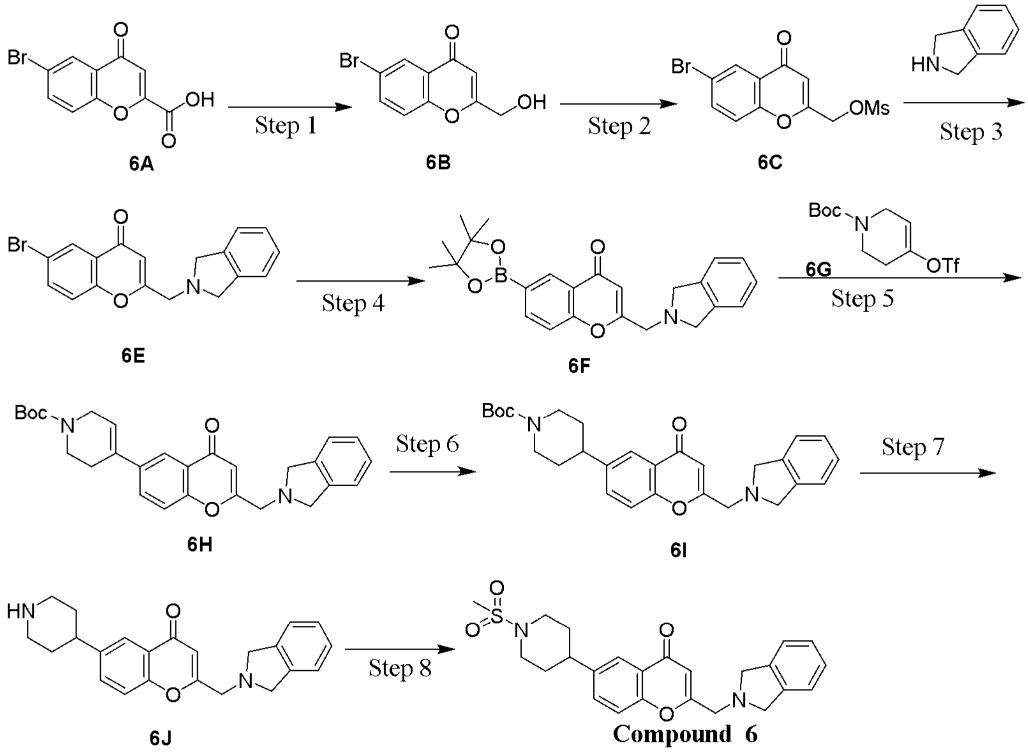


Step 1:

In a 500 mL single-neck flask, 6-bromochromone-2-carboxylic acid (6A) (12 g, 44.62 mmol) was dissolved in tetrahydrofuran (360 mL). N,N'-carbonyldiimidazole (7.24 g, 44.62 mmol) was added in an ice bath. After the addition was completed, the resulting mixture was stirred at room temperature for 2 h. Sodium borohydride (2.53 g, 66.93 mmol) was added slowly in portions in an ice bath. After the addition was completed, the resulting mixture was stirred and reacted at room temperature overnight. After the reaction was completed, methanol was added to quench the reaction in an ice bath, and the reaction product was diluted with ethyl acetate (600 mL). The organic phase was washed three times with water (300 mL), washed once with saturated aqueous sodium chloride solution (100 mL), dried over anhydrous sodium sulfate and filtered. The filtrate was concentrated to obtain compound **6B** (3.2 g, yield: 28%).

Step 2:

In a 250 mL single-neck flask, compound **6B** (3.2 g, 12.55 mmol) was dissolved in dry dichloromethane (128 mL), and in an ice bath, triethylamine (4.44 g, 43.93 mmol) was added. After the addition was completed, methylsulfonyl chloride (2.16 mg, 18.83 mmol) was slowly added in an ice bath and the resulting mixture was stirred at room temperature for 2 h. After the reaction was completed, the reaction solution was diluted with dichloromethane (200 mL), and the organic phase was washed twice with water (100 mL), dried over anhydrous sodium sulfate, and filtered. The filtrate was concentrated to obtain compound 6**C** (3.4 mg, yield: 81%), which was directly used in the next step.

Step 3:

In a 250 mL single-neck flask, compound **6C** (3.4 g, 10.21 mmol) was dissolved in dry dichloromethane (102 mL), and in an ice bath, triethylamine (3.62 g, 35.73 mmol) was added. After the addition was completed, isoindoline (1.28 g, 10.72 mmol) was slowly added in an ice bath and the resulting mixture was stirred at room temperature overnight. After the reaction was completed, the reaction solution was diluted with dichloromethane (200 mL), and the organic phase was washed twice with water (100 mL), dried over anhydrous sodium sulfate and filtered. The filtrate was concentrated and purified by column chromatography (PE:EA = 3:1) to obtain compound **6E** (2.4 g, yield: 66%).

Step 4:

Compound **6E** (1.6 g, 4.49 mmol), pinacol diborate (1.71 g, 6.74 mmol), [1,1'-bis(diphenylphosphino)ferrocene] palladium (II) dichloride (0.66 g, 0.9 mmol) and potassium acetate (1.54 g, 15.71 mmol) were dissolved in 1,4-dioxane (80

mL), the resulting mixture was subjected to nitrogen replacement 3 times and allowed to reacted at 80°C for 2 h. After the reaction was completed and the reaction product was cooled to room temperature, the reaction solution was directly used in the next step.

Step 5:

Water (20 mL) was added to the above reaction solution of **6F** (1.81 g, 4.49 mmol) to quench the previous reaction, tert-butyl 3,6-dihydro-4-[[(trifluoromethyl)sulfonyl]oxy]-1(2H)-picolinate **(6G)** (2.23 g, 6.74 mmol), [1,1'-bis(diphenylphosphino)ferrocene]palladium (II) dichloride (0.66 g, 0.9 mmol) and potassium carbonate (2.17 g, 15.71 mmol) were supplemented, and the resulting mixture was subjected to nitrogen replacement 3 times and allowed to reacted at 100°C overnight. After the reaction was completed, the reaction solution was diluted with ethyl acetate (400 mL). The organic phase was washed three times with water (200 mL), washed once with saturated aqueous sodium chloride solution (100 mL), dried over anhydrous sodium sulfate and filtered. The filtrate was concentrated and purified by column chromatography (PE:EA = 1:1) to obtain compound **6H** (1.2 g, yield: 58%).

Step 6:

Compound **6H** (1.2 g, 2.62 mmol) was dissolved in methanol (120 ml), palladium hydroxide on carbon (0.18 g, 1.28 mmol) was added, and the resulting mixture was subjected to hydrogen replacement three times and reacted at room temperature overnight. After the reaction was completed, the reaction solution was filtered through diatomaceous earth pad, and the filtrate was concentrated to obtain compound **61** (1.15 g, yield: 95%).

Step 7:

**61** (500 mg, 1.09 mmol), dichloromethane (8 ml) were added to a 25 ml single-neck flask. Trifluoroacetic acid (4 ml) was added, and the resulting mixture was reacted at room temperature for 1 h. After the reaction was completed, the reaction solution was concentrated to obtain the compound **6J** (390 mg, yield: 99%).

Step 8:

In a 100 mL single-neck flask, compound **6J** (390 mg, 1.08 mmol) was dissolved in dry dichloromethane (20 mL), and in an ice bath, triethylamine (382 mg, 3.78 mmol) was added. After the addition was completed, methylsulfonyl chloride (186 mg, 1.62 mmol) was slowly added in an ice bath and the resulting mixture was stirred at room temperature for 2 h. After the reaction was completed, the reaction solution was diluted with dichloromethane (100 mL), and the organic phase was washed twice with water (20 mL), dried over anhydrous sodium sulfate, and filtered. The filtrate was concentrated and separated and purified by preparative liquid chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% ammonium acetate); gradient: isocratic elution using 30%-80% acetonitrile; cycle time: 15 min) to obtain **compound 6** (25 mg, yield: 5%).

LCMS m/z = 439.2 $[M+H]^+$;
$^1H$ NMR (400 MHz, DMSO) δ 7.88 (d, 1H), 7.74 (d, 1H), 7.61 (d, 1H), 7.23 (d, J4H), 6.41 (s, 1H), 4.03 (s, 4H), 3.93 (s, 2H), 3.70 (d, 2H), 2.91 (s, 3H), 2.83 (dd, 3H), 1.93 (d, 2H), 1.71 (td, 2H).

**Example 7:**

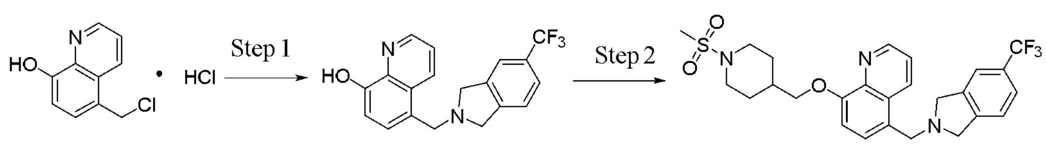


4A 7B Compound 7

Step 1:

Compound 4A (200 mg, 0.87 mmol) and potassium carbonate (720 mg, 5.21 mmol) were dissolved in acetonitrile (10 ml), then 5-trifluoromethylisoindoline hydrochloride (194 mg, 0.87 mmol) was added and the resulting mixture was

reacted at 70°C for 5 h. After the reaction was completed as monitored by TLC, the reaction product was cooled to room temperature, filtered, the filter cake was washed once with methanol (10 ml), the filtrate was collected and concentrated to obtain crude compound 7B (480 mg, 160%).

LC-MS (ESI):m/z = 345.0 $[M+H]^+$.

Step 2:

Compound 7B (200 mg, 0.58 mmol), intermediate 2 (160 mg, 0.58 mmol) and cesium carbonate (290 mg, 0.87 mmol) were added to a reaction flask, then dissolved in DMF (10 ml) and the resulting mixture was reacted at 100°C for 1 h. After the reaction was completed, the reaction product was cooled to room temperature and diluted by adding water (100 ml). An off-white solid was precipitated, which was then hot slurried twice with methanol (40 ml), filtered, and the filter cake was collected to obtain compound 7 (80 mg, 26.52%).

1H NMR (400 MHz, DMSO) δ 8.88 - 8.87 (m, 1H), 8.68 - 8.64 (m, 1H), 7.58 (s, 1H), 7.56 - 7.39 (m, 4H), 7.17 - 7.10 (m, 1H), 4.22 (s, 2H), 4.08 - 4.07 (m, 2H), 3.92 - 3.76 (m, 4H), 3.65 - 3.63 (m, 2H), 2.88 (s, 3H), 2.82 - 2.77 (m, 2H), 2.01 - 1.96 (m, 3H), 1.52 - 1.40 (m, 2H).

LC-MS (ESI):m/z = 520.70 $[M+H]^+$.

**Example 8:**

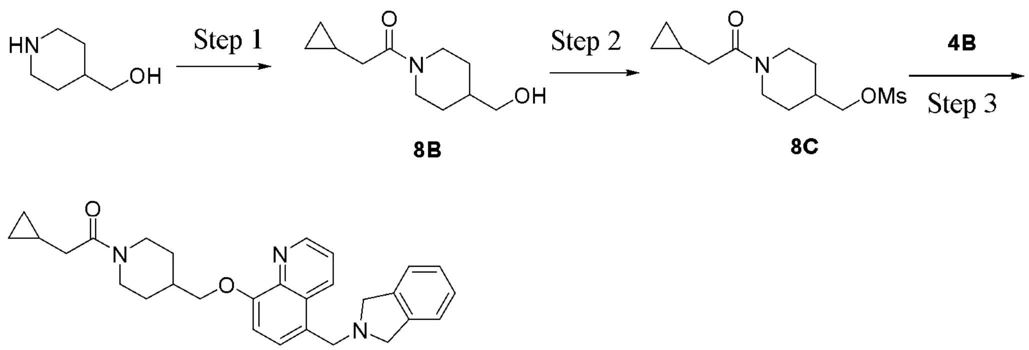


8B

8C

Compound 8

Step 1:

Cyclopropylacetic acid (1.0 g, 10 mmol) was weighed and dissolved in dichloromethane (40 mL), then EDCI. HCl (2.30 g, 12 mmol), HOBT (1.62 g, 12 mmaol) and triethylamine (3.03 g, 30 mmol) were successively added. After the addition was completed, the resulting mixture was stirred at room temperature for 10 min, then piperidine-4-methanol (1.15 g, 10 mmol) was added. After the addition was completed, the resulting mixture was stirred at room temperature for 18 h. After the reaction was completed, dichloromethane (100 mL) and water (100 mL) were added to the reaction solution. The aqueous phase was separated, and the organic phase was dried over anhydrous ammonium sulfate, filtered and concentrated to obtain a crude product, which was purified by column chromatography (eluent: EA, color developing with phosphomolybdic acid) to obtain the target compound **8B** (0.58 g, 29.43%).

LC-MS (ESI):m/z = 198.20 $[M+H]^+$.

Step 2:

**Compound 8B** (0.58 g, 2.94 mmol) was weighed and dissolved in dichloromethane (10 mL), then triethylamine (0.89 g, 8.8 mmol) was added. After the addition was completed, the temperature was cooled to 0°C, then methylsulfonyl chloride (0.40 g, 3.49 mmol) was added, and then the resulting mixture was stirred at room temperature for 1 h. The reaction was monitored by TLC (EA, the disappearance of the raw materials was the end point of the reaction, and color developing with phosphomolybdic acid). After the reaction was completed, dichloromethane (100 ml) was added to the reaction solution, and then the resulting mixture was successively washed with aqueous sodium bicarbonate solution, saturated aqueous ammonium chloride solution and water, dried over no sodium sulfate, filtered and concentrated to obtain a crude product of target compound **8C** (0.90 g, >100%), which was directly used in the next reaction.

LC-MS (ESI):m/z = 276.2 $[M+H]^+$.

Step 3:

Compound **8C** (0.237 g, crude product) and compound **4B** (0.19 g, 0.69 mmol) were weighed and added to a reaction flask, then DMF (10 mL) and cesium carbonate (0.33 g, 1.72 mmol) were added. After the addition was completed, the resulting mixture was heated to 100°C and reacted for 1 h, and the reaction was monitored by LCMS. After the reaction was completed, water (50 mL) was added to the reaction solution, then the resulting mixture was extracted with ethyl acetate (100 mL*2). The organic phases were combined, washed with water and saturated aqueous sodium chloride solution, dried over anhydrous sodium sulfate, filtered, dried over anhydrous sodium sulfate, filtered and concentrated to obtain a crude product, which was purified by Pre-TLC (DCM:MeOH = 10:1) to obtain the target compound 8 (72 mg, 18.38%).

LC-MS (ESI):m/z = 456.7 $[M+H]^+$.

$^1H$ NMR (400 MHz, DMSO) δ 8.87 - 8.75 (m, 1H), 8.68 - 8.66 (m, 1H), 7.54 - 7.49 (m, 2H), 7.19 - 7.11 (m, 5H), 4.49 - 4.45 (m, 1H), 4.19 (s, 2H), 4.06 - 4.00 (m, 2H), 3.93 - 3.89 (m, 1H), 3.81 (s, 4H), 3.10 - 3.04 m, 1H), 2.64 - 2.58 (m, 1H), 2.33 - 2.23 (m, 2H), 2.17 - 2.14 (m, 1H), 1.94 - 1.87 (m, 2H), 1.41 - 1.10 (m, 2H), 0.99 - 0.94 (m, 1H), 0.54 - 0.38 (m, 2H), 0.14 - 0.10 (m, 2H).

**Example 9:**

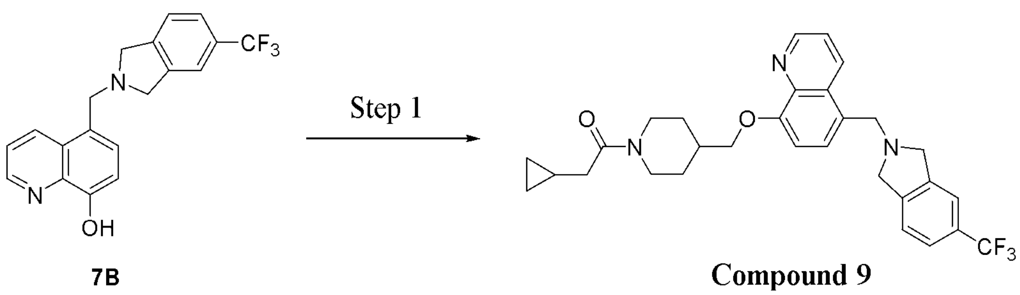


7B

Compound 9

Step 1:

Compound 7B (230 mg, 0.69 mmol), compound 8C (190 mg, 0.69 mmol) and cesium carbonate (330 mg, 1.03 mmol) were added to the reaction flask, then dissolved in DMF (10 ml) and the resulting mixture was reacted at 100°C for 1 h. After the reaction was completed as monitored by TLC, the reaction product was cooled to room temperature, diluted by adding water (100 ml), then the resulting mixture was extracted twice with ethyl acetate (100 ml*2). The organic phases were combined, dried over anhydrous sodium sulfate, concentrated, and purified by Pre-TLC (DCM:MeOH = 10:1) to obtain the target compound 9 (70 mg, 19.41%).

1H NMR (400 MHz, CDCl3) δ 8.90 - 8.88 (m, 1H), 8.62 - 8.59 (m, 1H), 7.40 - 7.33 (m, 3H), 7.19 - 7.17(m, 2H), 6.93 - 6.90 (m, 1H), 4.69 - 4.66 (m, 1H), 4.17 - 4.12 (m, 2H), 4.09 - 4.02 (m, 1H), 3.99 - 3.94 (m, 1H), 3.90 - 3.83 (m, 4H), 3.07 - 3.01 (m, 1H), 2.62 - 2.55 (m, 1H), 2.40 - 2.28 (m, 1H), 2.24 - 2.22 (m, 2H), 2.12 - 2.09 (m, 1H), 1.95 - 1.92 (m, 1H), 1.33 - 1.23 (m, 2H), 1.23 - 1.16 (m, 1H), 1.04 - 0.93 (m, 1H), 0.52 - 0.47(m, 2H), 0.13 - 0.10 (m, 2H).

LC-MS (ESI):m/z = 524.70 $[M+H]^+$.

**Example 10:**

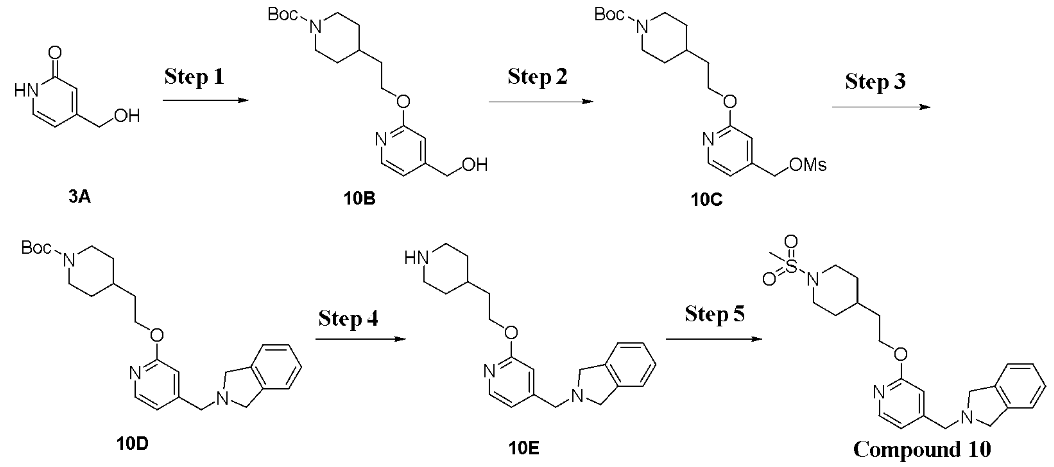


3A 10B 10C

10D 10E Compound 10

Step 1:

**3A** (1.0 g, 7.96 mmol), tert-butyl 4-(2-bromoethyl) piperidine-1-carboxylate (2.79 g, 9.55 mmol), and potassium carbonate (2.20 g, 15.92 mmol) were dissolved in N,N-dimethylformamide (20 mL), and the resulting mixture was reacted at 100°C overnight. After the reaction was cooled to room temperature, water (100 mL) was added to the reaction system to quench the reaction, and the resulting mixture was extracted with ethyl acetate (100 mL*2). The organic phase was dried, concentrated and separated by column chromatography (PE/EA = 80%/20%) to obtain **10B** (820 mg, 30%).

LC-MS (ESI): m/z= 337.2 $[M+H]^+$.

Step 2:

**10B** (820 mg, 2.44 mmol) was dissolved in dichloromethane (10 mL), and triethylamine (617 mg, 6.10 mmol) and methylsulfonyl chloride (559 mg, 4.88 mmol) were added dropwise at 0-5°C. After the addition was completed, the resulting mixture was reacted at room temperature overnight. Water (20 mL) was added to the reaction system to quench the reaction, the resulting mixture was extracted with dichloromethane (20 mL*2), and the organic phase was dried to obtain crude product **10C.**

LC-MS (ESI): m/z= 359.3 $[M+H]^+$.

Step 3:

**10C** (600 mg, 1.45 mmol) was dissolved in dichloromethane (5 mL), and triethylamine (293 mg, 2.90 mmol) and 2,3-dihydroisoindole hydrochloride (270 mg, 1.74 mmol) were added dropwise at 0-5°C. After the addition was completed, the resulting mixture was reacted at room temperature overnight. The reaction was concentrated and separated by column chromatography (PE/EA=70%/30%) to obtain **10D** (250 mg, 39%)

LC-MS (ESI): m/z= 438.2 $[M+H]^+$.

Step 4:

**10D** (250 mg, 0.57 mmol) was dissolved in dichloromethane (6 mL), and trifluoroacetic acid (1.5 mL) was added dropwise at 0-5°C. After the addition was completed, the resulting mixture was reacted at room temperature for 2 h. The reaction was concentrated to obtain crude product **10E.**

LC-MS (ESI): m/z= 338.5 $[M+H]^+$.

Step 5:

**10E** (190 mg, 0.56 mmol) was dissolved in dichloromethane (5 mL), and triethylamine (170 mg, 1.68 mmol) and methylsulfonyl chloride (128 mg, 1.12 mmol) were added dropwise at 0-5°C. After the addition was completed, the resulting mixture was reacted at room temperature for 2.5 h. After the reaction was concentrated, the residue was separated and purified by preparative liquid phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% trifluoroacetic acid); gradient: isocratic elution using 20%-70% acetonitrile; cycle time: 15 min) to obtain compound **10** (130 mg, 55%).

LC-MS (ESI): m/z= 416.2 [M+H]+.

1H NMR (400 MHz, DMSO-d6) δ 8.10-8.09 (d,1H), 7.24 - 7.17 (m, 4H), 6.99-6.98 (d,1H), 6.77 (s, 1H), 4.32-4.29 (t, 2H), 3.87-3.85 (m, 6H), 3.55-3.52 (d, 2H), 2.82 (s, 3H), 2.70-2.65 (m, 2H), 1.82-1.79 (d,2H), 1.71-1.66 (q, 2H), 1.62-1.55 (m, 1H), 1.28-1.18 (m, 2H).

**Example 11:**

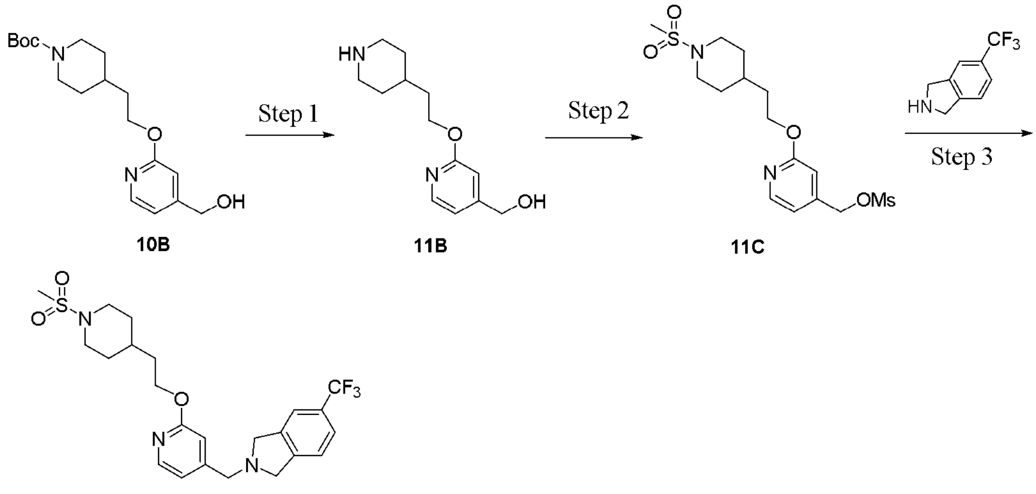


10B 11B 11C

Compound 11

Step 1:

Compound **10B** (0.33 g, 0.98 mmol) was dissolved in DCM (10 mL), and trifluoroacetic acid (3 mL) was added at room temperature, then the resulting mixture was stirred at room temperature for 1 h. The reaction solution was directly subjected to rotary evaporation, and the residue was used directly for the next step.
LC-MS (ESI): m/z= 237.3 [M+H]+

Step 2:

The crude **11B** was dissolved in DCM (15 mL), methylsulfonyl chloride (0.19 mL, 2.44 mmol) was then added, and the resulting mixture was reacted at room temperature for 16 h. After the reaction was completed, the reaction product was concentrated, and the resulting residue was purified by column chromatography (DCM:MeOH = 10:1) to obtain **11C** (0.35 g, 90%).

LC-MS (ESI): m/z= 393.5 [M+H]+.

Step 3:

Compound **11C** (350 mg, 0.89 mmol) and compound 5-trifluoromethylisoindoline hydrochloride (300 mg, 1.33 mmol) were dissolved in dichloromethane (15 mL), triethylamine (270 mg, 2.67 mmol) was then added and the resulting mixture was reacted at room temperature for 12 h. After the reaction was completed, the reaction product was concentrated, and the resulting crude product was purified by column chromatography (DCM:MeOH = 10:1) to obtain **compound 11** (95 mg, 22.07%).

LC-MS (ESI): m/z= 484.7 [M+H]+.

$^1$H NMR (400 MHz, $CDCl_3$) δ 8.10 (d, 1H), 7.54 - 7.41 (m, 2H), 7.29 (d, 1H), 6.93 (d, 1H), 6.78 (s, 1H), 4.35 (t, 2H), 4.00 (s, 4H), 3.88 (s, 2H), 3.79 (d, 2H), 2.76 (s, 3H), 2.65 (td, 2H), 1.88 (d, 2H), 1.77 (dd, 2H), 1.69 - 1.62 (m, 1H), 1.39 (ddd, 2H).

**Example 12:**

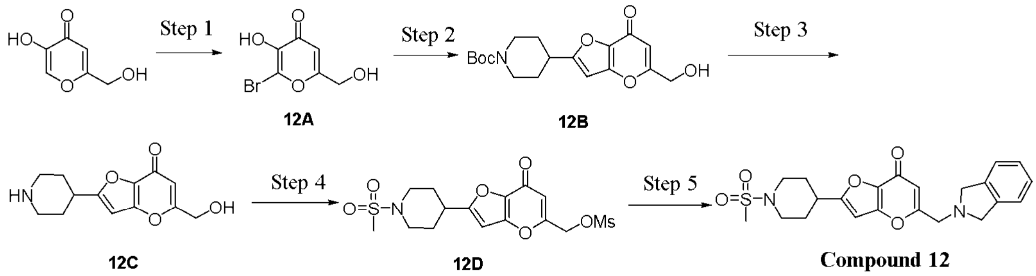


12A 12B 12C 12D Compound 12

Step 1:

Kojic acid (12.0 g, 84.42 mmol) was dissolved in tetrahydrofuran (100 mL), NBS (30.0 g, 168.61 mmol) and $NH_4OAc$ (3.25 g, 42.21 mmol) were successively added, and the reaction solution was warmed to 70°C and stirred for 6 h. After the reaction was completed, 150 mL of water was added to quench the reaction, and the resulting mixture was extracted with EA (100 mL × 3). The organic phases were combined, washed with saturated aqueous sodium chloride solution (200 mL × 1), dried over anhydrous sodium sulfate, filtered, concentrated under reduced pressure, and purified by column chromatography (DCM:MeOH = 1:0-10:1) to obtain compound **12A** (15.0 g, 80%).
LC-MS (ESI): m/z= 221.10 $[M+H]^+$.

Step 2:

At room temperature, the compound **12A** (5.0 g, 22.62 mmol), 1-Boc-4-ethynylpiperidine (4.73 g, 22.62 mmol), bis(triphenylphosphine)palladium dichloride (3.18 g, 4.52 mmol) and cuprous iodide (1.72 g, 9.05 mmol) were dissolved in tetrahydrofuran (100 mL), triethylamine (6.87 g, 67.86 mmol) was then added under nitrogen atmosphere, and the reaction solution was warmed to 40°C and stirred for 4 h. After the reaction was completed, the reaction product was filtered, and the filtrate was concentrated under reduced pressure and purified by column chromatography (DCM:MeOH = 1:0-10:1) to obtain compound **12B** (1.6 g, 20%).
LC-MS (ESI): m/z= 294.2 [M-*t*-Bu+H]$^+$.

Step 3:

Compound **12B** (1.6 g, 4.01 mmol) was dissolved in DCM (20 mL), trifluoroacetic acid (4 mL) was added, and the resulting mixture was reacted at room temperature for 2 h. After the reaction was completed, the reaction product was concentrated to obtain the compound **12C** (1.0 g, crude product), which was directly used in the next reaction without purification.
LC-MS (ESI): m/z= 250.2 $[M+H]^+$.

Step 4:

Compound **12C** (1.0 g, 4.01 mmol) and TEA (1.83 g, 4.50 mmol) were dissolved in DCM (20 mL), methylsulfonyl chloride (1.48 g, 12.92 mmol) was then added, and the resulting mixture was reacted at room temperature for 3 h. The reaction solution was concentrated and the residue was purified by column chromatography (ethyl acetate:petroleum ether = 0:1-1:1) to obtain compound **12D** (450 mg, 28%).
LC-MS (ESI): m/z= 406.3 $[M+H]^+$.

Step 5:

Compound **12D** (300 mg, 0.74 mmol) and isoindole hydrochloride (160 mg, 1.04 mmol) were dissolved in acetonitrile (20 mL), DIPEA (290 mg, 2.22 mmol) was then added, and the reaction solution was stirred at room temperature overnight. After the reaction was completed, the reaction product was concentrated, and the resulting crude product was purified by column chromatography (DCM:MeOH = 1:0-10:1) to obtain **compound 12** (250 mg, 78%).
LC-MS (ESI): m/z= 429.5 $[M+H]^+$.
$^1H$ NMR (400 MHz, DMSO-d6) δ 7.42-7.30 (m, 4H), 6.89 (s, 1H), 6.68 (s, 1H), 4.75-4.45 (m, 6H), 3.68-3.58 (m, 2H), 3.07-2.96 (m, 1H), 2.94-2.84 (m, 5H), 2.16-2.05 (m, 2H), 1.80-1.64 (m, 2H).

**Example 13:**

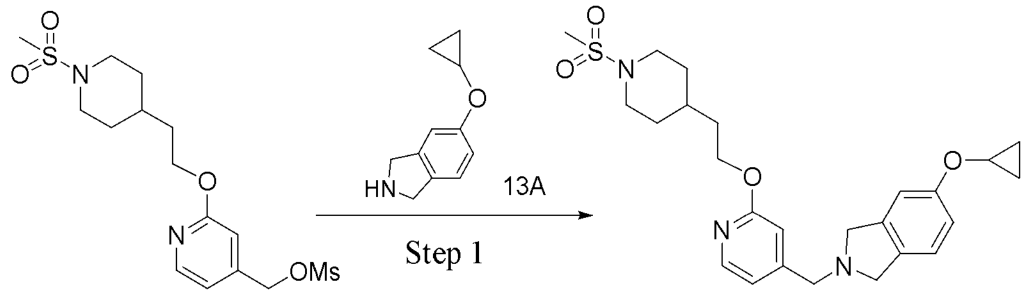


11C

Compound 13

Step 1:

Compound **11C** (350 mg, 0.89 mmol) and compound 13A (280 mg, 0.97 mmol) were dissolved in dichloromethane (15 mL), triethylamine (270 mg, 2.67 mmol) was then added and the resulting mixture was reacted at room temperature for 16 h. After the reaction was completed, the reaction product was concentrated, and the resulting crude product was purified by column chromatography (DCM:MeOH = 20:1) to obtain **compound 13** (45 mg, 10.7%).
LC-MS (ESI): m/z= 472.7 $[M+H]^+$.
$^1H$ NMR (400 MHz, $CDCl_3$) δ 8.09 (d, 1H), 7.08 (d, 1H), 6.93 (d, 1H), 6.88 (d, 2H), 6.78 (s, 1H), 4.34 (t, 2H), 3.91 (d, 4H), 3.85 (s, 2H), 3.79 (d, 2H), 3.70 (dt, 1H), 2.76 (s, 3H), 2.65 (td, 2H), 1.88 (d, 2H), 1.76 (q, 2H), 1.66 (ddd, 1H), 1.39 (ddd, 2H), 0.75 (d, 4H).

**Example 14:**

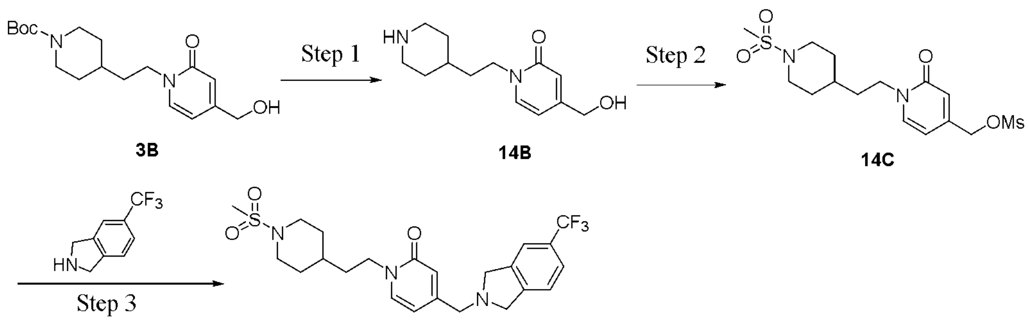


3B

14B

14C

Compound 14

Step 1:

**3B** (0.33 g, 0.98 mmol) was dissolved in DCM (10 mL), and trifluoroacetic acid (3 mL) was added at room temperature, then the resulting mixture was stirred at room temperature for 1 h. The reaction solution was directly subjected to rotary evaporation, and the residue was used directly for the next step.
LC-MS (ESI): m/z= 237.3 $[M+H]^+$.

Step 2:

Compound **14B** (0.23 g, 0.98 mmol) and TEA (0.45 g, 4.40 mmol) were dissolved in DCM (15 mL), methylsulfonyl chloride (0.19 mL, 2.44 mmol) was then added, and the resulting mixture was reacted at room temperature for 16 h. After the reaction was completed, the reaction product was concentrated, and the resulting residue was purified by column chromatography (DCM:MeOH = 10:1) to obtain **14C** (0.25 g, 65%).
LC-MS (ESI): m/z= 393.5 $[M+H]^+$.

Step 3:

Compound **14C** (250 mg, 0.64 mmol) and compound 5-trifluoromethylisoindoline hydrochloride (210 mg, 0.96 mmol) were dissolved in dichloromethane (15 mL), triethylamine (190 mg, 1.92 mmol) was then added and the resulting mixture was reacted at room temperature for 16 h. After the reaction was completed, the reaction product was concentrated, and the resulting crude product was purified by column chromatography (DCM:MeOH = 10:1) to obtain **compound 14** (105 mg, 33.93%).
LC-MS (ESI): m/z=484.1 $[M+H]^+$.
$^1H$ NMR (400 MHz, $CDCl_3$) δ 7.48 (d, 1H), 7.45 (s, 1H), 7.30 (d, 1H), 7.22 (d, 1H), 6.56 (s, 1H), 6.32 (dd, 1H), 4.03 - 3.94 (m, 6H), 3.80 (d, 2H), 3.73 (s, 2H), 2.77 (s, 3H), 2.66 (dd, 2H), 1.89 (d, 2H), 1.73 (dd, 2H), 1.54 - 1.31 (m, 3H).

**Example 15:**

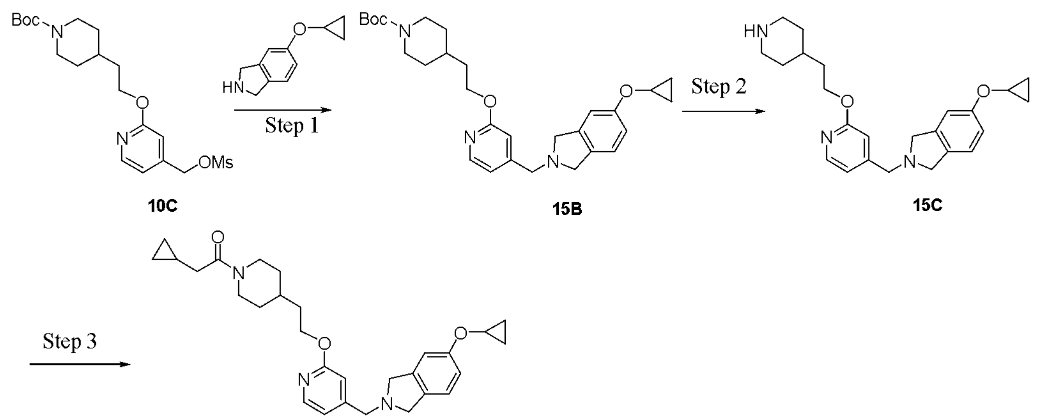


10C
15B
15C
Compound 15

Step 1:

Compound **10C** (350 mg, 0.84 mmol) and compound 5-cyclopropoxyisoindoline hydrochloride (290 mg, 1.01 mmol) were dissolved in dichloromethane (15 mL), triethylamine (340 mg, 3.36 mmol) was then added and the resulting mixture was reacted at room temperature for 16 h. After the reaction was completed, the reaction product was concentrated, and the resulting crude product was purified by column chromatography (PE:EA = 1:1) to obtain compound **15B** (350 mg, 84.41%).
LC-MS (ESI): m/z= 494.6 $[M+H]^+$.

Step 2:

Compound **15B** (350 mg, 0.71 mmol) was dissolved in dichloromethane (5 mL), hydrogen chloride dioxane solution (10 mL, 4.0 mmol/L) was then added, and the resulting mixture was reacted at room temperature for 2 h. After the reaction was completed, the reaction product was concentrated to obtain compound **15C** (250 mg, 84.41%).
LC-MS (ESI): m/z= 394.4 $[M+H]^+$.

Step 3:

Compound **15C** (250 mg, 0.64 mmol) and triethylamine (200 mg, 1.95 mmol) were dissolved in dichloromethane (15 mL). In an ice-water bath, cyclopropylacetyl chloride (150 mg, 1.27 mmol) was added dropwise. After the addition was completed, the resulting mixture was reacted at room temperature for 2 h. After the reaction was completed, the reaction product was concentrated and then purified by silica gel column chromatography (dichloromethane:methanol = 20:1) to obtain compound **15** (50 mg, 16.4%).
LC-MS (ESI): m/z= 476.3 $[M+H]^+$.
$^1H$ NMR (400 MHz, $CDCl_3$) δ 8.10 (d, 1H), 7.09 (d, 1H), 6.96 (d, 1H), 6.92 - 6.85 (m, 2H), 6.80 (s, 1H), 4.63 (d, 1H), 4.34 (t, 2H), 3.95 (d, 2H), 3.91 - 3.77 (m, 2H), 3.70 (ddd, 1H), 3.00 (dd, 2H), 2.55 (dd, 2H), 2.27 (d, 2H), 1.80-1.78 (m, 5H), 1.32 - 1.27 (m, 2H), 1.23 - 1.14 (m, 2H), 1.06-1.02 (m, 2H), 0.76 (dd, 2H), 0.58 - 0.52 (m, 2H), 0.17 (q, J = 4.8 Hz, 2H).

**Example 16:**

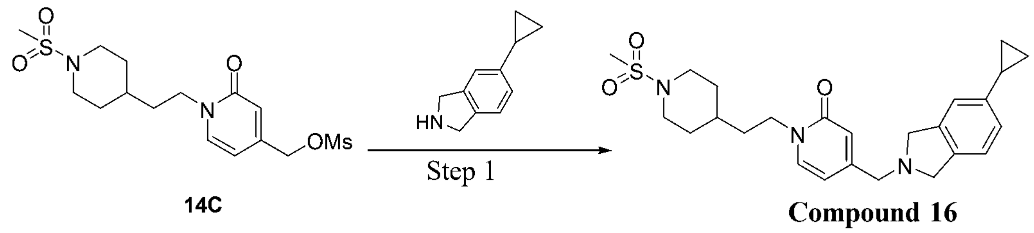


Step 1:

Compound **14C** (300 mg, 0.76 mmol) and compound 5-cyclopropylisoindoline hydrochloride (220 mg, 1.14 mmol) were dissolved in dichloromethane (15 mL), triethylamine (0.31 mg, 3.06 mmol) was then added and the resulting mixture was reacted at room temperature for 16 h. After the reaction was completed, the reaction product was concentrated, and the resulting crude product was purified by column chromatography (DCM:MeOH = 10:1) to obtain compound P0 (55 mg, 15.88%).
LC-MS (ESI): m/z=456.5 $[M+H]^+$
$^1H$ NMR (400 MHz, $CDCl_3$) δ 7.20 (d, 1H), 7.06 (d, 1H), 6.93 (d, 1H), 6.90 (s, 1H), 6.54 (s, 1H), 6.36 (d, 1H), 4.01 - 3.94 (m, 2H), 3.91 (s, 4H), 3.79 (d, 2H), 3.71 (s, 2H), 2.76 (s, 3H), 2.65 (t, 2H), 1.93 - 1.83 (m, 3H), 1.72 (dd, 3H), 1.44 - 1.33 (m, 2H), 0.96 - 0.89 (m, 2H), 0.68 - 0.61 (m, 2H).

**Example 17:**

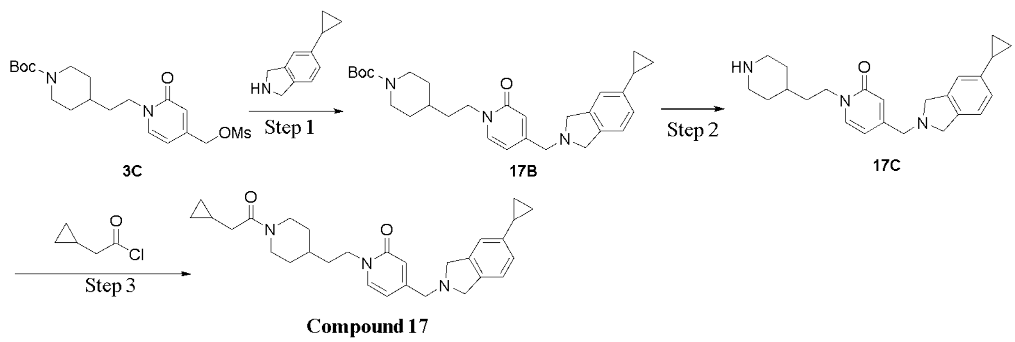


Step 1:

Compound **3C** (350 mg, 0.84 mmol) and compound 5-cyclopropylisoindoline hydrochloride (250 mg, 1.26 mmol) were dissolved in dichloromethane (15 mL), triethylamine (0.34 g, 3.36 mmol) was then added and the resulting mixture was reacted at room temperature for 16 h. After the reaction was completed, the reaction product was concentrated, and the resulting crude product was purified by column chromatography (DCM:MeOH = 10:1) to obtain compound **17B** (320 mg, 79.76%).
LC-MS (ESI): m/z= 478.4 $[M+H]^+$.

Step 2:

Compound **17B** (320 mg, 0.67 mmol) was dissolved in dichloromethane (5 mL), hydrogen chloride dioxane solution (10 mL, 4.0 mmol/L) was then added, and the resulting mixture was reacted at room temperature for 2 h. After the reaction was completed, the reaction product was concentrated to obtain compound **17C** (260 mg, 100%).
LC-MS (ESI): m/z= 378.6 $[M+H]^+$.

Step 3:

Compound **17C** (250 mg, 0.69 mmol) and triethylamine (209 mg, 2.06 mmol) were dissolved in dichloromethane (15 mL). In an ice-water bath, cyclopropylacetyl chloride (160 mg, 1.38 mmol) was added dropwise. After the addition was completed, the resulting mixture was reacted at room temperature for 2 h. After the reaction was completed, the reaction product was concentrated and then purified by silica gel column chromatography (dichloromethane:methanol = 20:1) to obtain **compound 17** (50 mg, 15.7%).

LC-MS (ESI): m/z= 460.7 $[M+H]^+$.

$^1H$ NMR (400 MHz, $CDCl_3$) δ 7.20 (d, 1H), 7.07 (d, 1H), 6.93 (d, 1H), 6.90 (s, 1H), 6.54 (s, 1H), 6.35 (d, 1H), 4.64 (d, 1H), 3.96 (td, 2H), 3.91 (s, 4H), 3.83 (d, 1H), 3.71 (s, 2H), 3.08 - 2.94 (m, 1H), 2.61 - 2.49 (m, 1H), 2.27 (d, 2H), 1.86 - 1.64 (m, 5H), 1.59 (ddd, 1H), 1.18 (dd, 2H), 1.10 - 0.99 (m, 1H), 0.97 - 0.91 (m, 2H), 0.70 - 0.61 (m, 2H), 0.61 - 0.51 (m, 2H), 0.17 (q, 2H)

**Example 18:**

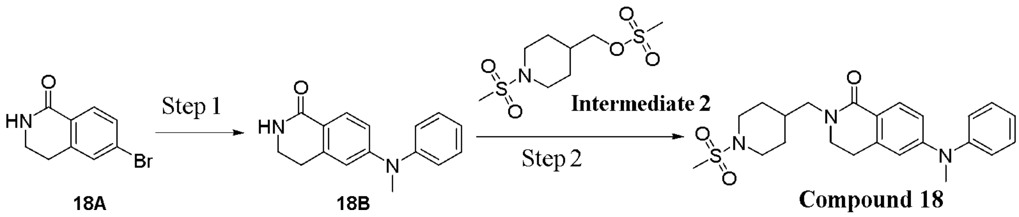


18A 18B Compound 18

Step 1:

**18A** (1 g, 4.42 mmol) and N-methylaniline (948 mg, 8.85 mmol) were dissolved in 1,4-dioxane (30 mL), RuPhos Pd G3 (370 mg, 0.44 mmol) and cesium carbonate (2.89 g, 8.85 mmol) were added and the resulting mixture was reacted at 100°C for 16 h. After the reaction was completed, water (50 mL) was added, and the resulting mixture was extracted with ethyl acetate (20 mL x 3). The organic phases were combined, concentrated, and purified by silica gel column chromatography (PE:EA = 1:1) to obtain the target compound **18B** (620 mg, 55.5%).
LCMS (ESI): m/z= 253.1 $[M+H]^+$

Step 2:

Compound **18B** (300 mg, 1.19 mmol) was dissolved in DMF (5 mL), sodium hydride (95 mg, 2.38 mmol, 40%) was added and stirred for 30 min, then **intermediate 2** (483 mg, 1.78 mmol) was added, and the resulting mixture was warmed to 60°C and stirred for 3 h. After the reaction was completed as monitored by TLC, water (20 mL) was added, and the resulting mixture was extracted with ethyl acetate (10 mL x 3). The organic phases were combined, concentrated, and separated and purified by preparative liquid phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% trifluoroacetic acid); gradient: isocratic elution using 20%-70% acetonitrile; cycle time: 15 min) to obtain compound **18** (100 mg, 19.7%).

LCMS (ESI): m/z= 428.2 $[M+H]^+$
$^1H$ NMR (400 MHz, DMSO-$d_6$) δ 7.66 (d, 1H), 7.45 - 7.35 (m, 2H), 7.24 - 7.13 (m, 3H), 6.74 - 6.67 (m, 1H), 6.63 (d, 1H), 3.59 - 3.51 (m, 2H), 3.48 (t, 2H), 3.34 (d, 2H), 3.32 - 3.29 (m, 3H, overlapped with $H_2O$ peak), 2.88 - 2.80 (m, 5H), 2.72 - 2.62 (m, 2H), 1.86 - 1.75 (m, 3H), 1.75 - 1.65 (m, 2H), 1.30 - 1.16 (m, 2H).

**Example 19:**

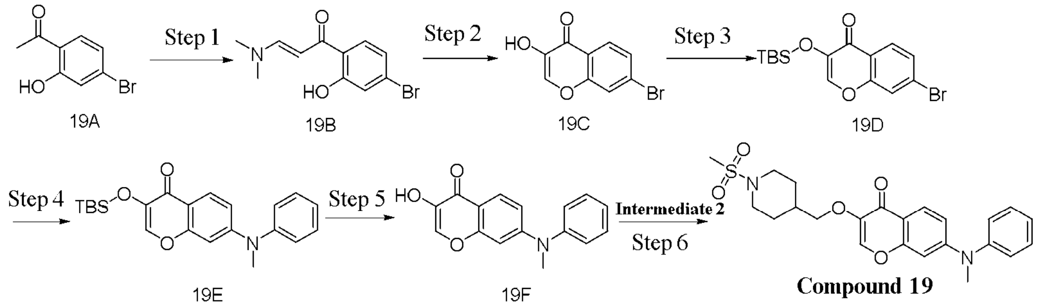


19A 19B 19C 19D

19E 19F Compound 19

Step 1:

**19A** (2 g, 9.3 mmol) was added to DMF-DMA (20 mL), and the resulting mixture was reacted at 100°C under stirring for 16 h. The reaction was complete as monitored by TLC, and the reaction product was concentrated to obtain a crude product of target compound **19B** (2.5 g, 99.5%), which was directly used in the next reaction.
LCMS (ESI): m/z= 270.1&272.1 [M+H]

Step 2:

Compound **19B** (2.5 g, 9.25 mmol) was dissolved in dichloromethane (50 mL), m-CPBA (4.8 g, 27.76 mmol) was slowly added at 0°C, and the resulting mixture was reacted at room temperature for 16 h. After the reaction was completed, the reaction product was filtered, and the filtrate was concentrated and separated by a silica gel chromatography column (PE:EA = 1:1) to obtain the target compound **19C** (1.1 g, two-step yield 49%).
LCMS (ESI): m/z= 241.1&243.1 [M+H]

Step 3:

Compound **19C** (1.1 g, 4.56 mmol) was dissolved in dichloromethane (20 mL), TBSCl (1.0 g, 6.85 mmol) and imidazole (465 mg, 6.85 mmol) were added, and the resulting mixture was reacted at room temperature for 1 h. After the reaction was completed, water (50 mL) was added, then the resulting mixture was extracted with dichloromethane (20 mL x 3). The organic phase was concentrated and separated by silica gel chromatography column (PE:EA = 7:1) to obtain the target compound **19D** (1.08 g, 66.6%).
LCMS (ESI): m/z= 355.1&357.1 [M+H]

Step 4:

**19D** (880 mg, 2.34 mmol) and N-methylaniline (500 mg, 4.68 mmol) were dissolved in toluene (20 mL), palladium acetate (87 mg, 0.23 mmol), BINAP (291 mg, 0.47 mmol) and cesium carbonate (1.5 g, 4.68 mmol) were added, and the resulting mixture was reacted at 100°C for 5 h. After the reaction was completed, water (50 mL) was added, and the resulting mixture was extracted with ethyl acetate (20 mL x 3). The organic phases were combined, concentrated, and purified by silica gel column chromatography (PE:EA = 2:1) to obtain the target compound **19E** (420 mg, 47.1%).
LCMS (ESI): m/z= 382.2 [M+H]

Step 5:

Compound **19E** (420 mg, 1.1 mmol) was dissolved in tetrahydrofuran (5 mL), tetrabutylammonium fluoride (1.7 mL, 1.7 mmol, 1 M) was added, and the resulting mixture was reacted at room temperature for 1 h. After the reaction was completed, water (10 mL) was added, and the resulting mixture was extracted with ethyl acetate (10 mL x 3) and concentrated to obtain the target compound **19F** (260 mg, 88.4%).
LCMS (ESI): m/z= 268.1 [M+H]

Step 6:

Compound **19F** (240 mg, 0.90 mmol) and intermediate 2 (487 mg, 1.80 mmol) were dissolved in acetonitrile (10 mL), cesium carbonate (585 mg, 1.80 mmol) was added and the resulting mixture was reacted at 80°C for 16 h. After the reaction was completed as monitored by TLC, water (30 mL) was added, and the resulting mixture was extracted with ethyl acetate (10 mL x 3). The organic phases were combined, concentrated, and separated and purified by preparative liquid

phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% ammonium bicarbonate); gradient: isocratic elution using 20%-70% acetonitrile; cycle time: 15 min) to obtain compound **19** (20 mg, 5.0%).

LCMS (ESI): m/z= 443.2 [M+H]

$^1$H NMR (400 MHz, DMSO-$d_6$) δ 8.11 (s, 1H), 7.80 (d, 1H), 7.53 - 7.45 (m, 2H), 7.35 - 7.25 (m, 3H), 6.79 - 6.72 (m, 1H), 6.71 - 6.64 (m, 1H), 3.83 - 3.72 (m, 2H), 3.64 - 3.52 (m, 2H), 3.40 - 3.32 (m, 3H, overlapped with $H_2O$ peak), 2.86 (s, 3H), 2.79 - 2.68 (m, 2H), 1.94 - 1.80 (m, 3H), 1.38 - 1.21 (m, 2H).

**Example 20:**

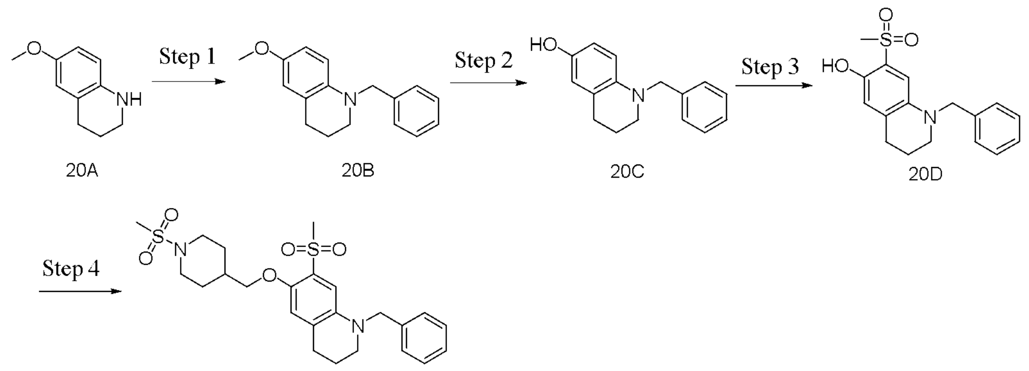


20A 20B 20C 20D

**Compound 20**

Step 1:

20A (10 g, 61.30 mmol), benzyl bromide (11.53 g, 67.43 mmol) and potassium carbonate (16.94 g, 122.6 mmol) were dissolved in N,N-dimethylformamide (60 mL), and the resulting mixture was stirred at 70°C for 2 h. After the reaction was completed, the reaction solution was diluted by adding water, and the resulting mixture was extracted with ethyl acetate 3 times. The organic phases were combined, subjected to rotary evaporation and slurried with petroleum ether to obtain product 20B (7.6 g, 49%).

LC-MS (ESI): m/z=254.20 $[M+H]^+$

Step 2:

20B (0.3 g, 1.18 mmol) was dissolved in dichloromethane (10 mL), then boron tribromide (1.03 g, 4.11 mmol) was added in an ice-water bath, and then the resulting mixture was stirred at room temperature for 4 h. The reaction solution was poured into water, then the resulting mixture was adjusted to basic with potassium carbonate, and the resulting product was extracted with ethyl acetate 3 times, subjected to rotary evaporation, then slurried with petroleum ether, and filtered to obtain product 20C (0.15 g, 53%).

LC-MS (ESI): m/z=240.30 $[M+H]^+$.

Step 3:

20C (0.5 g, 2.09 mmol), iodine (0.69 g, 2.72 mmol) and sodium methylsulfinate (0.28 g, 2.74 mmol) were dissolved in water (10 mL) and formic acid (2 mL) and then the resulting mixture was reacted at 110°C for 12 h. After the reaction was completed, the reaction product was diluted by adding water, and the resulting mixture was extracted with ethyl acetate 3 times. The organic phases were combined, subjected to rotary evaporation, and purified by normal phase chromatography (petroleum ether:ethyl acetate = 3:1) to obtain product 20D (0.2 g, 30%).

LC-MS (ESI): m/z=318.30 $[M+H]^+$.

Step 4:

20D (0.030 g, 0.095 mmol), (1-methanesulfonylpiperidin-4-yl)methyl methanesulfonate (0.036 g, 0.13 mmol) and potassium carbonate (0.033 g, 0.24 mmol) were dissolved in N,N-dimethylformamide (4 mL), and then the resulting

mixture was reacted at 80°C for 3 h. After the reaction was completed, the reaction product was diluted by adding water, and the resulting mixture was extracted with ethyl acetate 3 times. The organic phases were combined, subjected to rotary evaporation, and purified by HPLC. Preparative HPLC separation method: 1. Instrument: waters 2767 preparative liquid chromatographic instrument; chromatographic column: SunFire@ PrepC18 (19 mm × 250 mm); 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% ammonium acetate); b. gradient elution, mobile phase A: 30%-90%; c. flow rate: 20 mL/min. d. elution time: 15 min. retention time: 15 min. Compound 20 (7 mg, 15%) was obtained after lyophilization.

1H NMR (400 MHz, DMSO-d6) δ 7.39 - 7.30 (m, 2H), 7.27-7.21 (m, 3H), 6.94 (s, 1H), 6.86 (s, 1H), 4.45 (s, 2H), 3.92 (d, 2H), 3.58 (d, 2H), 3.36 (s, 1H), 3.12 (s, 3H), 2.85 (s, 3H), 2.84-2.79(m, 2H), 2.78-2.69 (m, 2H), 1.96 - 1.83 (m, 5H), 1.42-1.30 (m, 2H), 1.24 (s, 1H).

LC-MS (ESI): m/z=493.30[M+H]+.

**Example 21:**

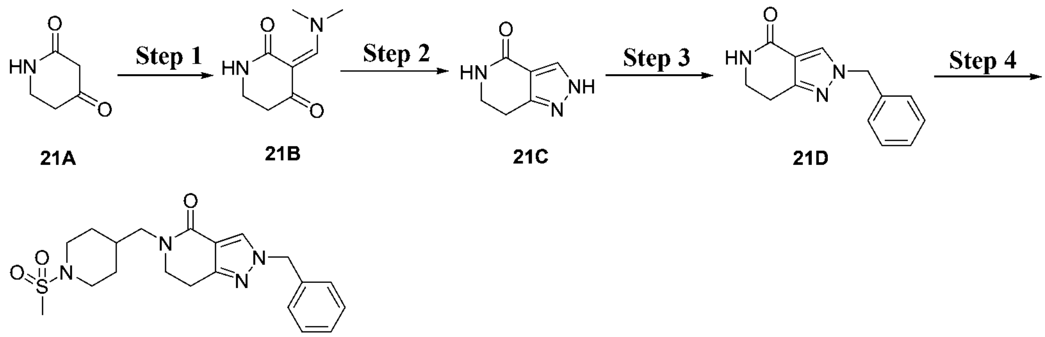

21A
21B
21C
21D

**Compound 21**

Step 1:

21A (2.0 g, 17.7 mmol) was dissolved in DMF-DMA (40 mL), and the resulting mixture was stirred at 90°C for 4 h. After the reaction was completed, the reaction solution was extracted with ethyl acetate three times, washed with saturated sodium chloride solution, dried over anhydrous sodium sulfate, concentrated under reduced pressure, and subjected to column chromatography (PE:EA = 2:1) to obtain compound 21B (2.4 g, 81%).
LCMS (ESI) : m/z= 169.2 [M+H]

Step 2:

Compound 21**B** (2.6 g, 15.46 mmol) was dissolved in ethanol (50 ml), $NH_2NH_2.H_2O$ (773 mg, 15.46 mmol) was added to the above reaction solution, and the resulting mixture was stirred at 100°C for 16 h. Column chromatography (MeOH:DCM=10:1) was performed to obtain the target compound **21C** (2.0 g, 94%).
LCMS (ESI) : m/z= 138.1 [M+H]

Step 3:

Compound 21**C** (200.0 mg, 1.46 mmol) was dissolved in tetrahydrofuran (4 mL), 60% sodium hydride (87.6 mg, 2.19 mmol) was added to the above reaction solution, and the resulting mixture was stirred at 25°C for 10 min. Benzyl bromide (299.6 mg, 1.75 mmol) was slowly added dropwise, and after the addition was completed, the reaction was transferred to 70°C and stirred for 16 h. After the reaction was completed, the reaction solution was extracted with ethyl acetate three times, washed with saturated sodium chloride solution, dried over anhydrous sodium sulfate, concentrated under reduced pressure, and subjected to column chromatography (MeOH:DCM=15:1) to obtain compound **21D** (120.0 mg, 36%).
LCMS (ESI) : m/z= 228.3 [M+H]

Step 4:

21**D** (120 mg, 0.53 mmol) was dissolved in tetrahydrofuran (4 mL), 60% sodium hydride (32.0 mg, 1.51 mmol) was added to the above reaction solution, and the resulting mixture was stirred at 25°C for 10 min. **Intermediate 2** (143.8 mg, 0.53 mmol) was slowly added, and after the addition was completed, the reaction was transferred to 70°C and stirred for 16 h. After the reaction was completed and the reaction was concentrated, the residue was separated and purified by preparative liquid phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% trifluoroacetic acid); gradient: isocratic elution using 20%-70% acetonitrile; cycle time: 15 min) to obtain the target **compound 21** (47 mg, 22%).

$^{1}H$ NMR (400 MHz, $CDCl_3$) δ 7.77 (s, 1H), 7.34 (t, 3H), 7.26 (d, 2H), 5.26 (s, 2H), 3.76 (d, 2H), 3.59 (t, 2H), 3.40 (d, 2H), 2.95 (t, 2H), 2.76 (s, 3H), 2.69 (t, 2H), 1.81 (d, 3H), 1.41 (dd, 2H).

LCMS (ESI) : m/z= 403.2 $[M+H]^+$.

**Example 22:**

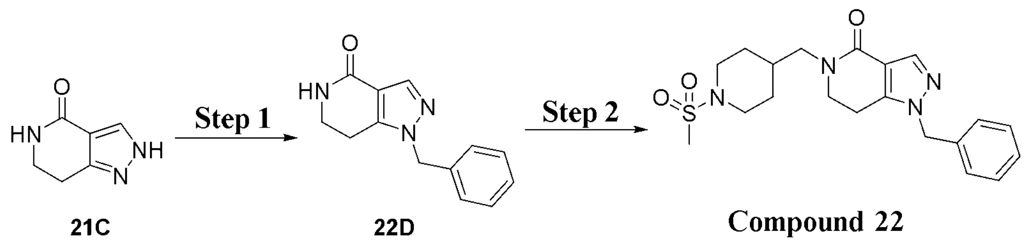


21C 22D Compound 22

Step 1:

Compound 21**C** (200.0 mg, 1.46 mmol) was dissolved in tetrahydrofuran (4 mL), 60% sodium hydride (87.6 mg, 2.19 mmol) was added to the above reaction solution, and the resulting mixture was stirred at 25°C for 10 min. Benzyl bromide (299.6 mg, 1.75 mmol) was slowly added dropwise, and after the addition was completed, the reaction was transferred to 70°C and stirred for 16 h. After the reaction was completed, the reaction solution was extracted with ethyl acetate three times, washed with saturated sodium chloride solution, dried over anhydrous sodium sulfate, concentrated under reduced pressure, and subjected to column chromatography (MeOH:DCM=15:1) to obtain compound **22D** (81.0 mg, 24%).

LCMS (ESI) : m/z= 228.3 $[M+H]^+$

Step 2:

22**D** (81.0 mg, 0.36 mmol) was dissolved in tetrahydrofuran (4 mL), 60% sodium hydride (21.7 mg, 0.54 mmol) was added to the above reaction solution, and the resulting mixture was stirred at 25°C for 10 min. **Intermediate 2** (97.7 mg, 0.36 mmol) was slowly added, and after the addition was completed, the reaction was transferred to 70°C and stirred for 16 h. After the reaction was completed and the reaction was concentrated, the residue was separated and purified by preparative liquid phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% trifluoroacetic acid); gradient: isocratic elution using 20%-70% acetonitrile; cycle time: 15 min) to obtain the target **compound 22** (29 mg, 14%).

$^{1}H$ NMR (400 MHz, $CDCl_3$) δ 7.90 (s, 1H), 7.37 - 7.28 (m, 3H), 7.15 (d, 2H), 5.29 (s, 2H), 3.76 (d, 2H), 3.56 (t, 2H), 3.37 (d, 2H), 2.79 (t, 2H), 2.76 (s, 3H), 2.69 (dd, 2H), 1.83 (d, 3H), 1.47 - 1.32 (m, 2H).

LCMS (ESI) : m/z= 403.2 $[M+H]^+$.

**Example 23:**

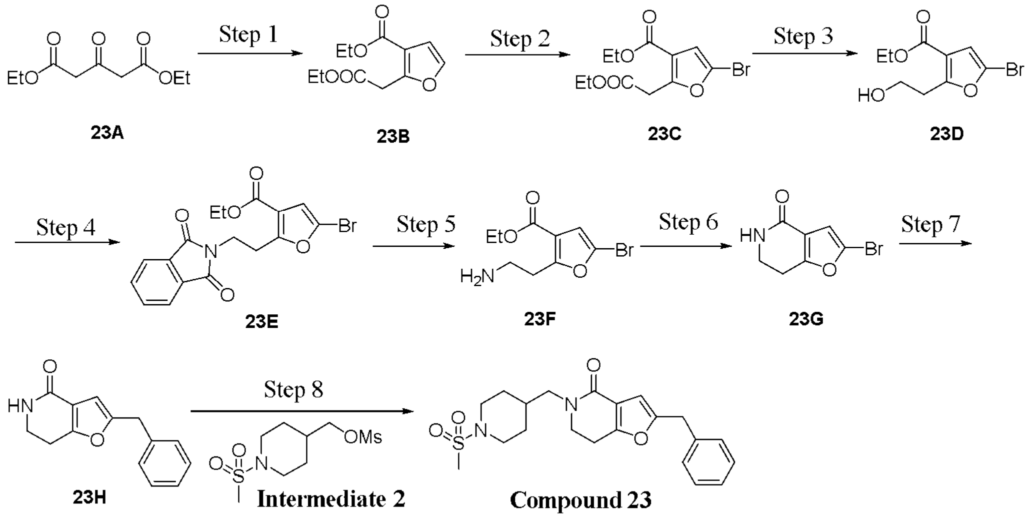


23A 23B 23C 23D

23E 23F 23G

23H Intermediate 2 Compound 23

Step 1:

23A (10.0 g, 49.5 mmol) was dissolved in pyridine (30 mL), and the resulting mixture was cooled to 0°C. Under $N_2$ atmosphere, 2-chloroacetaldehyde (4.7 g, 59.4 mmol) was added dropwise to the above reaction solution, and after the addition was completed, the resulting mixture was warmed to 50°C and reacted for 1.5 h. After the reaction was completed, water was added to quench the reaction, and the reaction solution was extracted with ethyl acetate three times, washed with saturated sodium chloride solution, dried over anhydrous sodium sulfate, concentrated under reduced pressure, and subjected to column chromatography (PE:EA = 4:1) to obtain compound 23B (5.7 g, 51%).
LCMS (ESI) : m/z= 227.2 $[M+H]^+$

Step 2:

Compound **23B** (5.6 g, 24.8 mmol) was dissolved in acetonitrile (60 mL), N-bromosuccinimide (5.3 g, 29.7 mmol) was added to the above reaction solution, and the resulting mixture was stirred at 25°C for 1.5 h. After the reaction was completed, the reaction product was concentrated under reduced pressure, and the pH was adjusted to neutral with saturated sodium bicarbonate. The resulting mixture was extracted with chloroform, the organic layer was concentrated under reduced pressure, and subjected to column chromatography (PE:EA = 3:1) to obtain the target compound **23C** (7.1 g, 94%).
LCMS (ESI) : m/z= 306.1 $[M+H]^+$

Step 3:

Compound **23C** (7.0 g, 22.9 mmol) was dissolved in ethanol (70 mL), sodium borohydride (2.6 mg, 68.7 mmol) was slowly added in an ice bath, and after 15 min the reaction was transferred to 25°C and stirred for 3 h. After the reaction was completed, the reaction was cooled to 0°C, the pH was adjusted to weakly acidic with 6 mol/L HCl, and the resulting mixture was extracted with chloroform and concentrated under reduced pressure. Column chromatography (PE:EA = 2:1) was performed to obtain compound **23D** (4.3 g, 71%).
LCMS (ESI) : m/z= 262.9 $[M+H]^+$

Step 4:

**23D** (4.1 g, 15.6 mmol), phthalimide (2.3 g, 15.6 mmol) and triphenylphosphine (4.1 g, 15.6 mmol) were dissolved in THF (40 mL). Under $N_2$ protection, the temperature was cooled to 0°C, and diisopropyl azodicarboxylate (3.2 g, 15.6 mmol) was added dropwise to the above reaction solution. After the dropwise addition was completed, the reaction was transferred to 25°C and stirred for 1 h. After the reaction was completed, saturated sodium bicarbonate was added to quench the reaction, the resulting mixture was extracted with chloroform, and the organic layer was concentrated under reduced pressure. Column chromatography (DCM:MeOH=12:1) was used for separation and purification to obtain the target compound **23E** (4.4 g, 72%).
LCMS (ESI) : m/z= 392.1 $[M+H]^+$

Step 5:

Compound **23E** (2.3 g, 5.8 mmol) was dissolved in ethanol (20 mL), and hydrazine hydrate (0.6 mL, 11.4 mmol) was added dropwise to the above reaction solution, and the resulting mixture was stirred at 50°C for 1 h. After the reaction was completed, the reaction solution was extracted with dichloromethane and methanol, dried over anhydrous sodium sulfate, concentrated under reduced pressure, and separated and purified by column chromatography (DCM:MeOH = 10:1) to obtain **23F** (1.4 g, 91%).
LCMS (ESI) : m/z= 262.1 $[M+H]^+$

Step 6:

1,5,7-triazidobicyclo(4.4.0)dec-5-ene (220.0 mg, 1.6 mmol) was added to a solution of **23F** (1.4 g, 5.3 mmol) in THF (6 mL), and the resulting mixture was stirred at 75°C for 30 min. After the reaction was completed, water was added to quench the reaction, and the resulting mixture was extracted with ethyl acetate, dried over anhydrous sodium sulfate, concentrated under reduced pressure, and subjected to column chromatography (PE:EA = 1:1) to obtain compound **23G** (780.0 mg, 68%).
LCMS (ESI) : m/z= 216.0 $[M+H]^+$

Step 7:

Under $N_2$ protection, **23G** (200.0 mg, 0.93 mmol), manganese (112.4 mg, 2.05 mmol) and TFA (0.1) were dissolved in acetonitrile (6 mL), and the resulting mixture was stirred for 10 min, then cobalt bromide (22.0 mg, 0.09 mmol) was added and the resulting mixture was stirred at 35°C for 4 h. After the reaction was completed, the reaction product was quenched with saturated ammonium chloride solution, and the resulting solution was extracted with ethyl acetate three times, washed with saturated sodium chloride solution, dried over anhydrous sodium sulfate, concentrated under reduced pressure, and subjected to column chromatography (PE:EA = 1:1) to obtain compound **23H** (121.0 mg, 57%).
LCMS (ESI) : m/z= 228.3 $[M+H]^+$

Step 8:

**23H** (121.0 mg, 0.53 mmol) was dissolved in THF (3 mL), 60% sodium hydride (25.0 mg, 0.63 mmol) was added to the above reaction solution at 0°C, and the resulting mixture was stirred for 15 min, and then **intermediate 2** (140.0 mg, 0.53 mmol) was added. After the addition was completed, the reaction was transferred to 70°C and stirred for 16 h. After the reaction was completed, the reaction was cooled to room temperature, and after the reaction was concentrated, the residue was separated and purified by preparative liquid phase chromatography (instrument: waters 2767 preparative liquid chromatographic instrument; chromatographic column: SunFire@ Prep C18 (19 mm × 150 mm); composition of mobile phase: Mobile phase A: acetonitrile; and mobile phase B: water (containing 0.1% trifluoroacetic acid); gradient: isocratic elution using 20%-70% acetonitrile; cycle time: 15 min) to obtain the target **compound 23** (37.0 mg, 17%).
$^1H$ NMR (400 MHz, CDCl3) δ 7.34 - 7.28 (m, 2H), 7.25 - 7.21 (m, 3H), 6.34 (s, 1H), 3.94 (s, 2H), 3.77 (d, 2H), 3.60 (t, 2H), 3.36 (d, 2H), 2.91 (t, 2H), 2.77 (s, 3H), 2.69 (td, 2H), 1.86 - 1.77 (m, 3H), 1.39 (dt, 2H).
LCMS (ESI) : m/z= 403.2 $[M+H]^+$.

**Example 24:**

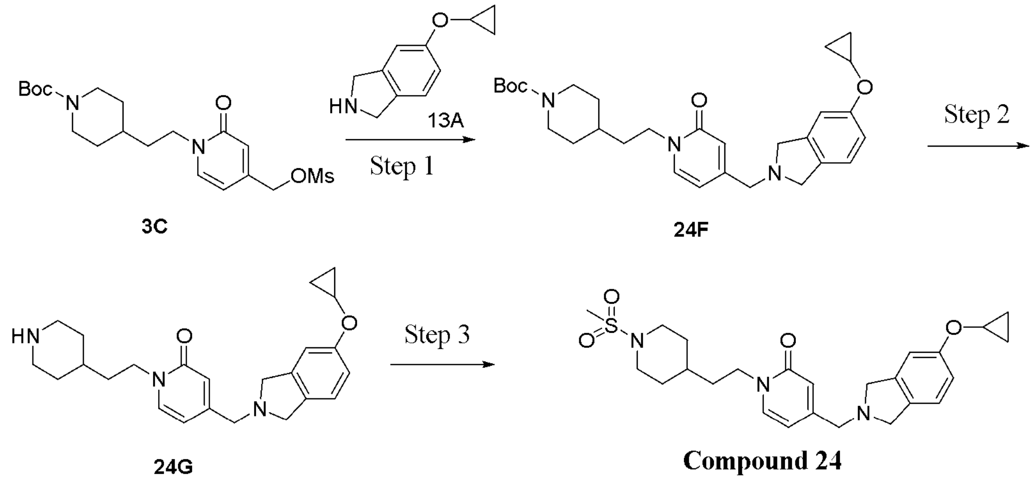


3C

24F

24G

Compound 24

Step 1:

Compound **3C** (1.2 g, 2.89 mmol) and compound **13A** (0.92 g, 3.18 mmol) were dissolved in DCM (20 mL), triethylamine (0.73 g, 7.23 mmol) was then added, and the reaction solution was stirred at room temperature for 3 h. After the reaction was completed, 50 mL of water was added to quench the reaction, and the resulting mixture was extracted with DCM (50 mL × 2). The organic phase was dried over anhydrous sodium sulfate, filtered and concentrated. The resulting crude product was purified by column chromatography (PE:EA = 1:0-1:1) to obtain compound **24F** (0.75 g, 53%).
LC-MS (ESI): m/z= 494.3 $[M+H]^+$.

Step 2:

Compound **24F** (750 mg, 1.56 mmol) was dissolved in dichloromethane (15 mL), trifluoroacetic acid (3 mL) was then added, and the reaction solution was stirred at room temperature for 2 h. After the reaction was completed, the reaction product was concentrated to obtain compound **24G** (0.80 g, crude product), which was directly used in the next reaction without purification.
LC-MS (ESI): m/z= 394.5 $[M+H]^+$.

Step 3:

Compound **24G** (0.36 g, 0.91 mmol) was dissolved in dichloromethane (10 mL), triethylamine (0.28 g, 2.77 mmol) and methylsulfonyl chloride (0.16 g, 1.39 mmol) were successively added in an ice-water bath, and the reaction solution was warmed to room temperature and stirring was continued for 3 h. After the reaction was completed, the reaction product was concentrated, and the crude product was purified by high performance preparative liquid chromatography to obtain compound **24** (15 mg, 3%).
Preparation method: Instrument: waters 2767 preparative liquid chromatographic instrument; chromatographic column: SunFire @ Prep C18 (19 mm × 250 mm). The sample was dissolved in DMF and filtered with a 0.45 μm filter to prepare a sample liquid. Preparative chromatography conditions: composition of mobile phases A and B: Mobile phase A: acetonitrile, and mobile phase B: water (containing 1% trifluoroacetic acid), gradient elution, mobile phase A: 15%-60%, flow rate: 12 mL/min. elution time: 20 min.
LC-MS (ESI): m/z= 472.2 $[M+H]^+$.
$^1$H NMR (400 MHz, DMSO) δ 7.82 (d, 1H), 7.30 (d, 1H), 7.13 (s, 1H), 7.02 (d, 1H), 6.58 (s, 1H), 6.37 (d, 1H), 4.80-4.35 (m, 6H), 3.93 (t, 2H), 3.86-3.80 (m, 1H), 3.55-3.51 (m, 2H), 2.84 (s, 3H), 2.69-2.62 (m, 2H), 1.86-1.76 (m, 2H), 1.64-1.54 (m, 2H), 1.40-1.30 (m, 1H), 1.27-1.16 (m, 2H), 0.80-0.74 (m, 2H), 0.66-0.60 (m, 2H).

**Example 25:**

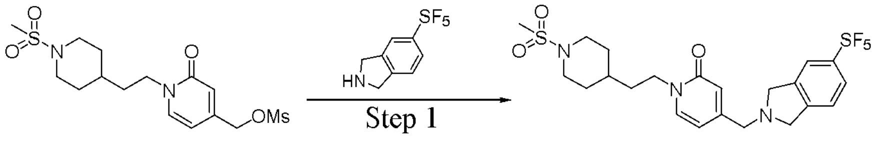


14C Compound 25

Step 1:

Compound **14C** (100 mg, 0.25 mmol) and compound 5-sulfur pentafluoride isoindoline hydrochloride (74 mg, 0.30 mmol) were dissolved in dichloromethane (15 mL), triethylamine (100 mg, 1.0 mmol) was then added and the resulting mixture was reacted at room temperature for 16 h. After the reaction was completed, the reaction product was concentrated, and the resulting crude product was purified by column chromatography (DCM:MeOH = 10:1) to obtain compound 25 (8 mg, 5.9%).
LC-MS (ESI): m/z=542.1 $[M+H]^+$.
$^1$H NMR (400 MHz, DMSO) δ 7.82 (d, 1H), 7.75 (dd, 1H), 7.65 (d, 1H), 7.47 (d, 1H), 6.35 (s, 1H), 6.25 (dd,1H), 3.99 - 3.86 (m, 6H), 3.71 (s, 2H), 3.52 (d, 2H), 2.83 (s, 3H), 2.65 (dd,2H), 1.81 (d, 2H), 1.58 (dd, 2H), 1.33 (d, 1H), 1.23 - 1.10 (m, 2H).

**Example 26:**

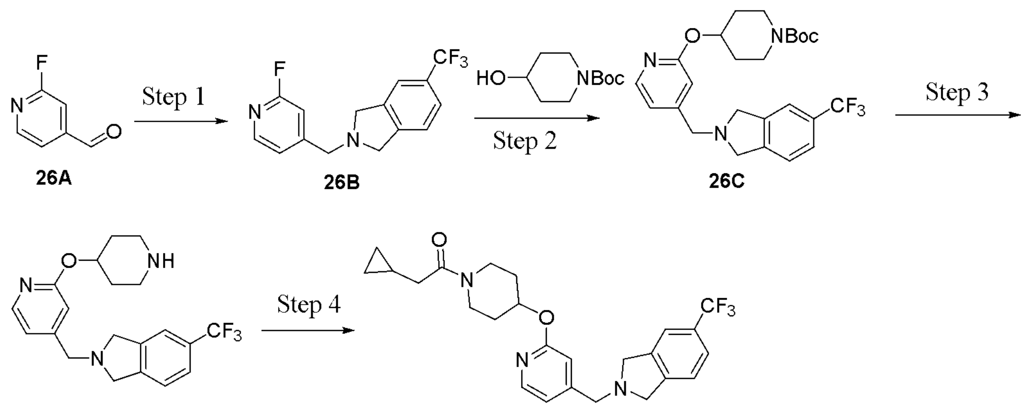


26A 26B 26C

26D 26

Step 1:

**26A** (2.0 g, 16.0 mmol) was dissolved in DCE (20 mL), 5-trifluoromethylisoindoline hydrochloride (3.5 g, 16.0 mmol) and sodium borohydride acetate (6.6 g, 32.0 mmol) were then added, and finally glacial acetic acid (0.6 g, 10.0 mmol) was added, and the resulting mixture was reacted at room temperature for 12 h. After the reaction was completed, saturated sodium biarbconate solution (30 mL) was added to the reaction solution, and the resulting mixture was extracted with dichloromethane (20 mL × 2). The organic layers were combined, washed with saturated aqueous sodium chloride solution (25 mL × 1), dried over anhydrous sodium sulfate, filtered, and concentrated. The residue was separated and purified by silica gel column chromatography (PE/EA = 4/1) to obtain **26B** (4.0 g, 84.3%).
LC-MS (ESI): m/z = 297.1 $[M+H]^+$.

Step 2:

N-Boc-4-hydroxypiperidine (0.8 g, 4.0 mmol) was dissolved in DMF (20 mL), sodium hydride (0.16 g, 4.0 mmol) was then added, and the resulting mixture was stirred at room temperature for 0.5 h, and then **26B** (0.6 g, 2.0 mmol) was added. The resulting mixture was reacted overnight at room temperature, quenched with a small amount of methanol, and concentrated. The residue was separated and purified by silica gel column chromatography (PE/EA = 4/1) to obtain **26C** (0.5 g, 50.1%).
LC-MS (ESI): m/z = 478.2 $[M+H]^+$.

Step 3:

Compound **26C** (0.5 g, 1.0 mmol) was dissolved in dichloromethane (4.0 mL), trifluoroacetic acid (3.0 mL) was then added, and the mixture was reacted at room temperature for half an hour and then concentrated to obtain compound **26D** (0.5 g, 96%), which was directly used in the next step.
LC-MS (ESI): m/z = 378.2 $[M+H]^+$.

Step 4:

**26D** (0.5 g, 1.0 mmol) was dissolved in DMF (8.0 mL), and cyclopropylacetic acid (121.2 mg, 1.2 mmol) and N-methylimidazole (327.8 mg, 4.0 mmol) were added. At 0°C, N,N,N',N'-tetramethylchloroformamidinium hexafluorophosphate (336.0 mg, 1.1 mmol) was added and the resulting mixture was stirred at room temperature for 20 min. After the reaction was completed, water (20 mL) was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (dichloromethane: methanol (v/v)=20:1) to obtain **compound 26** (150.0 mg, 32%).
LC-MS (ESI): m/z = 460.2 $[M+H]^+$.
$^1H$ NMR (400 MHz, $CDCl_3$) δ 8.09 (d, 1H), 7.52 - 7.38 (m, 2H), 7.29 (d, 1H), 6.93 - 6.91 (m, 1H), 6.77 (s, 1H), 5.83 - 5.28 (m, 1H), 3.96 - 3.93 (m, 5H), 3.87 (s, 2H), 3.78 - 3.64 (m, 1H), 3.59 - 3.46 (m, 1H), 3.46 - 3.32 (m, 1H), 2.31 (d, 2H), 2.06 - 1.93 (m, 2H), 1.80 - 1.77 (m, 2H), 1.14 - 0.97 (m, 1H), 0.63 - 0.51 (m, 2H), 0.19 (q, 2H).
$^{19}F$ NMR (377 MHz, $CDCl_3$) δ -60.02 (s).

**Example 27:**

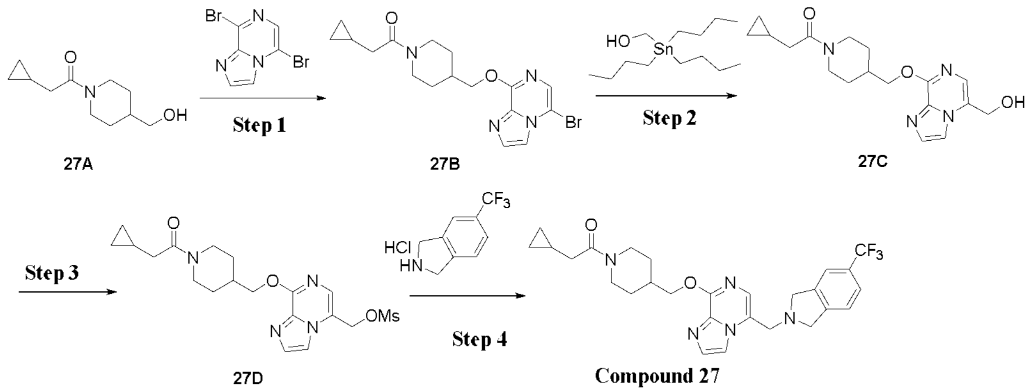


27A 27B 27C 27D Compound 27

Step 1:

27A (1.2 g, 6.1 mmol) (synthesized according to patent EP 2881384 A1) was weighed, added to a 100 mL eggplant flask, dissolved in 20 mL of DMF, and cooled in ice-water bath. Then NaH (60% in kerosene) (480 mg, 12 mmol) was added, and the resulting mixture was reacted at this temperature for 30 min. 5,8-dibromoimidazo[1,2-a]pyrazine (1.7 g, 6.0 mmol) was added, and the reaction was continued for 1 h. After the raw materials completely disappeared, saturated ammonium chloride solution was added until the system was weakly acidic, and the resulting mixture was filtered. The filter residue was washed with water and dried to obtain the target compound 27B (1.84 g, yield: 78.2%).
LC-MS (ESI): m/z = 393.3, 393.5$[M+H]^+$.

Step 2:

Compound 27B (1.84 g, 4.7 mmol) was dissolved in 1,4-dioxane (30 mL), then (tributyltin)methanol (1.6 g, 5 mmol) and chloro(2-dicyclohexylphosphino-2',4',6'-triisopropyl-1,1'-biphenyl)[2-(2'-amino-1,1'-biphenyl)]palladium(II) (Xphos-pd-G2) (390 mg, 0.5 mmol) were added, the obtained system was subjected to nitrogen replacement and then reacted at 90°C for 4 h. After cooling, the reaction product was concentrated, and the resulting residue was purified by silica gel column chromatography (petroleum ether:ethyl acetate (v:v) = 1:1) to obtain compound 27C (722 mg, yield: 44.5%) LC-MS (ESI): m/z = 345.4$[M+H]^+$.

Step 3:

Compound 27C (344 mg, 1 mmol) was dissolved in dichloromethane (10 mL), then triethylamine (1 mL) and methylsulfonyl chloride (125 mg, 1.1 mmol) were added and the resulting mixture was stirred at room temperature for 1 h. The resulting mixture was concentrated directly and the resulting residue redissolved with ethyl acetate, filtered, and the filtrate was concentrated to obtain the target compound 27D (185 mg, crude product).

Step 4:

Compound 27D (185 mg, crude product) was dissolved in DMF (5 mL), 5-(trifluoromethyl)isoindoline hydrochloride (112 mg, 0.5 mmol) and potassium carbonate (138 mg, 1 mmol) were added, and the resulting mixture was reacted at 70°C for 2 h. Water (20 mL) was added, and ethyl acetate (30 mL × 3) was used for extraction, the organic phase was collected, washed with saturated aqueous sodium chloride solution, and dried over anhydrous sodium sulfate, then filtered, the filtrate was collected, and concentrated, the resulting residue was purified by column chromatography (dichloromethane:anhydrous methanol (v:v) = 1:0-5:1) to obtain compound 27 (87 mg, yield: 33.8%).

$^1$H NMR (400 MHz, $CDCl_3$) δ7.97 - 7.94 (m, 1H), 7.67 - 7.65 (m, 1H), 7.50 - 7.46 (m, 1H), 7.44 (s, 1H), 7.33 (s, 1H), 7.30 - 7.26 (m, 1H), 4.76 - 4.66 (m, 1H), 4.49 - 4.32 (m, 2H), 4.13 - 4.09 (m, 2H), 4.03 - 3.98 (m, 4H), 3.93 - 3.86 (m, 1H), 3.12 - 3.03 (m, 1H), 2.68 - 2.58 (m, 1H), 2.34 - 2.20 (m, 3H), 2.12 - 1.90 (m, 2H), 1.40 - 1.27 (m, 2H), 1.13 - 0.99 (m, 1H), 0.62 - 0.50 (m, 2H), 0.27 - 0.15 (m, 2H).

LC-MS (ESI): m/z = 514.2$[M+H]^+$

**Example 28:**

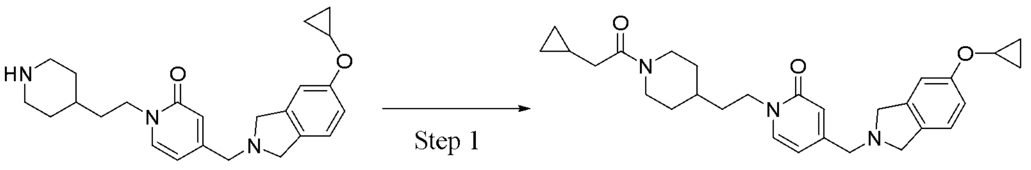


24G

Compound 28

Step 1:

Compound **24G** (0.36 g, 0.91 mmol) was dissolved in dichloromethane (10 mL), triethylamine (0.37 g, 3.64 mmol) and cyclopropylacetyl chloride (0.22 g, 1.82 mmol) were successively added in an ice-water bath, and the reaction solution was warmed to room temperature and stirring was continued for 5 h. After the reaction was completed, the reaction product was concentrated, and the crude product was purified by high performance preparative liquid chromatography to obtain compound **28** (20 mg, 4%).

Preparation method: Instrument: waters 2767 preparative liquid chromatographic instrument; chromatographic column: SunFire @ Prep C18 (19 mm × 250 mm). The sample was dissolved in DMF and filtered with a 0.45 μm filter to prepare a sample liquid. Preparative chromatography conditions: composition of mobile phases A and B: Mobile phase A: acetonitrile, and mobile phase B: water (containing 1% trifluoroacetic acid), gradient elution, mobile phase A: 20%-60%, flow rate: 12 mL/min. elution time: 20 min.

LC-MS (ESI): m/z= 476.3 $[M+H]^+$.

$^1$H NMR (400 MHz, DMSO) δ 7.83 (d, 1H), 7.32 (d, 1H), 7.13 (s, 1H), 7.03 (d, 1H), 6.58 (s, 1H), 6.37 (d, 1H), 4.70-4.30 (m, 7H), 3.93 (t, 2H), 3.88-3.76 (m, 3H), 3.01-2.87 (m, 1H), 2.23 (d, 2H), 1.78-1.67 (m, 2H), 1.60-1.44 (m, 3H), 1.15-0.90 (m, 3H), 0.82-0.75 (m, 2H), 0.65-0.60 (m, 2H), 0.48-0.40 (m, 2H), 0.14-0.08 (m, 2H).

**Example 29:**

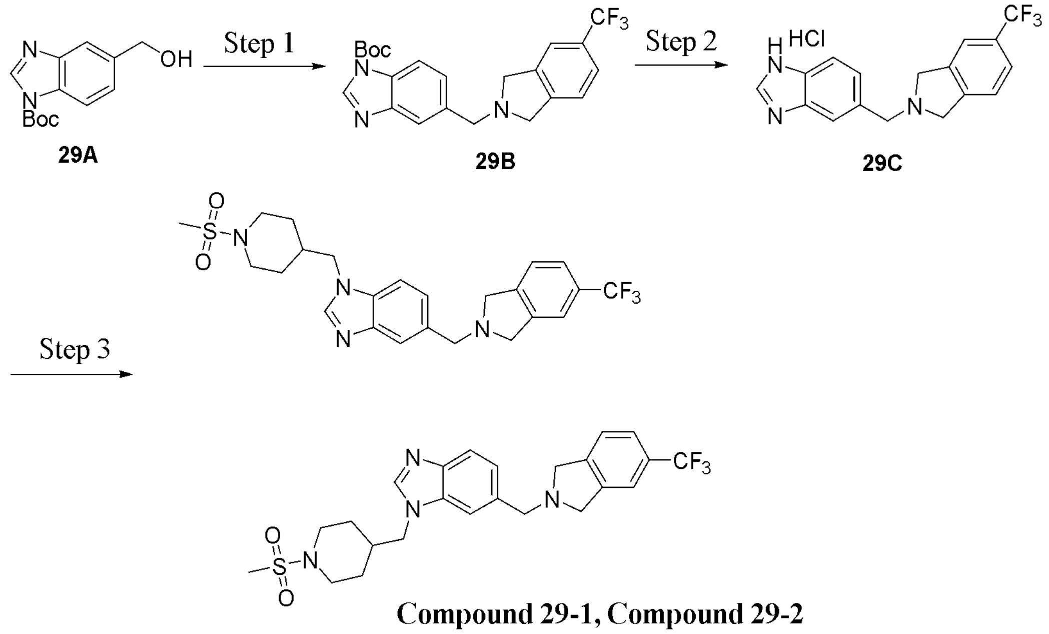


29A 29B 29C

Compound 29-1, Compound 29-2

Step 1:

**29A** (550 mg, 2.22 mmol), triethylamine (338 mg, 3.32 mmol) and methanesulfonic anhydride (579 mg, 3.32 mmol) were dissolved in 10 mL of DCM, and the resulting mixture was reacted at 0°C for 0.5 h. After the disappearance of raw materials as monitored by LCMS, 5-trifluoromethylisoindole hydrochloride (740 mg, 3.32 mmol) and triethylamine (676 mg, 6.64 mmol) were added. After the disappearance of active ester as monitored by TLC, the reaction solution was added dropwise to 20 mL of ice water, and the resulting mixture was extracted with 20 mL of dichloromethane three times. The organic phases were combined, concentrated and purified by preparative silica gel column chromatography (PE:EA = 1:1) to obtain the target compound **29B** (230 mg, 25%).
LCMS (ESI): m/z= 418.5 $[M+H]^+$

Step 2:

**29B** (230 mg, 0.55 mmol) was dissolved in 4 mL of methanol, 4 mL of 4N hydrogen chloride dioxane was added, and the resulting mixture was reacted for 3 h. After the disappearance of the raw materials as monitored by TLC, the reaction solution was concentrated to obtain compound **29C** (140 mg, 80%), which was directly used in the next step.
LCMS (ESI): m/z= 318.1 $[M+H]^+$

Step 3:

NaH (20 mg, 0.50 mmol, 60% in mineral oil) was suspended in 5 ml of anhydrous THF, **29C** (80 mg, 0.25 mmol) was added in an ice bath, and the resulting mixture was reacted for 1 h. The excess solvent in the reaction solution was removed by rotary evaporator, and 5 mL of anhydrous 1,4-dioxane and intermediate 2 (74 mg, 0.28 mmol) were added, and the resulting mixture was reacted at 80°C for 2 h. After the reaction was completed as monitored by LCMS, the reaction product was purified by silica gel column chromatography (DCM:MeOH-20:1) and then by SFC to obtain the target compound 29-1 (7.2 mg, 6%) **and** compound 29-2 (13.3 mg, 11%).

**Analytical method:**

instrument: SHIMADZU LC-30AD, chiral column: Chiralcel AS column, mobile phase: A for CO2; B for 0.05% DEA in IPA, gradient: B 5%-40%, flow rate: 3 mL/min, column pressure: 100 bar, column temperature: 35°C
detection wavelength: 220 nm, cycle time: 3.0 min

**Preparation method:**

instrument: Waters 150 Prep-SFC A, chiral column: Chiralcel AS column, mobile phase: A for CO2; B for 0.1% NH3•H2O in IPA, gradient: B 40%, flow rate: 100 mL/min, column pressure: 100 bar, column temperature: 25°C, detection wavelength: 220 nm, cycle time: 3.8 min.

**Compound 29-1:**

LCMS (ESI): m/z=493.8 $[M+H]^+$
7.2 mg, retention time: 1.833 min. $^1H$ NMR (400 MHz, DMSO) δ 8.18 (s, 1H), 7.67-7.58 (m, 3H), 7.57-7.53 (m, 1H), 7.47-7.43 (m, 1H), 7.24 (d, 1H), 4.21-4.18 (m, 2H), 4.01 (s, 2H), 3.93 (s, 4H), 3.54 (d, 2H), 2.85-2.78(s, 3H), 2.68-2.57 (m, 2H), 1.96 (s, 1H), 1.60-1.55(m, 2H), 1.40 - 1.21 (m, 2H).

**Compound 29-2:**

LCMS (ESI): m/z=493.8 $[M+H]^+$
13.3 mg, retention time: 2.175 min. $^1H$ NMR (400 MHz, DMSO) δ 8.19 (s, 1H), 7.69- 7.59 (m, 3H), 7.57-7.53 (m, 1H), 7.47-7.43 (m, 1H), 7.30 (d, 1H), 4.21-4.18 (m, 2H), 3.98 (s, 2H), 3.92 (s, 4H), 3.55 (d, 2H), 2.85-2.78(s, 3H), 2.68-2.57 (m, 2H), 1.96 (s, 1H), 1.60-1.55(m, 2H), 1.40 - 1.21 (m,2H).

**Example 30:**

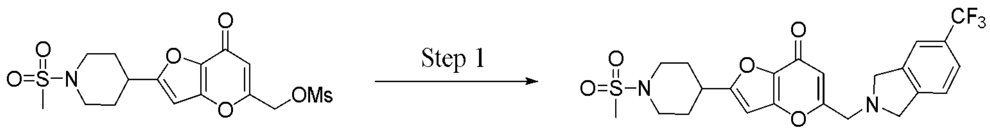


12D

Compound 30

Step 1:

Compound **12D** (100 mg, 0.25 mmol) and 5-trifluoromethylisoindoline hydrochloride (80 mg, 0.36 mmol) were dissolved in acetonitrile (10 mL), DIPEA was then added (100 mg, 0.78 mmol), and the reaction solution was stirred at room temperature overnight. After the reaction was completed, the reaction product was concentrated, and the resulting crude product was purified by column chromatography (DCM:MeOH = 1:0-10:1) to obtain **compound 30** (35 mg, 28%).
LC-MS (ESI): m/z= 497.60 $[M+H]^+$.
$^1H$ NMR (400 MHz, DMSO-$d_6$) δ 7.63 (s, 1H), 7.57 (d, 1H), 7.47 (d, 1H), 6.88 (s, 1H), 6.44 (s, 1H), 4.06 (s, 4H), 3.91 (s, 2H), 3.66-3.56 (m, 2H), 3.03-2.94 (m, 1H), 2.93-2.84 (m, 5H), 2.15-2.05 (m, 2H), 1.75-1.63 (m, 2H).

**Example 31:**

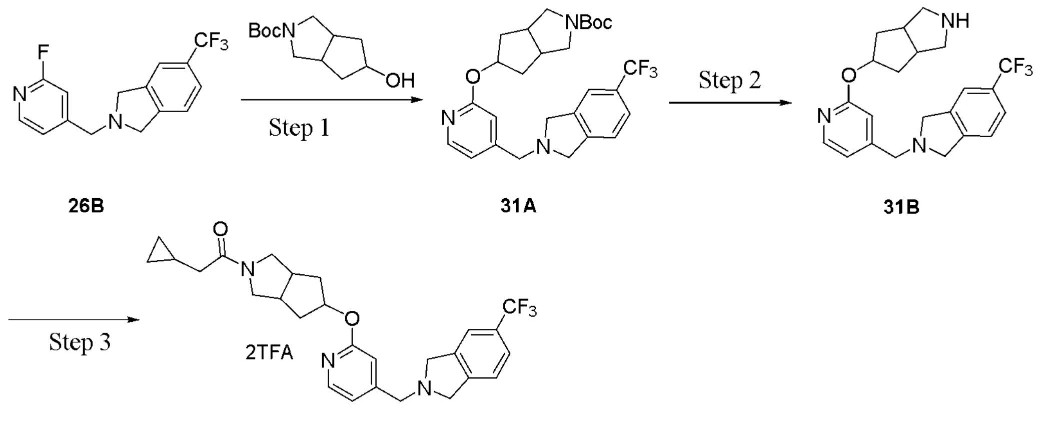


26B 31A 31B

Compound 31

Step 1:

Tert-butyl 5-hydroxyhexahydrocyclopenta[C]pyrrole-2(1H)-carboxylate (1.4 g, 6.0 mmol) was dissolved in DMF (20 mL), sodium hydride (0.24 g, 6.0 mmol) was then added, and the resulting mixture was stirred at room temperature for 0.5 h, and then **26B** (0.9 g, 3.0 mmol) was added. The resulting mixture was reacted overnight at room temperature, quenched with a small amount of methanol, and concentrated. The residue was separated and purified by silica gel column chromatography (PE/EA = 4/1) to obtain **31A** (1.3 g, 86.6%).

LC-MS (ESI): m/z = 504.2 $[M+H]^+$.

Step 2:

Compound **31A** (1.3 g, 2.5 mmol) was dissolved in dichloromethane (10 mL). Trifluoroacetic acid (5 mL) was then added, the resulting mixture was reacted at room temperature for half an hour and then concentrated to obtain compound **31B** (1.3 g, 95%), which was directly used in the next step.

LC-MS (ESI): m/z = 404.2 $[M+H]^+$.

Step 3:

**31B** (1.3 mg, 2.5 mmol) was dissolved in DMF (10 mL), and cyclopropylacetic acid (303.0 mg, 3.0 mmol) and N-methylimidazole (819.6 mg, 10 mmol) were added. At 0°C, N,N,N',N'-tetramethylchloroformamidinium hexafluorophosphate (840.0 mg, 2.85 mmol) was added and the resulting mixture was stirred at room temperature for 20 min. After the reaction was completed, water (20 mL) was added to the reaction solution, and the resulting mixture was extracted with ethyl acetate (15 mL $\times$ 3). The organic layers were combined, washed with saturated aqueous sodium chloride solution (25 mL $\times$ 3), dried over anhydrous sodium sulfate, filtered, and concentrated. The residue was separated and purified by silica gel column chromatography (dichloromethane: methanol (v/v)=20:1) to obtain **compound 31** (450.0 mg, 37%).

LC-MS (ESI): m/z = 486.2 $[M+H]^+$.

$^1$H NMR (400 MHz, $CDCl_3$) $\delta$ 8.24 (d, 1H), 7.64 (d, 1H), 7.56 (s, 1H), 7.43 (d, 1H), 7.11 (d, 1H), 6.75 (s, 2H), 5.62 - 5.43 (m, 1H), 4.74-4.65 (m, 4H), 4.54 - 4.28 (m, 2H), 3.86 - 3.50 (m, 4H), 3.06 - 2.76 (m, 2H), 2.47 - 2.33 (m, 1H), 2.31 (d, 2H), 2.28 - 2.12 (m, 1H), 1.96 - 1.83 (m, 2H), 1.26 (d, 1H), 1.05 - 0.98 (m, 1H), 0.62 - 0.47 (m, 2H), 0.18 (d, 2H).

$^{19}$F NMR (377 MHz, $CDCl_3$) $\delta$ -60.59 (s), -73.88 (s).

**Example 32:**

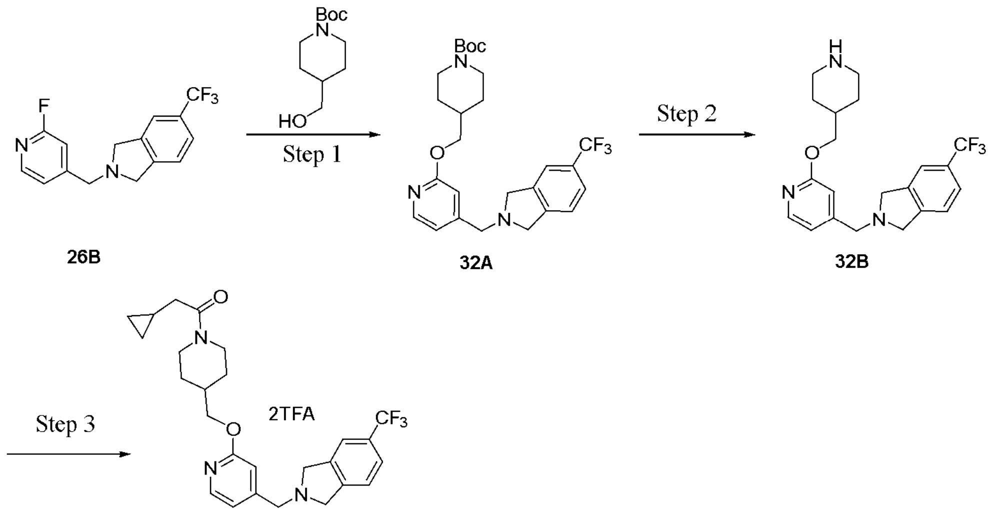


26B 32A 32B

**Compound 32**

Step 1:

N-BOC-4-piperidine methanol (1.3 g, 6.0 mmol) was dissolved in DMF (20 mL), sodium hydride (0.24 g, 6.0 mmol) was then added, and the resulting mixture was stirred at room temperature for 0.5 h, and then **26B** (0.9 g, 3.0 mmol) was added. The resulting mixture was reacted overnight at room temperature, quenched with a small amount of methanol, and concentrated. The residue was separated and purified by silica gel column chromatography (PE/EA = 4/1) to obtain **32A** (1.4 g, 95.2%).

LC-MS (ESI): m/z = 492.2 $[M+H]^+$.

Step 2:

Compound **32A** (1.4 g, 2.8 mmol) was dissolved in dichloromethane (5.0 mL). Trifluoroacetic acid (5.0 mL) was then added, the resulting mixture was reacted at room temperature for half an hour and then concentrated to obtain compound **32B** (1.4 g, 96%), which was directly used in the next step.

LC-MS (ESI): m/z = 392.2 $[M+H]^+$.

Step 3:

**32B** (1.4 g, 2.8 mmol) was dissolved in DMF (15.0 mL), and cyclopropylacetic acid (340.0 mg, 3.4 mmol) and N-methylimidazole (920.0 mg, 12.2 mmol) were added. At 0°C, N,N,N',N'-tetramethylchloroformamidinium hexafluorophosphate (940.1 mg, 3.2 mmol) was added and the resulting mixture was stirred at room temperature for 20 min. After the reaction was completed, 20 mL of water was added to the reaction solution, and the resulting mixture was extracted with ethyl acetate (25 mL × 3). The organic layers were combined, washed with saturated aqueous sodium chloride solution (25 mL × 3), dried over anhydrous sodium sulfate, filtered, and concentrated. The residue was separated and purified by silica gel column chromatography (dichloromethane: methanol (v/v) = 20:1), and then preparative acidic HPLC to obtain **compound 32** (550.0 mg, 42%).

LC-MS (ESI): m/z = 474.2 $[M+H]^+$.

$^1$H NMR (400 MHz, $CDCl_3$) δ 8.46 (s, 2H), 8.04 (d, 1H), 7.46 (d, 1H), 7.37 (s, 1H), 7.24 (d, 1H), 6.83 - 6.82 (m, 1H), 6.66 (s, 1H), 4.47 (d, 5H), 4.20 (s, 2H), 3.99 (d, 2H), 3.72 (d, 1H), 2.90 (t, 1H), 2.44 (t, 1H), 2.12 (d, 2H), 2.00 - 1.80 (m, 1H), 1.73 - 1.64 (m, 2H), 1.11 (s, 2H), 0.91 - 0.73 (m, 1H), 0.43 - 0.29 (m, 2H), -0.01 (q, 2H).

$^{19}$F NMR (377 MHz, $CDCl_3$) δ -60.55 (s), -73.71 (s).

**Example 33:**

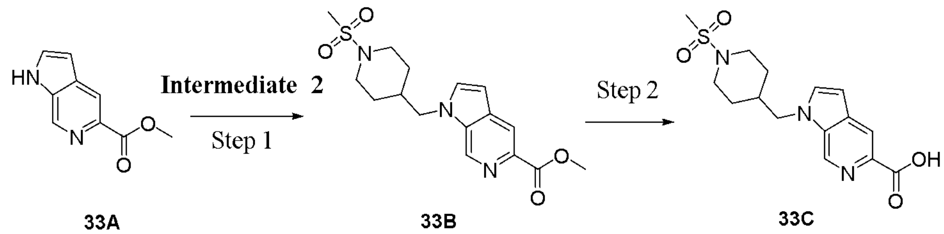


33A 33B 33C

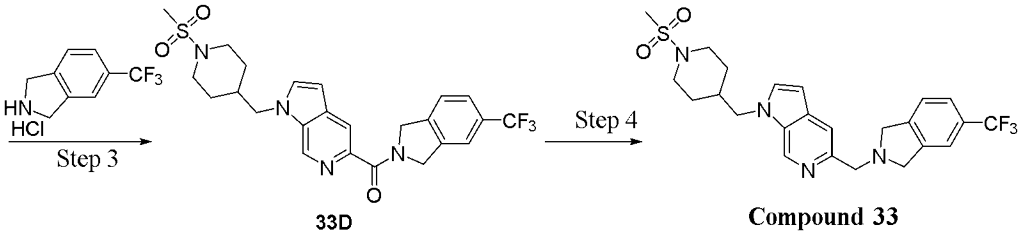


33D Compound 33

Step 1:

The compound **intermediate 2** (0.77 g, 2.84 mmol) and compound **33A** (0.50 g, 2.84 mmol) were weighed and added to a 100 mL single-neck flask and dissolved in DMF (10 mL), and potassium carbonate (1.17 g, 8.44 mmol) was added. After the addition was completed, the resulting mixture was stirred at 80 degrees for 16 h. After the reaction was completed as monitored by TLC (petroleum ether:ethyl acetate = 1:1), water (20 mL) was added and stirred for 5 min, and the resulting mixture was extracted with ethyl acetate (20 mL). The organic phase was separated, dried over anhydrous sodium sulfate and concentrated under reduced pressure, and the crude product was purified by column chromatography (eluent: petroleum ether: ethyl acetate = 1:1) to obtain the target compound **33B** (0.6 g, yield: 60%).
LCMS m/z =352.1 $[M +1]^+$

Step 2:

To a 100 mL single-neck flask was added compound **33B** (600 mg, 1.71 mmol), which was dissolved in methanol (10 mL), and lithium hydroxide monohydrate (140 mg, 3.42 mmol) and water (5 mL) were added. After the addition was completed, the system was subjected to nitrogen protection and stirred at 20°C for 16 h. Upon complete depletion of raw materials as monitored by LCMS, the reaction solution was adjusted to acidic with 2N hydrochloric acid, and then the resulting mixture was extracted with ethyl acetate (20 mL), concentrated under reduced pressure to obtain the target compound **33C** (0.4 g, yield: 69%).
LCMS m/z =338.1 $[M +1]^+$

Step 3:

The compound **33C** (0.50 g, 1.48 mmol) and 5-trifluoromethylisoindoline hydrochloride (0.33 g, 1.48 mmol) were weighed and added to a 100 mL single-neck flask and dissolved in DMF (10 mL), and 1-propylphosphoric anhydride (1.41 g, 2.22 mmol) was added. After the addition was completed, the resulting mixture was stirred at 20 degrees for 2 h. Upon complete depletion of raw materials as monitored by LCMS, water (20 mL) was added and stirred for 5 min, and the resulting mixture was extracted with ethyl acetate (20 mL). The organic phase was separated, dried over anhydrous sodium sulfate and concentrated under reduced pressure, and the crude product was purified by column chromatography (eluent: dichloromethane: methanol=10:1) to obtain the target compound **33D** (0.4 g, yield: 53%).
LCMS m/z =507.1 $[M +1]^+$

Step 4:

The compound **33D** (0.25 g, 0.49 mmol) was weighted, and dissolved in THF (10 mL), and borane dimethyl sulfide complex (0.15 g, 2.97 mmol) was added. After the addition was completed, the resulting mixture was stirred at 50 degrees for 16 h. Upon complete depletion of raw materials as monitored by LCMS, the reaction solution was quenched by adding methanol (10 mL), and the resulting mixture was concentrated under reduced pressure to obtain a crude product of the target compound, which was subjected to preparative HPLC to obtain compound **33** (6 mg, 2.5%).

Preparation method: Instrument: waters 2767 preparative liquid chromatographic instrument; chromatographic column: SunFire@ Prep C18 (19 mm × 250 mm); the sample was dissolved in DMF and filtered with a 0.45 μm filter to prepare a sample liquid; Preparative chromatography conditions: a. composition of mobile phases A and B: Mobile phase

A: acetonitrile, and mobile phase B: water (containing 5 mM aqueous ammonia); b. gradient elution, mobile phase A: 40%-80%; c. flow rate: 15 mL/min; d. elution time: 20 min; retention time: 10.20 min.

LCMS m/z =493.2 $[M+1]^+$

$^1$H NMR (400 MHZ, DMSO) δ8.86 (s, 1H), 7.54-7.60 (m, 4H), 7.44-7.46 (m, 1H), 6.47 (d, 1H), 4.22 (d, 2H), 4.05 (s, 2H), 4.00 (s, 4H), 3.54 (d, 2H), 2.81 (s, 3H), 2.63 (t, 2H), 1.94-2.02 (m, 1H), 1.55 (d, 2H), 1.23-1.35 (m, 2H).

**Example 34:**

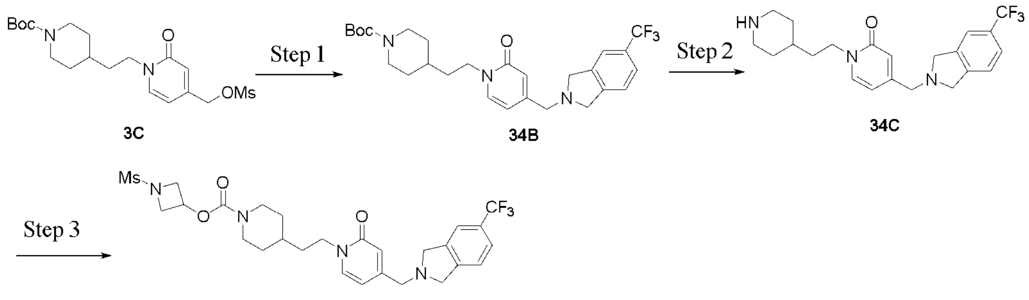


3C

34B

34C

**Compound 34**

Step 1:

Compound **3C** (350 mg, 0.84 mmol) and compound 5-trifluoromethylisoindoline hydrochloride (282 mg, 1.26 mmol) were dissolved in dichloromethane (15 mL), triethylamine (0.34 g, 3.36 mmol) was then added and the resulting mixture was reacted at room temperature for 16 h. After the reaction was completed, the reaction product was concentrated, and the resulting crude product was purified by column chromatography (EA/PE = 0-100%) to obtain the compound **34B** (350 mg, 82.43%).

LC-MS (ESI): m/z= 506.3 $[M+H]^+$.

Step 2:

Compound **34B** (350 mg, 0.69 mmol) was dissolved in dichloromethane (5 mL), trifluoroacetic acid (1.5 mL) was then added, and the resulting mixture was reacted at room temperature for 2 h. After the reaction was completed, the reaction product was concentrated to obtain compound **34C** (280 mg, 100%).

LC-MS (ESI): m/z= 406.3 $[M+H]^+$.

Step 3:

1-(methanesulfonyl)azetidin-3-ol (125 mg, 0.83 mmol) was dissolved in tetrahydrofuran (10 mL), CDI (135 mg, 0.83 mmol) was added, and the resulting mixture was stirred at room temperature for 30 min. Triethylamine (254 mg, 2.49 mmol) was added, and the resulting mixture was stirred at room temperature for another 15 min; Then a solution of compound **34C** (280 mg, 0.69 mmol) and triethylamine (209 mg, 2.06 mmol) in tetrahydrofuran (5 mL) were added to the reaction system followed by stirring at 60°C overnight. After the reaction was completed, the reaction product was concentrated and then subjected to column chromatography (dichloromethane:methanol = 10:1) to obtain **compound 34** (150 mg, 37.31%).

LC-MS (ESI): m/z= 583.7 $[M+H]^+$.

$^1$H NMR (400 MHz, DMSO-D6) δ 7.64 (d, 2H), 7.56 (d, 1H), 7.46 (d, 1H), 6.35 (s, 1H), 6.25 (dd, 1H), 5.05-4.98(m, 1H), 4.18-4.10(m, 2H), 3.99 - 3.92 (m, 6H), 3.90 - 3.81 (m, 4H),3.71(s, 2H), 3.28(s, 2H),3.04(s, 3H), 1.73(d, 2H), 1.62 - 1.50 (m, 2H),

1.48 - 1.37 (m, 1H), 1.15 - 1.02 (m, 2H).

**Example 35:**

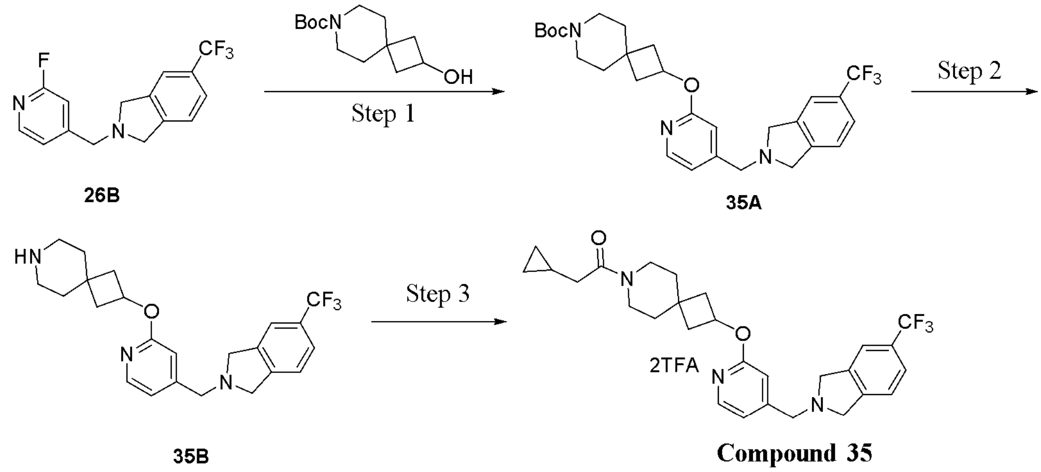


26B 35A 35B Compound 35

Step 1:

Tert-butyl 2-hydroxy-7-azaspiro[3.5]nonane-7-carboxylate (1.5 g, 6.0 mmol) was dissolved in DMF (20 mL), sodium hydride (0.24 g, 6.0 mmol) was then added, and the resulting mixture was stirred at room temperature for 0.5 h, and then **26B** (0.9 g, 3.0 mmol) was added. The resulting mixture was reacted overnight at room temperature, quenched with a small amount of methanol, and concentrated. The residue was separated and purified by silica gel column chromatography (PE/EA = 4/1) to obtain **35A** (1.0 g, 64.5%).
LC-MS (ESI): m/z = 518.2 $[M+H]^+$.

Step 2:

Compound **35A** (1.0 g, 1.9 mmol) was dissolved in dichloromethane (10 mL). Trifluoroacetic acid (6 mL) was then added, and the resulting mixture was reacted at room temperature for half an hour and then concentrated to obtain compound **35B** (0.9 g, 91%), which was directly used in the next step.
LC-MS (ESI): m/z = 418.2 $[M+H]^+$.

Step 3:

**35B** (0.6 g, 1.4 mmol) was dissolved in DMF (10 mL), and cyclopropylacetic acid (170.0 mg, 1.7 mmol) and N-methylimidazole (459.2 mg, 5.6 mmol) were added. At 0°C, N,N,N',N'-tetramethylchloroformamidinium hexafluorophosphate (470.04 mg, 1.6 mmol) was added and the resulting mixture was stirred at room temperature for 20 min. After the reaction was completed, water (20 mL) was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (dichloromethane: methanol (v/v) = 20:1), and then preparative acidic HPLC to obtain **compound 35** (210.0 mg, 30%).
LC-MS (ESI): m/z = 500.2 $[M+H]^+$.
$^1H$ NMR (400 MHz, $CDCl_3$) δ 8.49 (s, 2H), 8.26 (d, 1H), 7.68 (d, 1H), 7.58 (s, 1H), 7.45 (d, 1H), 7.00 (s, 1H), 6.83 (s, 1H), 5.29 - 5.09 (m, 1H), 4.68 (s, 4H), 4.37 (s, 2H), 3.61- 3.39 (m, 4H), 2.59 - 2.39 (m, 2H), 2.33 (d, 2H), 1.98 (s, 2H), 1.67 (s, 4H), 1.07 - 0.93 (m, 1H), 0.56 (d, 2H), 0.20 (d, 2H).
$^{19}F$ NMR (377 MHz, $CDCl_3$) δ -60.58 (s), -73.77 (s).

**Example 36:**

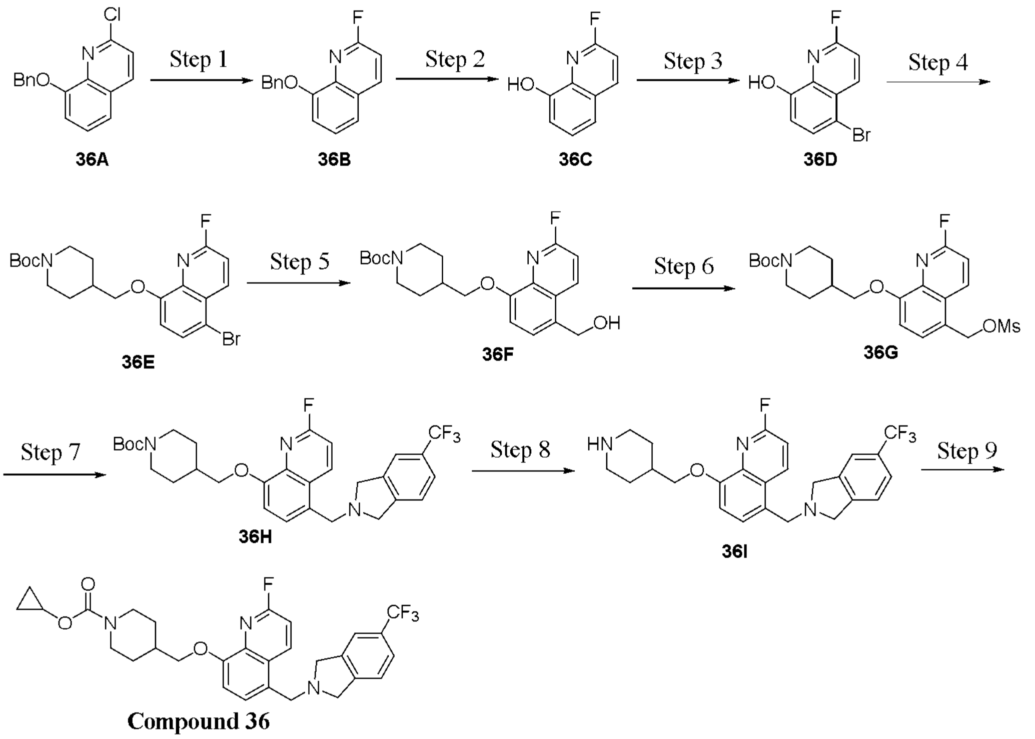


36A 36B 36C 36D 36E 36F 36G 36H 36I

Compound 36

Step 1:

Compound 36A (5.0 g, 18.54 mmol) and tetramethylammonium fluoride (7.0 g, 74.16 mmol) were added to the reaction flask, then DMSO (50 ml) was added, and the resulting mixture was reacted at 80°C for 16 h. After the reaction was completed as monitored by TLC, the reaction product was cooled to room temperature, diluted by adding water (200 ml), and the resulting mixture was extracted twice with ethyl acetate (150 ml*2). The organic phases were combined, dried over anhydrous sodium sulfate, concentrated, and purified by silica gel column chromatography (PE:EA = 10:1) to obtain compound 36B (4.3 g, yield: 91.57%).

LC-MS(ESI): m/z = 254.1$[M+H]^+$.

Step 2:

Compound 36B (4.3 g, 16.98 mmol) was added to the reaction flask, methanol (100 ml) was added, and palladium/carbon (0.85 g, 20% wt) was then added, a hydrogen balloon was inserted, and the resulting mixture was reacted at room temperature for 3 h. After the reaction was completed as monitored by TLC, the reaction product was filtered, and the filter cake was washed once with methanol (20 ml). The filtrate was collected, concentrated, and purified by silica gel column chromatography (PE:EA = 12:1) to obtain compound 36C (2.6 g, yield: 93.49%).

LC-MS(ESI): m/z = 164.20$[M+H]^+$.

Step 3:

Compound 36C (2.5 g, 15.32 mmol) was added to a reaction flask, dissolved in ACN (50 ml), then 1,3-dibromo-5,5-dimethylhydantoin (2.62 g, 9.20 mmol) was added, and the resulting mixture was reacted at room temperature for 4 h. After the reaction was completed as monitored by TLC, the reaction product was concentrated and purified by a silica gel column (PE:EA = 15:1) to obtain compound 36D (2.4 g, yield: 64.72%).

LC-MS(ESI): m/z = 243.90$[M+H]^+$.

Step 4:

Compound 36D (2.4 g, 9.92 mmol), intermediate 5 (2.9 g, 9.92 mmol), and cesium carbonate (4.84 g, 14.88 mmol) were added to the reaction flask and dissolved in DMF (50 ml), and the resulting mixture was reacted at 100°C for 1 h. After the reaction was completed as monitored by TLC, the reaction product was cooled to room temperature, and diluted by adding water (200 ml), and then the resulting mixture was extracted twice with ethyl acetate (100 ml*2). The

organic phases were combined, dried over anhydrous sodium sulfate, concentrated, and purified by silica gel column chromatography (PE:EA = 15:1) to obtain compound 36E (2.14 g, yield: 49.10%).

LC-MS(ESI): m/z = 341.0 $[M+H]^+$.

Step 5:

Compound 36E (2.1 g, 4.78 mmol), tri-n-butyltin methanol (1.70 g, 5.26 mmol) and Xphos Pd G2 (0.37 g, 0.48 mmol) were added to the reaction flask and dissolved in 1,4-dioxane (40 ml), and then the resulting mixture was reacted at 90°C for 4 h. After the reaction was completed as monitored by TLC, the reaction product was cooled to room temperature, concentrated under reduced pressure, and purified by silica gel column chromatography (PE:EA = 12:1) to obtain compound 36F (0.5 g, yield: 26.79%).

LC-MS(ESI): m/z = 291.2$[M+H]^+$.

Step 6:

Compound 36F (0.4 g, 1.02 mmol) was added to a reaction flask and dissolved in DCM (20 ml), and triethylamine (0.29 ml, 2.04 mmol) was then added, then methylsulfonyl chloride (132 mg, 1.12 mmol) was added, and the resulting mixture was reacted at room temperature for 3 h. After the reaction was completed as monitored by TLC, the reaction product was concentrated under reduced pressure to obtain compound 36G (0.48 g, yield: 100.4%), which was directly used in the next step.

Step 7:

Compound 36G (0.48 g, 1.02 mmol), 5-trifluoromethylisoindoline hydrochloride (230 mg, 1.02 mmol) and potassium carbonate (0.56 g, 4.08 mmol) were added to the reaction flask and dissolved in acetonitrile (10 ml), and the resulting mixture was reacted at 70°C for 3 h. After the reaction was completed as monitored by TLC, the reaction product was concentrated under reduced pressure and purified by silica gel column chromatography (PE:EA = 1:2) to obtain compound 36H (0.4 g, yield: 70.08%).

LC-MS(ESI) m/z = 460.2$[M+H]^+$.

Step 8:

Compound 36H (0.4 g, 0.71 mmol) was added to a reaction flask, then DCM (8 ml) and TFA (2 ml) were added, and the resulting mixture was reacted at room temperature for 1 h. After the reaction was completed as monitored by TLC, the reaction product was concentrated under reduced pressure to obtain compound 36I (0.31 g, yield: 95.02%).

LC-MS(ESI) m/z = 460.2$[M+H]^+$.

Step 9:

Compounds cyclopropanol (31 mg, 0.52 mmol) and CDI (91 mg, 0.56 mmol) were added to a reaction flask and dissolved in THF (10 ml), and the resulting mixture was stirred at room temperature for 30 min, then triethylamine (0.5 ml, 3.5 mmol) was added, and finally compound 36I (160 mg, 0.35 mmol) was added and the resulting mixture was reacted at 70°C for 16 h. After the reaction was completed as monitored by TLC, the reaction product was cooled to room temperature, concentrated under reduced pressure, and purified by silica gel column chromatography (DCM:EA = 1:1) to obtain compound 36 (38 mg, yield: 19.97%).

$^1$H NMR (400 MHz, DMSO-$d_6$) δ 10.80 (s, 1H), 8.25 (d, 1H), 7.58 (s, 1H), 7.53 (d,1H), 7.42 (d, 1H), 7.11 (d, 1H), 7.06 (d, 1H), 6.52 (d, 1H), 4.06 (s, 2H), 4.01 - 3.97 (m, 1H), 3.95 (d, 2H), 3.87 (s, 4H), 3.31 (s, 2H), 2.84 - 2.77 (m, 2H), 2.15 - 2.03 (m, 1H), 1.91 (d, 2H), 1.28 - 1.19 (m, 2H), 0.66 - 0.58 (m, 4H).

LC-MS(ESI) m/z = 544.50$[M+H]^+$.

**Example 37**

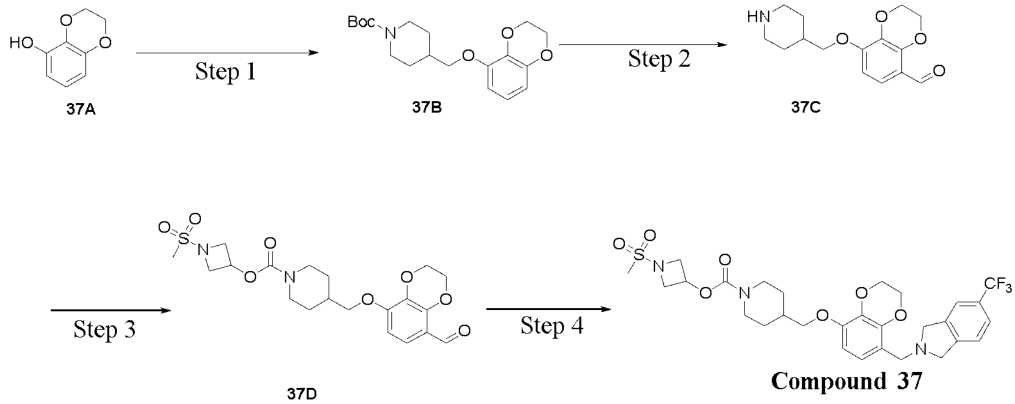


37A 37B 37C 37D Compound 37

Step 1:

Compound 37A (1.52 g, 10 mmol) was dissolved in DMF (30 mL), 1-BOC-4-bromomethylpiperidine (3.34 g, 12 mmol) was added, cesium carbonate (4.89 g, 15 mmol) was then added, and the resulting mixture was heated to 80°C and reacted for 3 h. After the reaction was completed, water (60 mL) was added and the resulting mixture was extracted with EA (20 mL x 3). The organic phase was washed with saturated aqueous sodium chloride solution, the organic phase was dried and concentrated, and the resulting crude product was subjected to column chromatography (EA/PE = 0-50%) to obtain compound 37B (2.1 g, 62%).
LC-MS (ESI): m/z = 294.2 $[M-^tBu+H]^+$.

Step 2:

Compound 37B (1.5 g, 4.29 mmol) was dissolved in dichloromethane (30 mL), and the resulting mixture was cooled to -15°C. $SnCl_4$ (1.91 g, 7.33 mmol) was added, and the resulting mixture was reacted at -15°C for 15 min. 1,1-dichlorodimethyl ether (493 mg, 4.29 mmol) was then added and the reaction was continued for 30 min. After the reaction was completed as monitored by TLC, the reaction solution was slowly added dropwise into ice water, silica gel was added to the system, which was concentrated and subjected to rotary evaporation, and purified by column chromatography (MeOH/DCM = 0-30%) to obtain compound 37C (1 g, 84%).
LC-MS (ESI): m/z= 278.1 $[M+H]^+$.

Step 3:

1-(methanesulfonyl)azetidin-3-ol (382 mg, 2.52 mmol) was dissolved in tetrahydrofuran (15 mL), CDI (408 mg, 2.52 mmol) was added, and the resulting mixture was stirred at room temperature for 30 min. Triethylamine (771 mg, 7.56 mmol) was added, and the resulting mixture was stirred at room temperature for another 15 min; Then a solution of compound 37C (500 mg, 1.80 mmol) and triethylamine (552 mg, 5.41 mmol) in tetrahydrofuran (15 mL) were added to the reaction system followed by stirring at 60°C overnight. The reaction solution was concentrated and then subjected to column chromatography (MeOH/DCM = 0-15%) to obtain 37D (290 mg, 35%).
LC-MS (ESI): m/z= 455.1$[M+H]^+$

Step 4:

Compound 37D (50 mg, 0.11 mmol) and compound 5-trifluoromethylisoindoline hydrochloride (49 mg, 0.22 mmol) were dissolved in DCE (10 mL), glacial acetic acid (7 mg, 0.11 mmol) was added, and the resulting mixture was stirred at room temperature for 5 min. Sodium triacetoxyborohydride (47 mg, 0.22 mmol) was then added, and the resulting mixture was stirred at room temperature overnight. The reaction solution was concentrated and then purified by column chromatography (MeOH/DCM = 0-15%) to obtain compound 37 (9 mg, 13%).
LC-MS (ESI): m/z = 626.6 $[M+H]^+$.
$^1H$ NMR (400 MHz, DMSO-$d_6$) $\delta$ 7.59 (s, 1H), 7.53 (d, 1H), 7.44 (d, 1H), 6.80 (d, 1H), 6.53 (d, 1H), 5.06-5.00(m, 1H), 4.28-4.20(m, 4H), 4.18 -4.12 (m, 2H), 4.06 - 3.99 (m, 2H),3.89(s, 4H), 3.87-3.83(m, 2H),3.80(d, 2H), 3.77(s, 2H), 3.27(s, 2H), 3.04(s, 3H), 2.00 - 1.92 (m, 1H),1.82 - 1.74 (m, 2H), 1.15 - 1.02 (m, 2H).

**Example 38:**

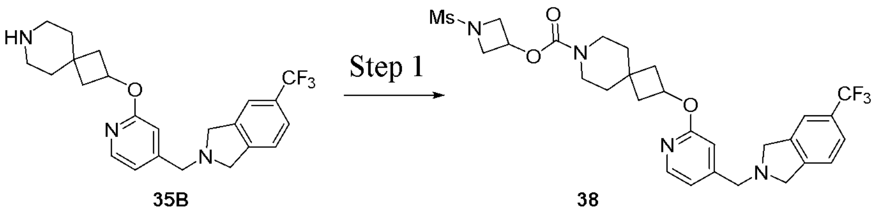


35B 38

Step 1:

1-methylsulfonylazetidin-3-ol (220.0 mg, 1.5 mmol) was dissolved in tetrahydrofuran (10 mL), CDI (240.0 mg, 1.5 mmol) was then added, and the resulting mixture was stirred and reacted at room temperature for 0.5 h. A solution of 35B (0.42 g, 1.0 mmol) in tetrahydrofuran (10 mL), and triethylamine (300.0 mg, 3.0 mmol) were added, and the resulting mixture was heated to 60°C and reacted overnight. After the reaction was completed as monitored by TLC, the reaction product was concentrated and the residue was separated and purified by silica gel column chromatography (dichloromethane: methanol (v/v)=20:1) to obtain compound 38 (210.0 mg, 41%).

LC-MS (ESI): $m/z$ = 595.2 $[M+H]^+$.

$^1H$ NMR (400 MHz, $CDCl_3$) δ 8.11 (s, 1H), 7.49 (d, 2H), 7.31 (s, 1H), 6.94 (s, 1H), 6.78 (s, 1H), 5.27 - 5.20 (m, 1H), 5.16 - 5.06 (m, 1H), 4.18 - 4.14 (m, 2H), 4.12 - 3.70 (m, 7H), 3.49 - 3.32 (m, 4H), 2.89 (s, 3H), 2.50 - 2.45 (m, 2H), 1.96 - 1.91 (m, 2H), 1.66 - 1.61 (m, 4H).

$^{19}F$ NMR (377 MHz, $CDCl_3$) δ -60.06 (s).

**Example 39:**

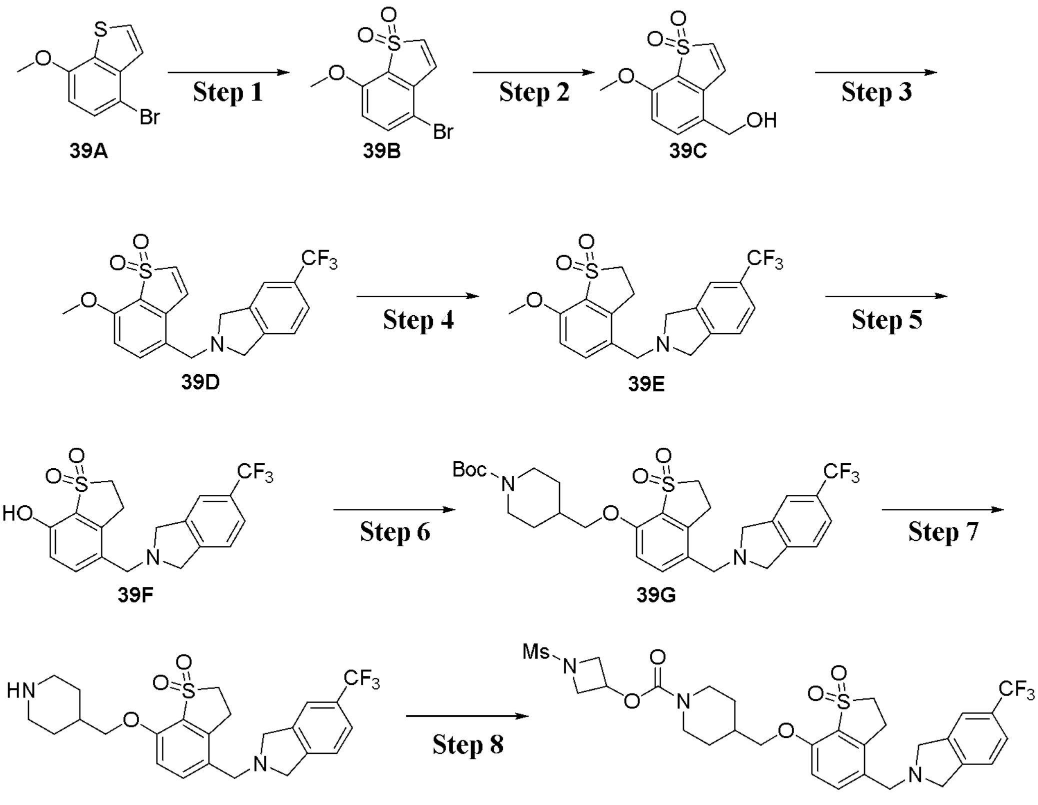


39A 39B 39C 39D 39E 39F 39G 39H Compound 39

Step 1:

39A (5.50 g, 22.62 mmol, synthesis reference: WO 2022/56269, 2022, A1) was dissolved in dichloromethane (100 mL), and m-chloroperoxybenzoic acid (13.78 g, 67.86 mmol) was added at 0-5°C. After the addition was completed, the resulting mixture was reacted at room temperature overnight. After the reaction was completed, the reaction solution was filtered, and the filtrate was concentrated and purified by column chromatography (petroleum ether/ethyl acetate = 4/1, (v/v)) to obtain 39B (5.50 g, 88%).

LC-MS (ESI): m/z = 275.0 $[M+H]^+$.

Step 2:

39B (5.50 g, 19.99 mmol) was dissolved in 1,4-dioxane (100 mL), and (tributyltin)methanol (9.63 g, 29.98 mmol) was added at room temperature. After the addition was completed, the resulting mixture was subjected to nitrogen replacement three times, and then chloro(2-dicyclohexylphosphino-2',4',6'-triisopropyl-1,1'-biphenyl)[2-(2'-amino-1,1'-biphenyl)]palladium(II) (1.57 g, 2.00 mmol) was added. After the addition was completed, the resulting mixture was subjected to nitrogen replacement 3 times, and reacted at 100°C overnight. After the reaction was completed, the reaction solution was filtered, and the filtrate was concentrated and purified by column chromatography (petroleum ether/ethyl acetate = 4/1, (v/v)) to obtain 39C (2.50 g, 55%).

LC-MS (ESI): m/z= 227.1 $[M+H]^+$.

Step 3:

39C (1.60 g, 7.07 mmol) was dissolved in dichloromethane (40 mL), and triethylamine (2.86 g, 28.28 mmol) and methylsulfonyl chloride (0.85 g, 7.42 mmol) were added at 0°C-5°C. After the addition was completed, the resulting mixture was reacted at room temperature for 30 min, and 5-trifluoromethylisoindoline hydrochloride (1.90 g, 8.48 mmol) was added at room temperature. After the addition was completed, the resulting mixture was reacted at room temperature overnight. After the reaction was completed, the reaction solution was concentrated and purified by column chromatography (petroleum ether/ethyl acetate = 3/7, (v/v)) to obtain compound 39D (1.5 g, 53%).

LC-MS (ESI): m/z= 396.1 $[M+H]^+$ .

Step 4:

39D (650 mg, 1.64 mmol) was dissolved in methanol (30 mL), and palladium on carbon (175 mg, 0.16 mmol) was added at room temperature. After the addition was completed, the resulting mixture was subjected to hydrogen replacement three times, and reacted at room temperature overnight. After the reaction was completed, the reaction solution was concentrated to obtain crude compound 39E.

LC-MS (ESI): m/z= 398.1 $[M+H]^+$ .

Step 5:

39E (530 mg, 1.33 mmol) was dissolved in dichloromethane (15 mL), and boron tribromide (1.00 g, 3.99 mmol) at 0-5°C. After the addition was completed, the mixture was reacted at room temperature overnight. After the reaction was completed, the solvent in the reaction flask was poured off, and the black solid in the reaction flask was quenched with saturated sodium bicarbonate, and the resulting mixture was extracted with ethyl acetate (50 mL*2). The organic phase was concentrated, and the residue was purified by column chromatography (petroleum ether/ethyl acetate = 1/4, (v/v)) to obtain compound 39F (320 mg, 62%).

LC-MS (ESI): m/z=384.1 $[M+H]^+$.

Step 6:

39F (0.37 g, 0.97 mmol) was dissolved in N,N-dimethylformamide (8 mL), tert-butyl 4-(bromomethyl)piperidine-1-carboxylate (0.32 g, 1.16 mmol), potassium carbonate (0.27 g, 1.95 mmol) and potassium iodide (16.10 mg, 0.097 mmol) were added at room temperature, and the resulting mixture was reacted at 80°C overnight. After the reaction was completed, the reaction was concentrated and the residue was purified by column chromatography (petroleum ether/ethyl acetate = 1/1, (v/v)) to obtain 39G (0.21 g, 37%).

LC-MS (ESI): m/z= 528.8 $[M-55]^+$.

Step 7:

39G (210 mg, 0.36 mmol) was dissolved in dichloromethane (4 mL), and trifluoroacetic acid (1 mL) was added at 0°C-5°C. After the addition was completed, the mixture was reacted at room temperature for 2 hrs. After the reaction was completed, the reaction was concentrated to obtain crude compound 39H, which was directly used in the next reaction.

Step 8:

1-methylsulfonylazetidin-3-ol (47.62 mg, 0.32 mmol) was dissolved in tetrahydrofuran (4 mL), and N,N'-carbonyldiimidazole (51.08 mg, 0.32 mmol) was added at room temperature. After the addition was completed, the resulting mixture was reacted at room temperature for 4.5 h, and triethylamine (63.75 mg, 0.63 mmol) was added dropwise to the reaction system at room temperature. After the addition was completed, the resulting mixture was reacted at room temperature for 2 h, and 39H (100 mg, 0.21 mmol) was added dropwise at 0°C-5°C. After the addition was completed, the resulting mixture was reacted at 60°C overnight. After the reaction was completed, the reaction solution was concentrated and purified by preparative HPLC to obtain compound 39 (25 mg, 18%).

LC-MS (ESI): m/z=658.2[M+H]$^+$.

$^1$H NMR (400 MHz, DMSO-$d_6$) $\delta$ 7.60 - 7.54 (m, 3H), 7.5-7.434 (d, 1H), 7.09-7.07 (d, 1H), 5.06-5.00 (m, 1H), 4.17-4.13 (m, 2H), 4.03-3.98 (m, 4H), 3.92 - 3.86 (m, 8H), 3.53-3.49 (m, 2H), 3.37-3.28 (m, 2H), 3.04(s, 3H), 2.87-2.82 (m, 2H), 1.99 (s, 1H), 1.82-1.75 (m, 2H), 1.27-1.24 (m, 2H).

**Example 40:**

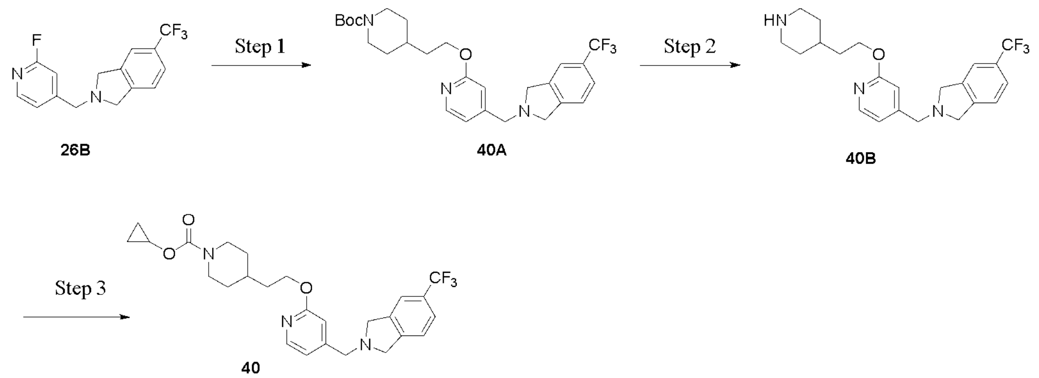


26B 40A 40B 40

Step 1:

N-BOC-4-piperidine ethanol (1.37 g, 6.0 mmol) was dissolved in DMF (20 mL), sodium hydride (0.24 g, 6.0 mmol) was then added, and the resulting mixture was stirred at room temperature for 0.5 h, and then 26B (0.9 g, 3.0 mmol) was added. The resulting mixture was reacted overnight at room temperature, quenched with a small amount of methanol, and concentrated. The residue was separated and purified by silica gel column chromatography (petroleum ether/ethyl acetate = 4/1 (v/v)) to obtain 40A (1.5 g, 96%).

LC-MS (ESI): *m*/*z* = 506.2 [M+H]$^+$.

Step 2:

Compound 40A (1.5 g, 2.9 mmol) was dissolved in dichloromethane (5.0 mL). Trifluoroacetic acid (5.0 mL) was then added, and the resulting mixture was reacted at room temperature for half an hour and then concentrated to obtain compound 40B (1.4 g, 96%), which was directly used in the next step.

LC-MS (ESI): *m*/*z* = 406.2 [M+H]$^+$.

Step 3:

Cyclopropanol (220.0 mg, 1.5 mmol) was dissolved in tetrahydrofuran (10 mL), CDI (240.0 mg, 1.5 mmol) was then added, and the resulting mixture was stirred and reacted at room temperature for 0.5 h. A solution of 40B (0.4 g, 1.0 mmol) in tetrahydrofuran (10 mL), and triethylamine (300.0 mg, 3.0 mmol) were added, and the resulting mixture was

heated to 60°C and reacted overnight. After the reaction was completed as monitored by TLC, the reaction product was concentrated and the residue was separated and purified by silica gel column chromatography (dichloromethane: methanol (v/v)=20:1) to obtain compound 40 (290.0 mg, 59%).

LC-MS (ESI): *m/z* = 490.2 $[M+H]^+$.

$^1H$ NMR (400 MHz, $CDCl_3$) δ 8.11 (d, 1H), 7.54 - 7.40 (m, 2H), 7.29 (d, 1H), 6.93 (s, 1H), 6.78 (s, 1H), 4.34 (t, 2H), 4.22 - 3.81 (m, 9H), 2.76 - 2.70 (m, 2H), 1.79 - 1.64 (m, 5H), 1.19 (s, 2H), 0.72 - 0.59 (m, 4H).

$^{19}F$ NMR (377 MHz, $CDCl_3$) δ -60.03 (s).

**Example 41:**

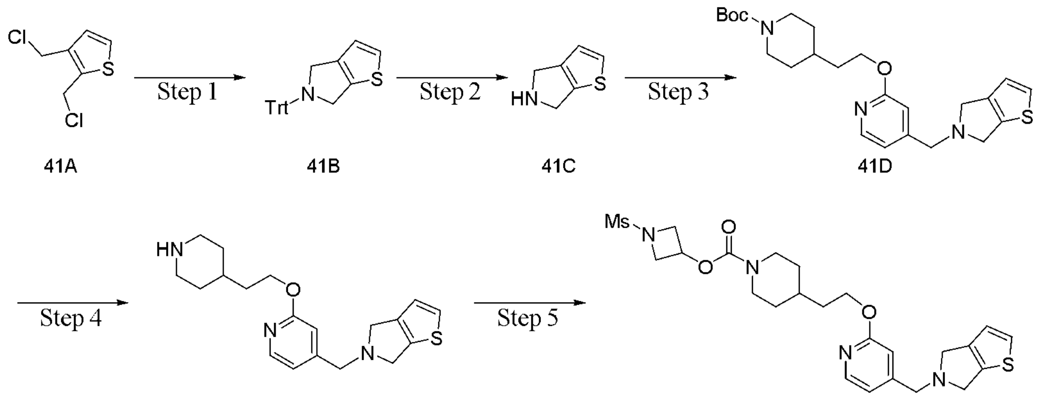


41A 41B 41C 41D

41E Compound 41

Step 1:

41A (1.20 g, 6.63 mmol, synthesis reference: Organic Letters, 2012, vol. 14, # 6, p. 1508 - 1511) was dissolved in N,N-dimethylformamide (20 mL), and tritylamine (1.72 g, 6.63 mmol), potassium carbonate (2.75 g, 19.89 mmol) and potassium iodide (0.11 g, 0.66 mmol) were added at room temperature. After the addition was completed, the resulting mixture was reacted at 85°C overnight. After the reaction was completed, water (70 mL) was added to the reaction solution to quench the reaction, and the resulting mixture was extracted with ethyl acetate (70 mL*2). The organic phase was washed with water (70 mL) and saturated aqueous sodium chloride solution (70 mL), dried over anhydrous sodium sulfate, and concentrated. The residue was purified by column chromatography (petroleum ether/ethyl acetate = 19/1, (v/v)) to obtain 41B (300 mg, 12%).

Step 2:

41B (300 mg, 0.82 mmol) was dissolved in dichloromethane (3 mL) and trifluoroacetic acid (3 mL) was added at 0-5°C. After the addition was completed, the mixture was reacted at room temperature for 2 hrs. After the reaction was completed, the reaction was concentrated to obtain crude compound 41C, which was directly used in the next reaction.

LC-MS (ESI): m/z= 126.0 $[M+H]^+$.

Step 3:

10C (0.25 g, 0.60 mmol,) was dissolved in dichloromethane (10 mL), and triethylamine (212.50 mg, 2.10 mmol) and 41C (0.11 g, 0.90 mmol) were added at 0-5°C. After the addition was completed, the resulting mixture was reacted at room temperature overnight. After the reaction was completed, the reaction was concentrated and the residue was purified by column chromatography (petroleum ether/ethyl acetate = 1/1, (v/v)) to obtain 41D (170 mg, 63%).

LC-MS (ESI): m/z= 444.2 $[M+H]^+$.

Step 4:

41D (125 mg, 0.28 mmol) was dissolved in dichloromethane (4 mL), and trifluoroacetic acid (1 mL) was added at 0°C-5°C. After the addition was completed, the mixture was reacted at room temperature for 2 hrs. After the reaction was completed, the reaction was concentrated to obtain crude compound 41E, which was directly used in the next reaction.

Step 5:

1-methylsulfonylazetidin-3-ol (63.50 mg, 0.42 mmol) was dissolved in tetrahydrofuran (6 mL), and N,N'-carbonyldiimidazole (68.10 mg, 0.42 mmol) was added at room temperature. After the addition was completed, the resulting mixture was reacted at room temperature for 4.5 h, and triethylamine (85.00 mg, 0.84 mmol) was added dropwise to the reaction system at room temperature. After the addition was completed, the resulting mixture was reacted at room temperature for 2 h, and 41E (96 mg, 0.28 mmol) was added dropwise at 0°C-5°C. After the addition was completed, the resulting mixture was reacted at 60°C overnight. After the reaction was completed, the reaction solution was concentrated and purified by preparative HPLC to obtain compound 41 (12 mg, 8%).

LC-MS (ESI): m/z= 521.2 $[M+H]^+$.

$^1H$ NMR (400 MHz, DMSO-$d_6$) δ8.09-8.08 (d, 1H), 7.42-7.41 (d, 1H), 6.98-6.97 (d, 1H), 6.88-6.87 (d, 1H), 6.76 (s, 1H), 5.05 -4.99 (m, 1H), 4.31-4.26 (m, 2H), 4.16 - 4.12 (m, 2H), 3.93-3.92 (m, 6H), 3.87 - 3.79 (m, 4H), 3.03 (s, 3H), 2.86-2.70 (m, 2H), 1.73-1.62 (m, 5H), 1.11-1.09 (s, 2H).

**Example 42:**

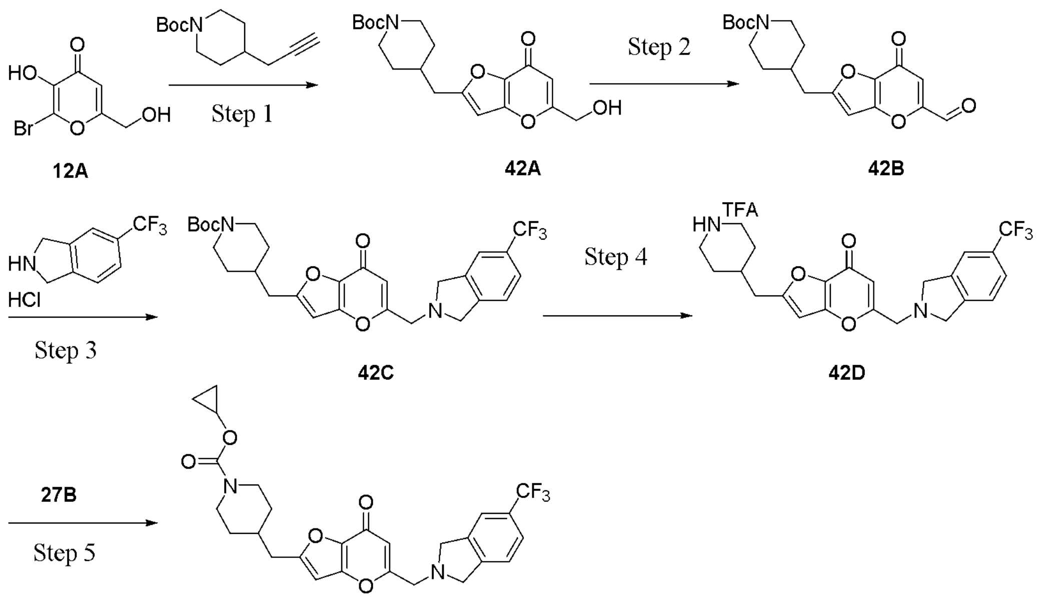


Compound 42

Step 1:

At room temperature, compound 12A (3.0 g, 13.57 mmol), tert-butyl 4-(prop-2-yn-1-yl)piperidine-1-carboxylate (3.03 g, 13.57 mmol), bis(triphenylphosphine)palladium dichloride (0.95 g, 1.36 mmol) and cuprous iodide (0.52 g, 2.72 mmol) were dissolved in tetrahydrofuran (50 mL), triethylamine (4.11 g, 40.71 mmol) was then added under nitrogen atmosphere, and the reaction solution was warmed to 40°C and stirred for 4 h. After the reaction was completed, the reaction product was filtered, and the filtrate was concentrated under reduced pressure, and then purified by column chromatography (eluent: petroleum ether: ethyl acetate = 1:1 (v/v)) to obtain the target compound 42A (0.76 g, yield: 15%).

LC-MS (ESI): *m*/*z*= 308.2 $[M\text{-}t\text{-}Bu+H]^+$.

Step 2:

42A (440 mg, 1.21 mmol) was dissolved in DCM (20 mL), and Dess-Martin oxidant (617 mg, 1.45 mmol) was added in portions at room temperature. After the addition was completed, the resulting mixture was reacted at room temperature for two hours. The reaction was quenched with saturated aqueous sodium thiosulfate solution, and the aqueous phase was extracted with dichloromethane. The organic layers were combined, washed with saturated NaCl solution, dried over anhydrous $Na_2SO_4$, filtered and subjected to rotary evaporation; The residue was subjected to silica gel column chromatography (dichloromethane: methanol (v/v)=10:1) to obtain target compound 42B (420 mg, yield: 96%)

LC-MS (ESI): *m*/*z* = 306.2 [M-*t*-Bu+H]$^+$.

Step 3:

42B (420 mg, 1.16 mmol) was dissolved in methanol (10 mL), then 5-trifluoromethylyl-2,3-dihydro1H-isoindole hydrochloride (312 mg, 1.40 mmol) was added, and the resulting mixture was stirred at room temperature for 1 h. Sodium cyanoborohydride (88 mg, 1.40 mmol) was added, and the resulting mixture was reacted at room temperature for one hour. The reaction solution was quenched with saturated aqueous sodium bicarbonate solution, the aqueous phase was extracted with dichloromethane, and the organic layers were combined, washed with saturated NaCl solution, dried over anhydrous $Na_2SO_4$, filtered, subjected to rotary evaporation and subjected to silica gel column chromatography (dichloromethane: methanol (v/v)=10:1) to obtain the target compound 42C (156 mg, yield: 25%).
LC-MS (ESI): *m*/*z* = 477.2 [M-*t*-Bu+H]$^+$.

Step 4:

42C (156 mg, 0.29 mmol) was dissolved in DCM (5 mL), then trifluoroacetic acid (1 mL) was added, and the resulting mixture was reacted at room temperature for 1 h. After the reaction was completed, the reaction solution was concentrated, and the crude product 42D was directly used in the next reaction without purification.

Step 5:

The crude product 42D obtained in the previous step was dissolved in DCM (20 mL), N,N-diisopropylethylamine (112 mg, 0.87 mmol) was added, and the resulting mixture was stirred at room temperature for five minutes. 27B (129 mg, 0.58 mmol) was added, and the resulting mixture was reacted at room temperature for 1 h. The reaction solution was quenched with water, and the aqueous phase was extracted with dichloromethanethe. The organic layers were combined, washed with saturated NaCl solution, dried over anhydrous $Na_2SO_4$, filtered, concentrated, and subjected to column chromatography (dichloromethane: methanol (v/v) = 10:1) to obtain the target compound 42 (40 mg, yield: 27%).
LC-MS (ESI): *m*/*z* = 517.6 [M+H]$^+$.
$^1$H NMR (400 MHz, $CDCl_3$) $\delta$ 7.49 (d, 1H), 7.46 (s, 1H), 7.31 (d, 1H), 6.46 (s, 1H), 6.32 (s, 1H), 4.11 (s, 4H), 4.08 - 4.03 (m, 1H), 3.88 (s, 2H), 2.77 - 2.67 (m, 4H), 2.05 - 1.90 (m, 2H), 1.74 - 1.60 (m, 3H), 1.26 - 1.10 (m, 2H), 0.72 - 0.62 (m, 4H).

**Example 43:**

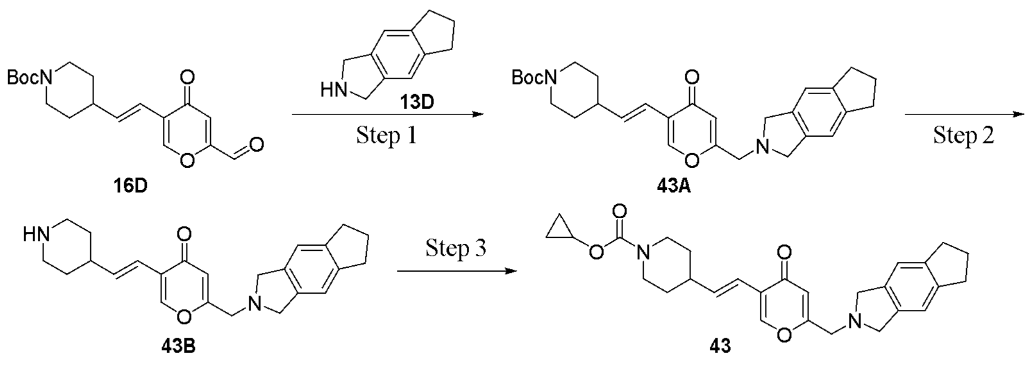

16D
43A
43B
43

Step 1:

**16D** (333.0 mg, 2.25 mmol) was dissolved in 1,2-dichloroethane (20 mL), then 13D (174.9 mg, 1.1 mmol) was added, and the resulting mixture was stirred at room temperature for 1 h. Sodium triacetoxyborohydride (466.0 mg, 2.2 mmol) was added, and the resulting mixture was reacted at room temperature overnight. The reaction solution was quenched with saturated aqueous sodium bicarbonate solution, the aqueous phase was extracted with dichloromethane, and the organic layers were combined and washed with saturated NaCl solution. The organic layers were combined, dried over $Na_2SO_4$, filtered, subjected to rotary evaporation, and subjected to silica gel column chromatography (dichloromethane: methanol (v/v) = 10:1) to obtain 43A (300.0 mg, 63.0%).

LC-MS (ESI): $m/z$ = 477.2 [M+H]$^+$.

Step 2:

Compound 43A (300.0 mg, 0.62 mmol) was dissolved in dichloromethane (3.0 mL). Trifluoroacetic acid (2.0 mL) was then added, and the resulting mixture was reacted at room temperature for half an hour and then concentrated to obtain compound 43B (310.0 mg, 97%), which was directly used in the next step.
LC-MS (ESI): $m/z$ = 377.2 [M+H]$^+$.

Step 3:

Cyclopropanol (44.0 mg, 0.75 mmol) was dissolved in tetrahydrofuran (5.0 mL), then CDI (120.0 mg, 0.75 mmol) was added, and the mixture was stirred and reacted at room temperature for 0.5 h. A solution of 43B (310.0 mg, 0.62 mmol) in tetrahydrofuran (5.0 mL), and triethylamine (191.0 mg, 1.9 mmol) were added, and the mixture was heated to 60°C and reacted overnight. After the reaction was completed as monitored by TLC, the reaction mixture was concentrated and the residue was separated and purified by silica gel column chromatography (petroleum ether: ethyl acetate (v/v) = 10:1) to obtain compound 43 (102.0 mg, 35%).
LC-MS (ESI): $m/z$ = 461.2 [M+H]$^+$.
$^1$H NMR (400 MHz, $CDCl_3$) δ 7.79 (s, 1H), 7.04 (s, 2H), 6.55 - 6.49 (m, 1H), 6.42 (d, 1H), 6.17 (d, 1H), 4.45 - 3.86 (m, 7H), 3.78 (d, 2H), 2.94 - 2.71 (m, 6H), 2.32 -2.15 (m, 1H), 2.11 -2.04 (m, 2H), 1.74 (d, 2H), 1.36 (d, 2H), 0.69 - 0.67 (m, 4H).

**Example 44**

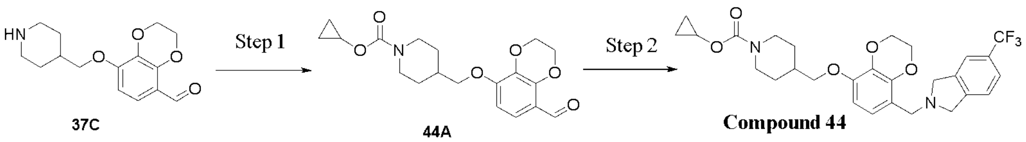


37C 44A Compound 44

Step 1:

Cyclopropanol (146 mg, 2.52 mmol) was dissolved in tetrahydrofuran (10 mL), CDI (408 mg, 2.52 mmol) was added, and the resulting mixture was stirred at room temperature for 30 min. Triethylamine (771 mg, 7.56 mmol) was added, and the resulting mixture was stirred at room temperature for another 15 min; Then a solution of compound 37C (500 mg, 1.80 mmol) and triethylamine (552 mg, 5.41 mmol) in tetrahydrofuran (15 mL) were added to the reaction system followed by stirring at 60°C overnight. The reaction solution was concentrated and then subjected to column chromatography (MeOH/DCM = 0-15%) to obtain 44A (140 mg, 21%).
LC-MS (ESI): m/z= 362.40[M+H]$^+$

Step 2:

Compound 44A (140 mg, 0.39 mmol) and compound 5-trifluoromethylisoindoline hydrochloride (173 mg, 0.78 mmol) were dissolved in DCE (10 mL), glacial acetic acid (23 mg, 0.39 mmol) was added, and the resulting mixture was stirred at room temperature for 5 min. Sodium triacetoxyborohydride (164 mg, 0.79 mmol) was then added, and then the resulting mixture was stirred at room temperature overnight. The reaction solution was concentrated and then purified by column chromatography (MeOH/DCM = 0-15%) to obtain compound **44** (30 mg, 14%).
LC-MS (ESI): m/z= 533.3 [M+H]$^+$.
$^1$H NMR (400 MHz, DMSO-$d_6$) δ 7.58 (s, 1H), 7.52 (d, 1H), 7.43 (d, 1H), 6.79 (d, 1H), 6.52 (d, 1H), 4.27-4.18(m, 4H), 4.02-3.94(m, 2H), 3.89(s, 4H), 3.81-3.75(m, 4H), 3.28(s, 1H), 2.85- 2.70 (m, 2H),1.92 - 1.87 (m, 1H), 1.77 - 1.69 (m, 2H),1.20 - 1.11 (m, 2H), 0.65- 0.56 (m, 4H).

**Example 45:**

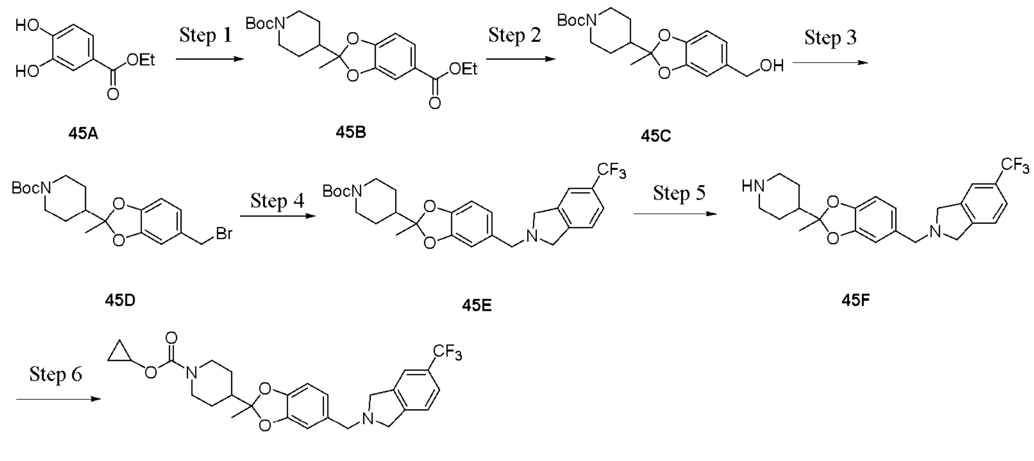


45A 45B 45C

45D 45E 45F

**Compound 45**

Step 1:

Ethyl 3,4-dihydroxybenzoate **45A** (1.1 g, 6.02 mmol), triruthenium dodecarbonyl (0.19 g, 0.30 mmol), and triphenylphosphine (0.16 g, 0.60 mmol) were dissolved in toluene (30 mL), and the resulting mixture was subjected to nitrogen protection, warmed to 90°C and stirred for 30 min. Then a solution of 1-Boc-4-ethynylpiperidine (2.14 g, 10.23 mmol) in toluene was added dropwise. After the dropwise addition was completed, the resulting mixture was warmed to 120°C, continuously stirred and reacted for 24 h. The reaction solution was cooled to room temperature, filtered through diatomaceous earth pad, and washed with ethyl acetate (20 mL × 2). The organic layers were combined, washed with saturated aqueous sodium chloride solution (30 mL), dried over anhydrous sodium sulfate, filtered, and concentrated. The residue was purified using a medium-pressure preparation instrument Biotage Isolera One (20 g silica gel column, eluent: 0-40% EA/PE) to obtain a mixture **45B** (2.1 g, 89.11%).

LC-MS (ESI): m/z = 292.2 $[M+H-100]^+$.

Step 2:

**45B** (0.95 g, 2.43 mmol) was dissolved in THF (20 mL), and lithium borohydride (0.064 g, 2.92 mmol) was added to the solution, and the resulting mixture was subjected to nitrogen protection and warmed to 70°C, continuously stirred and reacted for 18 h. The reaction solution was poured into 30 mL of diluted hydrochloric acid, and the resulting mixture was extracted with ethyl acetate (20 mL × 3). The organic phases were combined and washed with saturated aqueous sodium chloride solution (30 mL), dried over anhydrous sodium sulfate, filtered and concentrated. The residue was purified using a medium-pressure preparation instrument Biotage Isolera One (12 g silica gel column, eluent: 0-30% EA/PE) to obtain **45C** (0.45 g, 53%).

LC-MS (ESI): m/z = 332.1 $[M-OH]^+$.

Step 3:

At room temperature, **45C** (0.3 g, 0.86 mmol) and carbon tetrabromide (0.29 g, 0.86 mmol) were dissolved in DCM (15 mL), and the resulting mixture was stirred for 5 min. Triphenylphosphine (0.25 g, 0.95 mmol) was slowly added to the solution, and the resulting mixture was subjected to nitrogen protection, stirred and reacted for 18 h. The reaction solution was poured into water, and the resulting mixture was extracted with dichloromethane (20 mL × 3). The organic layers were combined and washed with saturated aqueous sodium chloride solution (30 mL), dried over anhydrous sodium sulfate, filtered and concentrated. The residue was purified using a medium-pressure preparation instrument Biotage Isolera One (12 g silica gel column, eluent: 0-30% EA/PE) to obtain **45D** (0.22 g, 62.04%).

LC-MS (ESI): m/z = 357.0 $[M-55]^+$.

Step 4:

Compound **45D** (0.22 g, 0.53 mmol) and 5-trifluoromethylisoindoline hydrochloride (0.12 g, 0.53 mmol) were dissolved in DMF (10 mL), potassium carbonate (0.088 g, 0.64 mmol) was added, and the resulting mixture was stirred and reacted at 70°C for 18 h. The reaction solution was poured into ice water and the resulting mixture was extracted with ethyl acetate (20 mL × 3). The combined organic phase was washed with saturated aqueous sodium chloride solution (20 mL ×

2), dried over anhydrous sodium sulfate, filtered and concentrated. The residue was purified using a medium-pressure preparation instrument Biotage Isolera One (12 g silica gel column, eluent: 0-60% EA/PE) to obtain **45E** (0.215 g, 78.23%).
LC-MS (ESI): m/z= 519.3 $[M+H]^+$.

Step 5:

Compound **45E** (0.215 g, 0.41 mmol) was dissolved in DCM (10 mL), trifluoroacetic acid (3 mL) was added, and the resulting mixture was reacted at room temperature for 1 h. Upon complete depletion of raw materials as monitored by LCMS, the reaction solution was concentrated to obtain the trifluoroacetate of **45F** (0.20 g, 91%), which was directly used in the next step without purification.
LC-MS (ESI): m/z= 419.2 $[M+H]^+$.

Step 6:

Cyclopropanol (0.021 g, 0.36 mmol) and carbonyldiimidazole (0.058 g, 0.36 mmol) were dissolved in THF (10 mL), and the resulting mixture was stirred at room temperature for 30 min. Then triethylamine (0.073 g, 0.72 mmol) and the trifluoroacetate of compound **45F** (0.1 g, 0.19 mmol) were added, and the resulting mixture was subjected to nitrogen protection, warmed to 70°C, stirred and reacted for 18 h. The reaction solution was poured into ice water and the resulting mixture was extracted with ethyl acetate (20 mL × 3). The combined organic phase was washed with saturated aqueous sodium chloride solution (20 mL × 2), dried over anhydrous sodium sulfate, filtered and concentrated. The residue was purified using a medium-pressure preparation instrument Biotage Isolera One (12 g silica gel column, eluent: 0-5% MeOH/DCM) to obtain compound **45** (0.04 mg, 33.17%).
LC-MS (ESI): m/z= 503.3 $[M+H]^+$.
$^1H$ NMR (400 MHz, $CDCl_3$) δ 7.46 - 7.42 (m, 2H), 7.28 - 7.26 (m, 1H), 6.81 - 6.76 (m, 2H), 6.76 - 6.66 (m, 1H), 4.27 - 4.26 (m, 1H), 4.08 - 4.05(m, 1H), 4.04 (s, 4H), 3.80 (s, 2H), 2.72 - 3.66 (m, 2H), 2.00 - 1.95 (m, 1H), 1.85 - 1.82 (m, 2H), 1.62 - 1.50 (m, 4H), 1.43 - 1.37 (m, 2H), 0.69 - 0.63 (m, 4H).

**Example 46:**

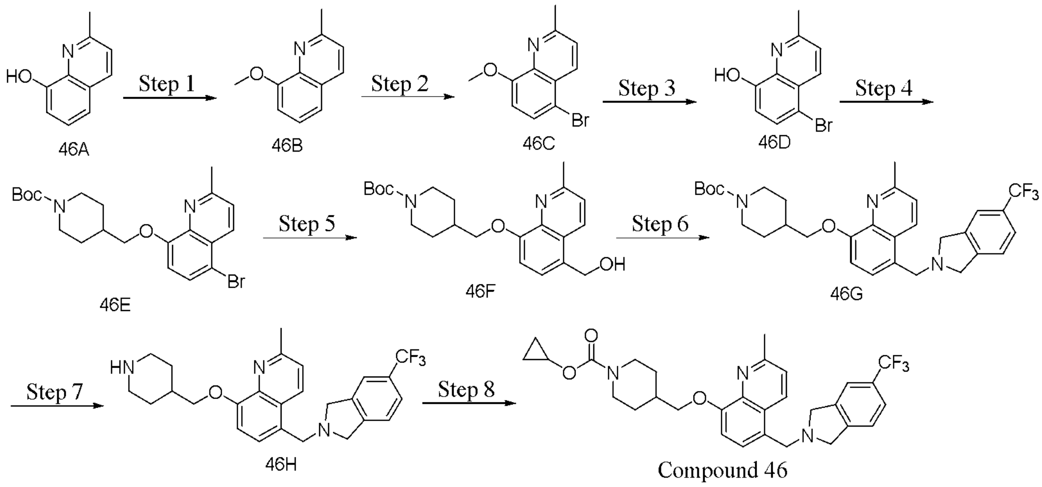


Step 1:

Compound 46A (1.00 g, 6.31 mmol) was dissolved in tetrahydrofuran (10 mL), sodium hydride (0.25 g, 6.31 mmol) was added, and the resulting mixture was stirred at 25°C for 30 min. Iodomethane (1.34 g, 9.48 mmol) was then added, and the resulting mixture was stirred at 25°C for 16 h. After the reaction was completed, the reaction product was concentrated, then slurried with water (20 mL), filtered, and washed twice with water. The filter cake was collected and dried to obtain the compound **46B** (0.9 g, 82.35%).
LCMS (ESI): m/z= 174.2 $[M+H]^+$

Step 2:

Compound 46B (1 g, 5.78 mmol) and N-bromosuccinimide (1.13 g, 6.36 mmol) were dissolved in tetrahydrofuran (25 mL), water (5 mL) was added, sulfuric acid (0.05 mL) was then added, and the resulting mixture was stirred at room temperature for 2 h. 20 mL of water was added, the resulting mixture was adjusted to PH=10 with saturated sodium bicarbonate and concentrated. The organic phase was removed, a large amount of solid was precipitated, filtered, and the filter cake was collected and dried to obtain compound 46C (1.2 g, 82.35%)
LCMS (ESI): m/z= 252.2 $[M+H]^+$.

Step 3:

Compound 46C (0.12 g, 0.49 mmol) was dissolved in dichloromethane (5 mL), boron tribromide (0.62 g, 2.49 mmol) was then added, and the resulting mixture was reacted at room temperature overnight. Water was added, the resulting mixture was extracted, and the organic phase was washed with saturated sodium bicarbonate, concentrated, and purified by column (ethyl acetate:petroleum ether = 5%-50%) to obtain compound 46D (0.1 g, 85.72%)
LCMS (ESI): m/z= 238.0 $[M+H]^+$.

Step 4:

Compound 46D (0.1 g, 0.42 mmol), tert-butyl 4-(bromomethyl)piperidine-1-carboxylate (0.12 g, 0.42 mmol) and cesium carbonate (0.27 g, 0.84 mmol) were dissolved in N,N dimethylformamide (5 mL), and the resulting mixture was stirred at 100°C for 3 h, cooled to room temperature, concentrated, and purified by column (ethyl acetate:petroleum ether = 5%-70%) to obtain compound 46E (0.12 g, 65.63%)
LCMS (ESI): m/z= 435.6 $[M+H]^+$.

Step 5:

Compound 46E (0.1 g, 0.23 mmol), (tributyltin)methanol (0.089 g, 0.28 mmol), chloro(2-dicyclohexylphosphino-2',4',6'-triisopropyl-1,1'-biphenyl)[2-(2'-amino-1,1'-biphenyl)]palladium(II) (0.018 g, 0.023 mmol) were dissolved in 1,4-dioxane (10 mL), and the resulting mixture was subjected to nitrogen protection, heated to 90°C and stirred for 3 h, cooled to room temperature, concentrated, and purified by column (ethyl acetate:petroleum ether = 5%-50%) to obtain compound 46F (0.08 g, 90.00%)
LCMS (ESI): m/z= 387.2 $[M+H]^+$.

Step 6:

Compound 46F (0.1 g, 0.26 mmol) and triethylamine (0.13 g, 1.3 mmol) were dissolved in dichloromethane (5 mL), methylsulfonyl chloride (0.033 g, 0.29 mmol) was then slowly added, and the resulting mixture was stirred at room temperature for half an hour. 5-(trifluoromethyl)isoindoline hydrochloride (0.070 g, 0.31 mmol) was then added, and the resulting mixture was stirred at room temperature for 3 h, concentrated, and purified by column (ethyl acetate:petroleum ether = 5%-50%) to obtain compound 46G (0.11 g, 76.14%)
LCMS (ESI): m/z=556.3 $[M+H]^+$.

Step 7:

Compound 46G (0.3 g, 0.54 mmol) was dissolved in dichloromethane (6 mL), trifluoroacetic acid (2 mL) was then added, and the resulting mixture was stirred at room temperature for 3 h, and concentrated to obtain crude 46H, which was directly used in the next step.
LCMS (ESI): m/z=456.3 $[M+H]^+$.

Step 8:

Cyclopropanol (0.029 g, 0.50 mmol), N,N'-carbonyldiimidazole (0.080 g, 0.49 mmol) and triethylamine (0.20 g, 1.98 mmol) were dissolved in tetrahydrofuran (5 mL), and the resulting mixture was stirred at room temperature for half an hour. Compound 46H (0.15 g, 0.33 mmol) was then added, and the resulting mixture was stirred at room temperature for 2 h, concentrated, and purified by reverse phase column (acetonitrile:water = 3%-70%) to obtain compound 46 (0.12 g, 67.39%)
$^1$H NMR (400 MHz, DMSO-$d_6$) δ 8.54 (d, 1H), 7.57 (s, 1H), 7.53 (d, 1H), 7.43-7.29 (m, 3H), 7.10 (d, 1H), 4.18 (s,

2H), 4.13 - 3.90 (m, 5H), 3.88 (s, 4H), 2.86-2.82 (m, 2H), 2.63 (s, 3H), 2.12-2.07(m, 1H), 1.88 (d, 2H), 1.26-1.22 (m, 2H), 0.69 - 0.56 (m, 4H).

LCMS (ESI): m/z=540.3[M+H]$^+$.

**Example 47:**

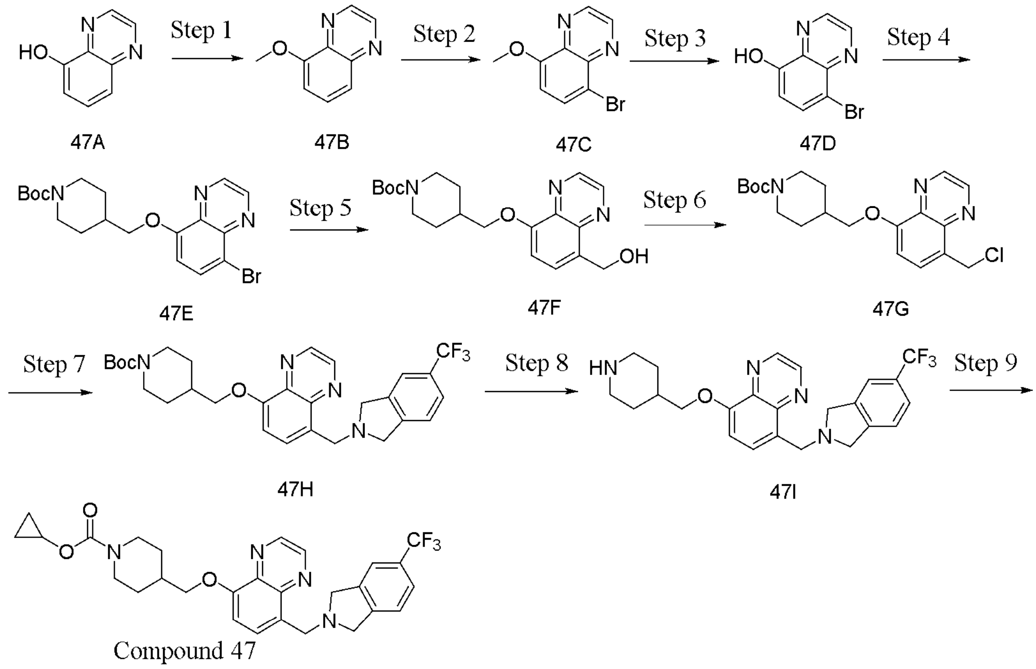


Step 1:

47A (8 g, 54.78 mmol) was dissolved in tetrahydrofuran (80 mL), then sodium hydride (3.29 g, 136.95 mmol) was slowly added in portions in an ice-water bath, and after half an hour, iodomethane (11.66 g, 82.17 mmol) was added, and then the resulting mixture was reacted at 60 degrees for 6 h. After the reaction was completed, the reaction product was diluted by adding water, and the resulting mixture was extracted with ethyl acetate. The organic phases were combined, subjected to rotary evaporation, and purified by normal phase chromatography (petroleum ether:ethyl acetate = 4:1) to obtain product 47B (6.4 g, 73%).
LC-MS (ESI): m/z=161.10 [M+H]$^+$

Step 2:

47B (3 g, 18.73 mmol) and N-bromosuccinimide (5.00 g, 28.09 mmol) were dissolved in acetonitrile (40 mL), and then the resulting mixture was reacted at 50 degrees for 4 h. After the reaction was completed, the reaction product was subjected to rotary evaporation, and purified by normal phase chromatography (petroleum ether:ethyl acetate = 4:1) to obtain product 47C (4.5 g, 100%).

LC-MS (ESI): m/z=239.1[M+H]$^+$.

Step 3:

47C (5 g, 20.91 mmol) was dissolved in dichloromethane (70 mL), then boron tribromide (78.58 g, 313.64 mmol) was slowly added in portions, and then the resulting mixture was reacted at 40 degrees overnight. After the reaction was completed, the reaction product was carefully poured into water and the reaction was quenched with saturated sodium bicarbonate, and then the combined organic phase was extracted with dichloromethane and subjected to rotary evaporation to obtain crude 47D (4 g, 85%).

LC-MS (ESI): m/z=224.9 [M+H]$^+$.

Step 4:

47D (2 g, 8.89 mmol), tert-butyl 4-[(methanesulfonyloxy)methyl]piperidine-1-carboxylate (3.91 g, 13.34 mmol) and potassium carbonate (3.07 g, 22.23 mmol) were dissolved in N,N-dimethylformamide (25 ml), and then the resulting mixture was reacted at 80 degrees for 3 h. After the reaction was completed, the reaction product was diluted by adding water, and the resulting mixture was extracted with ethyl acetate 3 times. The organic phases were combined, subjected to rotary evaporation, and purified by normal phase chromatography (petroleum ether:ethyl acetate = 3:1) to obtain product 47E (2.3 g, 61%).
LC-MS (ESI): m/z=422.2 $[M+H]^+$

Step 5:

47E (2 g, 4.74 mmol), chloro(2-dicyclohexylphosphino-2',4',6'-triisopropyl-1,1'-biphenyl)[2-(2'-amino-1,1'-biphenyl)]palladium(II) (0.37 g, 0.47 mmol) and (tributylstannyl)methanol (2.28 g, 7.11 mmol) were dissolved in 1,4-dioxane (25 mL), and the resulting mixture was subjected to nitrogen replacement 3 times, and then reacted at 90 degrees for 6 h. After the reaction was completed, the reaction product was directly subjected to rotary evaporation, spiked onto silica gel and purified by normal phase chromatography (petroleum ether:ethyl acetate = 1:2) to obtain product 47F (0.6 g, 34%).
LC-MS (ESI): m/z=374.3 $[M+H]^+$

Step 6:

47F (0.32 g, 0.86 mmol) was dissolved in dichloromethane (8 mL), N,N-dimethylformamide (0.05 mL) was added, oxalyl chloride (0.13 g, 1.02 mmol) was slowly added in portions in an ice-water bath, and the reaction was continued for 1 h. After the reaction was completed, the reaction was quenched with saturated sodium bicarbonate solution, and then the combined organic phase was extracted with dichloromethane 3 times and subjected to rotary evaporation to obtain 47G (0.3 g, 89%).
LC-MS (ESI): m/z=392.1 $[M+H]^+$

Step 7:

47G (0.3 g, 0.77 mmol), 5-(trifluoromethyl)-2,3-dihydro-1H-isoindole (0.19 g, 1.00 mmol) and potassium carbonate (0.21 g, 1.54 mmol) were dissolved in N,N-dimethylformamide (10 mL), and the resulting mixture was reacted at 80 degrees for 3 h. After the reaction was completed, the reaction product was diluted by adding water, and the resulting mixture was extracted with ethyl acetate. The organic phases were combined, subjected to rotary evaporation, and purified by normal phase chromatography (petroleum ether:ethyl acetate = 1:1) to obtain product 47H (0.1 g, 24%).
LC-MS (ESI): m/z=543.3 $[M+H]^+$

Step 8:

47H (0.090 g, 0.17 mmol) was dissolved in dichloromethane (4 mL), trifluoroacetic acid (1.53 g, 13.42 mmol) was added, and the resulting mixture was reacted at room temperature for 2 h. After the reaction was completed, the reaction product was directly subjected to rotary evaporation to obtain crude 47I (70 mg, 93%).
LC-MS (ESI): m/z=443.3 $[M+H]^+$.

Step 9:

Cyclopropanol (0.019 g, 0.32 mmol) and N,N-carbonyldiimidazole (0.052 g, 0.32 mmol) were dissolved in tetrahydrofuran (4 mL), and the resulting mixture was reacted at room temperature for 0.5 h. Then triethylamine (0.065 g, 0.64 mmol) and 47I (0.070 g, 0.16 mmol) were added, and then the resulting mixture was refluxed at 70 degrees overnight. After the reaction was completed, the reaction product was directly subjected to rotary evaporation and then preparative HPLC. Preparative HPLC separation method: 1. Instrument: waters 2767 preparative liquid chromatographic instrument; chromatographic column: SunFire@ PrepC18 (19 mm × 250 mm); 2. The sample was filtered with a 0.45 μm filter to prepare a sample liquid. 3. Preparative chromatography conditions: a. composition of mobile phases A and B: mobile phase A: acetonitrile; mobile phase B: water (containing 0.5% ammonium acetate); b. gradient elution, mobile phase A: 20%-60%; c. flow rate: 15 mL/min. Compound 47 (8 mg, 9.5%) was obtained after lyophilization.
$^1H$ NMR (400 MHz, DMSO-$d_6$) δ 8.97 (d, 1H), 8.92 (d, 1H), 7.84 (d, 1H), 7.59 (s, 1H), 7.54 (d, 1H), 7.43 (d, 1H), 7.30 (d, 1H), 4.44 (s, 2H), 4.08 (d, 2H), 3.99 (s, 4H), 3.27(s,5H), 2.82 (d, 1H), 1.27 (d, 4H), 0.66-0.59 (m, 4H).
LC-MS (ESI): m/z=527.2 $[M+H]^+$.

**Example 48:**

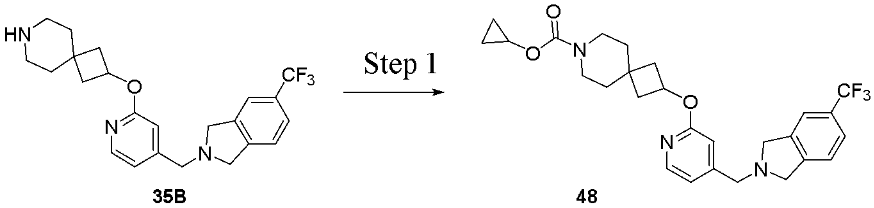


35B

48

Step 1

Cyclopropanol (87 mg, 1.5 mmol) was dissolved in tetrahydrofuran (10 mL), CDI (243 mg, 1.5 mmol) was then added, and the resulting mixture was stirred and reacted at room temperature for 0.5 h. A solution of 35B (418 mg, 1.0 mmol) in tetrahydrofuran (10 mL), and triethylamine (303 mg, 3.0 mmol) were added, and the resulting mixture was heated to 60°C and reacted overnight. After the reaction was completed as monitored by TLC, the reaction product was concentrated and the residue was separated and purified by silica gel column chromatography (dichloromethane: methanol (v/v) = 20:1) to obtain compound 48 (130 mg, 26%).

LC-MS (ESI): *m/z* = 502.2 $[M+H]^+$.

$^1H$ NMR (400 MHz, $CDCl_3$) $\delta$ 8.09 (d, 1H), 7.07 (d, 1H), 6.95 - 6.91 (m, 1H), 6.91 - 6.85 (m, 2H), 6.78 (s, 1H), 4.33 (t, 2H), 4.26 - 3.76 (m, 9H), 3.74 - 3.66 (m, 1H), 2.78 - 2.66 (m, 2H), 1.80 - 1.60 (m, 5H), 1.27 - 1.05 (m, 2H), 0.77 - 0.72 (m, 4H), 0.72 - 0.64 (m, 4H).

$^{19}F$ NMR (376 MHz, $CDCl_3$) $\delta$ -60.04 (s).

**Example 49:**

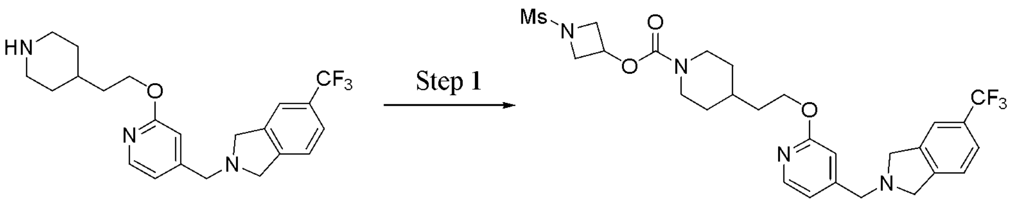


26D

Compound 49

Step 1:

1-methylsulfonylazetidin-3-ol (226.5 mg, 1.5 mmol) was dissolved in tetrahydrofuran (10 mL), CDI (240.0 mg, 1.5 mmol) was then added, and the resulting mixture was stirred and reacted at room temperature for 0.5 h. A solution of 26D (0.4 g, 1.0 mmol) in tetrahydrofuran (10 mL), and triethylamine (300.0 mg, 3.0 mmol) were added, and the resulting mixture was heated to 60°C and reacted overnight. After the reaction was completed as monitored by TLC, the reaction product was concentrated and the residue was separated and purified by silica gel column chromatography (dichloromethane: methanol (v/v)=20:1) to obtain compound 49 (250.0 mg, 57%).

LC-MS (ESI): *m/z* = 583.2 $[M+H]^+$.

$^1H$ NMR (400 MHz, $CDCl_3$) $\delta$ 8.10 (d, 1H), 7.51 - 7.40 (m, 2H), 7.29 (d, 1H), 6.93 - 6.92 (m, 1H), 6.78 (s, 1H), 5.20 - 5.04 (m, 1H), 4.35 (t, 2H), 4.21 - 3.94 (m, 10H), 3.88 (s, 2H), 2.94 - 2.66 (m, 5H), 1.83 - 1.66 (m, 5H), 1.26 - 1.16 (m, 2H).

$^{19}F$ NMR (377 MHz, $CDCl_3$) $\delta$ -60.02 (s).

**Example 50:**

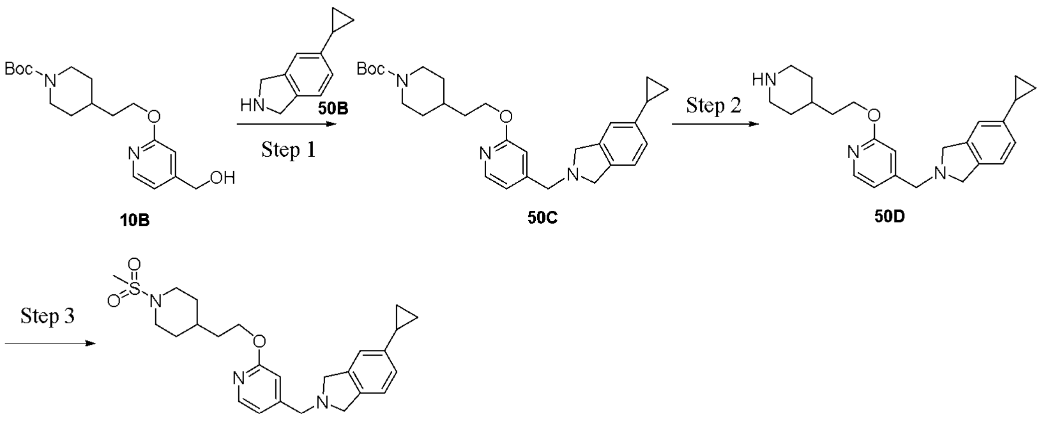


10B 50B 50C 50D

Compound 50

Step 1:

Compound 10B (0.65 g, 1.93 mmol) was dissolved in dichloromethane (20 mL), and triethylamine (0.37 g, 3.65 mmol) and methylsulfonyl chloride (0.28 g, 2.45 mmol) were successively added in an ice-water bath. The reaction solution was warmed to room temperature and stirring was continued for 1 h, triethylamine (0.37 g, 3.65 mmol) and compound 50B (0.38 g, 1.93 mmol) were then successively added, and the reaction solution was stirred at room temperature overnight. After the reaction was completed, the reaction product was concentrated, and the resulting crude product was purified by column chromatography (petroleum ether:ethyl acetate = 1:1 (v/v)) to obtain compound **50C** (0.55 g, 60%).
LC-MS (ESI): *m/z* = 478.70 $[M+H]^+$.

Step 2:

Compound 50C (0.55 g, 1.15 mmol) was dissolved in dichloromethane (10 mL), trifluoroacetic acid (3 mL) was added, and the resulting mixture was reacted at room temperature for 2 h. Upon complete depletion of raw materials as monitored by LCMS, the reaction solution was concentrated to obtain compound 50D (450 mg, crude product), which was directly used in the next step without purification.
LC-MS (ESI): *m/z* = 378.50 $[M+H]^+$.

Step 3:

Compound 50D (0.2 g, 0.53 mmol) was dissolved in dichloromethane (10 mL), and triethylamine (0.16 g, 1.59 mmol) and methylsulfonyl chloride (0.1 g, 0.87 mmol) were successively added in an ice-water bath. The reaction solution was warmed to room temperature and stirring was continued for 3 h. Upon complete depletion of raw materials as monitored by LCMS, the reaction solution was concentrated and purified by preparative HPLC to obtain compound 50 (75 mg, 31%).
LC-MS (ESI): *m/z* = 456.50 $[M+H]^+$.
$^1H$ NMR (400 MHz, DMSO-d6) δ 8.08 (d, 1H), 7.08 (d, 1H), 6.97 (d, 1H), 6.93-6.89 (m, 2H), 6.76 (s, 1H), 4.30 (t, 2H), 3.84-3.77 (m, 6H), 3.56-3.48 (m, 2H), 2.82 (s, 3H), 2.72-2.62 (m, 2H), 1.92-1.84 (m, 1H), 1.83-1.76 (m, 2H), 1.71-1.64 (m, 2H), 1.60-1.52 (m, 1H), 1.28-1.17 (m, 2H), 0.93-0.87 (m, 2H), 0.63-0.57 (m, 2H).

**Example 51:**

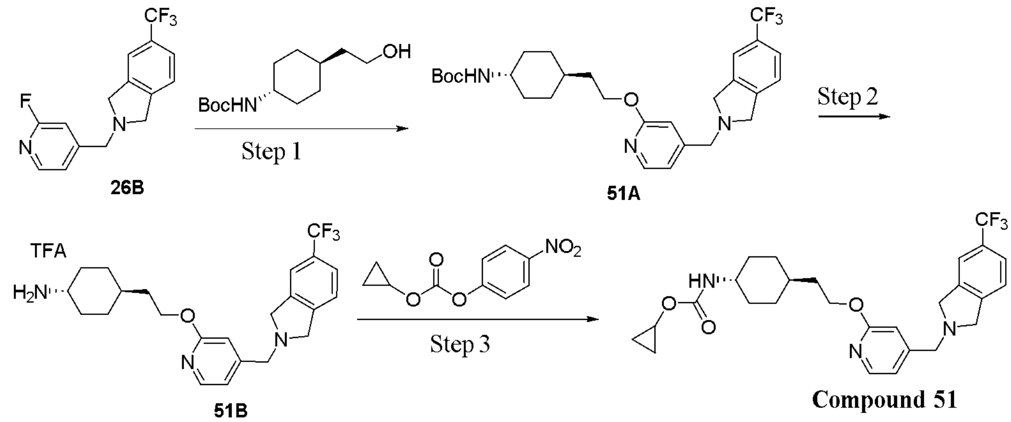


26B 51A 51B Compound 51

Step 1:

Trans-1-(BOC-amino)-4-(2-hydroxyethyl)cyclohexane (1.5 g, 6 mmol) was dissolved in THF (20 mL), NaH (0.3 g, 7 mmol) was added in an ice-water bath, and the resulting mixture was transferred to room temperature and reacted for 1 h. **26B** (0.9 g, 3 mmol) was added, and the resulting mixture was reacted at room temperature for 24 h. After the reaction was completed, water (10 mL) was added to quench the reaction, and the resulting mixture was extracted with EA (20 mL × 2). The organic layers were combined, washed with saturated aqueous sodium chloride solution (25 mL × 1), dried over anhydrous sodium sulfate, filtered, and concentrated. The residue was separated and purified by silica gel column chromatography (PE/EA = 1/1) to obtain **51A** (1.1 g, 70%)

LC-MS (ESI): *m/z* = 420.6 $[M+H-100]^+$.

Step 2:

Compound **51A**(1.1 g, 1.9 mmol) was dissolved in dichloromethane (10 mL). Trifluoroacetic acid (8.0 mL) was then added, and the resulting mixture was reacted at room temperature for 10 min and then concentrated to obtain compound **51B** (900 mg, 92%), which was directly used in the next step.

LC-MS (ESI): *m/z* = 420.6 $[M+H]^+$.

Step 3:

**51B** (900 mg, 1.7 mmol) was dissolved in dichloromethane (20 mL), DIEPA (0.8 g, 6.0 mmol) and **27B** (0.7 g, 3 mmol) were added, and the resulting mixture was stirred at room temperature for 30 min. After the reaction was completed, the solvent was concentrated and the residue was separated and purified by silica gel column chromatography (PE/EA=1/1) to obtain compound **51** (200 mg, 14%).

LC-MS (ESI): *m/z* = 504.6 $[M+H]^+$.

$^1H$ NMR (400 MHz, $CDCl_3$) δ 8.10 (d,1H), 7.47 (d, 1H), 7.44 (s, 1H), 7.29 (d, 1H), 6.92 (d, 1H), 6.77 (s, 1H), 4.41 (s, 1H), 4.31 (t, 2H), 4.07 - 4.03 (m, 1H), 4.02 (s, 4H), 3.87 (s, 2H), 3.44 (s, 1H), 2.01 (s, 2H), 1.85 (d, 2H), 1.68 (q, 2H), 1.45 (s, 1H), 1.21 - 1.01 (m, 4H), 0.65 (s, 4H).

$^{19}F$ NMR (400 MHz, $CDCl_3$) δ -59.81 (s).

**Example 52:**

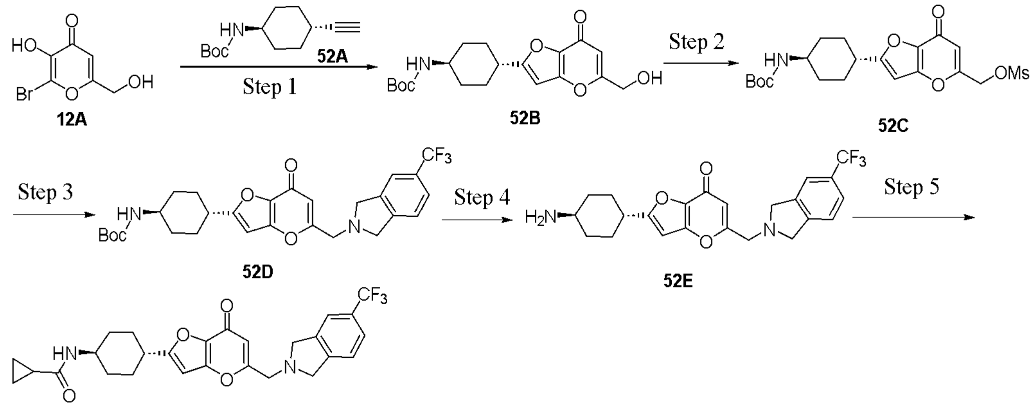


12A 52A 52B 52C 52D 52E

Compound 52

Step 1:

At room temperature, compound 12A (3.0 g, 13.57 mmol), compound 52A (3.33 g, 14.93 mmol), bis(triphenylphosphine)palladium dichloride (0.95 g, 1.36 mmol) and cuprous iodide (0.52 g, 2.71 mmol) were dissolved in tetrahydrofuran (100 mL), triethylamine (4.38 g, 43.28 mmol) was then added under nitrogen atmosphere, and the reaction solution was warmed to 40°C and stirred for 4 h. After the reaction was completed, the reaction product was filtered, and the filtrate was concentrated under reduced pressure, and purified by column chromatography (dichloromethane:ethyl acetate = 1:3 (v/v)) to obtain compound 52B (1.1 g, 23%).
LC-MS (ESI): *m*/*z* = 364.30 $[M+H]^+$.

Step 2:

Compound 52B (0.5 g, 1.38 mmol) was dissolved in dichloromethane (15 mL), and triethylamine (0.31 g, 3.10 mmol) and methylsulfonyl chloride (0.36 g, 2.06 mmol) were successively added in an ice-water bath. The reaction solution was warmed to room temperature, and then stirring was continued for 1 h. After the reaction was completed, the reaction product was concentrated, and the resulting crude product was purified by column chromatography (petroleum ether:ethyl acetate = 3:1 (v/v)) to obtain compound 52C (0.25 g, 41%).
LC-MS (ESI): *m*/*z* = 442.80 $[M+H]^+$.

Step 3:

Compound 52C (200 mg, 0.45 mmol) and 5-trifluoromethylisoindoline hydrochloride (130 mg, 0.58 mmol) were dissolved in DCM (10 mL), triethylamine (0.14 g, 1.38 mmol) was then added, and the reaction solution was stirred at room temperature overnight. After the reaction was completed, the reaction product was concentrated, and the resulting crude product was purified by column chromatography (petroleum ether:ethyl acetate = 1:1 (v/v)) to obtain compound 50D (0.15 g, 62%).
LC-MS (ESI): *m*/*z* = 533.20 $[M+H]^+$.

Step 4:

Compound 50D (0.15 g, 0.28 mmol) was dissolved in dichloromethane (10 mL), hydrogen chloride dioxane solution (3 mL) was then added, and the resulting mixture was reacted at room temperature for 2 h. Upon complete depletion of raw materials as monitored by LCMS, the reaction solution was concentrated to obtain compound 50E (120 mg, crude product), which was directly used in the next step without purification.
LC-MS (ESI): *m*/*z* = 433.20 $[M+H]^+$.

Step 5:

Compound 50E (0.12 g, 0.28 mmol) was dissolved in dichloromethane (10 mL), and triethylamine (0.085 g, 0.84 mmol) and cyclopropanecarbonyl chloride (0.044 g, 0.42 mmol) were successively added in an ice-water bath. After the dropwise addition was completed, the reaction was continued for 4 h in an ice-water bath. Upon complete depletion of raw materials as monitored by LCMS, the reaction solution was concentrated and purified by preparative HPLC to obtain

compound 52 (20 mg, 14%).

LC-MS (ESI): *m/z* = 501.50 $[M+H]^+$.

$^1$H NMR (400 MHz, DMSO-$d_6$) δ 7.98 (d, 1H), 7.62 (s, 1H), 7.57 (d, 1H), 7.47 (d, 1H), 6.79 (s, 1H), 6.43 (s, 1H), 4.05 (s, 4H), 3.90 (s, 2H), 3.63-3.52 (m, 1H), 2.08-2.00 (m, 2H), 1.94-1.86 (m, 2H), 1.55-1.43 (m, 4H), 0.92-0.80 (m, 1H), 0.68-0.58 (m, 4H).

**Example 53:**

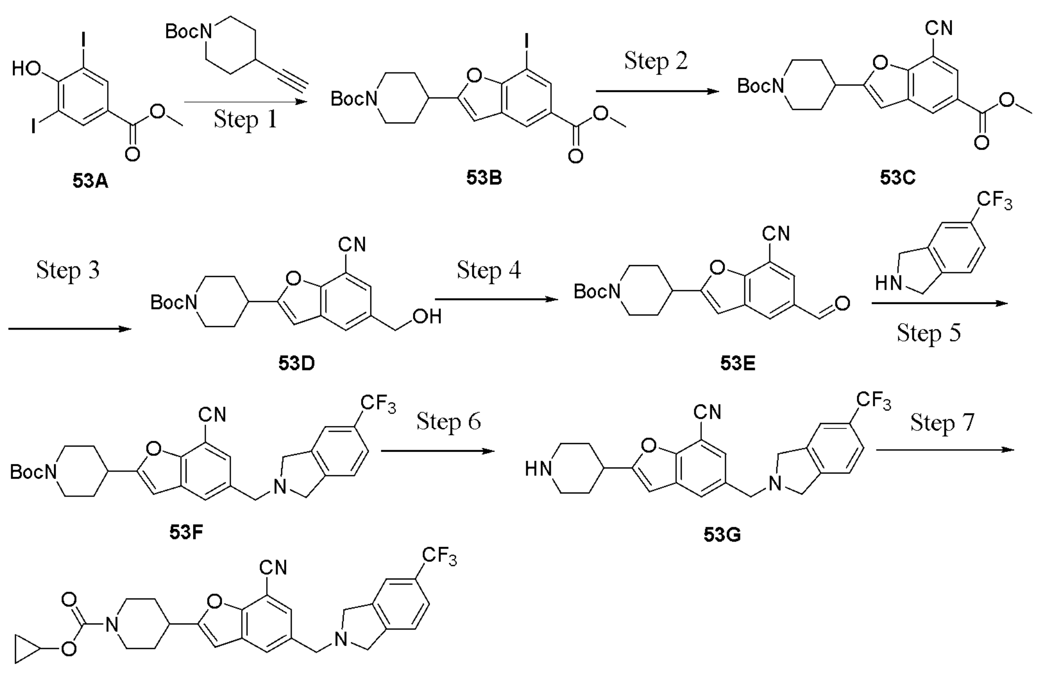


53A 53B 53C 53D 53E 53F 53G

**Compound 53**

Step 1:

At room temperature, compound 53A (400 mg, 0.99 mmol), tert-butyl 4-ethynylpiperidine-1-carboxylate (248 mg, 1.19 mmol) and cuprous oxide (71 mg, 0.50 mmol) were added to pyridine (10 mL), and the resulting mixture was subjected to nitrogen replacement three times. The reaction solution was warmed to 120°C and stirred for 4 h. After the reaction was completed, the reaction product was filtered, and the filtrate was concentrated under reduced pressure, and then purified by silica gel column chromatography (eluent: petroleum ether: ethyl acetate = 1:1 (v/v)) to obtain the target compound 53B (450 mg, 94%).

LC-MS (ESI): *m/z*= 430.1$[M-{}^tBu+H]^+$.

Step 2:

53B (450 mg, 0.92 mmol), tetrakis(triphenylphosphine)palladium (106 mg, 0.092 mmol) and zinc cyanide (162 mg, 1.38 mmol) were added to ultra-dry DMF (20 mL), and the resulting mixture was subjected to nitrogen replacement three times. The reaction solution was warmed to 100°C and stirred for 6 h. After the reaction was completed, the reaction product was filtered, and the filtrate was diluted with EA and washed twice with water. The organic layer was washed with saturated NaCl solution, dried over anhydrous $Na_2SO_4$, filtered and subjected to rotary evaporation; silica gel column chromatography (eluent: petroleum ether: ethyl acetate = 1:1 (v/v)) to obtain the target compound 53C (240 mg, 68%) LC-MS (ESI): *m/z* = 329.2 $[M-{}^tBu+H]^+$

Step 3:

53C (240 mg, 0.63 mmol) was dissolved in diethyl ether (10 mL), then lithium borohydride (69 mg, 3.15 mmol) was added, and the resulting mixture was stirred at room temperature for 1 h. The reaction solution was quenched with

saturated ammonium chloride solution, and the aqueous phase was extracted with EA. The organic layers were combined, washed with saturated NaCl solution, dried over anhydrous $Na_2SO_4$, filtered and subjected to rotary evaporation; silica gel column chromatography (eluent: petroleum ether: ethyl acetate = 1:1 (v/v)) to obtain the target compound 53D (220 mg, 98%).

LC-MS (ESI): *m*/*z* = 301.2$[M-^{t}Bu+H]^{+}$.

Step 4:

53D (220 mg, 0.62 mmol) was dissolved in DCM (10 mL), and Dess-Martin oxidant (393 mg, 0.93 mmol) was added in portions at room temperature. After the addition was completed, the resulting mixture was reacted at room temperature for two hours. The reaction was quenched with saturated aqueous sodium thiosulfate solution, and the aqueous phase was extracted with dichloromethane. The organic layers were combined, washed with saturated NaCl solution, dried over anhydrous $Na_2SO_4$, filtered and subjected to rotary evaporation; silica gel column chromatography (eluent: petroleum ether: ethyl acetate = 1:1 (v/v)) to obtain the target compound 53E (204 mg, 93%)

LC-MS (ESI): *m*/*z* = 299.2 $[M-^{t}Bu+H]^{+}$.

Step 5:

53E (184 mg, 0.52 mmol) was dissolved in methanol (10 mL), then 5-trifluoromethylyl-2,3-dihydro1H-isoindole hydrochloride (139 mg, 0.62 mmol) was added, and the resulting mixture was stirred at room temperature for 1 h. Sodium cyanoborohydride (49 mg, 0.78 mmol) was added, and the resulting mixture was reacted at room temperature for one hour. The reaction solution was quenched with saturated aqueous sodium bicarbonate solution, the aqueous phase was extracted with dichloromethane, and the organic layers were combined, washed with saturated NaCl solution, dried over anhydrous Na2SO4, filtered, subjected to rotary evaporation, and subjected to silica gel column chromatography (eluent: dichloromethane: methanol (v/v) = 10:1) to obtain the target compound 53F (204 mg, 75%).

LC-MS (ESI): *m*/*z* = 470.2$[M-^{t}Bu+H]^{+}$.

Step 6:

53F (204 mg, 0.39 mmol) was dissolved in DCM (5 mL), then trifluoroacetic acid (1 mL) was added, and the resulting mixture was reacted at room temperature for 1 h. After the reaction was completed, the reaction solution was concentrated, and the crude product 53G was directly used in the next reaction without purification.

Step 7:

Cyclopropanol (34 mg, 0.59 mmol) was dissolved in tetrahydrofuran (5.0 mL), CDI (96 mg, 0.59 mmol) was then added, and the resulting mixture was stirred and reacted at room temperature for 0.5 h. A solution of the product 53G obtained in the previous step in tetrahydrofuran (5.0 mL), and triethylamine (191.0 mg, 1.9 mmol) were added, and the resulting mixture was heated to 60°C and reacted overnight. After the reaction was completed as monitored by TLC, the reaction product was concentrated and the residue was separated and purified by silica gel column chromatography (eluent: petroleum ether: ethyl acetate (v/v) = 1:3) to obtain compound 53 (90 mg, 46%).

LC-MS (ESI): *m*/*z* = 510.2 $[M+H]^{+}$.

$^{1}H$ NMR (400 MHz, Chloroform-d) $\delta$ 7.79 (s, 1H), 7.58 (d, 1H), 7.53 - 7.39 (m, 2H), 7.29 (d, 1H), 6.44 (s, 1H), 4.46 - 3.78 (m, 9H), 3.07 - 2.82 (m, 3H), 2.12 (d, 2H), 1.70 (d, 2H), 0.80 - 0.62 (m, 4H).

**Example 54:**

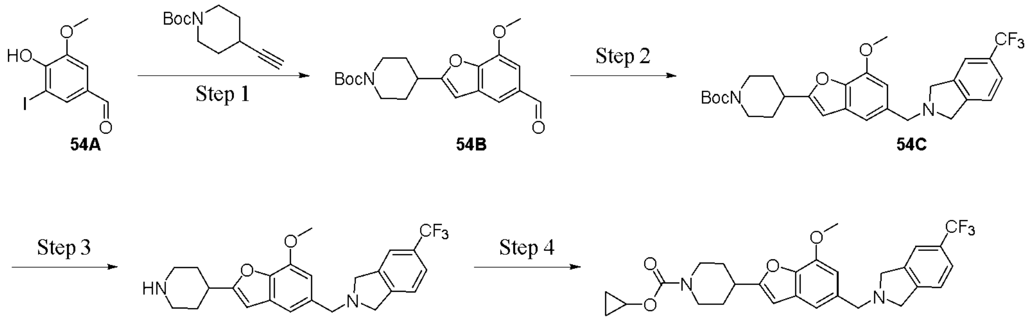


54A 54B 54C

54D 54

Step 1:

54A (2.78 g, 10.0 mmol) was dissolved in pyridine (10 mL), 1-Boc-4-ethynylpiperidine (2.52 g, 12.0 mmol) and cuprous oxide (0.72 g, 5.0 mmol) were then added, and the resulting mixture was heated to 120°C, stirred and reacted for 4 h. The reaction product was concentrated to remove the solvent, and the residue was separated and purified by silica gel column chromatography (petroleum ether: ethyl acetate (v/v) = 10:1) to obtain compound 54B (2.0 g, 55%).

LC-MS (ESI): *m*/*z* = 360.1 $[M+H]^+$.

Step 2:

54B (2.0 g, 5.56 mmol) was dissolved in 1,2-dichloroethane (20 mL), then 5-trifluoromethylyl-2,3-dihydro1H-isoindole hydrochloride (1.24 g, 5.56 mmol) and sodium borohydride acetate (2.36 g, 11.16 mmol) were added, and finally acetic acid (0.27 g, 4.45 mmol) was added, and the resulting mixture was reacted at room temperature overnight. Saturated aqueous sodium bicarbonate solution was added for quenching, and the aqueous phase was extracted with dichloromethane. The organic layers were combined, washed with saturated NaCl solution, dried over anhydrous $Na_2SO_4$, filtered and subjected to rotary evaporation to obtain the target compound 54C (2.2 g, 74%).

LC-MS (ESI): *m*/*z* = 531.2 $[M+H]^+$.

Step 3:

54C (2.0 g, 3.77 mmol) was dissolved in DCM (10 mL), then trifluoroacetic acid (4.0 mL) was added, and the resulting mixture was reacted at room temperature for 1 h. After the reaction was completed, the reaction solution was concentrated to obtain compound 54D, which was directly used in the next reaction.

Step 4:

Cyclopropanol (170.0 mg, 3.0 mmol) was dissolved in tetrahydrofuran (10.0 mL), CDI (490.0 mg, 3.0 mmol) was then added, and the resulting mixture was stirred and reacted at room temperature for 0.5 h. Triethylamine (601.0 mg, 6.0 mmol) and a solution of 54D (860.0 mg, 2.0 mmol) in tetrahydrofuran (5.0 mL) were successively added, and the resulting mixture was heated to 60°C and reacted overnight. After the reaction was completed as monitored by TLC, the reaction product was concentrated and the residue was separated and purified by silica gel column chromatography (petroleum ether: ethyl acetate (v/v) = 5:1) to obtain compound 54 (500.0 mg, 48%).

LC-MS (ESI): *m*/*z* = 515.2 $[M+H]^+$.

$^1$H NMR (400 MHz, DMSO) δ 7.59 (s, 1H), 7.55 (d, 1H), 7.44 (d, 1H), 7.11 (s, 1H), 6.88 (s, 1H), 6.56 (s, 1H), 4.03 - 3.98 (m, 2H), 3.91 (d, 9H), 3.27 (s, 2H), 3.07 - 2.87 (m, 3H), 2.07 - 1.94 (m, 2H), 1.61 - 1.42 (m, 2H), 0.65 - 0.62 (m, 4H).

$^{19}$F NMR (377 MHz, $CDCl_3$) δ -58.30 (s).

**Example 55:**

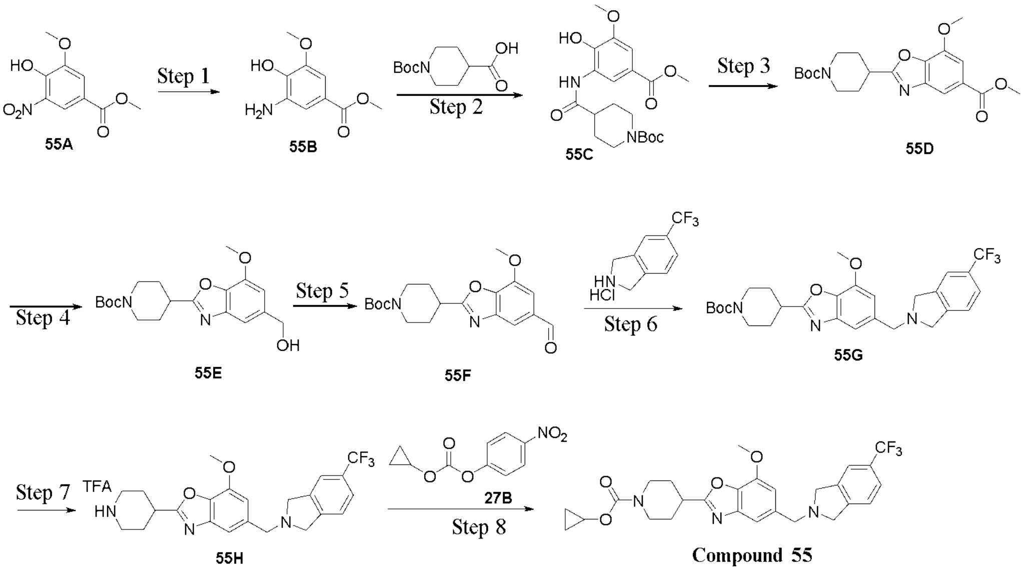


Step 1:

**55A** (11.4 g, 50 mmol) was dissolved in methanol (40 mL), Pd/C (1.1 g, 10% wt) was added, and hydrogen replacement was performed 3 times using hydrogen balloon, and the resulting mixture was reacted at room temperature for 18 h. After the reaction was completed, the reaction solution was filtered and concentrated to obtain 55B (9.2 g, 94%).
LC-MS (ESI): m/z = 198 $[M+H]^+$.

Step 2:

N-BOC-4-piperidinecarboxylic acid (3.9 g, 20 mmol) was dissolved in DMF (40 mL), and **55B** (4.6 g, 20 mmol) and N-methylimidazole (6.6 g, 80 mmol) were added. At 0°C, TCFH (8.4 g, 30 mmol) was added, and the resulting mixture was stirred at room temperature for 20 min. After the reaction was completed, 40 mL of water was added to the reaction solution, and the resulting mixture was extracted with ethyl acetate (40 mL × 3). The organic layers were combined, washed with saturated aqueous sodium chloride solution (30 mL × 3), dried over anhydrous sodium sulfate, filtered, and concentrated. The residue was separated and purified by silica gel column chromatography (dichloromethane: methanol (v/v) = 20:1) to obtain **55C** (5.3 g, 65%).
LC-MS (ESI): m/z = 409 $[M+H]^+$.

Step 3:

Triphenylphosphine (7.9 g, 30 mmol) was dissolved in toluene (50 mL), DDQ (6.8 g, 30 mmol) was added, and the resulting mixture was stirred at room temperature for 20 min. Then 55C (4.1 g, 10 mmol) was added, and the resulting mixture was reacted at 110°C for 12 h. After the reaction was completed, ethyl acetate (50 mL) was added to the reaction solution, and the resulting mixture was washed with saturated aqueous sodium chloride solution (30 mL × 3), dried over anhydrous sodium sulfate, filtered, and concentrated. The residue was separated and purified by silica gel column chromatography (ethyl acetate: petroleum ether (v/v) = 1:1) to obtain **55D** (3.5 g, 90%).
LC-MS (ESI): m/z = 335 $[M+H-56]^+$.

Step 4:

Compound **55D** (3.1 g, 8 mmol) was dissolved in THF (30 mL). At 0°C, DABAL-H (24 mL, 1 M) was added, and the resulting mixture was transferred to room temperature and reacted for 1 h. After the reaction was completed, diluted hydrochloric acid (30 mL) was added to quench the reaction, and ethyl acetate (20 mL × 2) was added for extraction. The organic phases were combined, washed with saturated aqueous sodium chloride solution (30 mL × 3), dried over anhydrous sodium sulfate, filtered, and concentrated. The residue was separated and purified by silica gel column chromatography (ethyl acetate: petroleum ether (v/v) = 2:1) to obtain **55E** (2.2 g, 75%).
LC-MS (ESI): m/z = 307 $[M+H-56]^+$.

Step 5:

**55E** (2.2 g, 6 mmol) was dissolved in ethyl acetate (20 mL), IBX (3.4 g, 12 mmol) was added, and the resulting mixture was reacted at 80°C for 12 h. After the reaction was completed, the reaction product was filtered and concentrated to obtain **55F** (2.1 g, 97%).

Step 6:

**55F** (1.8 g, 5 mmol) was dissolved in DCE (20 mL), 5-trifluoromethylisoindoline hydrochloride (1.1 g, 5.0 mmol), sodium borohydride acetate (1.4 g, 6.8 mmol) and 5 drops of glacial acetic acid were successively added, and the resulting mixture was reacted at room temperature for 1 h. After the reaction was completed, 10 mL of water was added to the reaction solution, the resulting mixture was extracted with DCM (20 mL × 2), and the organic layers were combined, washed with saturated aqueous sodium chloride solution (25 mL × 1), dried over anhydrous sodium sulfate, filtered, and concentrated. The residue was separated and purified by silica gel column chromatography (PE/EA = 2/1) to obtain **55G** (2.1 g, 80%).

Step 7:

Compound **55G** (2.1 g, 4 mmol) was dissolved in dichloromethane (10 mL). Trifluoroacetic acid (8.0 mL) was then added, and the resulting mixture was reacted at room temperature for 10 min and then concentrated to obtain compound **55H** (2.0 g, 90%), which was directly used in the next step.

Step 8:

**55H** (862 mg, 2.0 mmol) was dissolved in dichloromethane (20 mL), DIEPA (516 mg, 4.0 mmol) and **27B** (669 mg, 3 mmol) solution were added, and the resulting mixture was stirred at room temperature for 30 min. After the reaction was completed, the solvent was concentrated and the residue was separated and purified by silica gel column chromatography (PE/EA = 1/1) to obtain **25C** (300 mg, 30%).

LC-MS (ESI): *m/z* = 516.6 $[M+H]^+$.

$^1H$ NMR (400 MHz, CDC13) δ 7.46 (d, 1H), 7.42 (s, 1H), 7.28 (s, 2H), 6.98 (s, 1H), 4.36 - 4.03 (m, 3H), 4.01 (s, 3H), 3.96 (d, 6H), 3.22 - 3.09 (m, 1H), 3.10 - 2.93 (m, 2H), 2.15 (d, 2H), 2.04 - 1.84 (m, 2H), 0.69 (s, 4H).

$^{19}F$ NMR (400 MHz, $CDCl_3$) δ -59.98 (s).

**Example 56:**

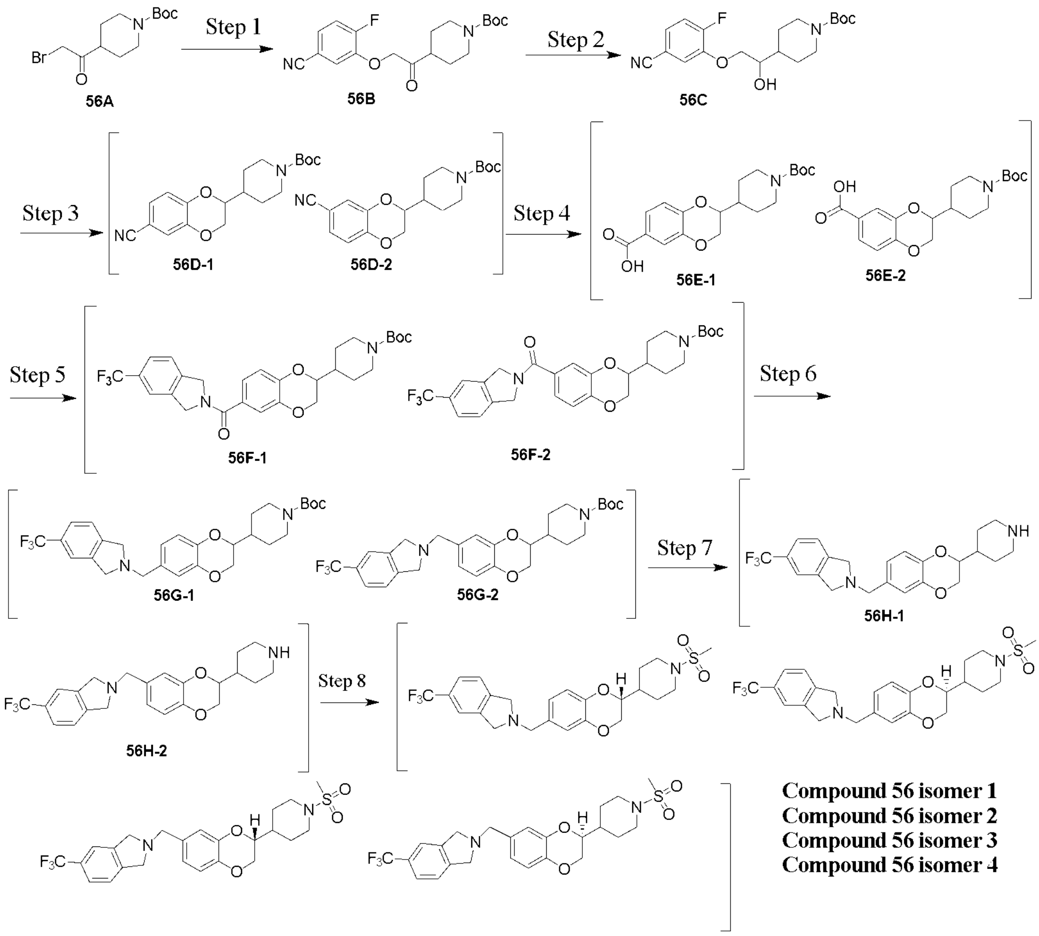


Step 1:

**56A** (4.4 g, 14.3 mmol), 4-fluoro-3-hydroxybenzonitrile (1.9 g, 14.3 mmol) and potassium carbonate (4.96 g, 35.9 mmol) were dissolved in acetonitrile (50 mL), and the resulting mixture was reacted at room temperature for 12 h. After the reaction was completed, water (30 mL) was added to the reaction system to quench the reaction, and the resulting mixture was extracted with ethyl acetate (30 mL*2), washed with saturated aqueous sodium chloride solution (20 mL), dried over anhydrous sodium sulfate, and filtered. The filtrate was concentrated to obtain crude **56B** (5.1 g, 98%).
LC-MS (ESI): m/z = 363.4 $[M+H]^+$.

Step 2:

Compound **56B** (5.1 g, 14.0 mmol) was dissolved in methanol (50 mL), sodium borohydride (1.6 g, 42.0 mmol) was added in an ice bath, and after the addition was completed, the resulting mixture was reacted at room temperature for 2 h. After the reaction was completed, water (30 mL) was added to the reaction system, and the resulting mixture was extracted with ethyl acetate (30 mL*3), washed with saturated aqueous sodium chloride solution (20 mL), dried over anhydrous sodium sulfate, and filtered. The filtrate was concentrated to obtain crude **56C** (5.1 g, 100%).
LC-MS (ESI): m/z = 365.1 $[M +1]^+$

Step 3:

Compound **56C** (5.1 g, 14.0 mmol) and potassium carbonate (4.83 g, 35.0 mmol) were dissolved in DMF (50 mL), and the resulting mixture was heated to 85°C and reacted for 12 h. The reaction was cooled to room temperature, then water (100 mL) was added to the reaction system to quench the reaction, and the resulting mixture was extracted with ethyl acetate (100 mL*2), washed with saturated aqueous sodium chloride solution (20 mL), dried over anhydrous sodium sulfate, and filtered. The filtrate was concentrated and the residue was separated and purified by silica gel column chromatography (petroleum ether: ethyl acetate (v/v) = 5:1) to obtain a mixture of **56D-1** and **56D-2** (2.1 g, 44%).
LC-MS (ESI): m/z = 345.2 $[M +1]^+$

Step 4:

The mixture of compounds **56D-1** and **56D-2** (816 mg, 2.37 mmol) and potassium hydroxide (1.99 g, 35.5 mmol) were dissolved in ethanol (10 mL) and water (5 mL), and the resulting mixture was reacted at 100°C for 24 h. After the reaction was stopped as monitored by LCMS, the reaction was cooled to room temperature, and adjusted to PH = 6-7 with diluted hydrochloric acid, and the resulting mixture was washed with ethyl acetate (30 mL*2) and saturated aqueous sodium chloride solution (20 mL), dried over anhydrous sodium sulfate, and filtered. The filtrate was concentrated to obtain a mixture of crude products **56E-1** and **56E-2** (608 mg, 70%).
LC-MS (ESI): m/z = 364.1[M+H]$^+$

Step 5:

The mixture of compounds **56E-1** and **56E-2** (608 mg, 1.67 mmol), HATU (954 mg, 2.51 mmol) and DIPEA (433 mg, 3.34 mmol) were dissolved in dichloromethane (20 mL), and the resulting mixture was reacted at room temperature for 15 min. 5-(trifluoromethyl)isoindoline hydrochloride (412 mg, 1.84 mmol) was added to the reaction system, and the resulting mixture was stirred at room temperature for 3 h. After the reaction was stopped as monitored by LCMS, water (10 ml) was added to quench the reaction, and the resulting mixture was extracted with dichloromethane (20 mL*2), washed with saturated aqueous sodium chloride solution (20 mL), dried over anhydrous sodium sulfate, and filtered. The filtrate was concentrated and the residue was separated and purified by silica gel column chromatography (petroleum ether: ethyl acetate (v/v) = 3:1) to obtain a mixture of **56F-1** and **56F-2** (455 mg, 51%)
LC-MS (ESI): m/z = 533.2[M+H]$^+$

Step 6:

The mixture of compounds **56F-1** and **56F-2** (455 mg, 0.85 mmol) was dissolved in anhydrous toluene (10 ml), 1 M DABAL-H (1.1 ml) was added at 0°C, this temperature was maintained and the resulting mixture was reacted for 6 h. After the reaction was completed as monitored by TLC, water (1.1 ml) and 15% NaOH (1.1 ml) were added, water (2.2 ml) was then added, and the resulting mixture was stirred, and dried over anhydrous sodium sulfate. The solid was filtered, washed with dichloromethane (10 ml), the organic phase was concentrated, and the residue was separated and purified by silica gel column chromatography (petroleum ether: ethyl acetate (v/v) = 2:1) to obtain a mixture of 56G-1 and **56G-2** (133 mg, 30%)
LC-MS (ESI): m/z = 519.2 [M+H]$^+$

Step 7:

The mixture of compounds **56G-1** and **56G-2** (133 mg, 0.25 mmol) was dissolved in dichloromethane (2 mL), trifluoroacetic acid (1 ml) was added, and the resulting mixture was reacted at room temperature for 2 h. After the reaction was completed as monitored by LCMS, the reaction product was directly concentrated to obtain a mixture of crude products **56H-1** and **56H-2** (107 mg), which was directly used in the next reaction without purification.
LC-MS (ESI): m/z = 419.1 [M+H]$^+$.

Step 8:

The mixture of compounds **56H-1** and **56H-2** (107 mg, 0.25 mmol) was dissolved in anhydrous dichloromethane (2 mL), triethylamine (126 mg, 1.25 mmol) was added, and the resulting mixture was stirred for 15 min. Methylsulfonyl chloride (34 mg, 0.30 mmol) was added, and the resulting mixture was reacted at room temperature for 3 h. The reaction solution was concentrated and the residue was separated and purified by silica gel column chromatography (petroleum ether: ethyl acetate (v/v) = 2:1) to obtain compound **56** (59 mg, 38%). The mixture of compound **56** was separated by SFC to obtain four isomers, compound **56-1** (12 mg, retention time 4.224 min, 8%), **56-2** (13 mg, retention time 4.748 min, 7%), **56-3** (6 mg, retention time 5.571 min, 1%) and **56-4** (5 mg, retention time 6.168 min, 2%)
Separation conditions of preparative chromatography: 1. Instrument: SHIMADZU LC-30AD sf; 2. chromatographic column: Chiral AD column; 3. mobile phase system: A for n-hexane; B for 0.05% DEA in ethanol and acetonitrile; 4. gradient: B 40%; 5. flow rate: 3 mL/min

Compound **56-1:**

LC-MS (ESI): m/z = 497.2 [M+H]$^+$.
$^1$H NMR (400 MHz, $CD_3OD$) δ 7.54 - 7.48 (m, 2H), 7.42 - 7.36 (m,1H), 6.94 - 6.89 (m, 1H), 6.89 - 6.79 (m, 2H), 4.36 -

4.28 (m , 1H), 4.07 - 3.99 (m, 1H), 3.95 (s, 4H), 3.80 (s, 2H), 3.78 - 3.74 (m, 1H), 3.31 - 3.29 (m, 2H), 2.82 (s, 3H), 2.79 - 2.69 (m, 2H), 2.15 - 2.02 (m, 1H), 1.90 - 1.77 (m, 2H), 1.63 - 1.50 (m, 2H).

Compound **56-2:**

LC-MS (ESI): m/z = 497.2 $[M+H]^+$.
$^1H$ NMR (400 MHz, $CD_3OD$) δ 7.54 - 7.48 (m, 2H), 7.42 - 7.36 (m,1H), 6.94 - 6.89 (m, 1H), 6.89 - 6.79 (m, 2H), 4.36 - 4.28 (m , 1H), 4.07 - 3.99 (m, 1H), 3.95 (s, 4H), 3.80 (s, 2H), 3.78 - 3.74 (m, 1H), 3.31 - 3.29 (m, 2H), 2.82 (s, 3H), 2.79 - 2.69 (m, 2H), 2.15 - 2.02 (m, 1H), 1.90 - 1.77 (m, 2H), 1.63 - 1.50 (m, 2H).

Compound **56-3:**

LC-MS (ESI): m/z = 497.2 $[M+H]^+$.
$^1H$ NMR (400 MHz, $CD_3OD$) δ 7.54 - 7.48 (m, 2H), 7.42 - 7.36 (m,1H), 6.94 - 6.89 (m, 1H), 6.89 - 6.79 (m, 2H), 4.36 - 4.28 (m , 1H), 4.07 - 3.99 (m, 1H), 3.95 (s, 4H), 3.80 (s, 2H), 3.78 - 3.74 (m, 1H), 3.31 - 3.29 (m, 2H), 2.82 (s, 3H), 2.79 - 2.69 (m, 2H), 2.15 - 2.02 (m, 1H), 1.90 - 1.77 (m, 2H), 1.63 - 1.50 (m, 2H).

Compound **56-4:**

LC-MS (ESI): m/z = 497.2 $[M+H]^+$.
$^1H$ NMR (400 MHz, $CD_3OD$) δ 7.54 - 7.48 (m, 2H), 7.42 - 7.36 (m,1H), 6.94 - 6.89 (m, 1H), 6.89 - 6.79 (m, 2H), 4.36 - 4.28 (m , 1H), 4.07 - 3.99 (m, 1H), 3.95 (s, 4H), 3.80 (s, 2H), 3.78 - 3.74 (m, 1H), 3.31 - 3.29 (m, 2H), 2.82 (s, 3H), 2.79 - 2.69 (m, 2H), 2.15 - 2.02 (m, 1H), 1.90 - 1.77 (m, 2H), 1.63 - 1.50 (m, 2H).

**Example 57**

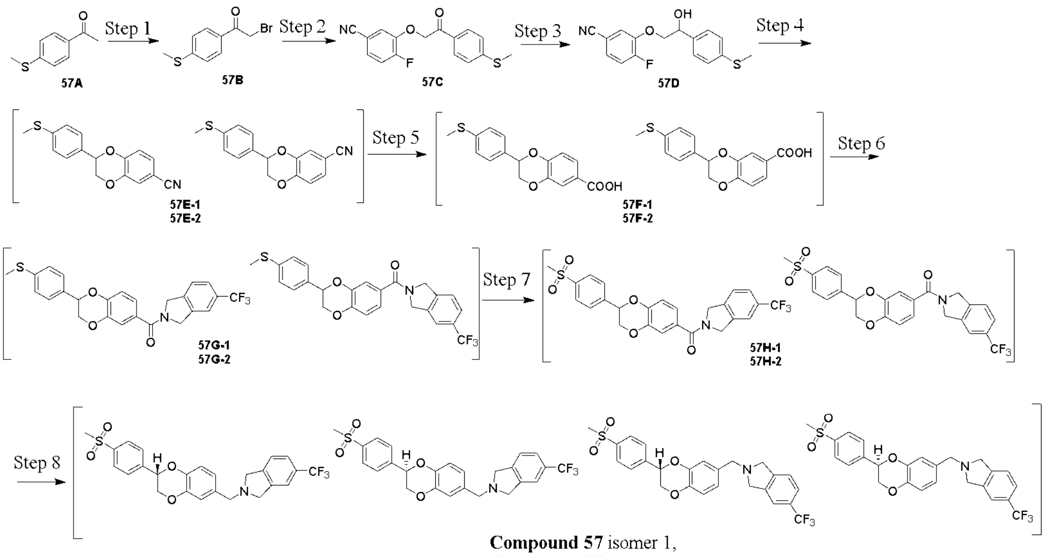


**Compound 57** isomer 1,
**Compound 57** isomer 2,
**Compound 57** isomer 3,
**Compound 57** isomer 4

Step 1:

At room temperature, the compound **57A** (4.0 g, 24.06 mmol) was dissolved in methanol (80 mL), and the resulting mixture was cooled to 0°C. Liquid bromine (3.85 g, 24.06 mmol) was slowly added dropwise, and the resulting mixture was stirred at 0°C for 2 h. After the reaction was completed, the reaction product was adjusted to pH = 7 with saturated sodium bicarbonate solution, and subjected to rotary evaporation to remove methanol. The remaining reaction solution was extracted with ethyl acetate (30 mL × 2), and the combined organic phase was washed with saturated aqueous sodium chloride solution (20 mL), dried over anhydrous sodium sulfate, and filtered. The filtrate was concentrated and the residue was separated and purified by silica gel column chromatography (petroleum ether: ethyl acetate (v/v) =

3:1) to obtain compound **57B** (4.5 g, 76%).
LC-MS (ESI): m/z = 245.0&247.0 $[M+H]^+$.

Step 2:

At room temperature, **57B** (4.5 g, 18.36 mmol), 4-fluoro-3-hydroxybenzonitrile (2.52 g, 18.36 mmol), and potassium carbonate (6.34 g, 45.90 mmol) were dissolved in acetonitrile (50 mL), and the resulting mixture was reacted at room temperature for 16 h. After the reaction was completed, the reaction solution was filtered. The filtrate was concentrated and the residue was separated and purified by silica gel column chromatography (petroleum ether: ethyl acetate (v/v) = 2:1) to obtain compound **57C** (5.0 g, 90%).
LC-MS (ESI): m/z = 302.0 $[M+H]^+$.

Step 3:

At room temperature, the compound **57C** (5.0 g, 16.59 mmol) was dissolved in anhydrous tetrahydrofuran (50 mL), and then sodium borohydride (1.26 g, 33.18 mmol) was slowly added at room temperature. After the reaction was completed, the reaction solution was filtered, and the filtrate was concentrated under reduced pressure to obtain compound **57D** (4.0 g, 79%), which was directly used in the next reaction without purification.
LC-MS (ESI): m/z = 304.1 $[M+H]^+$.

Step 4:

At room temperature, the compound **57D** (4.0 g, 13.19 mmol) and potassium carbonate (3.64 g, 26.38 mmol) were dissolved in N,N-dimethylformamide (40 mL), and the resulting mixture was warmed to 80°C and reacted for 12 h. After the reaction was completed, the reaction product was cooled to room temperature, and the reaction solution was filtered. The filtrate was concentrated and the residue was separated and purified by silica gel column chromatography (petroleum ether: ethyl acetate (v/v) = 4:1) to obtain a mixture of compounds **57E-1** and **57E-2** (0.85 g, 23%).
LC-MS (ESI): m/z = 284.0 $[M+H]^+$.

Step 5:

At room temperature, the mixture of compounds **57E-1** and **57E-2** (0.85 g, 3.00 mmol) was dissolved in a mixed system of 6 M potassium hydroxide solution (10 mL) and ethanol (10 mL), and the resulting mixture was warmed to 100°C and reacted for 36 h. After the reaction was completed, the reaction product was cooled to room temperature, and subjected to rotary evaporation to remove ethanol. The pH of the remaining reaction solution was adjusted to 2 with 4 M hydrochloric acid solution, and a solid was precipitated, the solid was washed with water (5 mL), and the collected solid was concentrated under reduced pressure to obtain a mixture of compounds **57F-1** and **57F-2** (0.75 g, 83%), which was directly used in the next reaction without purification.
LC-MS (ESI): m/z = 303.0 $[M+H]^+$.

Step 6:

At room temperature, the mixture of compounds **57F-1** and **57F-2** (0.75 g, 2.48 mmol), 5-trifluoromethylisoindoline hydrochloride (0.61 g, 2.73 mmol), 2-(7-azobenzotriazole)-N,N,N',N'-tetramethyluronium hexafluorophosphate (1.22 g, 3.22 mmol) and N,N-diisopropylethylamine (0.96 g, 7.44 mmol) were dissolved in N,N-dimethylformamide (10 mL), and the resulting mixture was reacted at room temperature for 2 h. After the reaction was completed, water (15 mL) was added to the reaction system, and the resulting mixture was extracted with ethyl acetate (20 mL × 2). The combined organic phase was washed with saturated aqueous sodium chloride solution (10 mL), dried over anhydrous sodium sulfate, and filtered. The filtrate was concentrated and the residue was separated and purified by silica gel column chromatography (petroleum ether: ethyl acetate (v/v) = 1:1) to obtain a mixture of compounds **57G-1** and **57G-2** (0.75 g, 64%).
LC-MS (ESI): m/z = 472.5 $[M+H]^+$.

Step 7:

At room temperature, the mixture of compounds **57G-1** and **57G-2** (0.75 g, 1.59 mmol) was dissolved in chloroform (10 mL), then m-chloroperoxybenzoic acid (1.37 g, 7.95 mmol) was added, and the resulting mixture was reacted at room temperature for 2 h. After the reaction was completed, saturated sodium bicarbonate solution (15 mL) and saturated sodium thiosulfate solution (15 mL) were added to the reaction system, and the resulting mixture was extracted

with dichloromethane (30 mL × 2). The combined organic phase was washed with saturated aqueous sodium chloride solution (10 mL), dried over anhydrous sodium sulfate, and filtered. The filtrate was concentrated and the residue was separated and purified by silica gel column chromatography (petroleum ether: ethyl acetate (v/v) = 1:4) to obtain a mixture of compounds **57H-1** and **57H-2** (0.41 g, 51%).

LC-MS (ESI): m/z = 504.6 $[M+H]^+$.

Step 8:

At room temperature, the mixture of compounds **57H-1** and **57H-2** (0.41 g, 0.81 mmol) was dissolved in toluene (2 mL), 1 M diisobutylaluminum hydride (0.81 mL) was slowly added at 0°C, and the resulting mixture was stirred at 0°C for 2 h. After the reaction was completed, water (5 mL) was added to the reaction system, and the resulting mixture was extracted with ethyl acetate (10 mL × 2). The combined organic phase was washed with saturated aqueous sodium chloride solution (5 mL), dried over anhydrous sodium sulfate, and filtered. The filtrate was concentrated and the residue was separated and purified by silica gel column chromatography (petroleum ether: ethyl acetate (v/v) = 2:3) to obtain a mixture of compound **57** (0.15 g, 37%). The mixture of compound **57** was separated by SFC to obtain four isomers, compound **57-1** (32 mg, retention time 0.61 min, **8%), 57-2** (28 mg, retention time 0.82 min, 7%), **57-3** (4.5 mg, retention time 1.05 min, 1%) and **57-4** (9.5 mg, retention time 1.39 min, 2%)

Separation conditions of preparative chromatography: 1. Instrument: SHIMADZU LC-30AD sf; 2. chromatographic column: Chiral AD column; 3. mobile phase system: A for n-hexane; B for 0.05% DEA in ethanol and acetonitrile; 4. gradient: B 40%; 5. flow rate: 3 mL/min

Compound **57-1:**

$^1H$ NMR (400 MHz, $CD_3OD$) δ 8.04 - 7.99 (m, 2H), 7.78 - 7.74 (m, 2H), 7.55 - 7.49 (m, 2H), 7.42 - 7.38 (m, 1H), 7.01 - 6.97 (m, 2H), 6.96 - 6.93 (m, 1H), 5.36 - 5.32 (m, 1H), 4.49 - 4.45 (m, 1H), 4.10 - 4.04 (m, 1H), 3.97 (s, 4H), 3.85 (s, 2H), 3.14 (s, 3H).
LC-MS (ESI): m/z = 490.2 $[M+H]^+$.

Compound **57-2:**

$^1H$ NMR (400 MHz, $CD_3OD$) δ 8.04 - 7.99 (m, 2H), 7.78 - 7.73 (m, 2H), 7.53 - 7.49 (m, 2H), 7.42 - 7.37 (m, 1H), 7.01 - 6.97 (m, 2H), 6.96 - 6.93 (m, 1H), 5.35 - 5.32 (m, 1H), 4.49 - 4.45 (m, 1H), 4.10 - 4.04 (m, 1H), 3.97 (s, 4H), 3.84 (s, 2H), 3.13 (s, 3H).
LC-MS (ESI): m/z = 490.2 $[M+H]^+$.

Compound **57-3:**

$^1H$ NMR (400 MHz, $CD_3OD$) δ 8.04 - 7.99 (m, 2H), 7.79 - 7.73 (m, 2H), 7.55 - 7.49 (m, 2H), 7.43 - 7.38 (m, 1H), 7.10 - 7.06 (m, 1H), 6.96 - 6.88 (m, 2H), 5.37 - 5.32 (m, 1H), 4.50 - 4.45 (m, 1H), 4.10 - 4.04 (m, 1H), 3.98 (s, 4H), 3.86 (s, 2H), 3.14 (s, 3H).
LC-MS (ESI): m/z = 490.2 $[M+H]^+$.

Compound **57-4:**

$^1H$ NMR (400 MHz, $CD_3OD$) δ 8.03 - 7.99 (m, 2H), 7.78 - 7.74 (m, 2H), 7.56 - 7.49 (m, 2H), 7.43 - 7.39 (m, 1H), 7.10 - 7.06 (m, 1H), 6.97 - 6.89 (m, 2H), 5.37 - 5.32 (m, 1H), 4.49 - 4.46 (m, 1H), 4.09 - 4.04 (m, 1H), 3.99 (s, 4H), 3.87 (s, 2H), 3.14 (s, 3H).
LC-MS (ESI): m/z = 490.2 $[M+H]^+$.

## Example 58

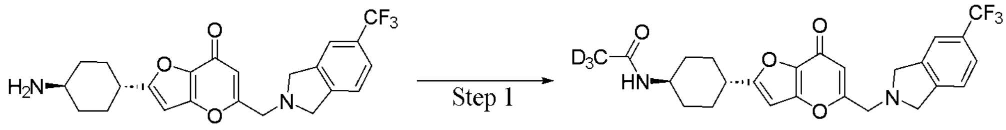


52E Compound 58

Step 1:

Compound 52E (0.2 g, 0.46 mmol) was dissolved in dichloromethane (10 mL), and triethylamine (0.14 g, 1.38 mmol) and deuterated acetyl chloride (0.056 g, 0.69 mmol) were successively added in an ice-water bath. After the dropwise addition was completed, the reaction was continued for 4 h in an ice-water bath. Upon complete depletion of raw materials as monitored by LCMS, the reaction solution was concentrated and purified by preparative HPLC to obtain compound 58 (45 mg, 20%).

LC-MS (ESI): *m/z* = 478.20 $[M+H]^+$.

$^1H$ NMR (400 MHz, DMSO-$d_6$) δ 7.81-7.75 (m, 2H), 7.70 (d, 1H), 7.59 (d, 1H), 6.77 (s, 1H), 6.64 (s, 1H), 4.65 (s, 4H), 4.53 (s, 2H), 3.61-3.51 (m, 1H), 2.84-2.73 (m, 1H), 2.09-2.00 (m, 2H), 1.93-1.85 (m, 2H), 1.56-1.43 (m, 2H), 1.35-1.23 (m, 2H).

**Example 59**

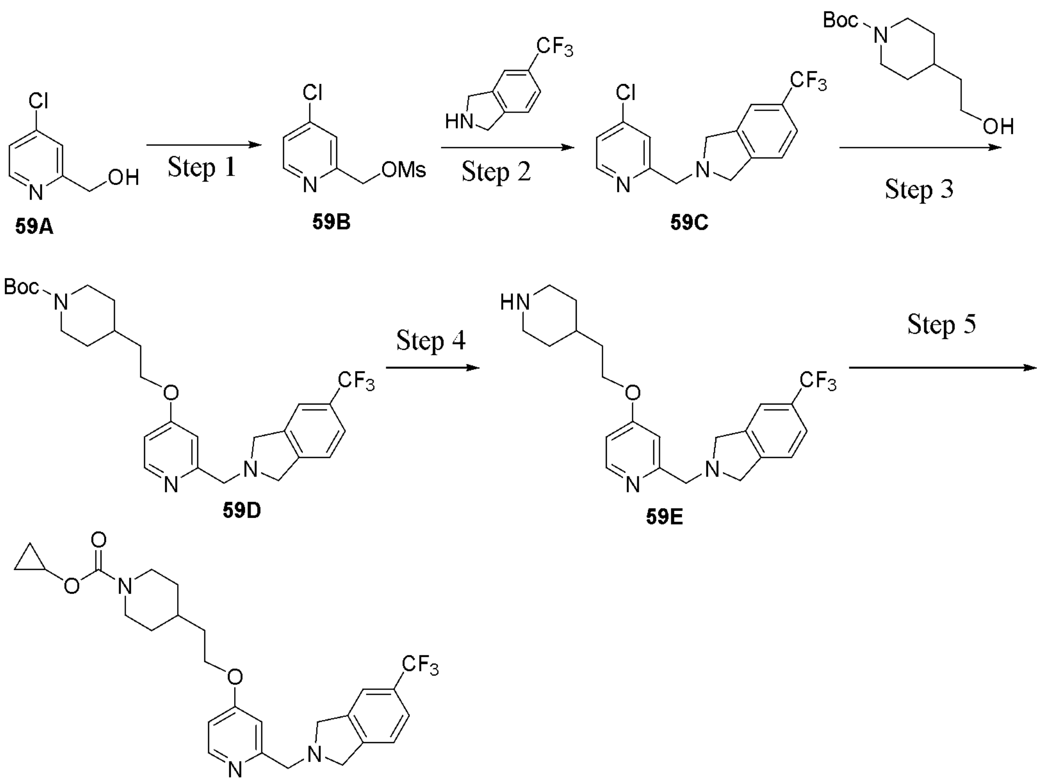


59A 59B 59C 59D 59E

Compound 59

Step 1:

Compound **59A** (1.0 g, 6.99 mmol) and TEA (2.12 g, 20.97 mmol) were dissolved in DCM (20 mL), and methylsulfonyl chloride (0.65 mL, 8.39 mmol) was added in an ice-water bath, and the resulting mixture was reacted at room temperature for 0.5 h. After the completion of the reaction, the reaction solution was directly used in the next step without further treatment.

Step 2:

Compound 5-trifluoromethylisoindoline hydrochloride (1.31 g, 6.99 mmol) was added to the reaction solution obtained in the previous step, and dissolved in dichloromethane (20 mL). Triethylamine (2.12 g, 20.97 mmol) was then added, and the resulting mixture was reacted at room temperature for 16 h. After the reaction was completed, the reaction product was concentrated, and the resulting crude product was purified by column chromatography (DCM:MeOH = 10:1) to obtain compound **59C** (750 mg, two-step yield 34.31%).
LC-MS (ESI): m/z=313.3 $[M+H]^+$.

Step 3:

Compound tert-butyl 4-(2-hydroxyethyl)piperidine-1-carboxylate (0.88 g, 3.84 mmol) was dissolved in DMF (15 mL), sodium hydride (0.092 g, 3.84 mmol) was added in an ice-water bath, and the resulting mixture was stirred for 1 h. **59C** (0.60 g, 1.92 mmol) was added to the reaction system, then the resulting mixture was reacted at room temperature for 16 h. After the reaction was completed, the reaction product was concentrated, and the resulting crude product was purified by column chromatography (PE:EA = 1:1) to obtain compound **59D** (250 mg, 25.75%).
LC-MS (ESI): m/z=506.2 $[M+H]^+$.

Step 4:

**59D** was dissolved in DCM (10 mL), hydrogen chloride dioxane solution (4 mol/L, 5 mL) was added at room temperature, and then the resulting mixture was stirred at room temperature for 1 h. The reaction solution was directly subjected to rotary evaporation, and the residue was directly used in the next step.
LC-MS (ESI): m/z= 406.5 $[M+H]^+$.

Step 5:

Cyclopropanol (0.040 g, 0.69 mmol) was dissolved in tetrahydrofuran (15 mL), CDI (0.11 g, 0.69 mmol) was added at room temperature, and the resulting mixture was stirred at room temperature for 0.5 h. Then triethylamine (0.19 g, 1.84 mmol) was added, and the stirring was continued for 0.5 h. Then **59E** (0.22 g, 0.46 mmol) was added to the reaction system, and the resulting mixture was warmed to 60°C and further reacted for 16 h. The reaction solution was directly subjected to rotary evaporation, and the resulting crude product was purified by preparative HPLC to obtain compound 59 (80 mg, 32.33%)
LC-MS (ESI): m/z=490.3 $[M+H]^+$.
$^1H$ NMR (400 MHz, $CDCl_3$) δ 8.53 (d, 1H), 7.82 (s, 1H), 7.76 (d, 1H), 7.64 (d, 1H), 7.22 (d, 1H), 7.14 (dd, 1H), 4.75 - 4.66 (m, 6H), 4.20 (t, 2H), 3.98 - 3.82 (m, 4H), 2.76 (s, 2H), 1.71 (dd, 4H), 1.10 (d, 2H), 0.71 - 0.56 (m, 4H).

**Example 60**

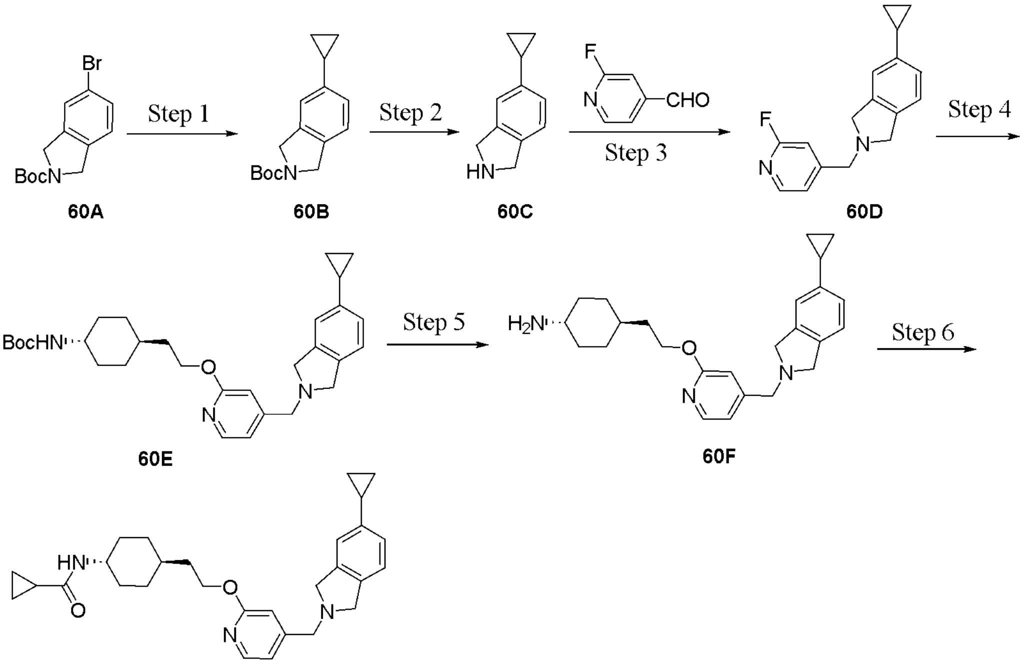


**Compound 60**

Step 1:

**60A** (10 g, 34 mmol), cyclopropyl boronic acid (5.8 g, 67 mmol), bis(triphenylphosphine)palladium dichloride (2.4 g, 2.4 mmol) and potassium phosphate (21 g, 102 mmol) were dissolved in mixed solvents of toluene (200 mL) and water (20 mL), and the resulting mixture was reacted at 100°C under nitrogen atmosphere overnight. After the reaction was completed, the reaction product was cooled to room temperature, water (200 mL) was added to the reaction solution, and the resulting mixture was extracted with ethyl acetate (300 mL × 3). The organic layers were combined, washed with saturated sodium bicarbonate solution (200 mL), dried over anhydrous sodium sulfate, filtered, and concentrated. The residue was separated and purified by silica gel column chromatography (petroleum ether: ethyl acetate (v/v) = 20:1) to obtain **60B** (5.7 g, 66%).

LC-MS (ESI): m/z = 204.1 $[M+H-56]^+$.

Step 2:

**60B** (5.7 mg, 22 mmol) was dissolved in a solution of 4 M hydrogen chloride in 1,4-dioxane (80 mL), and the resulting mixture was reacted at room temperature overnight. After 16 h, the reaction mixture was subjected to rotary evaporation under reduced pressure to remove the solvent. Saturated sodium bicarbonate solution (25 mL) was added to the residue and the mixture was extracted with ethyl acetate (20 mL X 3). The organic layers were combined, dried over anhydrous sodium sulfate and concentrated. The residue was separated and purified by silica gel column chromatography (dichloromethane: methanol (v/v) = 20:1) to obtain **60C** (3.4 g, 96%).

LC-MS (ESI): m/z = 160.1 $[M+H]^+$.

Step 3:

2-fluoropyridine-4-carbaldehyde (2.5 g, 20 mmol) was dissolved in DCE (40 mL), **60C** (3.2 g, 20 mmol), sodium borohydride acetate (4.2 g, 3.6 mmol) and 1 mL of glacial acetic acid were successively added, and the resulting mixture was reacted at room temperature for 1 h. After the reaction was completed, 20 mL of water was added to the reaction solution, and the resulting mixture was extracted with DCM (30 mL × 2). The organic layers were combined, washed with saturated aqueous sodium chloride solution (30 mL ×1), dried over anhydrous sodium sulfate, filtered, and concentrated. The residue was separated and purified by silica gel column chromatography (PE/EA = 1/1) to obtain **60D** (4.3 mg, 80%).

LC-MS (ESI): *m/z* = 269.2 $[M+H]^+$.

Step 4:

Trans-1-(BOC-amino)-4-(2-hydroxyethyl)cyclohexane (4.8 g, 20.0 mmol) was dissolved in THF (100 mL), sodium hydride (0.8 g, 20.0 mmol) was then added, and the resulting mixture was stirred at room temperature for 0.5 h. **60D** (2.7 g, 10 mmol) was then added, and the resulting mixture was reacted at room temperature overnight. 30 mL of water was added to the reaction solution, and the resulting mixture was extracted with ethyl acetate (50 mL × 2). The organic layers were combined, washed with saturated aqueous sodium chloride solution (25 mL × 1), dried over anhydrous sodium sulfate, filtered, and concentrated. The residue was separated and purified by silica gel column chromatography (PE/EA = 5/1) to obtain **60E** (3.5 g, 85%).

LC-MS (ESI): m/z = 492.3 $[M+H]^+$.

Step 5:

Compound **60E** (3.5 g, 8.5 mmol) was dissolved in dichloromethane (20 mL), trifluoroacetic acid (12 mL) was then added, and the resulting mixture was reacted at room temperature for half an hour and then concentrated to obtain compound 60F (3.2 g, 90%), which was directly used in the next step.

LC-MS (ESI): m/z = 392.3 $[M+H]^+$.

Step 6:

**60F** (391 mg, 1.0 mmol) was dissolved in DMF (10 mL), and cyclopropanecarboxylic acid (86 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 65** (140 mg, 36%).

LC-MS (ESI): m/z = 460.3 $[M+H]^+$.

$^1$H NMR (400 MHz, $CDCl_3$) δ 8.09 (d, 1H), 7.06 (d, 1H), 6.94 (s, 1H), 6.92 (s, 1H), 6.89 (s, 1H), 6.78 (s, 1H), 5.50 (d, 1H), 4.30 (t, 2H), 3.92 (s, 4H), 3.85 (s, 2H), 3.77 - 3.69 (m, 1H), 2.08 - 1.96 (m, 2H), 1.88 - 1.85 (m, 3H), 1.68 (q, 2H), 1.47 - 1.45 (m, 1H), 1.27 - 1.24 (m, 1H), 1.20 - 1.03 (m, 4H), 0.98 - 0.86 (m, 4H), 0.76 - 0.55 (m, 4H).

**Example 61**

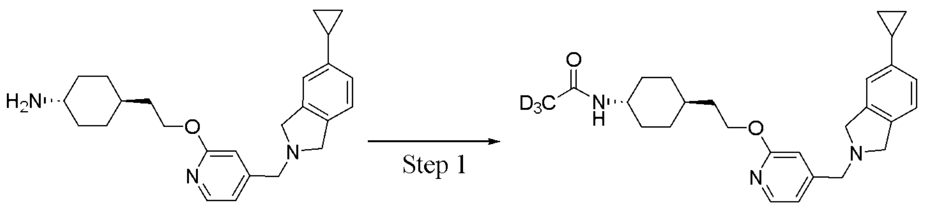


60F

Compound 61

Step 1:

Compound **60F** (391 mg, 1.0 mmol) was dissolved in dichloromethane (10 mL), and triethylamine (151 mg, 1.5 mmol) was then added. Deuterated acetyl chloride (98 mg, 1.2 mmol) was added dropwise at -30°C, and the resulting mixture was transferred to room temperature and reacted for 20 min. A small amount of methanol was added to quench the reaction, the reaction solution was concentrated, and the residue was separated and purified by silica gel column chromatography (PE/EA = 1/1) to obtain compound **61** (140 mg, 32%).

LC-MS (ESI): m/z = 437.3 $[M+H]^+$.

$^1$H NMR (400 MHz, $CDCl_3$) δ 8.09 (d,1H), 7.06 (d,1H), 6.94 (d,1H), 6.92 (s, 1H), 6.89 (s, 1H), 6.78 (s, 1H), 5.28 (d,1H), 4.30 (t, 2H), 3.92 (s, 4H), 3.85 (s, 2H), 3.77 - 3.68(m, 1H), 2.00 (s, 2H), 1.91 - 1.83 (m, 3H), 1.68 (q, 2H), 1.49 - 1.41 (m, 1H), 1.20 - 1.02 (m, 4H), 0.96 - 0.81 (m, 2H), 0.71 - 0.53 (m, 2H).

**Example 62**

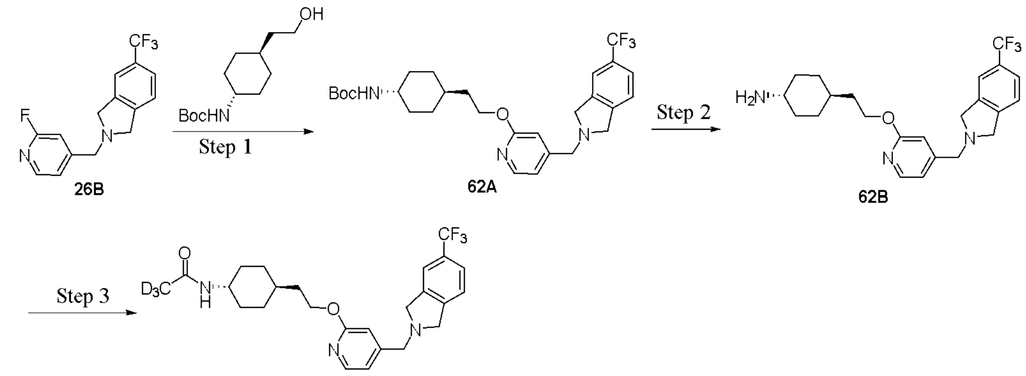


26B 62A 62B

**Compound 62**

Step 1:

Trans-1-(BOC-amino)-4-(2-hydroxyethyl)cyclohexane (4.8 g, 20.0 mmol) was dissolved in THF (100 mL), sodium hydride (0.8 g, 20.0 mmol) was then added, and the resulting mixture was stirred at room temperature for 0.5 h. **26B** (3.0 g, 10 mmol) was then added, and the resulting mixture was reacted at room temperature overnight. 30 mL of water was added to the reaction solution, and the resulting mixture was extracted with ethyl acetate (50 mL × 2). The organic layers were combined, washed with saturated aqueous sodium chloride solution (25 mL × 1), dried over anhydrous sodium sulfate, filtered, and concentrated. The residue was separated and purified by silica gel column chromatography (PE/EA = 5/1) to obtain **62A** (3.8 g, 73%).

LC-MS (ESI): m/z = 520.6 $[M+H]^+$.

Step 2:

Compound **62A** (3.8 g, 7.3 mmol) was dissolved in dichloromethane (10 mL). Trifluoroacetic acid (6 mL) was then added, and the resulting mixture was reacted at room temperature for half an hour and then concentrated to obtain compound **62B** (3.5 g, 92%), which was directly used in the next step.

LC-MS (ESI): m/z = 420.5 $[M+H]^+$.

Step 3:

Compound **62B** (419 mg, 1.0 mmol) was dissolved in dichloromethane (10 mL), and triethylamine (151 mg, 1.5 mmol) was then added. Deuterated acetyl chloride (98 mg, 1.2 mmol) was added dropwise at -30°C, and the resulting mixture was transferred to room temperature and reacted for 20 min. A small amount of methanol was added to quench the reaction, the reaction solution was concentrated, and the residue was separated and purified by silica gel column chromatography (PE/EA = 1/1) to obtain **62A** (150 mg, 32%).

LC-MS (ESI): m/z = 465.5 $[M+H]^+$.

$^1$H NMR (400 MHz, $CDCl_3$) δ 8.10 (d, 1H), 7.47 (d, 1H), 7.44 (s, 1H), 7.28 (d, 1H), 6.91 (d, 1H), 6.77 (s, 1H), 5.34 (d, 1H), 4.31 (t, 2H), 3.99 (s, 4H), 3.86 (s, 2H), 3.80 - 3.65 (m, 1H), 2.00 (s, 2H), 1.84 (s, 2H), 1.71 - 1.66 (m, 2H),1.46 (s, 1H), 1.20 - 1.01 (m, 4H).

$^{19}$F NMR (376 MHz, $CDCl_3$) δ -60.00 (s).

**Example 63**

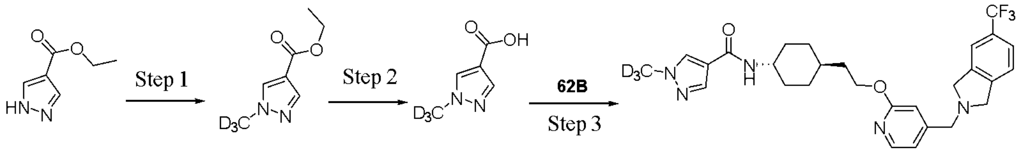


63A 63B 63C Compound 63

Step 1:

**63A** (2.6 g, 18.56 mmol) was dissolved in N,N-dimethylformamide (30 mL), and cesium carbonate (9.07 g, 27.84 mmol) and deuterated iodomethane (3.23 g, 22.27 mmol) were successively added at 0°C-5°C. After the addition was completed, the resulting mixture was reacted at room temperature overnight. After the reaction was completed, water (200 mL) was added to the reaction solution to quench the reaction, and the resulting mixture was extracted with ethyl acetate (200 mL*2). The organic phases were combined, washed once each with water (200 mL) and saturated aqueous sodium chloride solution (200 mL), dried over anhydrous sodium sulfate and concentrated to obtain **63B** (2.7 g, yield: 92%).
LC-MS (ESI): m/z= 158.2 $[M+H]^+$.

Step 2:

**63B** (2.7 g, 17.18 mmol) was dissolved in ethanol (30 mL), and a solution of sodium hydroxide (1.38 g, 34.61 mmol) in water (10 mL) was added at 0°C-5°C. After the addition was completed, the resulting mixture was reacted at room temperature overnight. After the reaction was completed, the reaction was concentrated. After concentration, the residue was dissolved in water, and adjusted to pH = 2-3 with 1 N hydrochloric acid. A large amount of white solid was precipitated, and the resulting mixture was subjected to suction filtration. The filter cake was concentrated to obtain **63C** (1.30 g, yield: 56%).
LC-MS (ESI): m/z= 130.2 $[M+H]^+$.

Step 3:

62B (419 mg, 1.0 mmol) was dissolved in DMF (10 mL), and 63C (129 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 63** (140 mg, 26%).
LC-MS (ESI): m/z = 531.4 $[M+H]^+$.
$^1H$ NMR (400 MHz, $CDCl_3$) δ 8.11 (d, 1H), 7.80 (s, 1H), 7.68 (s, 1H), 7.47 (d, 1H), 7.44 (s, 1H), 7.29 (d, 1H), 6.92 (d, 1H), 6.78 (s, 1H), 5.59 (d, 1H), 4.33 (t, 2H), 4.01 (s, 4H), 3.88 (s, 3H), 2.06 (s, 2H), 1.89 (s, 2H), 1.19 - 1.12 (m, 2H), 1.49 (s, 1H), 1.27 - 1.07 (m, 4H).
$^{19}F$ NMR (376 MHz, $CDCl_3$) δ -60.02 (s).

**Example 64**

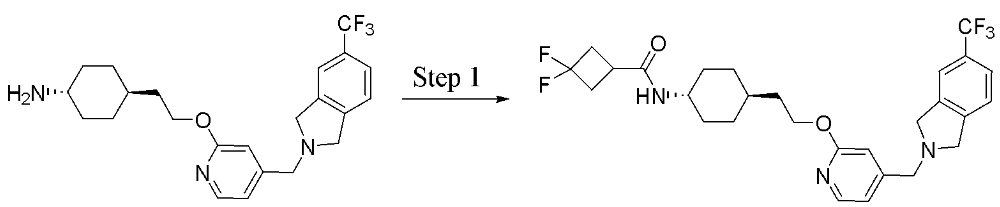


62B Compound 64

Step 1

**62B** (419 mg, 1.0 mmol) was dissolved in DMF (10 mL), and 3,3-difluorocyclobutane carboxylic acid (136 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 64** (160 mg, 30%).

LC-MS (ESI): m/z = 538.7[M+H]$^+$.

$^1$H NMR (400 MHz, $CDCl_3$) δ 8.10 (d, 1H), 7.47 (d, 1H), 7.44 (s, 1H), 7.28 (d, 1H), 6.91 (d, 1H), 6.77 (s, 1H), 5.33 (d, 1H), 4.32 (t, 2H), 4.00 (s, 4H), 3.87 (s, 2H), 3.73 (s, 1H), 2.99 - 2.77 (m, 2H), 2.72 - 2.62 (m, 3H), 2.05 - 2.18 (m, 4H), 1.71 - 1.66 (m, 2H), 1.47 (s, 1H), 1.20 - 1.03 (m, 4H).

$^{19}$F NMR (376 MHz, $CDCl_3$) δ -60.01 (s), -79.80 (d), -96.81 (d).

**Example 65**

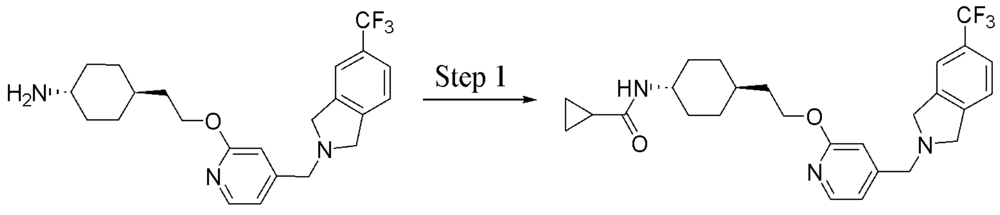


62B

Compound 65

Step 1:

**626B** (419 mg, 1.0 mmol) was dissolved in DMF (10 mL), and cyclopropanecarboxylic acid (86 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 65** (130 mg, 27%).

$^1$H NMR (400 MHz, DMSO-$d_6$) δ 8.10 (d, 1H), 7.88 (d, 1H), 7.62 (s, 1H), 7.56 (d, 1H), 7.46 (d, 1H), 6.98 (d, 1H), 6.78 (s, 1H), 4.28 (t, 2H), 3.94 (s, 4H), 3.87 (s, 2H), 3.52 - 3.44 (m, 1H), 1.79 - 1.77 (m, 4H), 1.62 - 1.58 (m, 2H), 1.50 - 1.46 (m, 1H), 1.23 - 1.11 (m, 1H), 1.05 - 0.96 (m, 2H), 1.02 - 0.88 (m, 2H), 0.66 - 0.57 (m, 4H).

$^{19}$F NMR (377 MHz, DMSO) δ -58.34 (s).

**Example 66**

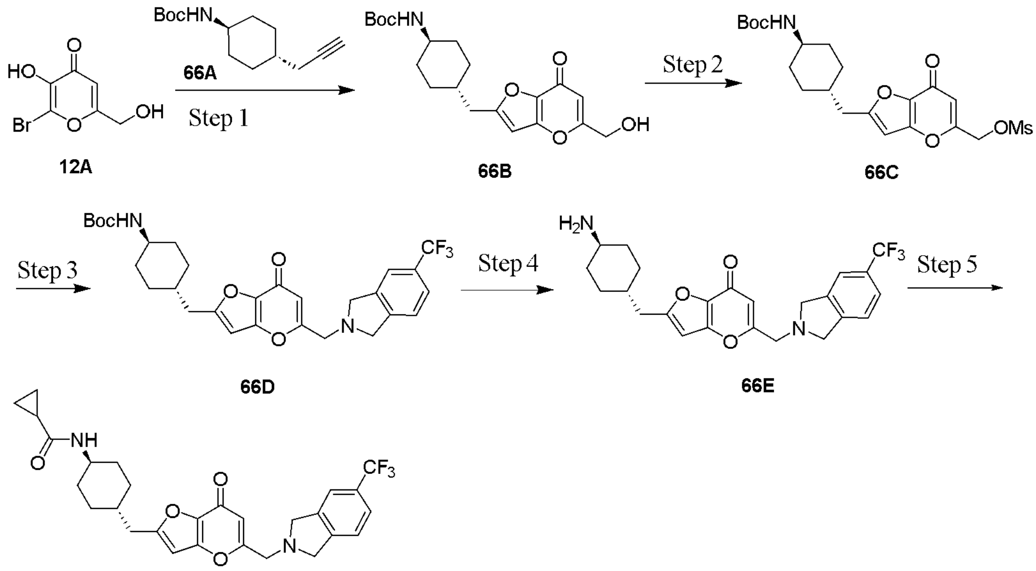


Compound 66

Step 1:

At room temperature, compound 12A (3.5 g, 15.84 mmol), compound 66A (4.14 g, 17.42 mmol), bis(triphenylphosphine)palladium dichloride (1.11 g, 1.58 mmol) and cuprous iodide (0.60 g, 3.17 mmol) were dissolved in tetrahydrofuran (100 mL), triethylamine (5.11 g, 50.50 mmol) was then added under nitrogen atmosphere, and the reaction solution was warmed to 40°C and stirred for 4 h. After the reaction was completed, the reaction product was filtered, and the filtrate was concentrated under reduced pressure, and purified by column chromatography (dichloromethane:ethyl acetate = 1:3 (v/v)) to obtain compound 66B (1.1 g, 18%).

LC-MS (ESI): *m*/*z* = 378.20 $[M+H]^+$.

Step 2:

Compound 66B (0.9 g, 2.38 mmol) was dissolved in dichloromethane (20 mL), and triethylamine (0.48 g, 4.76 mmol) and methylsulfonyl chloride (0.41 g, 3.57 mmol) were successively added in an ice-water bath. The reaction solution was warmed to room temperature, and then stirring was continued for 1 h. After the reaction was completed, the reaction product was concentrated, and the resulting crude product was purified by column chromatography (petroleum ether:ethyl acetate = 3:1 (v/v)) to obtain compound 66C (0.7 g, 64%).

LC-MS (ESI): *m*/*z* = 400.00 $[M\text{-}t\text{-}Bu+H]^+$.

Step 3:

Compound 66C (700 mg, 1.54 mmol) and 5-trifluoromethylisoindoline hydrochloride (520 mg, 2.34 mmol) were dissolved in DCM (20 mL), triethylamine (0.47 g, 4.62 mmol) was then added, and the reaction solution was stirred at room temperature overnight. After the reaction was completed, the reaction product was concentrated, and the resulting crude product was purified by column chromatography (petroleum ether:ethyl acetate = 1:1 (v/v)) to obtain compound 66D (0.5 g, 60%).

LC-MS (ESI): *m*/*z* = 491.20 $[M\text{-}t\text{-}Bu+H]^+$.

Step 4:

Compound 66D (0.5 g, 0.91 mmol) was dissolved in dichloromethane (10 mL), hydrogen chloride dioxane solution (5 mL) was then added, and the resulting mixture was reacted at room temperature for 2 h. Upon complete depletion of raw materials as monitored by LCMS, the reaction solution was concentrated to obtain compound 66E (400 mg, crude product), which was directly used in the next step without purification.

LC-MS (ESI): *m*/*z* = 447.10 $[M+H]^+$.

Step 5:

Compound 66E (150 mg, 0.34 mmol) was dissolved in dichloromethane (10 mL), and triethylamine (100 mg, 0.99 mmol) and cyclopropanecarbonyl chloride (53 mg, 0.51 mmol) were successively added in an ice-water bath. After the dropwise addition was completed, the reaction was continued for 4 h in an ice-water bath. Upon complete depletion of raw materials as monitored by LCMS, the reaction solution was concentrated and purified by preparative HPLC to obtain compound 66 (25 mg, 12%).

LC-MS (ESI): $m/z$ = 515.20 $[M+H]^+$.

$^1H$ NMR (400 MHz, DMSO-$d_6$) δ 7.90 (d, 1H), 7.78 (s, 1H), 7.70 (d, 1H), 7.59 (d, 1H), 6.81 (s, 1H), 6.64 (s, 1H), 4.66 (s, 4H), 4.53 (s, 2H), 3.50-3.45 (m, 1H), 2.69 (d, 2H), 1.82-1.74 (m, 2H), 1.71-1.65 (m, 2H), 1.52-1.44 (m, 1H), 1.25-1.03 (m, 5H), 0.65-0.55 (m, 4H).

**Example 67:**

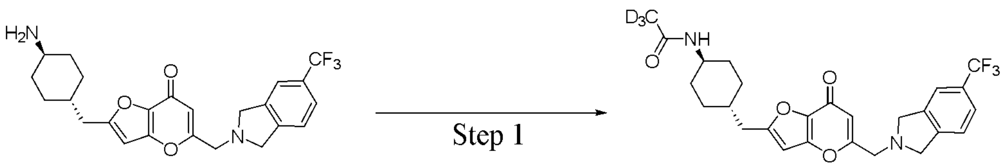


66E

Compound 67

Step 1:

Compound 66E (250 mg, 0.56 mmol) was dissolved in dichloromethane (10 mL), and triethylamine (170 mg, 1.68 mmol) and deuterated acetyl chloride (68 mg, 0.84 mmol) were successively added in an ice-water bath. After the dropwise addition was completed, the reaction was continued for 4 h in an ice-water bath. Upon complete depletion of raw materials as monitored by LCMS, the reaction solution was concentrated and purified by preparative HPLC to obtain compound 67 (35 mg, 10%).

LC-MS (ESI): $m/z$ = 492.20 $[M+H]^+$.

$^1H$ NMR (400 MHz, DMSO-$d_6$) δ 7.77 (s, 1H), 7.72-7.66 (s, 2H), 7.59 (d, 1H), 6.80 (s, 1H), 6.63 (s, 1H), 4.63 (s, 4H), 4.50 (s, 2H), 3.48-3.41 (m, 1H), 2.69 (d, 2H), 1.80-1.64 (m, 5H), 1.25-1.03 (m, 4H).

**Example 68**

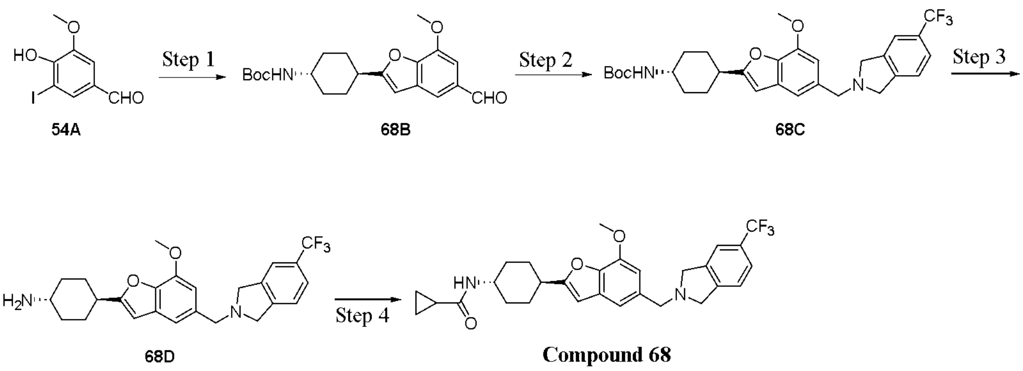


54A 68B 68C

68D Compound 68

Step 1:

Compound **54A** (2.8 g, 10 mmol) was dissolved in pyridine (20 mL), trans-1-(BOC-amino)-4-ethynylcyclohexane (2.2 g, 10 mmol) and cuprous oxide (715 mg, 5 mmol) were added, and the resulting mixture was stirred at 120°C for 2 h. After the reaction was completed, 20 mL of water was added to the reaction solution, and the resulting mixture was extracted with ethyl acetate (15 mL × 3). The organic layers were combined, washed with saturated aqueous sodium

chloride solution (25 mL × 3), dried over anhydrous sodium sulfate, filtered, and concentrated. The residue was separated and purified by silica gel column chromatography (PE/EA(v/v ) = 5:1) to obtain **compound 68B** (3.2 g, 85%).

LC-MS (ESI): m/z = 318.4[M+H-56]$^+$.

Step 2:

**68B** (3.2 g, 8.5 mmol) was dissolved in DCE (40 mL), 5-trifluoromethylisoindoline hydrochloride (2.2 g, 10.0 mmol) and sodium borohydride acetate (6.6 g, 32.0 mmol) were then added, and finally glacial acetic acid (1 mL) was added, and the resulting mixture was reacted at room temperature for 2 h. After the reaction was completed, saturated sodium biarbconate solution (30 mL) was added to the reaction solution, and the resulting mixture was extracted with dichloromethane (20 mL × 2). The organic layers were combined, washed with saturated aqueous sodium chloride solution (25 mL × 1), dried over anhydrous sodium sulfate, filtered, and concentrated. The residue was separated and purified by silica gel column chromatography (PE/EA = 4/1) to obtain **68C** (3.8 g, 83%).

LC-MS (ESI): m/z = 489.6[M+H-56]$^+$.

Step 3:

Compound **68C** (3.8 g, 7.0 mmol) was dissolved in dichloromethane (10 mL), trifluoroacetic acid (15 mL) was then added, and the resulting mixture was reacted at room temperature for half an hour and then concentrated to obtain compound **68D** (3.2 g, 91%), which was directly used in the next step.

LC-MS (ESI): m/z = 444.5[M+H]$^+$.

Step 4:

**68D** (444 mg, 1.0 mmol) was dissolved in DMF (10 mL), and cyclopropanecarboxylic acid (86 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 68** (171 mg, 33%).

$^1$H NMR (400 MHz, DMSO-$d_6$) δ 7.97 (d, 1H), 7.59 (s, 1H), 7.54 (d, 1H), 7.44 (d, 1H), 7.10 (s, 1H), 6.86 (d, 1H), 6.51 (s, 1H), 3.90 (s, 9H), 3.63 - 3.55 (m, 1H), 2.76 - 2.67 (m, 1H), 2.09 (d, 2H), 1.91 (d, 2H), 1.58 - 1.43 (m, 3H), 1.39 - 1.30 (m, 2H), 0.70 - 0.57 (m, 4H).

$^{19}$F NMR (376 MHz, DMSO) δ -58.30 (s).

**Example 69**

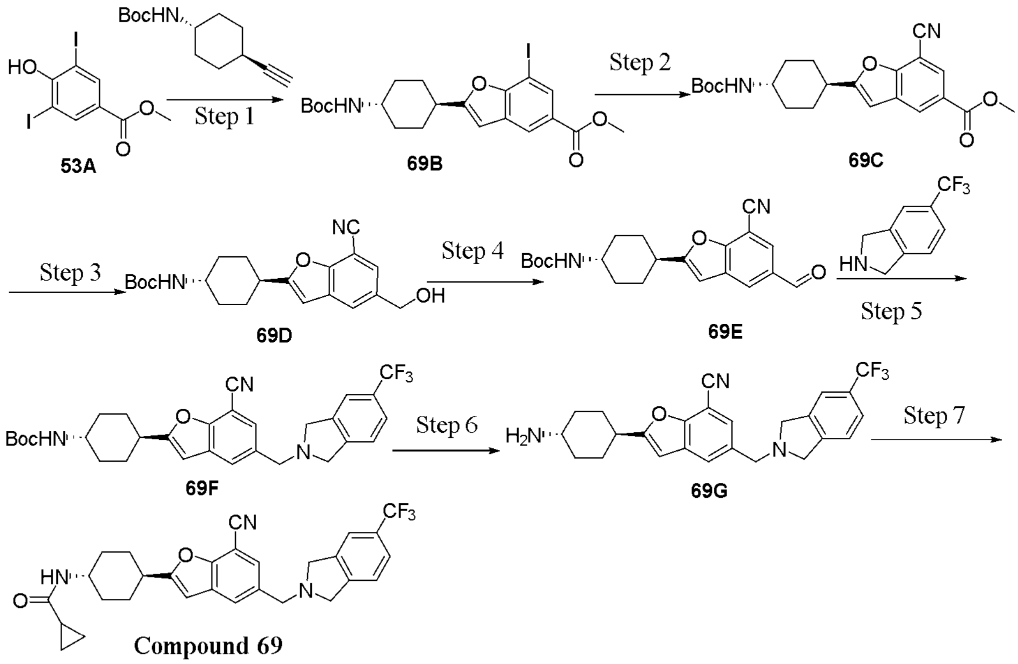


53A 69B 69C 69D 69E 69F 69G Compound 69

Step 1:

At room temperature, compound 53A (2.80 g, 6.95 mmol), tert-butyl ((1r,4r)-4-ethynylcyclohexyl)carbamate (1.71 g, 7.65 mmol) and cuprous oxide (500 mg, 3.48 mmol) were added to pyridine (50 mL), and the resulting mixture was subjected to nitrogen replacement three times. The reaction solution was warmed to 120°C and stirred for 4 h. After the reaction was completed, the reaction product was filtered, and the filtrate was concentrated under reduced pressure, and then purified by silica gel column chromatography (eluent: petroleum ether: ethyl acetate = 1:1 (v/v)) to obtain the target compound 69B (2.70 g, 78%).

LC-MS (ESI): *m/z*= 444.1[M-$^t$Bu+H]$^+$.

Step 2:

69B (2.70 g, 5.41 mmol), tetrakis(triphenylphosphine)palladium (630 mg, 0.54 mmol) and zinc cyanide (950 mg, 8.12 mmol) were added to ultra-dry DMF (50 mL), and the resulting mixture was subjected to nitrogen replacement three times. The reaction solution was warmed to 100°C and stirred for 6 h. After the reaction was completed, the reaction product was filtered, and the filtrate was diluted with EA and washed twice with water. The organic layer was washed with saturated NaCl solution, dried over anhydrous $Na_2SO_4$, filtered and subjected to rotary evaporation; silica gel column chromatography (eluent: petroleum ether: ethyl acetate = 1:1 (v/v)) to obtain the target compound 69C (1.70 g, 79%) LC-MS (ESI): *m/z* = 343.2 [M-$^t$Bu+H]$^+$

Step 3:

69C (1.70 g, 4.27 mmol) was dissolved in diethyl ether (10 mL), then lithium borohydride (280 mg, 12.85 mmol) was added, and the resulting mixture was stirred at room temperature for 1 h. The reaction solution was quenched with saturated ammonium chloride solution, and the aqueous phase was extracted with EA. The organic layers were combined, washed with saturated NaCl solution, dried over anhydrous $Na_2SO_4$, filtered and subjected to rotary evaporation; silica gel column chromatography (eluent: petroleum ether: ethyl acetate = 1:1 (v/v)) to obtain the target compound 69D (1.20 g, 76%).

LC-MS (ESI): *m/z* = 315.2[M-$^t$Bu+H]$^+$.

Step 4:

69D (1.20 g, 3.24 mmol) was dissolved in EA (50 mL), IBX oxidant (1.36 g, 4.86 mmol) was added at room temperature, and then the resulting mixture was heated to 70°C and reacted for 16 h. After the reaction was completed, the reaction product was filtered, and the filter cake was washed three times with EA. The filtrate was subjected to rotary

evaporation to obtain the target compound 69E (1.10 g, 92%)
LC-MS (ESI): *m/z* = 313.2 [M-$^t$Bu+H]$^+$.

Step 5:

69E(1.1 g, 2.99 mmol) was dissolved in methanol (50 mL), then 5-trifluoromethylyl-2,3-dihydro1H-isoindole hydrochloride (800 mg, 3.59 mmol) was added, and the resulting mixture was stirred at room temperature for 1 h. Sodium cyanoborohydride (230 mg, 3.59 mmol) was added, and the resulting mixture was reacted at room temperature for one hour. The reaction solution was quenched with saturated aqueous sodium bicarbonate solution, the aqueous phase was extracted with dichloromethane, and the organic layers were combined, washed with saturated NaCl solution, dried over anhydrous Na2SO4, filtered, subjected to rotary evaporation, and subjected to silica gel column chromatography (eluent: dichloromethane: methanol (v/v) = 10:1) to obtain the target compound 69F (460 mg, 29%).
LC-MS (ESI): *m/z* = 484.2[M-$^t$Bu+H]$^+$.

Step 6:

69F (230 mg, 0.43 mmol) was dissolved in DCM (5 mL), then trifluoroacetic acid (1 mL) was added, and the resulting mixture was reacted at room temperature for 1 h. After the reaction was completed, the reaction solution was concentrated, and the crude product 69G was directly used in the next reaction without purification.

Step 7:

69G obtained in the previous step was dissolved in DMF (10 mL), cyclopropanecarboxylic acid (56 mg, 0.65 mmol) and N-methylimidazole (140 mg, 1.71 mmol) were added, and the resulting mixture was stirred and reacted at room temperature for 0.5 h. TCFH (180 mg, 0.64 mmol) was then added, and the resulting mixture was reacted at room temperature for 2 h, and the reaction was completed as monitored by TLC. The reaction solution was diluted with EA, washed three times with water, and the organic phase was dried over anhydrous $Na_2SO_4$, filtered and subjected to rotary evaporation; the residue was separated and purified by silica gel column chromatography (eluent: petroleum ether: ethyl acetate (v/v) = 1:3) to obtain compound 69 (47 mg, 22%).
LC-MS (ESI): *m/z* = 508.3 [M+H]$^+$.
$^1$H NMR (400 MHz, DMSO-$d_6$) $\delta$ 8.00 (d, 1H), 7.93 (d, 1H), 7.72 (d, 1H), 7.60 (d, 1H), 7.58 - 7.51 (m, 1H), 7.44 (d, 1H), 6.76 (d, 1H), 3.98 (s, 2H), 3.92 (s, 4H), 3.66 - 3.54 (m, 1H), 2.84 (t, 1H), 2.14 (d, 2H), 1.93 (d, 2H), 1.60 - 1.43 (m, 3H), 1.43 - 1.28 (m, 2H), 0.74 - 0.46 (m, 4H).

**Example 70**

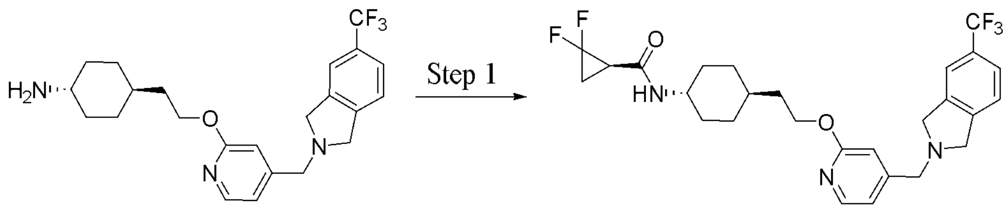


62B

Compound 70

Step 1:

62B (419 mg, 1.0 mmol) was dissolved in DMF (10 mL), and (1R)-2,2-difluorocyclopropane-1-carboxylic acid (122 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 70** (140 mg, 27%).
$^1$H NMR (400 MHz, $CDCl_3$) $\delta$ 8.10 (d, 1H), 7.47 (d, 1H), 7.44 (s, 1H), 7.28 (d, 1H), 6.92 (d, 1H), 6.77 (s, 1H), 5.65 (d, 1H), 4.32 (t, 2H), 4.00 (s, 4H), 3.87 (s, 2H), 3.77 (s, 1H), 2.31 - 2.14 (m, 1H), 2.14 - 1.93 (m, 4H), 1.86 (d, 2H), 1.71 - 1.58 (m,

3H), 1.47 (s, 1H), 1.22 - 0.99 (m, 4H).

$^{19}F$ NMR (376 MHz, $CDCl_3$) δ -60.00 (s), -123.60 (d), -139.42 (d).

**Example 71**

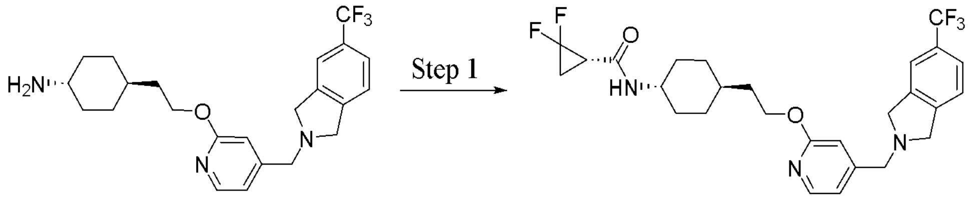


**62B** **Compound 71**

Step 1:

**62B** (419 mg, 1.0 mmol) was dissolved in DMF (10 mL), and (1S)-2,2-difluorocyclopropane-1-carboxylic acid (122 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain **compound 71** (160 mg, 30%).

$^{1}H$ NMR (400 MHz, $CDCl_3$) δ 8.13 (d, 1H), 7.50 (d, 1H), 7.46 (s, 1H), 7.31 (d, 1H), 6.96 (s, 1H), 6.80 (s, 1H), 5.42 (d, 1H), 4.32 (t, 2H), 3.98 (d, 6H), 3.81- 3.72 (m, 1H), 3.23- 2.15 (m, 1H), 2.12 - 2.02 (m, 5H), 1.73 - 1.57 (m, 3H), 1.48 (s, 1H), 1.20 - 1.17 (m, 4H).

$^{19}F$ NMR (376 MHz, $CDCl_3$) δ -60.09 (s), -123.78 (d), -139.64 (d).

**Example 72:**

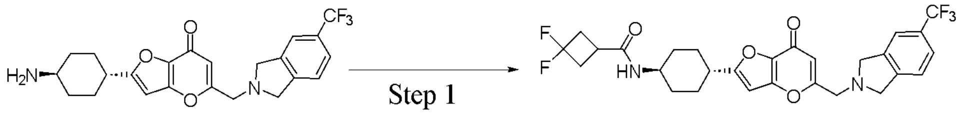


**52E** **Compound 72**

Step 1:

Compound 52E (250 mg, 0.58 mmol) and 3,3-difluorocyclobutane carboxylic acid (120 mg, 0.87 mmol) were dissolved in N,N-dimethylformamide (10 mL), and N-methylimidazole (140 mg, 1.74 mmol) and TCFH (240 mg, 0.87 mmol) were then successively added. After the addition was completed, the reaction was continued at room temperature for 3 h. Upon complete depletion of raw materials as monitored by LCMS, the reaction solution was concentrated, and purified by preparative HPLC to obtain compound 72 (70 mg, 22%).

LC-MS (ESI): $m/z$ = 551.10 $[M+H]^+$.

$^{1}H$ NMR (400 MHz, DMSO-$d_6$) δ 7.95 (d, 1H), 7.62 (s, 1H), 7.57 (d, 1H), 7.47 (d, 1H), 6.78 (s, 1H), 6.42 (s, 1H), 4.05 (s, 4H), 3.90 (s, 2H), 3.64-3.52 (m, 1H), 2.87-2.61 (m, 6H), 2.10-1.99 (m, 2H), 1.94-1.84 (m, 2H), 1.56-1.42 (m, 2H), 1.36-1.22 (m, 2H).

**Example 73:**

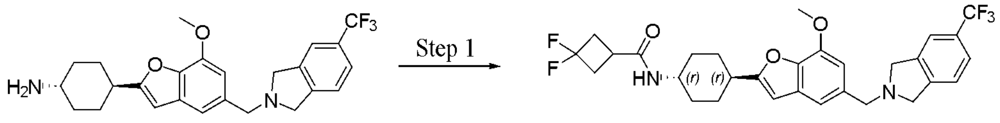


68D

Compound 73

Step 1:

**68D** (444 mg, 1.0 mmol) was dissolved in DMF (10 mL), and 3,3-difluorocyclobutane carboxylic acid (136 mg, 1.0 mmol) and N-methylimidazole (328 mg, 4.0 mmol) were added. At 0°C, N,N,N',N'-tetramethylchloroformamidinium hexafluorophosphate (421 mg, 1.5 mmol) was added and the resulting mixture was stirred at room temperature for 20 min. After the reaction was completed, 20 mL of water was added to the reaction solution, and the resulting mixture was extracted with ethyl acetate (15 mL × 3). The organic layers were combined, washed with saturated aqueous sodium chloride solution (25 mL × 3), dried over anhydrous sodium sulfate, filtered, and concentrated. The residue was separated and purified by silica gel column chromatography (PE/EA (v/v) = 1:1) to obtain compound 73 (150 mg, 27%).

LC-MS (ESI): *m/z* =563.3[M+H]$^+$.

$^1$H NMR (400 MHz, $CDCl_3$) δ 7.45 (d, 1H), 7.42 (s, 1H), 7.27 (d, H), 7.09 (s, 1H), 6.86 (s, 1H), 6.33 (s, 1H), 5.39 (d, 1H), 4.00 (s, 3H), 3.98 (s, 4H), 3.96 (s, 2H), 3.91 - 3.69 (m, 1H), 2.91 - 2.85 (m, 2H), 2.78 - 2.70 (m, 4H), 2.25 (d, 2H), 2.13 (d, 2H),1.72 - 1.54 (m, 2H), 1.32 - 1.24 (m, 2H).

$^{19}$F NMR (377 MHz MHz, $CDCl_3$) δ -59.96 (s), -79.81 (d), -96.69 (d).

**Example 74:**

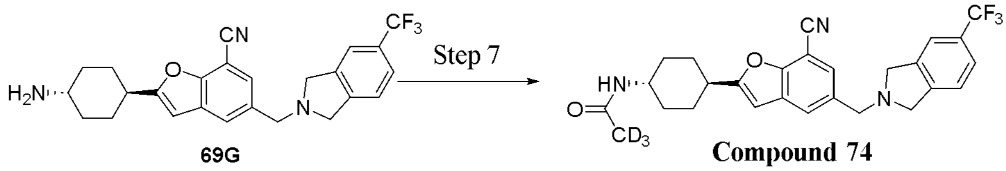


69G

Compound 74

Step 1:

69G (252 mg, 0.57 mmol) was dissolved in ultra-dry dichloromethane (10 mL), triethylamine (173 mg, 1.71 mmol) was added in an ice bath, and the resulting mixture was stirred for 5 min. Deuterated acetyl chloride (56 mg, 0.68 mmol) was then added, and after the addition was completed, the resulting mixture was slowly warmed to room temperature and reacted for 1 h, and the reaction was completed as monitored by TLC. The reaction solution was quenched with water (10 mL), and the resulting mixture was extracted with dichloromethane (10 mL × 3). The organic phase was dried over anhydrous $Na_2SO_4$, filtered and subjected to rotary evaporation; the residue was separated and purified by silica gel column chromatography (eluent: petroleum ether: ethyl acetate (v/v) = 3:1) to obtain compound 74 (130 mg, 47%).

LC-MS (ESI): *m/z* = 485.2 [M+H]$^+$.

$^1$H NMR (400 MHz, DMSO-$d_6$) δ 7.92 (d, 1H), 7.78 (d, 1H), 7.72 (d, 1H), 7.59 (d, 1H), 7.55 (d, 1H), 7.44 (d, 1H), 6.75 (s, 1H), 3.98 (s, 2H), 3.92 (s, 4H), 3.65- 3.51 (m, 1H), 2.89 - 2.76 (m, 1H), 2.13 (d, 2H), 1.92 (d, 2H), 1.2 - 1.44 (m, 2H), 1.40 - 1.26 (m, 2H).

$^{19}$F NMR (376 MHz, DMSO-$d_6$) δ -58.34.

**Example 75:**

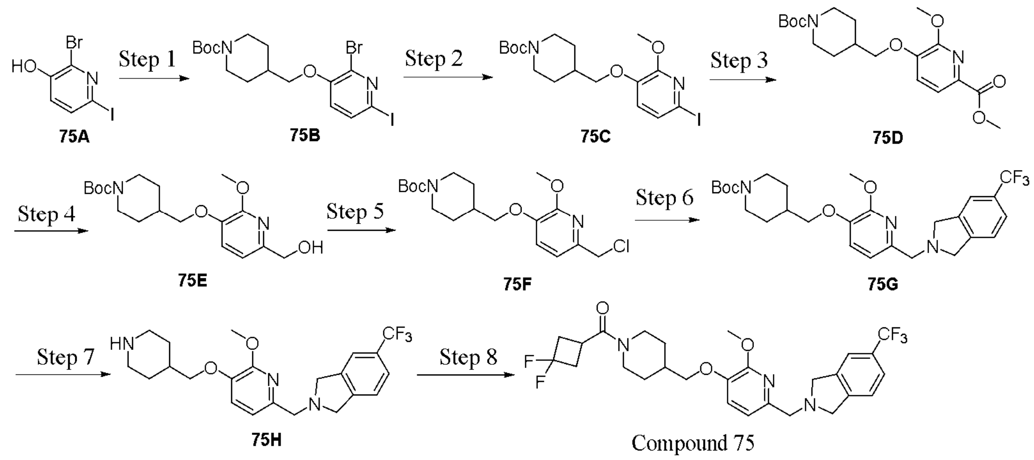


Step 1:

Compound **75A** (12.0 g, 40.13 mmol), tert-butyl 4-(bromomethyl)piperidine-1-carboxylate (11.13 g, 40.13 mmol) and potassium carbonate (11.1 g, 80.26 mmol) were added to acetonitrile (200 mL), and the resulting mixture was reacted at 80°C overnight, concentrated, separated and purified by silica gel chromatography column (EA:PE = 5:2) to obtain compound **75B** (15.0 g, 75.4%).
LC-MS (ESI): m/z= 441.1 $[M-56+H]^+$.

Step 2:

**75B** (5.0 g, 10.08 mmol), sodium methoxide (5.4 M/L methanol solution, 1.9 mL, 10.08 mmol) was dissolved in N-methylpyrrolidone (50 mL), and the resulting mixture was reacted at 100°C overnight. Water (50 mL) was added, and the resulting mixture was extracted with ethyl acetate (100 mL × 2), and washed with water (50 mL × 3). The organic phases were combined, concentrated, separated and purified by silica gel chromatography column (EA:PE = 5:2) to obtain compound **75C** (1.5 g, 33.2%).
LC-MS (ESI): m/z= 393.1 $[M-56+H]^+$.

Step 3:

Compound **75C** (1.5 g, 3.35 mmol) was dissolved in methanol (50 mL), and 1,1'-bis(diphenylphosphino) ferrocene]palladium dichloride (249 mg, 0.34 mmol) and triethylamine (1.02 g, 10.05 mmol) were added. The autoclave was filled with carbon monoxide to a pressure of about 2.0 Mpa, and the resulting mixture was reacted at 60°C overnight, concentrated, separated and purified by silica gel chromatography column (EA:PE = 5:1) to obtain compound **75D** (1.1, 86.6%).
LC-MS (ESI): m/z= 325.1 $[M-56+H]^+$.

Step 4:

Compound **75D** (1.0 g, 2.63 mmol) was dissolved in tetrahydrofuran (20 mL), lithium aluminum hydride (200 mg, 5.26 mmol) was added at 0°C, and the resulting mixture was stirred for 30 min. Then water (0.2 mL) was added, and the resulting mixture was stirred for 5 min, and then 10% aqueous sodium hydroxide solution (0.2 mL) was added. Water (0.6 mL) was then added, and anhydrous magnesium sulfate was added and the stirring was continued for 10 min. After filtration, the filtrate was concentrated, separated and purified by silica gel chromatography column (EA:PE = 3:1) to obtain compound **75E** (700 mg, 75.6%).
LC-MS (ESI): m/z= 353.1 [M+H]+.

Step 5:

Compound **75E** (0.70 g, 1.99 mmol) was dissolved in dichloromethane (20 mL), thionyl chloride (474 mg, 3.98 mmol) was added, N,N-dimethylformamide (3 drops) was added dropwise, and the resulting mixture was stirred at room temperature for 30 min, and concentrated to obtain the crude product of compound **75F,** which was directly used in the next step.

LC-MS (ESI): m/z= 315.1 [M-56+H]+.

Step 6:

The crude product of compound **75F** (1.99 mmol) was dissolved in acetonitrile (20 mL), 5-(trifluoromethyl) isoindoline hydrochloride (444 mg, 1.99 mmol) was added, N,N-diisopropylethylamine (770 mg, 5.97 mmol) was then added, and the resulting mixture was stirred at 80°C for 2 h, concentrated, separated and purified by silica gel chromatography column (MeOH:DCM = 1:10) to obtain compound **75G** (300 mg, two-step yield 28.9%).

LC-MS (ESI): m/z= 522.2 [M+H]+.

Step 7:

Compound **75G** (100 mg, 0.19 mmol) was dissolved in dichloromethane (2 mL), trifluoroacetic acid (0.5 mL) was added, and the resulting mixture was reacted under stirring at room temperature for 2 h, and then concentrated to obtain the crude product of compound **75H,** which was directly used in the next step.

LC-MS (ESI): m/z= 245.1 [M+H]+.

Step 8:

Compound 3,3-difluorocyclobutane-1-carboxylic acid (72 mg, 0.19 mmol) was dissolved in N,N-dimethylformamide (2 mL), HATU (72 mg, 0.19 mmol), the crude product of **75H** (0.19 mmol) and N,N-diisopropylethylamine (77 mg, 0.6 mmol) were added, and the resulting mixture was reacted at room temperature for 2 h. Water (10 mL) was added, and the resulting mixture was extracted with ethyl acetate (30 mL × 2), and washed with water (10 mL × 3). The organic phases were combined, concentrated, separated and purified by silica gel chromatography column (MeOH:DCM = 1:10, Rf = 0.3) to obtain **compound 75** (30 mg, two-step yield 29.2%).

LC-MS (ESI): m/z= 540.2 [M+H]+.

$^1$H NMR (400 MHz, DMSO-$d_6$) δ 7.61 (s, 1H), 7.55 (d, 1H), 7.45 (d, 1H), 7.26 (d, 1H), 6.95 (d, 1H), 4.39 (d, 1H), 4.02 - 3.98 (m, 4H), 3.86 - 3.84 (m, 5H), 3.83 - 3.76 (m, 2H), 3.29 - 3.22 (m, 2H), 3.06 - 2.92 (m, 1H), 2.84 - 2.69 (m, 4H), 2.68 - 2.58 (m, 1H), 2.09 - 1.88 (m, 1H), 1.87 - 1.63 (m, 2H), 1.24 - 1.11 (m, 2H).

**Example 76**

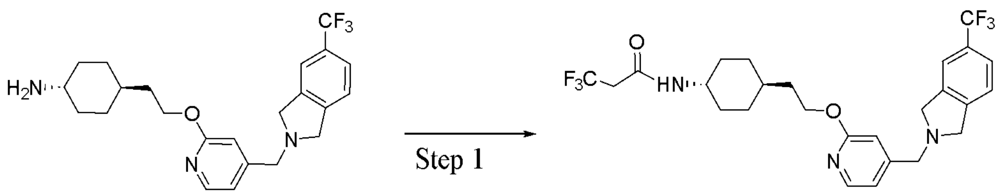


62B

Compound 76

Step 1:

Compound **62B** (419 mg, 1.0 mmol) was dissolved in dichloromethane (10 mL), and triethylamine (151 mg, 1.5 mmol) was then added. 3,3,3-fluoropropionyl chloride (176 mg, 1.2 mmol) was added dropwise at -30°C, and the resulting mixture was transferred to room temperature and reacted for 20 min. A small amount of methanol was added to quench the reaction, the reaction solution was concentrated, and the residue was separated and purified by silica gel column chromatography (PE/EA = 1/1) to obtain compound 76 (140 mg, 26%).

LC-MS (ESI): m/z = 530.2 $[M+H]^+$.

$^1$H NMR (400 MHz, $CDCl_3$) δ 8.10 (d, 1H), 7.47 (d, 1H), 7.44 (s, 1H), 7.29 (d, 1H), 6.92 (d, 1H), 6.77 (s, 1H), 5.58 (d, 1H), 4.32 (t, 2H), 4.00 (s, 4H), 3.85 (d, 2H), 3.77 (s, 1H), 3.02 (q, 2H), 2.06 - 1.96 (m, 2H), 1.94 - 1.80 (m, 2H), 1.72 - 1.67 (m, 2H), 1.54 - 1.43 (m, 1H), 1.23 - 1.06 (m, 4H).

$^{19}$F NMR (377 MHz, $CDCl_3$) δ -60.03 (s), -61.04 (s).

**Example 77**

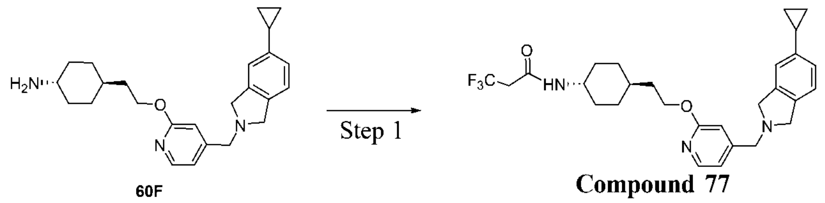


Step 1:

Compound **60F** (392 mg, 1.0 mmol) was dissolved in dichloromethane (10 mL), and triethylamine (151 mg, 1.5 mmol) was then added. 3,3,3-fluoropropionyl chloride (176 mg, 1.2 mmol) was added dropwise at -30°C, and the resulting mixture was transferred to room temperature and reacted for 20 min. A small amount of methanol was added to quench the reaction, the reaction solution was concentrated, and the residue was separated and purified by silica gel column chromatography (PE/EA = 1/1) to obtain compound **77** (180 mg, 36%).

LC-MS (ESI): m/z = 502.3 $[M+H]^+$.

$^1H$ NMR (400 MHz, $CDCl_3$) δ 8.09 (d, 1H), 7.06 (d, 1H), 6.97 - 6.91 (m, 2H), 6.90 (s, 1H), 6.79 (s, 1H), 5.52 (d, 1H), 4.31 (t, 2H), 3.95 (s, 4H), 3.87 (s, 2H), 3.77 - 3.74 (m, 1H), 3.02 (q, 2H), 2.05 - 2.00 (s, 2H), 1.92 - 1.84 (m, 3H), 1.68 (q, 2H), 1.54 - 1.40 (m, 1H), 1.22 - 1.06 (m, 4H), 0.97 - 0.88 (m, 2H), 0.68 - 0.61 (m, 2H).

$^{19}F$ NMR (377 MHz, $CDCl_3$) δ -61.03 (s).

**Example 78:**

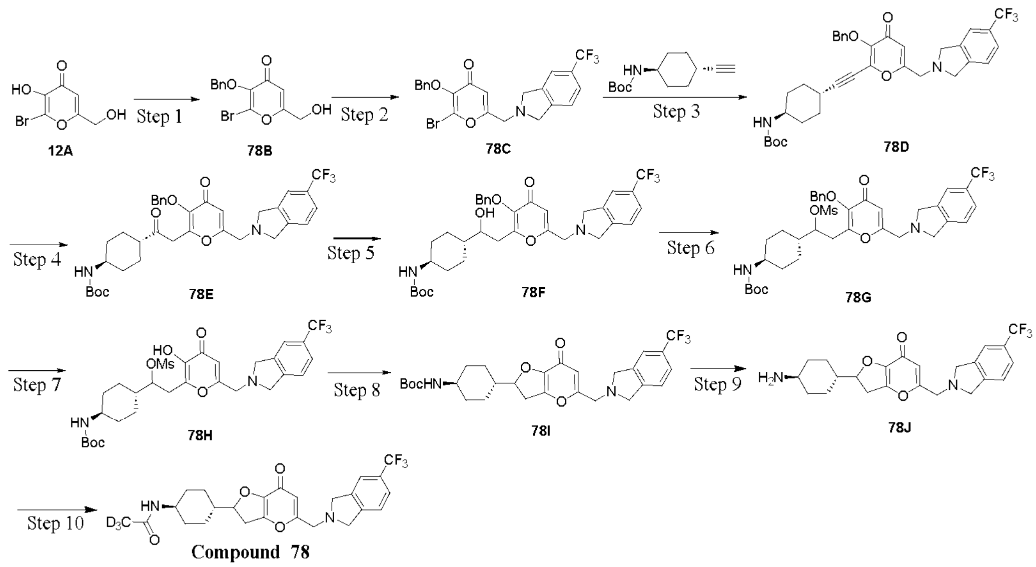


Step 1:

Compound 12A (10.0 g, 45.25 mmol) was dissolved in methanol (50 mL), and a solution of sodium hydroxide (2.5 g, 62.30 mmol) in water (50 mL) and benzyl bromide (9.5 g, 55.57 mmol) were then successively added. After the addition was completed, the resulting mixture was warmed to 50°C and reacted for 12 h. After the reaction was completed, the reaction product was concentrated and methanol was removed. 100 mL of water was added, and the resulting mixture was extracted with ethyl acetate (150 mL × 3). The organic layers were combined, washed with saturated aqueous sodium chloride solution (200 mL), dried over anhydrous sodium sulfate, concentrated and purified by column chromatography (petroleum ether: ethyl acetate (v/v) = 5:1) to obtain compound 78B (10.0 g, 71%).

LC-MS (ESI): *m*/*z* = 311.0 $[M+H]^+$.

Step 2:

Compound 78B (8.0 g, 25.71 mmol) was dissolved in dichloromethane (100 mL), and triethylamine (5.26 g, 51.93 mmol) and methylsulfonyl chloride (4.01 g, 34.97 mmol) were successively added in an ice-water bath. The reaction solution was warmed to room temperature and stirring was continued for 1 h. After complete depletion of raw materials as monitored by TLC, 5-trifluoromethylisoindoline hydrochloride (8.80 g, 39.34 mmol) and triethylamine (5.26 g, 51.93 mmol) were then added, and the reaction solution was stirred at room temperature overnight. After the reaction was completed, 150 mL of water was added, and the resulting mixture was extracted with ethyl acetate (150 mL × 3). The organic layers were combined, washed with saturated aqueous sodium chloride solution (200 mL), dried over anhydrous sodium sulfate, concentrated and purified by column chromatography (petroleum ether: ethyl acetate (v/v) = 1:1) to obtain compound 78C (3.5 g, 28%).
LC-MS (ESI): *m/z* = 480.10 $[M+H]^+$.

Step 3:

Compound 78C (2.5 g, 5.21 mmol) and trans-1-(Boc-amino)-4-ethynylcyclohexane (2.0 g, 1.72 mmol) were dissolved in toluene (50 mL), bis(triphenylphosphine)palladium dichloride (0.37 g, 0.52 mmol) and cuprous iodide (0.2 g, 1.03 mmol) were then successively added, triethylamine (1.58 g, 15.63 mmol) was then added under nitrogen atmosphere, and the reaction solution was warmed to 100°C and stirred for 12 h. After the reaction was completed, the reaction product was filtered, and the filtrate was concentrated under reduced pressure, and then purified by column chromatography (petroleum ether: ethyl acetate (v/v) = 1:1) to obtain compound 78D (1.3 g, 40%).
LC-MS (ESI): m/z = 623.50 $[M+H]^+$.

Step 4:

Compound 78D (1.3 g, 2.09 mmol) was dissolved in N,N-dimethylformamide (15 mL), tetrahydropyrrole (2 mL) was then added, and the reaction solution was warmed to 80°C and stirred for 12 h. After the reaction was completed, 30 mL of water was added, and the resulting mixture was extracted with ethyl acetate (40 mL × 3). The organic layers were combined, washed with saturated aqueous sodium chloride solution (100 mL), dried over anhydrous sodium sulfate, concentrated and purified by column chromatography (petroleum ether: ethyl acetate (v/v) = 1:1) to obtain compound 78E (1.0 g, 74%).
LC-MS (ESI): *m/z* = 641.30 $[M+H]^+$.

Step 5:

Compound 78E (1.0 g, 1.56 mmol) was dissolved in methanol (15 mL), sodium borohydride (0.12 g, 3.17 mmol) was then added, and the reaction solution was stirred at room temperature for 2 h. After the reaction was completed, the reaction product was concentrated and methanol was removed. 30 mL of water was added, and the resulting mixture was extracted with ethyl acetate (50 mL × 3). The organic layers were combined, washed with saturated aqueous sodium chloride solution (100 mL), dried over anhydrous sodium sulfate, concentrated and purified by column chromatography (petroleum ether: ethyl acetate (v/v) = 1:3) to obtain compound 78F (0.7 g, 70%).
LC-MS (ESI): *m/z* = 643.30 $[M+H]^+$.

Step 6:

Compound 78F (600 mg, 0.93 mmol) was dissolved in dichloromethane (10 mL), triethylamine (140 mg, 1.40 mmol) and methylsulfonyl chloride (130 mg, 1.12 mmol) were successively added in an ice-water bath, and the reaction solution was warmed to room temperature and stirring was continued for 1 h. After the reaction was completed, 15 mL of water was added, and the resulting mixture was extracted with ethyl acetate (20 mL × 3). The organic layers were combined, washed with saturated aqueous sodium chloride solution (50 mL), dried over anhydrous sodium sulfate, concentrated and purified by column chromatography (petroleum ether: ethyl acetate (v/v) = 1:1) to obtain compound 78G (450 mg, 67%).
LC-MS (ESI): *m/z* = 721.70 $[M+H]^+$.

Step 7:

Compound 78G (450 mg, 0.62 mmol) was dissolved in methanol (10 mL), Pd/C (13 mg, 0.12 mmol) was then added. After hydrogen replacement, the reaction solution was stirred at room temperature for 1 h. After the reaction was

completed, the reaction product was filtered and concentrated to obtain compound 78H (350 mg, 89%).

LC-MS (ESI): *m/z* = 631.90 [M+H]+.

Step 8:

Compound 78H (350 mg, 0.55 mmol) was dissolved in N,N-dimethylformamide (10 mL), potassium carbonate (150 mg, 1.1 mmol) was then added, and the reaction solution was stirred at room temperature for 3 h. After the reaction was completed, 20 mL of water was added, and the resulting mixture was extracted with ethyl acetate (20 mL × 3). The organic layers were combined, washed with saturated aqueous sodium chloride solution (40 mL), dried over anhydrous sodium sulfate, concentrated and purified by column chromatography (petroleum ether: ethyl acetate (v/v) = 1:3) to obtain compound 78I (80 mg, 27%).

LC-MS (ESI): *m/z* = 535.30 [M+H]+.

Step 9:

Compound 78I (80 mg, 0.24 mmol) was dissolved in dichloromethane (5 mL), hydrochloric acid/1,4-dioxane solution (5 mL) was added, and the resulting mixture was stirred at room temperature for 2 h. After the reaction was completed, the reaction product was concentrated to obtain compound 78J (65 mg, crude product), which was directly used in the next step without purification.

LC-MS (ESI): m/z = 435.30 [M+H]+.

Step 10:

Compound 78J (65 mg, 0.15 mmol) was dissolved in dichloromethane (5 mL), and triethylamine (46 mg, 0.45 mmol) and deuterated acetyl chloride (13 mg, 0.17 mmol) were successively added in an ice-water bath. The reaction solution was warmed to room temperature, and stirring was continued for 1 h. After the reaction was completed, 10 mL of water was added, and the resulting mixture was extracted with ethyl acetate (15 mL × 3). The organic layers were combined, washed with saturated aqueous sodium chloride solution (25 mL), dried over anhydrous sodium sulfate, concentrated and purified by column chromatography (dichloromethane: methanol (v/v) = 15:1) to obtain compound 78 (12 mg, 16%).

LC-MS (ESI): m/z = 480.30 [M+H]+.

$^{1}$H NMR (400 MHz, DMSO-$d_6$) δ 7.74 (s, 1H), 7.71-7.65 (m, 2H), 7.57 (d, 1H), 6.51 (s, 1H), 4.60-4.40 (m, 5H), 4.29 (s, 2H), 3.34-3.24 (m, 2H), 3.16-3.08 (m, 1H), 1.90-1.80 (m, 3H), 1.67-1.55 (m, 2H), 1.18-1.06 (m, 4H).

**Example 79:**

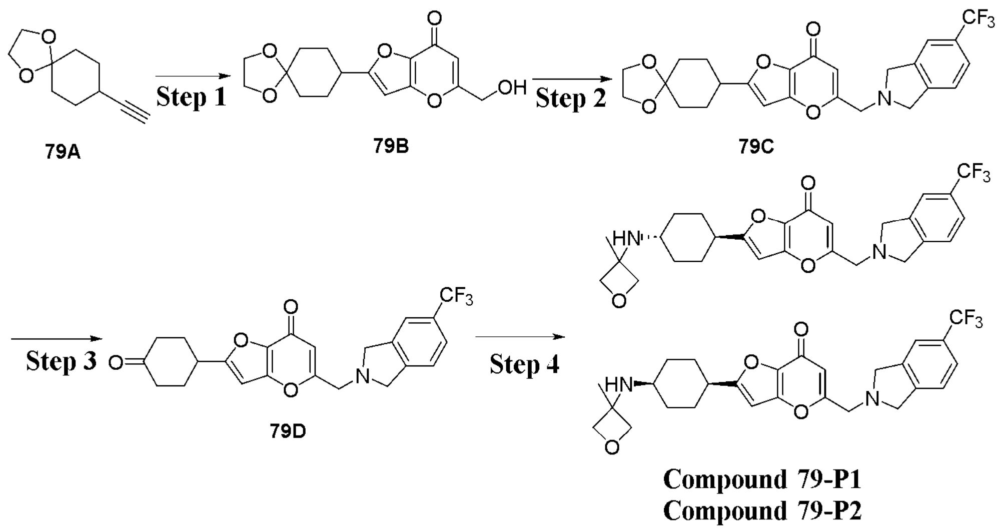


79A 79B 79C 79D

Compound 79-P1
Compound 79-P2

Step 1:

79A (2.00 g, 12.03 mmol), 78A (2.66 g, 12.03 mmol) and triethylamine (3.65 g, 36.09 mmol) were dissolved in tetrahydrofuran (40 mL), and the resulting mixture was subjected to nitrogen replacement 3 times. Bis(triphenylphosphorus)palladium dichloride (0.84 g, 1.20 mmol) and cuprous iodide (0.46 g, 2.41 mmol) were added. After the addition was completed, the resulting mixture was subjected to nitrogen replacement three times and reacted at 40°C overnight. After the reaction was completed, the reaction was concentrated and the residue purified by column chromatography (petroleum ether/ethyl acetate = 1/2, (v/v)) to obtain compound 79B (500 mg, yield: 13%).
LC-MS (ESI): m/z= 307.0 $[M+1]^+$

Step 2:

79B (0.50 g, 1.63 mmol) was dissolved in dichloromethane (10 mL), and triethylamine (659.76 mg, 6.52 mmol) and methylsulfonyl chloride (224.06 mg, 1.96 mmol) were added at 0°C-5°C. After the addition was completed, the resulting mixture was reacted at room temperature for 30 min, and 5-trifluoromethylisoindoline (366.08 mg, 1.96 mmol) was added at room temperature. After the addition was completed, the resulting mixture was reacted at room temperature overnight. After the reaction was completed, the reaction solution was concentrated and purified by column chromatography (petroleum ether/ethyl acetate = 1/1, (v/v)) to obtain compound 79C (570 mg, yield: 73%).
LC-MS (ESI): m/z= 476.2 $[M+H]^+$ .

Step 3:

79C (570 mg, 1.2 mmol) was dissolved in tetrahydrofuran (10 mL), and water (2 mL) and concentrated hydrochloric acid (2 mL) were added dropwise at room temperature. After the addition was completed, the mixture was reacted at room temperature for 2 hrs. After the reaction was completed, the reaction solution was concentrated and purified by column chromatography (petroleum ether/ethyl acetate = 1/1, (v/v)) to obtain compound 79D (450 mg, yield: 87%).
LC-MS (ESI): m/z= 432.2 $[M+H]^+$ .

Step 4:

79D (400 mg, 0.93 mmol), 3-methyl 3-aminooxetane (405.11 mg, 4.65 mmol) were dissolved in dichloroethane (10 mL), and the resulting mixture was subjected to nitrogen replacement three times. Tetraisopropyl titanate (768 mg, 2.7 mmol) was added dropwise at room temperature, and after the addition was completed, the resulting mixture was reacted at room temperature overnight. Then sodium borohydride (52.77 mg, 1.4 mmol) was added to the reaction system at 0°C-5°C, and after the addition was completed, the resulting mixture was reacted at room temperature for 1 h. After the reaction was completed, saturated sodium bicarbonate solution was added to the reaction system to quench the reaction, and the resulting mixture was filtered. The filtrate was extracted with dichloromethane (20 mL*2). The organic phases were combined and concentrated, and then purified by preparative HPLC to obtain compound 79-P1 and compound 79-P2.
Compound 79-P1: 50.00 mg, yield: 10%), LC-MS (ESI): m/z= 503.4 $[M+H]^+$.
$^1H$ NMR (400 MHz, DMSO-$d_6$) δ 9.40 (s, 1H), 7.72 (s, 1H), 7.66-7.64 (d, 1H), 7.56-7.54 (d, 1H), 6.78 (s, 1H), 6.57 (s, 1H), 4.77-4.75 (d, 2H), 4.44-4.40 (m, 6H), 4.30 (s, 2H), 3.33 -3.20(m, 1H), 2.89-2.78 (m, 1H), 2.14-2.05(m, 2H), 1.93-1.84 (m, 2H), 1.68 (s, 3H), 1.59-1.53 (m, 4H).
Compound 79-P2: LC-MS (ESI): m/z= 503.4 $[M+H]^+$

**Example 80, 81:**

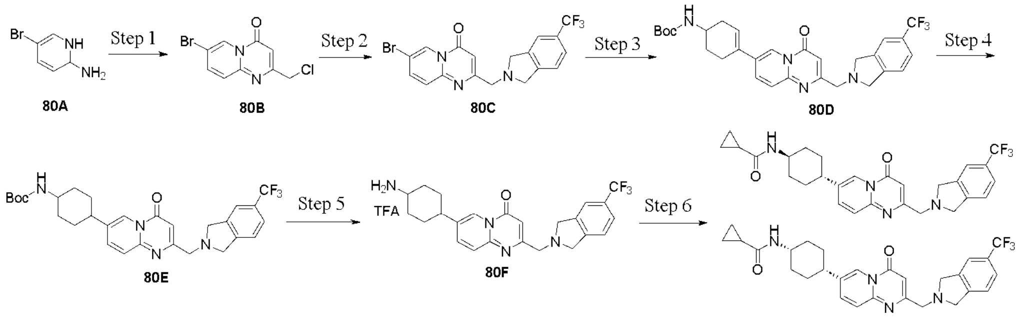


80A 80B 80C 80D 80E 80F

Compound 80 Compound 81

Step 1:

Compound **80A** (3.0 g, 17.24 mmol), ethyl 4-chloro-3-oxobutanoate (2.8 g, 17.24 mmol) and polyphosphoric acid (30 mL) were stirred at 110°C for 30 min, and the resulting mixture was adjusted to pH = 7-8 with saturated aqueous sodium bicarbonate solution, extracted with ethyl acetate (100 mL × 2), and washed with saturated aqueous sodium chloride solution (50 mL×). The organic phases were combined, concentrated, separated and purified by silica gel chromatography column (eluent: ethyl acetate/petroleum ether (v/v) = 20/100) to obtain compound **80B** (2.0 g, 42.7%).

LC-MS (ESI): m/z= 273.1 $[M+H]^+$.

Step 2:

**80B** (2.0 g, 7.35 mmol) and 5-(trifluoromethyl)isoindoline (1.4 mL, 7.48 mmol) were dissolved in acetonitrile (20 mL), triethylamine (2.2 g, 22.05 mmol) was added, and the resulting mixture was reacted at 60°C for 4 h, concentrated, separated and purified by silica gel chromatography column (eluent: ethyl acetate/petroleum ether (v/v) = 40/100) to obtain compound **80C** (2.0 g, 64.5%).

LC-MS (ESI): m/z= 424.2 $[M+H]^+$.

Step 3:

Compound **80C** (2.0 g, 4.73 mmol) was dissolved in dioxane (40 mL), water (8 mL), 1,1'-bis(diphenylphosphino) ferrocene]palladium dichloride (366 mg, 0.5 mmol), potassium carbonate (2.0 g, 14.19 mmol), tert-butyl (4-(4,4,5,5-tetramethyl-1,3,2-dioxaboran-2-yl)cyclohex-3-en-1-yl)carbamate (1.5 g, 4.73 mmol) were added, and the resulting mixture was reacted at 100°C overnight under nitrogen protection, concentrated, separated and purified by silica gel chromatography column (eluent: ethyl acetate/petroleum ether (v/v) = 40/100) to obtain compound **80D** (2.0, 78.1%).

LC-MS (ESI): m/z= 541.2 $[M+H]^+$.

Step 4:

Compound **80D** (1.0 g, 1.85 mmol) was dissolved in methanol (10 mL), palladium on carbon (200 mg) 1 was added and the resulting mixture was subjected to hydrogenation reaction at room temperature for 24 h. After filtration, the filtrate was concentrated, separated and purified by silica gel chromatography column (eluent: ethyl acetate/petroleum ether (v/v) = 40/100) to obtain compound **80E** (1.0 g, 99.9%).

LC-MS (ESI): m/z=543.2 $[M+H]^+$.

Step 5:

Compound **80E** (0.5 g, 0.92 mmol) was dissolved in dichloromethane (3 mL), trifluoroacetic acid (1 mL) was added, and the resulting mixture was stirred at room temperature for 1 h to obtain the reaction solution containing **80F,** which was directly used in the next step.

LC-MS (ESI): m/z= 443.2 $[M+H]^+$.

Step 6:

Triethylamine was added to the above reaction solution containing **80F** to adjust pH = 7-8. Cyclopropylformyl chloride (96 mg, 0.92 mmol) was added, and the resulting mixture was stirred at room temperature for 30 min,

concentrated, separated and purified by silica gel chromatography column (eluent: methanol/dichloromethane (v/v) = 10/100) to obtain a mixture (100 mg, 21.3%). After chiral preparation, the compound **80** (25 mg, 5.3%, retention time: 0.860 min) and the compound **81** (30 mg, 6.4%, retention time: 1.156 min) were obtained. Chiral preparation method: Instrument: Waters 150 MGM; chromatographic column: Chiralpak Column; mobile phase: A: carbon dioxide, and B: ethanol; isocratic elution: 70% mobile phase B; Flow rate: 100 mL/min; back pressure: 100 bar; Column temperature: 25°C; wavelength: 220 nm; elution time: 4.0 min.

**Compound 80:** $^1$H NMR (400 MHz, DMSO-$d_6$) δ8.84 (s, 1H), 8.12 - 8.00 (m, 2H), 7.74 - 7.65 (m, 1H), 7.66 - 7.54 (m, 2H), 7.52 - 7.42 (m, 1H), 6.49 (s, 1H), 4.07 (s, 4H), 3.93 (s, 2H), 3.30 - 3.29 (m, 1H), 2.82 - 2.71 (m, 1H), 1.94 - 1.79 (m, 2H), 1.77 - 1.61 (m, 7H), 0.74 - 0.59 (m, 4H).

LC-MS (ESI): m/z = 511.3[M+H]$^+$.

**Compound 81:** $^1$H NMR (400 MHz, DMSO-$d_6$) δ8.81 (s, 1H), 8.07 - 7.90 (m, 2H), 7.68 - 7.46 (m, 4H), 6.47 (s, 1H), 4.05 (s, 4H), 3.93 (s, 2H), 3.74 - 3.58 (m, 1H), 2.77 - 2.68 (m, 1H), 1.98 - 1.85 (m, 4H), 1.65 - 1.51 (m, 3H), 1.43 - 1.31 (m, 2H), 0.71 - 0.58 (m, 4H).

LC-MS (ESI): m/z = 511.3[M+H]$^+$.

**Example 82:**

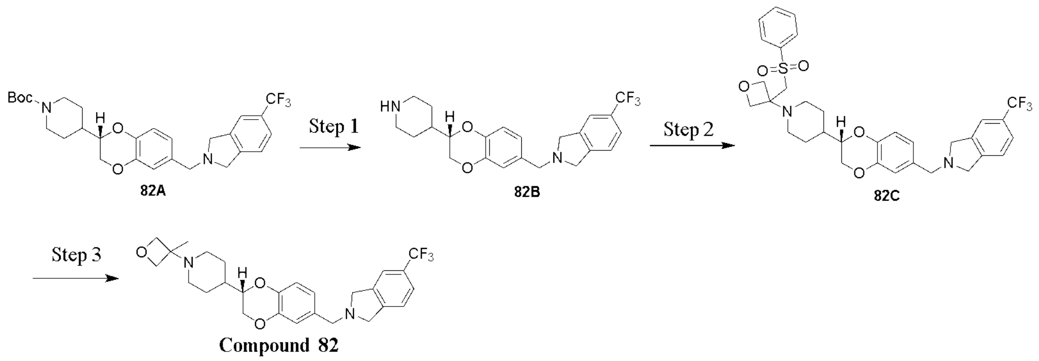


Step 1:

**82A** (0.1 g, 0.19 mmol) was dissolved in dichloromethane (2 mL), hydrogen chloride dioxane (1 mL) was added, and the resulting mixture was reacted at room temperature for 4 h. After the reaction was completed, aqueous sodium hydroxide solution (5 mL) was added to the reaction system to adjust PH = about 8, and the resulting mixture was extracted with dichloromethane (10 mL*2), dried over anhydrous sodium sulfate, and filtered. The filtrate was concentrated to obtain crude **82B** (76 mg, 95%).

LC-MS (ESI): m/z = 419.2 [M+H]$^+$.

Step 2:

Compound **82B** (76 mg, 0.18 mmol) was dissolved in methanol (4 mL), and 3-[(benzenesulfonyl)methylene] oxetane (50 mg, 0.24 mmol) was added. After the addition was completed, the resulting mixture was reacted at 50°C for 16 h. After the reaction was completed, water (3 mL) was added to the reaction system, and the resulting mixture was extracted with ethyl acetate (5 mL*3), washed with saturated aqueous sodium chloride solution (2 mL), dried over anhydrous sodium sulfate, and filtered. The filtrate was concentrated and the residue was separated and purified by silica gel column chromatography (petroleum ether: ethyl acetate (v/v) = 2:1) to obtain **82C** (105 mg, 92%).
LC-MS (ESI): m/z = 629.2 [M +1]$^+$

Step 3:

Compound **82C** (105 mg, 0.16 mmol) was dissolved in methanol (5 mL) and tetrahydrofuran (1 mL), magnesium (40 mg, 1.6 mmol) was added, and the resulting mixture was warmed to 40°C and reacted for 16 h. The reaction was cooled to room temperature, then water (5 mL) was added to the reaction system to quench the reaction, and the resulting mixture was extracted with ethyl acetate (5 mL*2), washed with saturated aqueous sodium chloride solution (3 mL), dried over anhydrous sodium sulfate, and filtered. The filtrate was concentrated and the residue was separated and purified by silica

gel column chromatography (petroleum ether: ethyl acetate (v/v) = 2:1) to obtain compound **82** (34 mg, 42%).

LC-MS (ESI): m/z = 489.2 [M +1]$^+$

$^1$H NMR (400 MHz, DMSO-$d_6$ ) δ 7.60 (s, 1H), 7.57 - 7.51 (m,1H), 7. 47 - 7.42 (m, 1H), 6.89 - 6.84 (m, 1H), 6.83 - 6.79 (m , 2H), 4.41 - 4.36 (m, 2H), 4.34 - 4.26 (m, 1H),4.14 - 4.06 (m, 2H), 4.02 - 3.90 (m, 2H), 3.89 - 3.82 (m, 4H), 3.78-3.72 (m, 2H), 2.03 - 1.96 (m, 2H), 1.92 - 1.82 (m, 2H), 1.70 - 1.63 (m, 1H), 1.61 - 1.56 (m, 2H), 1.46 -1.35 (m,2H), 1.24 (s,3H).

**Example 83:**

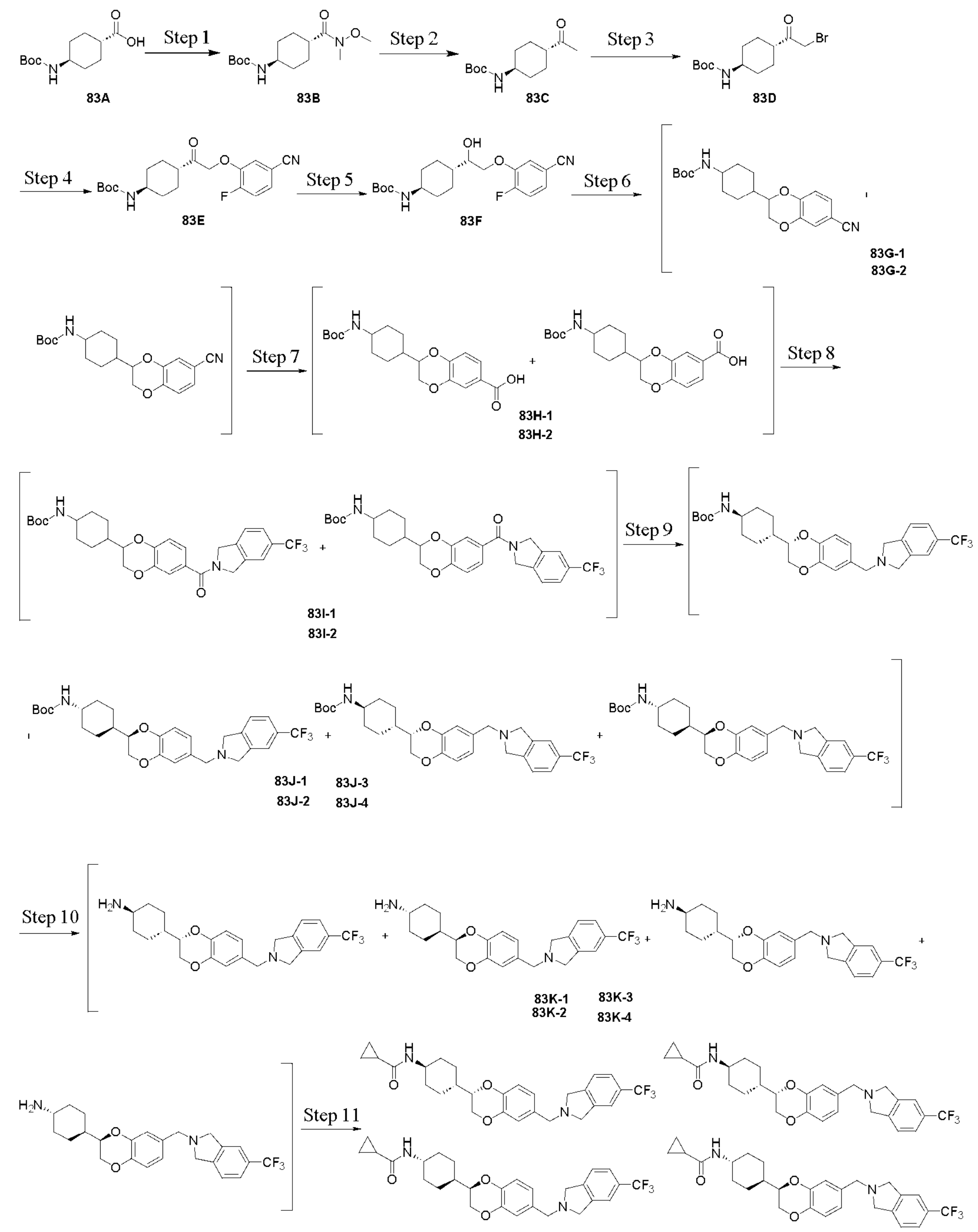


83A 83B 83C 83D 83E 83F 83G-1 83G-2 83H-1 83H-2 83I-1 83I-2 83J-1 83J-2 83J-3 83J-4 83K-1 83K-2 83K-3 83K-4

Step 1:

83A (50 g, 205.5 mmol), dimethylhydroxylamine hydrochloride (22 g, 226 mmol), HOBT (30.5 g, 226 mmol) and EDCI (43.3 g, 226 mmol) were added to dichloromethane (1 L), N-methylmorpholine (83 g, 822 mmol) was added, and the

resulting mixture was reacted under stirring at room temperature for 2 h. After the reaction was completed as monitored by LCMS, the organic phase was washed twice with saturated aqueous sodium chloride solution (1 L), dried over anhydrous sodium sulfate, and filtered. The filtrate was concentrated to obtain crude product 83B (52 g, 88.4% yield).

LC-MS(ESI): m/z=231.2 $[M+1]^+$.

Step 2:

At -10°C, methyl magnesium bromide (181.6 mL, 544.7 mmol, 3 M in $Et_2O$) was added dropwise to a solution of **83B** (52 g, 181.6 mmol) in tetrahydrofuran (500 mL). After the resulting mixture was stirred at 0°C for 4 h, saturated aqueous ammonium chloride solution (1 L) was added and the resulting mixture was extracted with ethyl acetate (200 mL x 3). The combined organic phase was dried over anhydrous sodium sulfate, filtered and concentrated to obtain crude product **83C** (43 g, 98% yield).

LC-MS(ESI): m/z=186.1 $[M+1]^+$.

Step 3:

At 0°C, N-bromosuccinimide (33.3 g, 187.1 mmol) was slowly added to a solution of **83C** (43 g, 178.2 mmol) in methanol (500 mL), then p-toluenesulfonic acid monohydrate (3.39 g, 17.8 mmol) was added, and the resulting mixture reacted at room temperature for 16 h. Water (500 mL) was added to the system, and the product was precipitated, filtered and dried to obtain **83D** (49 g, 85.9% yield).

LC-MS(ESI): m/z=264.1&266.1 $[M+1]^+$.

Step 4:

Using **83D** (49 g, 153 mmol) as the raw material, the operation method of step 1 of example 56 was referenced to obtain compound **83E** (54 g, 93.8% yield). LC-MS (ESI): m/z = 321.2 $[M+H]^+$.

Step 5:

Using **83E** (54 g, 143.5 mmol) as the raw material, the operation method of step 2 of example 56 was referenced to obtain compound **83F** (50 g, 92% yield).

LC-MS (ESI): m/z = 323.1 $[M+H]^+$.

Step 6:

Using **83F** (50 g, 132.1 mmol) as the raw material, the operation method of step 3 of example 56 was referenced to obtain a mixture of compounds **83G-1** and **83G-2** (34 g, 71.8% yield).

LC-MS (ESI): m/z = 303.1 $[M+H]^+$.

Step 7:

Using **83G** (30 g, 83.7 mmol) as the raw material, the operation method of step 4 of example 56 was referenced to obtain a mixture of compounds **83H-1** and **83H-2** (11 g, 34.8% yield).

LC-MS (ESI): m/z = 322.1 $[M+H]^+$.

Step 8:

Using **83H** (10 g, 26.5 mmol) as the raw material, the operation method of step 5 of example 56 was referenced to obtain a mixture of compounds **831-1** and **831-2** (8.5 g, 58.7% yield).

LC-MS (ESI): m/z = 492.2 $[M+H]^+$.

Step 9:

Using **83I** (5 g, 9.1 mmol) as the raw material, the operation method of step 6 of example 56 was referenced to obtain a mixture of compound 83J (2.5 g, 51.3%). **83J-1** (45.9 mg, retention time 1.021 min), **83J-2** (22.0 mg, retention time 1.231 min), **83J-3** (563.6 mg, retention time 1.408 min), **83J-4** (679.9 mg, retention time 2.321 min) was obtained by SFC resolution.

SFC separation conditions:

1. HPLC purification method;
instrument: SHIMADZU LC-20AP; chromatographic column: C18 column; mobile phase: A for 10 mmol/L $NH_4HCO_3$ in water; B for acetonitrile; gradient: B from 80% to 100% in 10 min; flow rate: 90 mL/min;
2. P1/P2 SFC purification method
instrument: SHIMADZU LC-20AP; chromatographic column: Chiral IA column; mobile phase: A for hexane; B for ethanol; gradient: B for 8%; flow rate: 60 mL/min;
3. P3/P4 SFC purification method
instrument: Waters 150 Prep-SFC; chromatographic column: Chiral AD column; mobile phase: A for CO2; B for methanol; gradient: B for 65%; flow rate: 100 mL/min;

LC-MS (ESI): m/z = 533.2 $[M+H]^+$

Step 10:

Using **83J-1**(45.9 mg, 0.09 mmol) as the raw material, the operation method of step 7 of example 56 was referenced to obtain compound **83K-1** (37 mg, 99% yield).
Using **83J-2**(22.0 mg, 0.04 mmol) as the raw material, the operation method of step 7 of example 56 was referenced to obtain compound **83K-2** (17 mg, 99% yield).
Using **83J-3(50** mg, 0.09 mmol) as the raw material, the operation method of step 7 of example 56 was referenced to obtain compound **83K-3** (40 mg, 99% yield).
Using **83J-4**(50 mg, 0.09 mmol) as the raw material, the operation method of step 7 of example 56 was referenced to obtain compound **83K-4** (40 mg, 99% yield).
LC-MS (ESI): m/z = 419.1 $[M+H]^+$.

Step 11:

Using **83K-1** (37 mg, 0.09 mmol) and cyclopropylformyl chloride (10 mg, 0.1 mmol) as the raw material, the operation method of step 8 of example 56 was referenced to obtain **compound 83-1** (5 mg, 11.7% yield).
Using **83K-2** (17 mg, 0.04 mmol) and cyclopropylformyl chloride (5 mg, 0.05 mmol) as the raw material, the operation method of step 8 of example 56 was referenced to obtain **compound 83-2** (3 mg, 15.3% yield).
Using **83K-3** (40 mg, 0.09 mmol) and cyclopropylformyl chloride (10 mg, 0.1 mmol) as the raw material, the operation method of step 8 of example 56 was referenced to obtain **compound 83-3** (10 mg, 21.6% yield).
Using **83K-4** (40 mg, 0.09 mmol) and cyclopropylformyl chloride (10 mg, 0.1 mmol) as the raw material, the operation method of step 8 of example 56 was referenced to obtain **compound 83-4** (10 mg, 21.6% yield).

Compound **83-1:**

LC-MS (ESI): m/z = 501.2 $[M+H]^+$.
$^1H$ NMR (400 MHz, Chloroform-d) δ 7.47 - 7.39 (m, 2H), 7.26 (d, 1H), 6.93 - 6.88 (m, 1H), 6.88 - 6.80 (m, 2H), 4.44 - 4.32 (m, 1H), 4.28 - 4.22 (m, 1H), 4.02 - 3.92 (m, 5H), 3.89 - 3.82 (m, 1H), 3.80 (s, 2H), 3.47 - 3.35 (m, 1H), 2.15 - 2.03 (m, 3H), 1.84 - 1.74 (m, 1H), 1.67 - 1.54 (m, 1H), 1.47 - 1.42 (m, 5H), 1.36 - 1.20 (m, 2H), 1.18 - 1.02 (m, 2H).

Compound **83-2**:

LC-MS (ESI): m/z = 501.2 $[M+H]^+$.
$^1H$ NMR (400 MHz, Chloroform-d) δ 7.47 - 7.39 (m, 2H), 7.26 (d, 1H), 6.93 - 6.88 (m, 1H), 6.88 - 6.80 (m, 2H), 4.44 - 4.32 (m, 1H), 4.28 - 4.22 (m, 1H), 4.02 - 3.92 (m, 5H), 3.89 - 3.82 (m, 1H), 3.80 (s, 2H), 3.47 - 3.35 (m, 1H), 2.15 - 2.03 (m, 3H), 1.84 - 1.74 (m, 1H), 1.67 - 1.54 (m, 1H), 1.47 - 1.42 (m, 5H), 1.36 - 1.20 (m, 2H), 1.18 - 1.02 (m, 2H).

Compound **83-3:**

LC-MS (ESI): m/z = 501.2 $[M+H]^+$.
$^1H$ NMR (400 MHz, Chloroform-d) δ 7.47 - 7.39 (m, 2H), 7.26 (d, 1H), 6.93 - 6.88 (m, 1H), 6.88 - 6.80 (m, 2H), 4.44 - 4.32 (m, 1H), 4.28 - 4.22 (m, 1H), 4.02 - 3.92 (m, 5H), 3.89 - 3.82 (m, 1H), 3.80 (s, 2H), 3.47 - 3.35 (m, 1H), 2.15 - 2.03 (m, 3H), 1.84 - 1.74 (m, 1H), 1.67 - 1.54 (m, 1H), 1.47 - 1.42 (m, 5H), 1.36 - 1.20 (m, 2H), 1.18 - 1.02 (m, 2H).

Compound **83-4:**

LC-MS (ESI): m/z = 501.2 $[M+H]^+$.

$^1H$ NMR (400 MHz, Chloroform-d) δ 7.47 - 7.39 (m, 2H), 7.26 (d, 1H), 6.93 - 6.88 (m, 1H), 6.88 - 6.80 (m, 2H), 4.44 - 4.32 (m, 1H), 4.28 - 4.22 (m, 1H), 4.02 - 3.92 (m, 5H), 3.89 - 3.82 (m, 1H), 3.80 (s, 2H), 3.47 - 3.35 (m, 1H), 2.15 - 2.03 (m, 3H), 1.84 - 1.74 (m, 1H), 1.67 - 1.54 (m, 1H), 1.47 - 1.42 (m, 5H), 1.36 - 1.20 (m, 2H), 1.18 - 1.02 (m, 2H).

**Example 84:**

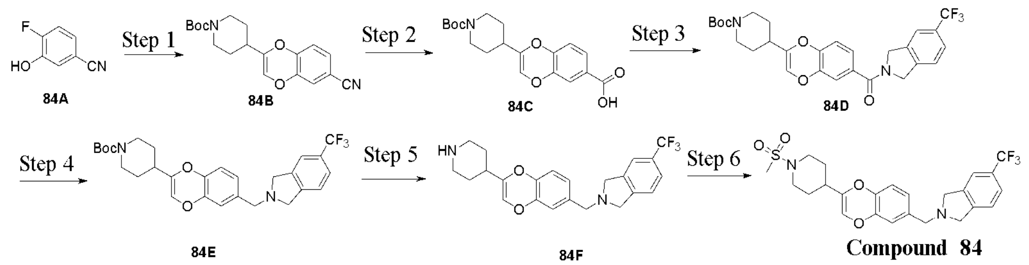

Step 1: Compound **84A** (1.4 g, 10.22 mmol), tert-butyl 4-(2-bromoacetyl)piperidine-1-carboxylate (3.12 g, 10.22 mmol) and potassium carbonate (4.23 g, 30.66 mmol) were added to acetonitrile (50 Ml), and the resulting mixture was stirred at 80°C overnight, and filtered. The filtrate was concentrated, separated and purified by silica gel chromatography column (eluent: ethyl acetate/petroleum ether (v/v) = 20/100) to obtain compound **84B** (3.0 g, 85.8%).

LC-MS (ESI): m/z= 343.1 $[M+H]^+$.

Step 2: Using compound **84B** (3.0 g, 8.77 mmol) as the raw material, the operation method of step 4 of example 56 was referenced to obtain compound **84C** (3.0 g, 94.7%).

LC-MS (ESI): m/z= 362.3 $[M+H]^+$.

Step 3: Using compound **84C** (3.0 g, 8.31 mmol) as the raw material, the operation method of step 5 of example 56 was referenced to obtain compound **84D** (2.5 g, 56.8%).

LC-MS (ESI): m/z= 531.2 $[M+H]^+$.

Step 4: Using compound **84D** (2.4 g, 4.53 mmol) as the raw material, the operation method of step 6 of example 56 was referenced to obtain compound **84E** (2.0 g, 85.6%).

LC-MS (ESI): m/z=517.2 $[M+H]^+$.

Step 5: Compound **84E** (0.5 g, 0.96 mol) was dissolved in dichloromethane (3 mL), trifluoroacetic acid (1 mL) was added, and the resulting mixture was stirred at room temperature for 1 h to obtain the reaction solution containing **84F,** which was directly used in the next step.

LC-MS (ESI): m/z= 417.2 $[M+H]^+$.

Step 6: Triethylamine was added to the above reaction solution containing **84F** to adjust pH = 7-8. Methanesulfonyl chloride (109 mg, 0.96 mmol) was added, and the resulting mixture was stirred at room temperature for 30 min, concentrated, separated and purified by silica gel chromatography column (eluent: methanol/dichloromethane (v/v) = 10/100) to obtain **compound 84** (50 mg, 10.5%).

LC-MS (ESI): m/z = 495.1$[M+H]^+$.

$^1H$ NMR (400 MHz, DMSO-$d_6$) δ7.66 - 7.51 (m, 2H), 7.48 - 7.37 (m, 1H), 6.93 - 6.83 (m, 1H), 6.77 - 6.67 (m, 2H), 6.11 (s, 1H), 3.87 (s, 4H), 3.72 (s, 2H), 3.65 - 3.53 (m, 2H), 2.86 (s, 3H), 2.77 - 2.66 (m, 2H), 2.20 - 2.04 (m, 1H), 1.92 - 1.76 (m, 2H), 1.63 - 1.40 (m, 2H).

**Example 85:**

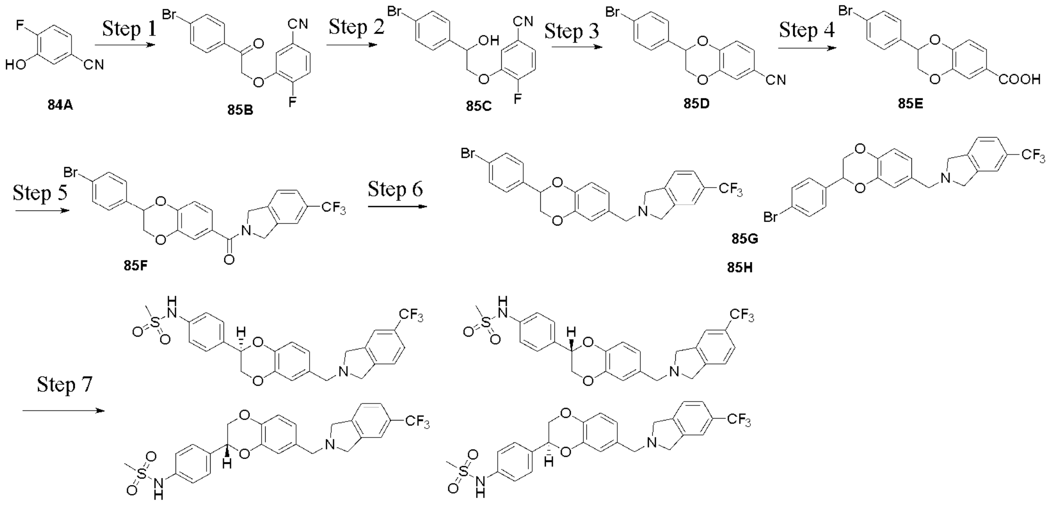


Compound 85-1 Compound 85-2 Compound 85-3 Compound 85-4

Step 1: Using compound **84A** (25.0 g, 182.48 mmol) and 2-bromo-1-(4-bromophenyl)ethan-1-one (50.1 g, 182.48 mmol) as the raw material, the operation method of step 1 of example 56 was referenced to obtain compound **85B** (29.0 g, 47.8%).

LC-MS (ESI): m/z= 334.1 $[M+H]^+$.

Step 2: Using compound **85B** (20.0 g, 60.06 mmol) as the raw material, the operation method of step 2 of example 56 was referenced to obtain compound **85C** (20.0 g, 99.5%).

LC-MS (ESI): m/z= 336.1 $[M+H]^+$.

Step 3: Using compound **85C** (20.0 g, 59.70 mmol) as the raw material, the operation method of step 3 of example 56 was referenced to obtain compound **85D** (6.0 g, 31.9%).

LC-MS (ESI): m/z= 316.1 $[M+H]^+$.

Step 4: Using compound **85D** (6.0 g, 19.05 mmol) as the raw material, the operation method of step 4 of example 56 was referenced to obtain compound **85E** (6.0 g, 94.3%).

LC-MS (ESI): m/z=335.1 $[M+H]^+$.

Step 5: Using compound **85E** (6.0 g, 17.96 mmol) as the raw material, the operation method of step 5 of example 56 was referenced to obtain compound **85F** (7.0 g, 77.5%).

LC-MS (ESI): m/z=504.3$[M+H]^+$.

Step 6: Compound **85F** (7.0 g, 13.9 mmol) was dissolved in tetrahydrofuran (70 mL), anhydrous lithium chloride (0.6 g, 13.9 mmol) was added, a solution of diethylzinc in toluene (1 M, 14 mL) was added under nitrogen protection at 0°C, polymethylhydrosiloxane (7 mL) was then added, and the resulting mixture was reacted at room temperature overnight, concentrated, separated and purified by silica gel chromatographycolumn (eluent: ethyl acetate/petroleum ether (v/v) = 20/100) to obtain a mixture of **compounds 85G** and **85H** (6.0 g, 88.3%).

LC-MS (ESI): m/z=490.3$[M+H]^+$.

Step 7: The mixture of compounds **85G** and **85H** (1.0 g, 2.04 mmol) was dissolved in N,N-dimethylformamide (20 mL), methanesulfonamide (965 mg, 10.2 mmol) and cuprous iodide (388 mg, 2.04 mmol) were added, and the resulting mixture was heated to 130°C and reacted overnight under nitrogen protection. After concentration, the reaction product was separated and purified by silica gel chromatography column (eluent: methanol/dichloromethane (v/v) = 10/100) to obtain a mixture (100 mg, 9.7%), which was separated by SFC to obtain four isomers, **compound 85-1** (5.0 mg, retention time 0.65 min, 1.9%), **compound 85-2** (6.0 mg, retention time 0.83 min, 2.3%), **compound 85-3** (5.0 mg, retention time 1.04 min, 1.9%) and **compound 85-4** (6.0 mg, retention time 1.38 min, 2.3%)

Chiral preparation method: Instrument: Waters 150 MGM; chromatographic column: Chiralpak Column; mobile phase: A: carbon dioxide, and B: ethanol; isocratic elution: 70% mobile phase B; Flow rate: 100 mL/min; back pressure: 100 bar; Column temperature: 25°C; wavelength: 220 nm; elution time: 4.0 min.

**Compound 85-1:**

LC-MS (ESI): m/z = 505.1 $[M+H]^+$.

$^1H$ NMR (400 MHz, DMSO-$d_6$) δ7.61 (s, 1H), 7.57 - 7.52 (m, 1H), 7.49 - 7.39 (m, 3H), 7.28 - 7.22 (m, 2H), 6.95 - 6.84 (m, 3H), 5.22 - 5.09 (m, 1H), 4.53 - 4.32 (m, 1H), 4.19 - 4.03 (m, 1H), 3.89 (s, 4H), 3.78 (s, 2H), 3.00 (s, 3H).

**Compound 85-2:**

LC-MS (ESI): m/z = 505.1 $[M+H]^+$.

$^1H$ NMR (400 MHz, DMSO-$d_6$) δ7.60 (s, 1H), 7.58 - 7.51 (m, 1H), 7.49 - 7.39 (m, 3H), 7.26 - 7.19 (m, 2H), 6.97 - 6.93 (m, 1H), 6.91 - 6.84 (m, 2H), 5.23 - 5.11 (m, 1H), 4.48 - 4.28 (m, 1H), 4.13 - 4.00 (m, 1H), 3.89 (s, 4H), 3.78 (s, 2H), 2.97 (s, 3H)..

**Compound 85-3:**

LC-MS (ESI): m/z = 505.1 $[M+H]^+$.

$^1H$ NMR (400 MHz, DMSO-$d_6$) δ 7.70 - 7.50 (m, 2H), 7.51 - 7.39 (m, 3H), 7.33 - 7.16 (m, 2H), 7.02 - 6.83 (m, 3H), 5.26 - 5.16 (m, 1H), 4.52 - 4.30 (m, 1H), 4.20 - 3.96 (m, 1H), 3.89 (s, 4H), 3.78 (s, 2H), 3.00 (s, 3H).

**Compound 85-4:**

LC-MS (ESI): m/z = 505.1 $[M+H]^+$.

$^1H$ NMR (400 MHz, DMSO-$d_6$) δ7.68 - 7.49 (m, 2H), 7.53 - 7.40 (m, 3H), 7.34 - 7.17 (m, 2H), 7.05 - 6.69 (m, 3H), 5.27 - 5.06 (m, 1H), 4.52 - 4.32 (m, 1H), 4.21 - 3.94 (m, 1H), 3.89 (s, 4H), 3.78 (s, 2H), 3.00 (s, 3H).

**Biological test:**

1. The purpose of the experimental study was intended to detect the testosterone level in the NCI-H295R cells by adopting the Enzyme linked immunosorbent assay (ELISA), so as to evaluate the inhibitory effects of the compounds on the testosterone level in the NCI-H295R cells.

The H295R cells were purchased from ATCC, the cell complete medium contained DMEM: F12+ 10% FBS +0.00625 mg/ml insulin +0.00625 mg/ml transferrin+6.25 ng/ml selenium +1.25 mg/ml BSA+0.00535 mg/ml linoleic acid, and the cells were cultured in a 37°C, 5% $CO_2$ incubator. The cells in the exponential growth phase were collected, and cultured in a cytokine-free medium (phenol red 1640-free +1% PS+10% css FBS+1.25 mg/ml BSA +0.00625 mg/ml insulin + 0.00625 mg/ml transferrin+6.25 ng/ml selenium+0.00535 mg/ml linoleic acid) for 3 days. The cells in the exponential growth phase were collected on the third day. The cell suspension was adjusted with a cytokine-free medium to a corresponding concentration and plated at 60000 cells/well, with the volume being 90 μL/well. Subsequently, 10 μL of the compounds at different concentrations was added and the plate was incubated in a $CO_2$ incubator for 3 days. After the incubation was completed, according to the operation instructions for testosterone Elisa kit (Beyotime, PT872), 50 μL of the cell culture supernatant was collected from each well and centrifuged at 500 g for 5 min at room temperature and then the resulting supernatant was collected. The samples were added to the corresponding wells at 25 μl/well, and then formulated horseradish peroxidase labeled testosterone was added at 75 μl/well. The mixture was fully mixed for 10 seconds. The reaction wells were sealed with a plate-sealing film (white) and the mixture was incubated at room temperature in the dark for 120 min. The plate was washed 3 times with a volume of 300 μl per well, and then placed onto thick absorbent paper and dried. Chromogenic reagent TMB solution was added at 100 μl/well. The reaction wells were sealed with a plate-sealing film (white) and the mixture was incubated at room temperature in the dark for 15-20 min. Subsequently, a termination solution was added at 50 μl/well, and after the mixture was fully mixed, the absorbance value $OD_{450}$ was immediately measured. The absorbance readings were processed using Graphpad Prim 8.0 software and S-shaped concentration curves were plotted and the $IC_{50}$ values were calculated using a four-parameter non-linear regression model. The results were processed according to formula (1), the inhibition rate of the compound at different concentrations was calculated, and the $IC_{50}$ value of the compound with an inhibition rate of 50% was calculated using Graphpad Prim 8.0 software, wherein $RLU_{compound}$ was the readout of the treated group, and $RLU_{control}$ was the average value of the solvent control group.

$$\text{Inhibition rate\%} = 100 - RLU_{compound} / RLU_{control} \times 100 \qquad \text{Formula (1)}$$

Table 1. Inhibitory activity on testosterone level in NCI-H295R cells

| Compound | Testosterone level in H295R (Elisa) | | $IC_{50}$/nM |
|---|---|---|---|
| | inh.% 1000 nM; | inh.% 100 nM | |
| Compound 4 | 89.2% | 81.5% | NA |
| Compound 10 | 85.4% | NA | 81.9 |
| Compound 16 | 89.4% | 51.7% | NA |
| Compound 30 | 80.9% | 59.1% | 35.3 |
| Compound 36 | 62% | 56.8% | NA |
| Compound 37 | 77% | 87.1% | NA |
| Compound 38 | 81% | 68.2% | NA |
| Compound 39 | 74.6% | 55.8% | NA |
| Compound 40 | NA | NA | 77 |
| Compound 41 | 67.9% | 51.5% | NA |
| Compound 42 | 82.6% | 76.9% | NA |
| Compound 43 | 77.8% | 75.6% | NA |
| Compound 48 | 78.2% | 77.5% | NA |
| Compound 49 | 65.2% | 57.7% | NA |
| Compound 50 | 89.4% | 51.7% | 129.9 |
| Compound 51 | 78.5% | 74.4% | NA |
| Compound 53 | 85.5% | 72.5% | NA |
| Compound 54 | 78.4% | 59.1% | NA |
| Compound 56-3 | 86% | 75% | 32 |
| Compound 57-1 | 86% | 75% | 21 |
| Compound 58 | NA | NA | 57 |
| Compound 59 | 76% | 60% | NA |
| Compound 62 | NA | NA | 151 |
| Compound 63 | 76.4% | 64.4% | 92 |
| Compound 65 | 75% | 71% | 168 |
| Compound 66 | 76.7% | 54.8% | NA |
| Compound 67 | 72% | 52% | NA |
| Compound 69 | 80% | 79% | NA |
| Compound 70 | 76.3% | 68.9% | NA |
| Compound 71 | 86.9% | 74.9% | NA |
| Compound 72 | 88% | 81% | NA |
| Compound 75 | 90.4% | 76.2% | NA |
| Compound 76 | 78% | 70% | NA |
| Compound 80 | 65.4% | 72.9% | NA |
| Compound 81 | 78.5% | 78.9% | NA |
| Compound 82 | 63.1% | 63.2% | NA |
| Compound 83-1 | 88 % | 73 % | 56 |
| Compound 84 | 83% | 74% | 38 |

(continued)

| Compound | Testosterone level in H295R (Elisa) | | $IC_{50}$/nM |
|---|---|---|---|
| | inh.% 1000 nM; | inh.% 100 nM | |
| Compound 85-1 | 73% | 64% | 82 |
| Conclusion: The compounds of the present invention, such as example compounds, have significant inhibitory effects on the testosterone level in NCI-H295R cells. | | | |

2. The purpose of the experimental study was intended to detect the pregnenolone level in the NCI-H295R cells by adopting the Enzyme linked immunosorbent assay (ELISA), so as to evaluate the inhibitory effects of the compounds on the pregnenolone level in the NCI-H295R cells.

The H295R cells were purchased from ATCC (CRL-2128), the cell complete medium contained DMEM: F12+ 10% FBS +0.00625 mg/ml insulin+0.00625 mg/mL transferrin+6.25 ng/mL selenium+1.25 mg/mL BSA+0.00535 mg/mL linoleic acid, and the cells were cultured in a 37°C, 5% $CO_2$ incubator. The cells in the exponential growth phase were collected, and cultured in a cytokine-free medium (phenol red 1640-free +1% PS+10% css FBS+1.25 mg/mL BSA +0.00625 mg/mL insulin+0.00625 mg/mL transferrin+6.25 ng/mL selenium+0.00535 mg/mL linoleic acid) for 3 days. The cells in the exponential growth phase were collected on the third day. The cell suspension was adjusted with a cytokine-free medium to a corresponding concentration and plated at 60000 cells/well, with the volume being 90 μL/well. Subsequently, 10 μL of the compounds at different concentrations was added and the plate was incubated in a $CO_2$ incubator for 3 days. After the incubation was completed, the cell culture supernatant was collected and centrifuged at 2°C-8°C at 800×g for 10 min. The resulting supernatant was collected. Determination was carried out according to the operation instructions for pregnenolone Elisa kit (Antibodies-A73791), and the $OD_{450}$ signal was read.

Data processing:

2.1 Standard curve plotting. The standard concentration was taken as the abscissa and the $OD_{450}$ value as the ordinate, the coordinate points of various standards were connected with a smooth line and a curve was fitted according to the four parameter logistic (4-PL) method.

2.2 Sample quantification: The corresponding concentration (CONC) of testosterone/pregnenolone in the sample was calculated using the absorbance value of the sample and the standard curve. According to formula (2), the inhibition rate of the compound at various concentrations was calculated, and S-shaped concentration curves were plotted and the $IC_{50}$ values (i.e., concentration of the compound at which the inhibition rate was 50%) were calculated using a three parameter non-linear regression model in Graphpad Prim 8.0 software. CONC $_{cpd}$ was the concentration of the treated group, and CONC $_{ctrl}$ was the average concentration value of the solvent control group.

$$\text{Inhibition rate\%} = 100\% - \text{CONC}_{cpd} / \text{CONC}_{ctrl} \times 100\% \qquad \text{Formula (2)}$$

Table 2

| Compound | Pregnenolone level in H295R (Elisa) | | |
|---|---|---|---|
| | inh.% 1000 nM | inh.% 100 nM | $IC_{50}$ nM |
| Compound 56-3 | NA | NA | 64 |
| Compound 57-1 | NA | NA | 106 |
| Compound 58 | NA | NA | 160 |
| Compound 62 | NA | NA | 142 |
| Compound 63 | NA | NA | 272 |
| Compound 65 | NA | NA | 319 |
| Compound 71 | 57.5% | 43.9% | NA |
| NA: not detected.<br>Conclusion: The compounds of the present invention, such as example compounds, have significant inhibitory effects on the pregnenolone level in NCI-H295R cells. | | | |

3. Pharmacokinetic test in rats

3.1 Experimental animals: Male SD rats (about 220 g, 6-8 weeks old, 6 rats/compound), purchased from Chengdu Dossy Experimental Animals Co., Ltd.

3.2 Experimental design: On the day of the experiment, the SD rats were randomly grouped by body weight. The animals were fasted with water available for 12-14 h one day before the administration, and were fed 4 h after the administration.

Table 3. Administration information

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G1 | 3 | Compound 40 | 2.5 | 0.5 | 5 | Plasma | Intravenous administration |
| G2 | 3 | Compound 40 | 10 | 1 | 10 | Plasma | Intragastric administration |
| G3 | 3 | Compound 51 | 2.5 | 0.5 | 5 | Plasma | Intravenous administration |
| G4 | 3 | Compound 51 | 10 | 1 | 10 | Plasma | Intragastric administration |
| G5 | 3 | Compound 53 | 2.5 | 0.5 | 5 | Plasma | Intravenous administration |
| G6 | 3 | Compound 53 | 10 | 1 | 10 | Plasma | Intragastric administration |
| G7 | 3 | Compound 54 | 2.5 | 0.5 | 5 | Plasma | Intravenous administration |
| G8 | 3 | Compound 54 | 10 | 1 | 10 | Plasma | Intragastric administration |
| G9 | 3 | Compound 56-3 | 2.5 | 0.5 | 5 | Plasma | Intravenous administration |
| G10 | 3 | Compound 56-3 | 10 | 1 | 10 | Plasma | Intragastric administration |
| G11 | 3 | Compound 57-1 | 2.5 | 0.5 | 5 | Plasma | Intravenous administration |
| G12 | 3 | Compound 57-1 | 10 | 1 | 10 | Plasma | Intragastric administration |
| G13 | 3 | Compound 60 | 2.5 | 0.5 | 5 | Plasma | Intravenous administration |
| G14 | 3 | Compound 60 | 10 | 1 | 10 | Plasma | Intragastric administration |
| G15 | 3 | Compound 62 | 2.5 | 0.5 | 5 | Plasma | Intravenous administration |
| G16 | 3 | Compound 62 | 10 | 1 | 10 | Plasma | Intragastric administration |
| G17 | 3 | Compound 65 | 2.5 | 0.5 | 5 | Plasma | Intravenous administration |

(continued)

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G18 | 3 | Compound 65 | 10 | 1 | 10 | Plasma | Intragastric administration |
| Vehicle for administration: 0.5% MC | | | | | | | |

Before and after the administration, 0.1 ml of blood was taken from the orbit of the animals under isoflurane anesthesia and placed in an EDTAK2 centrifuge tube. The blood was centrifuged at 5000 rpm and 4°C for 10 min to collect plasma. Blood sampling time points for the intravenous administration group: 0, 5 min, 15 min, 30 min, 1 h, 2 h, 4 h, 6 h, 8 h and 24 h; blood sampling time points for the intragastric administration group: 0, 5 min, 15 min, 30 min, 1 h, 2 h, 4 h, 6 h, 8 h and 24 h. Before analysis and detection, all samples were stored at -80°C.

Table 4. Pharmacokinetic parameters of test compounds in plasma of rats

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 40 | Intravenous administration (2.5 mg/kg) | 19.1 | 9.21 | 1951 | 9.77 |
| Compound 40 | Intragastric administration (10 mg/kg) | - | - | 4210 | 11.1 |
| Compound 51 | Intravenous administration (2.5 mg/kg) | 9.32 | 4.58 | 4002 | 8.52 |
| Compound 51 | Intragastric administration (10 mg/kg) | - | - | 13602 | 9.63 |
| Compound 53 | Intravenous administration (2.5 mg/kg) | 11 | 8.06 | 3106 | 13.2 |
| Compound 53 | Intragastric administration (10 mg/kg) | - | - | 6746 | 8.13 |
| Compound 54 | Intravenous administration (2.5 mg/kg) | 10.3 | 6.41 | 3560 | 10.3 |
| Compound 54 | Intragastric administration (10 mg/kg) | - | - | 5839 | 7.74 |
| Compound 56-3 | Intravenous administration (2.5 mg/kg) | 6.72 | 4.89 | 5166 | 9.73 |
| Compound 56-3 | Intragastric administration (10 mg/kg) | - | - | 12263 | 11.3 |
| Compound 57-1 | Intravenous administration (2.5 mg/kg) | 8.08 | 6.61 | 5186 | 8.09 |
| Compound 57-1 | Intragastric administration (10 mg/kg) | - | - | 24515 | 15 |
| Compound 60 | Intravenous administration (2.5 mg/kg) | 6.8 | 0.951 | 6076 | 1.75 |
| Compound 60 | Intragastric administration (10 mg/kg) | - | - | 27131 | 2.36 |
| Compound 62 | Intravenous administration (2.5 mg/kg) | 3.97 | 1.11 | 10796 | 3.81 |
| Compound 62 | Intragastric administration (10 mg/kg) | - | - | 41460 | 4.13 |
| Compound 65 | Intravenous administration (2.5 mg/kg) | 17.4 | 8.8 | 2153 | 8.25 |
| Compound 65 | Intragastric administration (10 mg/kg) | - | - | 7546 | 6.58 |
| Conclusion: The compounds of the present invention, such as the compounds in the examples, have good bioavailability and pharmacokinetic characteristics in rats. | | | | | |

4. Pharmacokinetic test in mice

4.1. Experimental animals: Male ICR mice, 20-25 g, 6 mice/compound, purchased from Chengdu Dossy Experimental Animals Co., Ltd.

4.2 Experimental design: on the day of the experiment, the ICR mice were randomly grouped according to their body weights. The animals were fasted with water available for 12-14 h one day before the administration, and were fed 4 h after the administration.

Table 5. Administration information

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G1 | 3 | Compound 38 | 2.5 | 0.5 | 5 | Plasma | Intravenous administration |
| G2 | 3 | Compound 38 | 10 | 1 | 10 | Plasma | Intragastric administration |
| G3 | 3 | Compound 50 | 2.5 | 0.5 | 5 | Plasma | Intravenous administration |
| G4 | 3 | Compound 50 | 10 | 1 | 10 | Plasma | Intragastric administration |
| G5 | 3 | Compound 56-3 | 2.5 | 0.5 | 5 | Plasma | Intravenous administration |
| G6 | 3 | Compound 56-3 | 10 | 1 | 10 | Plasma | Intragastric administration |
| G7 | 3 | Compound 58 | 2.5 | 0.5 | 5 | Plasma | Intravenous administration |
| G8 | 3 | Compound 58 | 10 | 1 | 10 | Plasma | Intragastric administration |
| G9 | 3 | Compound 63 | 2.5 | 0.5 | 5 | Plasma | Intravenous administration |
| G10 | 3 | Compound 63 | 10 | 1 | 10 | Plasma | Intragastric administration |

vehicle for intravenous administration: 5% DMA+5% HS-15+90% NS; vehicle for intragastric administration: 0.5% MC

Before and after administration, 0.06 mL of blood was taken from the orbit under isoflurane anesthesia and placed in an EDTAK2 centrifuge tube. The tube was centrifuged at 5000 rpm at 4°C for 10 min, and plasma was collected. The blood collection time points for the intravenous administration group and intragastric administration group were 0, 5 min, 15 min, 30 min, 1 h, 2 h, 4 h, 7 h and 24 h. Before analysis and detection, all samples were stored at -80°C, and the samples were quantitatively analyzed by LC-MS/MS.

Table 6. Pharmacokinetic parameters of test compounds in plasma of mice

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 38 | Intravenous administration (2.5 mg/kg) | 9.45 | 2.78 | 4364 | 4.11 |
| Compound 38 | Intragastric administration (10 mg/kg) | - | - | 13540 | 3.65 |
| Compound 50 | Intravenous administration (2.5 mg/kg) | 17.3 | 3.37 | 2364 | 3.00 |
| Compound 50 | Intragastric administration (10 mg/kg) | - | - | 7782 | 2.73 |
| Compound 56-3 | Intravenous administration (2.5 mg/kg) | 3.29 | 1.78 | 11422 | 6.94 |
| Compound 56-3 | Intragastric administration (10 mg/kg) | - | - | 39129 | 9.42 |
| Compound 58 | Intravenous administration (2.5 mg/kg) | 14.2 | 1.34 | 2962 | 2.77 |
| Compound 58 | Intragastric administration (10 mg/kg) | - | - | 9459 | 2.08 |
| Compound 63 | Intravenous administration (2.5 mg/kg) | 7.37 | 2.36 | 5534 | 4.7 |

(continued)

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 63 | Intragastric administration (10 mg/kg) | - | - | 20811 | 6.62 |
| Conclusion: The compounds of the present invention, such as the compounds in the examples, have good pharmacokinetic characteristics in mice. | | | | | |

5: Pharmacokinetic test in beagle dogs

Experimental animals: male beagle dogs, about 8-11 kg, 6 beagle dogs/compound, purchased from Beijing Marshall Biotechnology Co., Ltd.

Experimental method: on the day of the experiment, the beagle dogs were randomly grouped according to their body weights. The animals were fasted with water available for 12-14 h one day before the administration, and were fed 4 h after the administration. Administration was performed as per Table 7.

Test compounds: example compounds and control compounds.

Table 7. Administration information

| Group | Number | Administration information | | | | | |
|---|---|---|---|---|---|---|---|
| | Male | Test substance | Administration dosage (mg/kg) | Administration concentration (mg/mL) | Administration volume (mL/kg) | Collected sample | Administration mode |
| G1 | 3 | Compound 58 | 1 | 1 | 1 | Plasma | Intravenous administration |
| G2 | 3 | Compound 58 | 3 | 0.6 | 5 | Plasma | Intragastric administration |
| Note: vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: methylcellulose solution) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for both the intravenous administration group and the intragastric administration group were: 0, 5 min, 15 min, 30 min, 1 h, 2 h, 4 h, 6 h, 8 h, 10 h, 12 h, 24 h, 48 h, and 72 h. Before analysis and detection, all samples were stored at -80°C. The samples were analyzed quantitatively by LC-MS/MS.

Table 8. Pharmacokinetic parameters of test compounds in plasma of beagle dogs

| Test compound | Administration mode | CL (mL/min/kg) | $Vd_{ss}$ (L/kg) | $AUC_{0-t}$ (hr*ng/mL) | $T_{1/2}$ (h) |
|---|---|---|---|---|---|
| Compound 58 | Intravenous administration (1 mg/kg) | 1.24 | 0.87 | 13950 | 10.2 |
| Compound 58 | Intragastric administration (3 mg/kg) | - | - | 24753 | 6.79 |
| Conclusion: The compounds of the present invention, such as example compounds, have good pharmacokinetic characteristics in beagle dogs. | | | | | |

## Claims

1. A compound represented by formula (I), or a stereoisomer, a deuterated substance, a solvate, a co-crystal or a pharmaceutically acceptable salt thereof, **characterized in that**

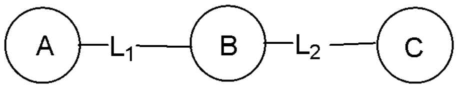

(I)

A is selected from 4- to 14-membered heterocyclyl, 5- or 6-membered cycloalkyl, or phenyl, wherein the heterocyclyl contains 1-3 heteroatoms selected from N, O or S; the heterocyclyl, cycloalkyl or phenyl is optionally substituted with 1-4 $R^A$;

B is selected from 6- to 12-membered bicyclic carbocyclyl, 6- to 12-membered bicyclic heterocyclyl, or 6- to 12-membered heteroaryl, wherein the heterocyclyl or heteroaryl contains 1-3 heteroatoms selected from N, O or S; the carbocyclyl, heterocyclyl or heteroaryl is optionally substituted with 1-4 $R^B$;

C is selected from phenyl, 6- to 12-membered bicyclic carbocyclyl, 6- to 12-membered partially unsaturated bicyclic heterocyclyl, or 8- to 14-membered tricyclic heterocyclyl, wherein the heterocyclyl contains 1-3 heteroatoms selected from N, O or S; the cycloalkyl, heterocyclyl or phenyl is optionally substituted with 1-4 $R^C$;

$L_1$ and $L_2$ are each independently selected from $W_1$-$R^L$-$W_2$, wherein $L_1$ is connected to A on the left side, and $L_2$ is connected to B on the left side; $L_1$ and $L_2$ are not both bonds;

$R^L$ is selected from a bond, $C_{1-4}$ alkylene, $C_{2-4}$ alkenylene, or 3- to 6-membered cycloalkyl, wherein the alkylene, alkenylene or cycloalkyl is optionally further substituted with 1-4 $R^{L1}$; each $R^{L1}$ is independently selected from halogen, =O, $C_{1-4}$ alkyl, $C_{2-4}$ alkenyl, $C_{1-4}$ alkoxy, or 3- to 6-membered cycloalkyl, wherein the alkyl, alkoxy or cycloalkyl is optionally further substituted with 1-4 substituents selected from halogen, CN, OH and $NH_2$;

$W_1$ and $W_2$ are each independently selected from a bond, -O-, -S-, -$NR^{W1}$-, - $CONR^{W1}$-, -$NR^{W1}CO$-, -C(=O)O-, or -OC(=O)-;

$R^{W1}$ is selected from H, $C_{1-4}$ alkyl, or halogen;

alternatively, B, $L_2$ and C, together with atoms to which they are connected, form tetracyclic heterocyclyl, optionally substituted with 1-4 groups selected from halogen, CN, OH, $NO_2$, =O, COOH, $C_{1-4}$ alkyl, $C_{2-6}$ alkenyl, $C_{2-6}$ alkynyl, -$OC_{1-4}$ alkyl or -O-halo $C_{1-4}$ alkyl;

each $R^A$ is independently selected from H, halogen, CN, OH, COOH, $C_{1-4}$ alkyl, $C_{2-6}$ alkenyl, $C_{2-6}$ alkynyl, -$OC_{1-4}$ alkyl, -NH-$S(O)_2$-$C_{1-4}$ alkyl, -$S(O)_2$-$C_{1-4}$ alkyl, - $S(O)_2$-$(CH_2)_p$-$R^a$, -P(O)-($C_{1-4}$ alkyl$)_2$, -C(O)-$OR^a$, -$(CH_2)_p$-C(O)-$(CH_2)_p$-$R^a$, - NHC(O)-$R^a$, -C(O)N($C_{1-4}$ alkyl$)_2$, -NHC(O)-$C_{1-4}$ alkyl or 3- to 6-membered heterocycloalkyl, or -$NHR^a$, wherein the alkyl, alkenyl, alkynyl or heterocycloalkyl is optionally further substituted with 1-4 groups selected from halogen, OH, $NH_2$, CN, -O-halo $C_{1-4}$ alkyl, deuterium or $C_{1-4}$ alkyl; the heterocycloalkyl contains 1-3 heteroatoms selected from N, O or S;

$R^a$ is selected from $C_{2-4}$ alkynyl, 3- to 5-membered cycloalkyl, 3- to 6-membered heterocycloalkyl, 5- or 6-membered heteroaryl, $C_{1-4}$ alkoxy, -O-$C_{3-6}$ cycloalkyl, deuterated $C_{1-4}$ alkyl, or $C_{1-4}$ alkyl, wherein the alkynyl, cycloalkyl, heterocycloalkyl, heteroaryl, or alkoxy is optionally further substituted with 1-4 groups selected from halogen, $C_{1-4}$ alkyl, halo $C_{1-4}$ alkyl, deuterated $C_{1-4}$ alkyl, 6-membered heterocycloalkyl, 5- or 6-membered heteroaryl, $C_{2-4}$ alkynyl, 3- to 5-membered cycloalkyl, -O-halo $C_{1-4}$ alkyl, OH, -$S(O)_2$-$CH_3$, or =$CH_2$, =CHF or =$CF_2$, wherein the alkyl is further substituted with 1-3 groups selected from OH or CN, and the heterocycloalkyl or heteroaryl contains 1-3 heteroatoms selected from N, O or S;

each $R^B$ is independently selected from H, halogen, CN, =O, OH, $NO_2$, -$SF_5$, $C_{1-4}$ alkyl, -P(O)-$(CH_3)_2$, -$S(O)_2$-$CH_3$, -$S(O)_2$-$CH_2R^a$-, $NH_2$, -$C_{1-4}$ alkoxy, $C_{2-6}$ alkenyl, $C_{2-6}$ alkynyl, 3- to 5-membered cycloalkyl, halo $C_{1-2}$ alkyl, 5- or 6-membered heteroaryl containing 1-3 heteroatoms selected from N, O or S, or 3- to 6-membered heterocycloalkyl containing 1-3 heteroatoms selected from N, O or S, wherein the alkoxy, cycloalkyl, heteroaryl or heterocycloalkyl is optionally further substituted with a $C_{1-2}$ alkyl group;

each $R^C$ is independently selected from H, halogen, CN, =O, OH, $NO_2$, $C_{1-4}$ alkyl, halo $C_{1-4}$ alkyl, -O-$C_{3-5}$ cycloalkyl, $C_{3-5}$ cycloalkyl, $C_{2-4}$ alkenyl, $C_{2-4}$ alkynyl, -$SCF_3$, -$SF_5$, -$(NH)_p$-P(O)($C_{1-4}$ alkyl$)_2$, or -$(CH_2)_p$-O-$C_{1-4}$ alkyl;

p is selected from 0, 1, 2, 3 or 4.

2. The compound represented by formula (I), or the stereoisomer, the deuterated substance, the solvate, the co-crystal, or the pharmaceutically acceptable salt thereof according to claim 1, **characterized in that**

A is selected from 4- to 6-membered monocyclic heterocyclyl, 7- to 9-membered bicyclic heterocyclyl, 5- or 6-membered cycloalkyl, or phenyl, wherein the heterocyclyl, cycloalkyl or phenyl is optionally substituted with 1-4 $R^A$;

each $R^A$ is independently selected from H, halogen, CN, OH, COOH, $C_{1-4}$ alkyl, $C_{2-4}$ alkenyl, $C_{2-4}$ alkynyl, -$OC_{1-4}$ alkyl, -NH-$S(O)_2$-$C_{1-2}$ alkyl, -$S(O)_2$-$C_{1-2}$ alkyl, - $S(O)_2$-$(CH_2)_p$-$R^a$, -P(O)-($C_{1-2}$ alkyl$)_2$, -C(O)-$OR^a$,

$-(CH_2)_p-C(O)-(CH_2)_p-R^a$, $-NHC(O)-R^a$, $-C(O)N(C_{1-4}\ alkyl)_2$, $-NHC(O)-C_{1-4}$ alkyl, 3- to 6-membered heterocycloalkyl, or $-NHR^a$, wherein the alkyl, alkenyl, alkynyl or heterocycloalkyl is optionally further substituted with 1-4 groups selected from halogen, OH, $NH_2$, CN, -O-halo $C_{1-2}$ alkyl, deuterium or $C_{1-4}$ alkyl; the heterocycloalkyl contains 1-3 heteroatoms selected from N, O or S;

$R^a$ is selected from $C_{2-4}$ alkynyl, cyclopropyl, cyclobutyl, 4- to 6-membered heterocycloalkyl, pyrazolyl, $C_{1-4}$ alkoxy, $-O-C_{3-6}$ cycloalkyl, deuterated $C_{1-2}$ alkyl, or $C_{1-2}$ alkyl, wherein the cycloprop-yl, cyclobutyl, heterocycloalkyl, pyrazolyl, or alkoxy is optionally further substituted with a group selected from halogen, $C_{1-2}$ alkyl, halo $C_{1-2}$ alkyl, deuterated $C_{1-2}$ alkyl, OH, $-S(O)_2-CH_3$, $=CH_2$, =CHF or $=CF_2$, and the alkyl is further substituted with 1-3 groups selected from OH or CN;

p is selected from 0, 1 or 2;

B is selected from 6-membered heteroaryl, 5- or 6-membered heterocyclyl fused 5- or 6-membered heteroaryl, 5- or 6-membered carbocyclyl fused 5- or 6-membered heteroaryl, 5- or 6-membered heteroaryl fused 5- or 6-membered heteroaryl, benzo 5- or 6-membered heteroaryl, 5- or 6-membered aryl fused 5- or 6-membered aryl, 4- to 6-membered heterocyclyl spiro 5- or 6-membered heteroaryl, or benzo 5- or 6-membered heterocyclyl, wherein the heterocyclyl, heteroaryl, carbocyclyl or aryl is optionally substituted with 1-4 $R^B$;

each $R^B$ is independently selected from halogen, CN, $-P(=O)(C_{1-2}\ alkyl)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;

C is selected from phenyl, 5- to 7-membered heterocyclyl fused phenyl, 5- or 6-membered carbocyclyl fused phenyl, or 5- or 6-membered heterocyclyl fused 5- or 6-membered heteroaryl, wherein the heterocyclyl or heteroaryl contains 1-3 heteroatoms selected from N, O or S; the phenyl, heterocyclyl, carbocyclyl or heteroaryl is optionally substituted with 1-4 $R^C$;

each $R^C$ is independently selected from CN, OH, $C_{1-2}$ alkyl, halo $C_{1-2}$ alkyl, -O-cyclopropyl, cyclopropyl, $C_{2-4}$ alkenyl, $C_{2-4}$ alkynyl, $-SCF_3$, $-SF_5$, $-(NH)_{0-1}-P(O)(C_{1-2}\ alkyl)_2$, or $C_{1-2}$ alkyl substituted with $C_{1-2}$ alkoxy;

$L_1$ is selected from a bond, $C_{1-2}$ alkylene-O, $C_{1-2}$ alkylene, $N(C_{1-2}$ alkylene)-$C_{1-2}$ alkylene, -O-, or $C_{2-4}$ alkenylene, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl;

$L_2$ is selected from a bond, $C_{1-2}$ alkylene, or $-N(CH_3)-$, wherein the alkylene is optionally further substituted with 1-3 $R^{L1}$; each $R^{L1}$ is independently selected from halogen, =O, $C_{1-2}$ alkyl, halo $C_{1-2}$ alkyl, $C_{2-4}$ alkenyl, $C_{1-2}$ alkoxy, halo $C_{1-2}$ alkoxy, or 3- to 6-membered cycloalkyl.

**3.** The compound represented by formula (I), or the stereoisomer, the deuterated substance, the solvate, the co-crystal, or the pharmaceutically acceptable salt thereof according to claim 2, **characterized in** having a structure of formula (I-1), (I-2), (I-3) or (I-4):

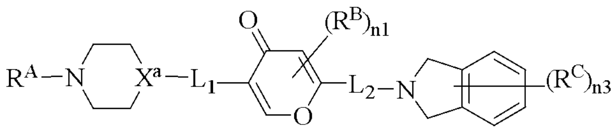

(I-1)

,

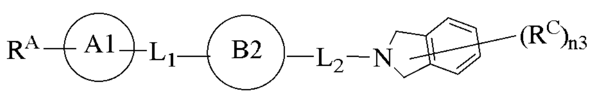

(I-2)

,

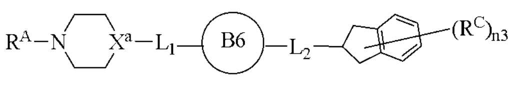

(I-3)

or

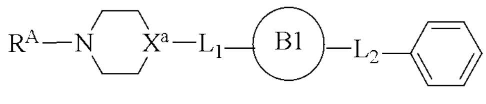

(I-4)

;

$X^a$ is selected from CH or N;

A1 is selected from the following groups:

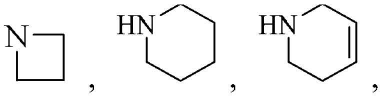

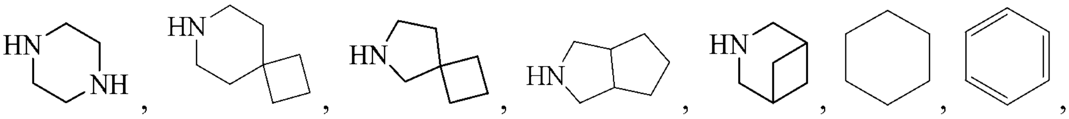

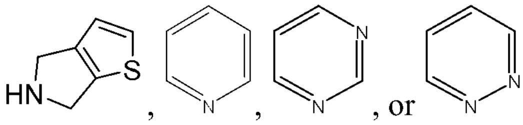;

B2 is selected from the following groups optionally substituted with 1-4 $R^B$:

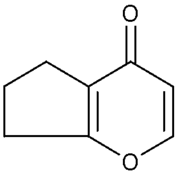, 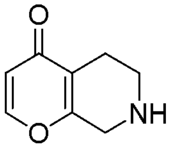, 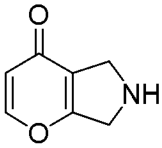, 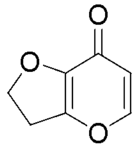, 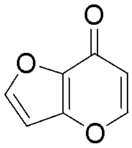, 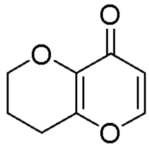,

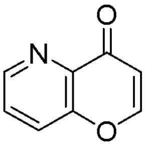, 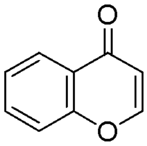, 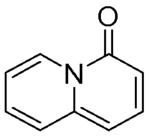, 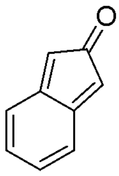, 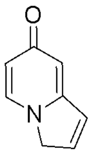, 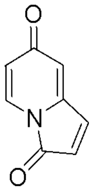, 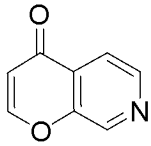,

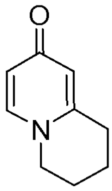, 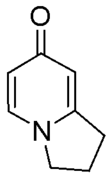, 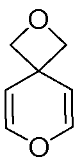, 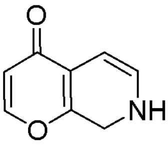, 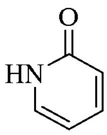, 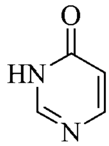, 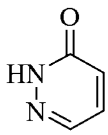, 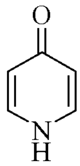,

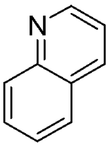, 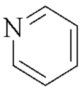, 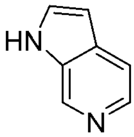, 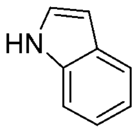, 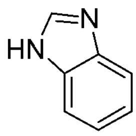, 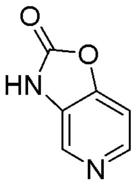, 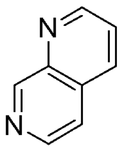,

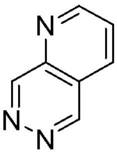, 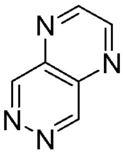, 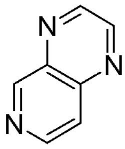, 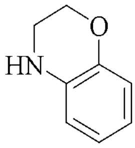, 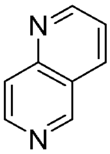, 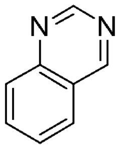, 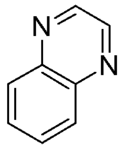,

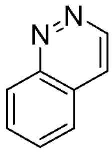, 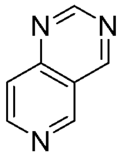, 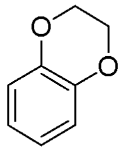, 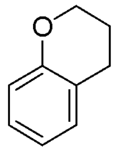, 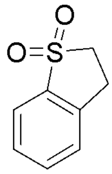, 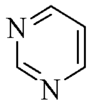, 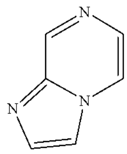, 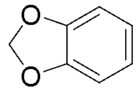,

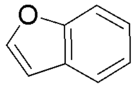, 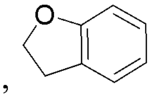, 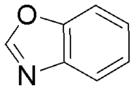, 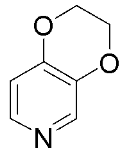, or 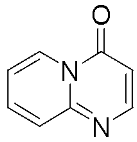;

B6 is selected from the following groups optionally substituted with 1-4 $R^B$:

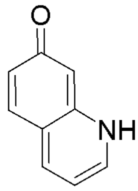 or 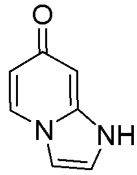;

B1 is selected from the following groups optionally substituted with 1-4 $R^B$:

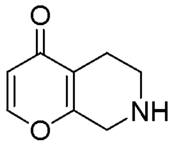, 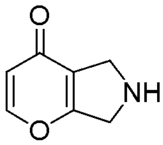, 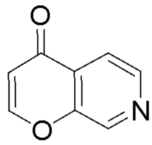, 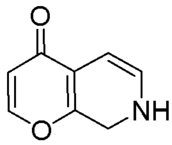, 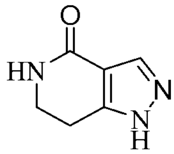,

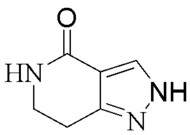, 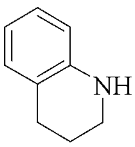, 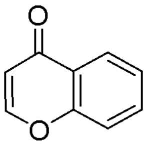, 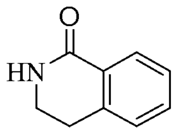, 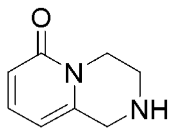,

or

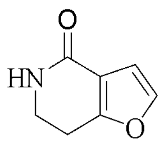;

alternatively.

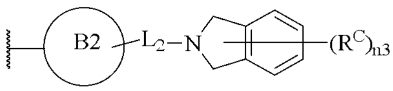

is

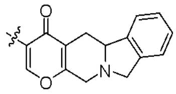;

$R^A$ is selected from $-S(O)_2-(CH_2)_p-R^a$, $-P(O)-(C_{1-2}$ alkyl$)_2$, $-C(O)-OR^a$, $-(CH_2)_p-C(O)-(CH_2)_p-R^a$, $-S(O)_2-C_{1-2}$ alkyl, $-NHC(O)-R^a$, $-C(O)N(C_{1-4}$ alkyl$)_2$, $-NHC(O)-C_{1-2}$ alkyl, 3- to 6-membered heterocycloalkyl, or $-NHR^a$, wherein the alkyl or heterocycloalkyl is optionally further substituted with 1-4 groups selected from halogen, OH, $NH_2$, CN, -O-halo $C_{1-2}$ alkyl, deuterium or $C_{1-2}$ alkyl; the heterocycloalkyl contains 1-3 heteroatoms selected from N, O or S;

each $R^B$ is independently selected from halogen, CN, $-P(=O)(C_{1-2}$ alkyl$)_2$, =O, $-SF_5$, $C_{1-2}$ alkyl, $-S(O)_2-C_{1-2}$ alkyl, $NH_2$, $C_{1-2}$ alkoxy, $C_{2-4}$ alkenyl, $C_{2-4}$ alkynyl, cyclopropyl, halo $C_{1-2}$ alkyl, or 5- or 6-membered heteroaryl, wherein the alkyl, alkoxy, cyclopropyl or heteroaryl is optionally further substituted with a $C_{1-2}$ alkyl group;

each $R^C$ is independently selected from CN, OH, $C_{1-2}$ alkyl, halo $C_{1-2}$ alkyl, $C_{2-4}$ alkenyl, $C_{2-4}$ alkynyl, -O-cyclopropyl, cyclopropyl, or $-SF_5$;

$R^a$ is selected from $C_{2-4}$ alkynyl, cyclopropyl, cyclobutyl, 4- to 6-membered heterocycloalkyl, pyrazolyl, $C_{1-4}$ alkoxy, -O-cyclopropyl, $-CD_3$, $-CH_2D$, $-CHD_2$, methyl, or ethyl, wherein the cyclopropyl, cyclobutyl, heterocycloalkyl, pyrazolyl or alkoxy is optionally further substituted with a group selected from halogen, $C_{1-2}$ alkyl, halo $C_{1-2}$ alkyl, deuterated $C_{1-2}$ alkyl, OH, $-S(O)_2-CH_3$, $=CH_2$, =CHF or $=CF_2$, and the methyl or ethyl is further substituted with 1-3 groups selected from OH or CN;

n1 is selected from 1, 2 or 3;

n3 is selected from 0, 1, 2 or 3.

4. The compound represented by formula (I), or the stereoisomer, the deuterated substance, the solvate, the co-crystal, or the pharmaceutically acceptable salt thereof according to claim 3, **characterized in that**

$R^A$ is selected from $-S(O)_2-(CH_2)_p-R^a$, $-S(O)_2-C_{1-2}$ alkyl, $-C(O)-OR^a$, $-C(O)-CH_2-R^a$, $-NHC(O)-R^a$, $-C(O)N(C_{1-4}$ alkyl$)_2$, $-NHC(O)-C_{1-2}$ alkyl, 3- to 6-membered heterocycloalkyl, or-$NHR^a$, wherein the alkyl or heterocycloalkyl is optionally further substituted with 1-4 groups selected from F, Cl, Br, OH, $NH_2$, CN, D, $-CH_3$ or $-CH_2CH_3$; the heterocycloalkyl contains 1-3 heteroatoms selected from N, O or S;

each $R^B$ is independently selected from F, Cl, Br, =O, CN, methyl, ethyl, methoxy, ethoxy, vinyl, propenyl, butenyl, ethynyl, propynyl, cyclopropyl, $-CF_3$, $-CHF_2$, $-CH_2F$, 5-membered heteroaryl, or 6-membered heteroaryl, wherein the methyl, ethyl, methoxy, ethoxy, cyclopropyl or heteroaryl is optionally further substituted with a group selected from methyl or ethyl;

each $R^C$ is independently selected from methyl, ethyl, $-CF_3$, $-CHF_2$, $-CH_2CH_2F$, $-CH_2CF_3$, $-CH_2CHF_2$, $-CH_2CH_2F$, vinyl, propenyl, ethynyl, propynyl, -O-cyclopropyl, cyclopropyl, or $-SF_5$;

$R^a$ is selected from ethynyl, propynyl, cyclopropyl, cyclobutyl, 4- to 6-membered heterocycloalkyl, pyrazolyl, methoxy, ethoxy, -O-cyclopropyl, $-CD_3$, methyl, or ethyl, wherein the cyclopropyl, cyclobutyl, heterocycloalkyl, pyrazolyl, methoxy or ethoxy is optionally further substituted with a group selected from F, Cl, Br, methyl, ethyl, $-CD_3$, $-CH_2D$, $-CHD_2$, OH, $-S(O)_2-CH_3$, $=CH_2$, =CHF or $=CF_2$, and the methyl or ethyl is further substituted with 1-3 groups selected from OH or CN;

n1 is selected from 1 or 2;

n3 is selected from 0, 1 or 2;

$L_1$ is selected from a bond, $-CH_2-O-$, $-CH_2CH_2-O-$, $-CH_2-$, -O-, $-CH_2CH_2-$, $-N(CH_3)-CH_2-$, ethylene, or propylene, wherein the $-CH_2-$ is optionally further substituted with 1-3 $R^{L1}$; each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$, $-OCHF_2$, $-OCH_2F$, cyclopropyl, or cyclobutyl;

$L_2$ is selected from a bond, $-CH_2-$, $-CH(CH_3)-$, or $-N(CH_3)-$, wherein the $-CH_2-$ or $-CH(CH_3)-$ is optionally further substituted with 1-3 $R^{L1}$; each $R^{L1}$ is independently selected from F, Cl, Br, =O, methyl, ethyl, $-CF_3$, $-CHF_2$, $-CH_2F$, vinyl, propenyl, methoxy, ethoxy, $-OCF_3$, $-OCHF_2$, $-OCH_2F$, cyclopropyl, or cyclobutyl.

5. The compound represented by formula (I), or the stereoisomer, the deuterated substance, the solvate, the co-crystal, or the pharmaceutically acceptable salt thereof according to claim 3 or 4, **characterized in that**

A1 is selected from the following groups:

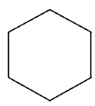;

B2 is selected from the following groups optionally substituted with 1-4 $R^B$:

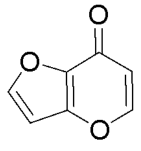, or 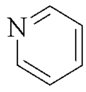;

$R^A$ is -NHC(O)-$R^a$;
each $R^B$ is independently selected from F, Cl, Br, =O, CN, methyl, ethyl, methoxy, or ethoxy, wherein the methyl, ethyl, methoxy or ethoxy is optionally further substituted with a group selected from methyl or ethyl;
each $R^C$ is independently selected from $-CF_3$, $-CHF_2$, $-CH_2CH_2F$, or cyclopropyl;
$R^a$ is selected from cyclopropyl, cyclobutyl, pyrazolyl, or $-CD_3$, wherein the cyclopropyl, cyclobutyl or pyrazolyl is optionally further substituted with a group selected from F, Cl, Br, methyl, ethyl, $-CD_3$, $-CH_2D$ or $-CHD_2$;
n3 is selected from 1 or 2;
$L_1$ is selected from a bond, $-CH_2$-O-, $-CH_2CH_2$-O-, ethylene, or propylene, wherein the $-CH_2$- is optionally further substituted with 1-3 $R^{L1}$; each $R^{L1}$ is independently selected from F, Cl, Br, methyl, ethyl, cyclopropyl, or cyclobutyl;
$L_2$ is selected from $-CH_2$-, wherein the $-CH_2$- is optionally further substituted with 1-3 $R^{L1}$; each $R^{L1}$ is independently selected from F, Cl, Br, methyl, ethyl, cyclopropyl, or cyclobutyl.

**6.** The compound represented by formula (I), or the stereoisomer, the deuterated substance, the solvate, the co-crystal, or the pharmaceutically acceptable salt thereof according to claim 1, **characterized in** having a structure of formula (I-5):

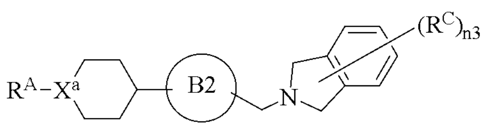

(I-5)

wherein:

$X^a$ is selected from CH or N;
each $R^A$ is independently selected from halogen, CN, OH, COOH, $C_{1-4}$ alkyl, - $OC_{1-4}$ alkyl, -NH-$S(O)_2$-$C_{1-4}$ alkyl, -$S(O)_2$-$C_{1-4}$ alkyl, -C(O)N($C_{1-4}$ alkyl)$_2$, -NHC(O)-$C_{1-4}$ alkyl, -C(O)-O$R^a$, $-(CH_2)_p$-C(O)-$(CH_2)_p$-$R^a$, -NHC(O)-$R^a$, -NH$R^a$, or 3- to 6-membered heterocycloalkyl, wherein the alkyl or heterocycloalkyl is optionally further substituted with 1-4 groups selected from halogen, OH, $NH_2$, CN, -O-halo $C_{1-4}$ alkyl, deuterium or $C_{1-4}$ alkyl, and the heterocycloalkyl contains 1-3 heteroatoms selected from N, O or S; preferably each $R^A$ is independently selected from halogen, CN, OH, COOH, $C_{1-2}$ alkyl, -$OC_{1-2}$ alkyl, -NH-$S(O)_2$-$C_{1-2}$ alkyl, - $S(O)_2$-$C_{1-2}$ alkyl, -C(O)N($C_{1-2}$ alkyl)$_2$, -NHC(O)-$C_{1-2}$ alkyl, -C(O)-O$R^a$, $-(CH_2)_p$-C(O)-$(CH_2)_p$-$R^a$, -NHC(O)-$R^a$, -NH$R^a$, or 3- or 4-membered heterocycloalkyl, wherein the alkyl or heterocycloalkyl is optionally further substituted with 1-4 groups selected from halogen, OH, $NH_2$, CN, -O-halo $C_{1-2}$ alkyl, deuterium or $C_{1-2}$ alkyl;
$R^a$ is selected from $C_{1-4}$ alkyl, deuterated $C_{1-4}$ alkyl, $C_{1-4}$ alkoxy, 3- to 5-membered cycloalkyl, 3- to 6-membered heterocycloalkyl, 5- or 6-membered heteroaryl, or -O-$C_{3-6}$ cycloalkyl, wherein the cycloalkyl, heterocycloalkyl or heteroaryl is optionally further substituted with 1-4 groups selected from OH, - $S(O)_2$-$CH_3$, halogen, $C_{1-4}$ alkyl, halo $C_{1-4}$ alkyl, deuterated $C_{1-4}$ alkyl or -O-halo $C_{1-4}$ alkyl, the alkyl is further substituted with 1-3 groups selected from OH or CN, and the heterocycloalkyl or heteroaryl contains 1-3 heteroatoms selected from N, O or S; preferably $R^a$ is selected from $C_{1-2}$ alkyl, deuterated $C_{1-2}$ alkyl, $C_{1-2}$ alkoxy, 3- or 4-membered cycloalkyl, 3- or 4-membered heterocycloalkyl, 5-membered heteroaryl, or -O-$C_{3-4}$ cycloalkyl, wherein the cycloalkyl, heterocycloalkyl or heteroaryl is optionally further substituted with 1-4 groups selected from OH, -$S(O)_2$-$CH_3$, halogen, $C_{1-2}$ alkyl, halo $C_{1-2}$ alkyl, deuterated $C_{1-2}$ alkyl or -O-halo $C_{1-2}$ alkyl, and the alkyl is further substituted with 1-3 groups selected from OH or CN;
each $R^C$ is independently selected from H, halogen, CN, OH, $C_{1-4}$ alkyl, halo $C_{1-4}$ alkyl, -O-$C_{3-5}$ cycloalkyl, or $C_{3-5}$ cycloalkyl, preferably H or halo $C_{1-2}$ alkyl, more preferably H or $CF_3$;
B2 is selected from the following groups optionally substituted with 1-4 $R^B$:

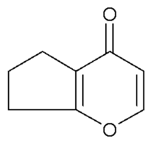, 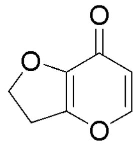, 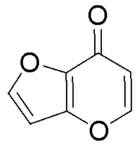, 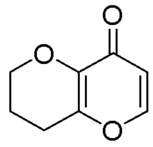, 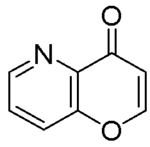, 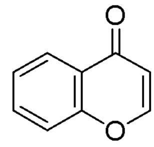,

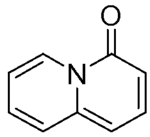, 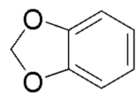, 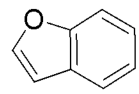, 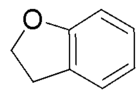, 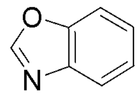, 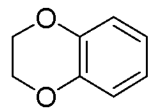,

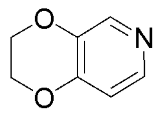, 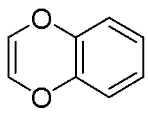, or 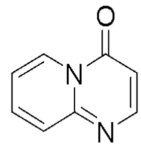;

each $R^B$ is independently selected from halogen, CN, =O, $C_{1-2}$ alkyl, $NH_2$, $C_{1-2}$ alkoxy, $C_{2-4}$ alkenyl, $C_{2-4}$ alkynyl, or halo $C_{1-2}$ alkyl;
n3 is selected from 0, 1 or 2;
p is selected from 0, 1 or 2.

7. The compound, or the stereoisomer, the deuterated substance, the solvate, the co-crystal, or the pharmaceutically acceptable salt thereof according to claim 1, **characterized in that** the compound is selected from one of the structures in Table I.

8. A pharmaceutical composition or pharmaceutical preparation, **characterized in that** the pharmaceutical composition or pharmaceutical preparation comprises the compound, or the stereoisomer, the deuterated substance, the solvate, the co-crystal, or the pharmaceutically acceptable salt thereof according to any one of claims 1-7, and a pharmaceutically acceptable carrier and/or excipient.

9. The pharmaceutical composition or pharmaceutical preparation according to claim 8, **characterized in that** the pharmaceutical composition or pharmaceutical preparation comprises 1-1500 mg of the compound, or the stereoisomer, the deuterated substance, the solvate, the co-crystal, or the pharmaceutically acceptable salt thereof according to any one of claims 1-7, and a carrier and/or excipient.

10. The compound, or the stereoisomer, the deuterated substance, the solvate, the co-crystal, or the pharmaceutically acceptable salt thereof according to any one of claims 1-7, or the composition according to claim 8 or 9 for use in the preparation of a drug for treating/preventing a CYP11A1-mediated disease.

11. The use according to claim 10, **characterized in that** the CYP11A1-mediated disease is treating steroid hormone-dependent cancer.

12. A method for treating a disease in a mammal, **characterized in that** the method comprises administering to a subject a therapeutically effective amount of the compound, or the stereoisomer, the deuterated substance, the solvate, or the pharmaceutically acceptable salt thereof according to any one of claims 1-7, wherein the therapeutically effective amount is preferably 1-1500 mg, and the disease is preferably prostate cancer.

# INTERNATIONAL SEARCH REPORT

International application No.
**PCT/CN2024/088772**

## A. CLASSIFICATION OF SUBJECT MATTER

C07D405/14(2006.01)i; C07D209/44(2006.01)i; C07D211/98(2006.01)i; C07D413/14(2006.01)i; A61K31/4439(2006.01)i; A61K31/351(2006.01)i; A61P35/00(2006.01)i

According to International Patent Classification (IPC) or to both national classification and IPC

## B. FIELDS SEARCHED

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS: CNTXT; ENTXTC; ENTXT; STN; Web of Science; CNKI: 细胞色素P450单加氧酶11A1, 吡喃类固醇激素依赖性癌症, 前列腺癌, prostatic cancer, steroid hormone dependent cancer, CYP11A1, inhibit, cancer, 结构式检索, structural formula search

## C. DOCUMENTS CONSIDERED TO BE RELEVANT

| Category* | Citation of document, with indication, where appropriate, of the relevant passages | Relevant to claim No. |
|---|---|---|
| PX | WO 2023084158 A1 (ORION CORP.) 19 May 2023 (2023-05-19)<br>description, pages 31-53, and claims 1-35 | 1-4, 8-12 |
| X | CN 110139861 A (ORION CORPORATION) 16 August 2019 (2019-08-16)<br>description, paragraphs [1215]-[1503], and claims 1-43 | 1-5, 7-12 |
| X | CN 115551831 A (ORION CORPORATION) 30 December 2022 (2022-12-30)<br>description, paragraphs [0493]-[1015], and claims 1-53 | 1-5, 7-12 |
| X | WO 2022117920 A1 (ORION CORP.) 09 June 2022 (2022-06-09)<br>description, pages 1-80 | 1-5, 8-12 |
| A | WO 2022226349 A1 (ESSA PHARMA INC.) 27 October 2022 (2022-10-27)<br>claims 1-81 | 1-12 |
| A | CN 105636965 A (ASTELLAS PHARMA INC.) 01 June 2016 (2016-06-01)<br>claims 1-19 | 1-12 |

☐ Further documents are listed in the continuation of Box C.   ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 July 2024** | **27 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/088772**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

Claim 12 relates to a method for treating a disease in a mammal, which falls within methods for treatment of a human or animal body by therapy, and falls within subject matter for which no search is required by the International Searching Authority as defined in PCT Rule 39.1(iv). The search is carried out on the basis that the method is reasonably expected to be the subject matter of a pharmaceutical use claim, i.e., "the use of preparing a drug for treating a disease in a mammal".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/088772**

| Patent document cited in search report | Publication date (day/month/year) | Patent family member(s) | Publication date (day/month/year) |
|---|---|---|---|
| WO 2023084158 A1 | 19 May 2023 | None | |
| CN 110139861 A | 16 August 2019 | CA 3047370 A1 | 28 June 2018 |
| | | EP 3868756 A1 | 25 August 2021 |
| | | MX 2019007373 A | 18 September 2019 |
| | | WO 2018115591 A1 | 28 June 2018 |
| | | JP 2020502229 A | 23 January 2020 |
| | | JP 7025432 B2 | 24 February 2022 |
| | | TW 202229264 A | 01 August 2022 |
| | | TWI 796205 B | 11 March 2023 |
| | | EA 201991513 A1 | 29 November 2019 |
| | | EA 039309 B1 | 12 January 2022 |
| | | AU 2021203497 A1 | 01 July 2021 |
| | | AU 2021203497 B2 | 10 November 2022 |
| | | RS 62198 B1 | 31 August 2021 |
| | | LT 3558981 T | 25 August 2021 |
| | | US 2020299280 A1 | 24 September 2020 |
| | | US 11098032 B2 | 24 August 2021 |
| | | AU 2017380282 A1 | 01 August 2019 |
| | | AU 2017380282 B2 | 24 June 2021 |
| | | EP 3558981 A1 | 30 October 2019 |
| | | EP 3558981 B1 | 26 May 2021 |
| | | PH 12019550111 A1 | 09 March 2020 |
| | | HRP 20211255 T1 | 12 November 2021 |
| | | KR 20190095950 A | 16 August 2019 |
| | | KR 102491308 B1 | 27 January 2023 |
| | | UA 124640 C2 | 20 October 2021 |
| | | KR 20230019496 A | 08 February 2023 |
| | | BR 112019012906 A2 | 03 December 2019 |
| | | TN 2019000189 A1 | 05 October 2020 |
| | | SI 3558981 T1 | 30 September 2021 |
| | | MA 47102 B1 | 30 September 2021 |
| | | ES 2880151 T3 | 23 November 2021 |
| | | HUE 056540 T2 | 28 February 2022 |
| | | MA 55983 A | 23 March 2022 |
| | | IL 285729 A | 30 September 2021 |
| | | IL 285729 B | 01 February 2022 |
| | | AR 110412 A1 | 27 March 2019 |
| | | CO 2019007321 A2 | 31 July 2019 |
| | | TW 201835072 A | 01 October 2018 |
| | | TWI 762544 B | 01 May 2022 |
| | | JP 2022078068 A | 24 May 2022 |
| | | JP 7286825 B2 | 05 June 2023 |
| | | PT 3558981 T | 06 July 2021 |
| | | IL 267484 A | 29 August 2019 |
| | | IL 267484 B | 30 September 2021 |
| | | PL 3558981 T3 | 06 December 2021 |
| | | DK 3558981 T3 | 26 July 2021 |
| | | CL 2019001728 A1 | 27 September 2019 |
| | | US 2021347765 A1 | 11 November 2021 |
| | | US 2019359601 A1 | 28 November 2019 |

Form PCT/ISA/210 (patent family annex) (July 2022)

**INTERNATIONAL SEARCH REPORT**
**Information on patent family members**

International application No.
**PCT/CN2024/088772**

| Patent document cited in search report | Publication date (day/month/year) | Patent family member(s) | Publication date (day/month/year) |
|---|---|---|---|
| | | US 10717726 B2 | 21 July 2020 |
| | | CY 1124461 T1 | 22 July 2022 |
| | | PE 20191137 A1 | 02 September 2019 |
| CN 115551831 A | 30 December 2022 | WO 2021229152 A1 | 18 November 2021 |
| | | JP 2023525137 A | 14 June 2023 |
| | | AU 2021269872 A1 | 24 November 2022 |
| | | US 2023192677 A1 | 22 June 2023 |
| | | EP 4149924 A1 | 22 March 2023 |
| | | KR 20230010723 A | 19 January 2023 |
| | | CA 3178067 A1 | 18 November 2021 |
| WO 2022117920 A1 | 09 June 2022 | EP 4255902 A1 | 11 October 2023 |
| | | KR 20230117187 A | 07 August 2023 |
| | | CA 3200556 A1 | 30 November 2021 |
| | | US 2024124431 A1 | 18 April 2024 |
| | | MX 2023006290 A | 21 August 2023 |
| | | JP 2023551340 A | 07 December 2023 |
| | | AU 2021391977 A1 | 13 July 2023 |
| WO 2022226349 A1 | 27 October 2022 | US 2022380378 A1 | 01 December 2022 |
| CN 105636965 A | 01 June 2016 | TW 201607951 A | 01 March 2016 |
| | | TWI 648281 B | 21 January 2019 |
| | | UA 116042 C2 | 25 January 2018 |
| | | CY 1120880 T1 | 29 May 2020 |
| | | US 2015231138 A1 | 20 August 2015 |
| | | US 9642852 B2 | 09 May 2017 |
| | | HK 1220192 A1 | 28 April 2017 |
| | | RS 57532 B1 | 31 October 2018 |
| | | EA 201690790 A1 | 31 August 2016 |
| | | EA 029075 B1 | 28 February 2018 |
| | | KR 20160071408 A | 21 June 2016 |
| | | KR 102248450 B1 | 06 May 2021 |
| | | LT 3059239 T | 10 September 2018 |
| | | PL 3059239 T3 | 31 October 2018 |
| | | HRP 20181183 T1 | 19 October 2018 |
| | | SA 516370956 B1 | 04 April 2019 |
| | | MX 2016004944 A | 11 July 2016 |
| | | MX 367857 B | 09 September 2019 |
| | | HUE 040208 T2 | 28 February 2019 |
| | | CA 2927518 A1 | 23 April 2015 |
| | | CA 2927518 C | 12 April 2022 |
| | | AR 098070 A1 | 27 April 2016 |
| | | SI 3059239 T1 | 31 August 2018 |
| | | NZ 719149 A | 27 September 2019 |
| | | ES 2685070 T3 | 05 October 2018 |
| | | EP 3059239 A1 | 24 August 2016 |
| | | EP 3059239 A4 | 26 April 2017 |
| | | EP 3059239 B1 | 27 June 2018 |
| | | JPWO 2015056771 A1 | 09 March 2017 |
| | | JP 6477484 B2 | 06 March 2019 |
| | | IL 244953 A0 | 31 May 2016 |
| | | IL 244953 B | 31 January 2019 |

Form PCT/ISA/210 (patent family annex) (July 2022)

**INTERNATIONAL SEARCH REPORT**
**Information on patent family members**

International application No.
**PCT/CN2024/088772**

| Patent document cited in search report | Publication date (day/month/year) | Patent family member(s) | | | Publication date (day/month/year) |
|---|---|---|---|---|---|
| | | SG | 11201602926 | UA | 30 May 2016 |
| | | PT | 3059239 | T | 09 October 2018 |
| | | MY | 177271 | A | 10 September 2020 |
| | | DK | 3059239 | T3 | 13 August 2018 |
| | | US | 2015111876 | A1 | 23 April 2015 |
| | | US | 9051339 | B2 | 09 June 2015 |
| | | AU | 2014335304 | B2 | 01 March 2018 |
| | | WO | 2015056771 | A1 | 23 April 2015 |
| | | PH | 12016500625 | B1 | 23 May 2016 |
| | | BR | 112016008654 | A2 | 01 August 2017 |
| | | BR | 112016008654 | B1 | 06 December 2022 |
| | | BR | 112016008654 | B8 | 14 February 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018115591 A1
- CN 114685415
- EP 2881384 A1
- WO 202256269 A1


### Non-patent literature cited in the description


- *Organic Letters*, 2012, vol. 14 (6), 1508-1511